# EUROPEAN PATENT SPECIFICATION

(11) **EP 1 175 402 B1**
(45) Date of publication and mention of the grant of the patent: **20.07.2005**
(21) Application number: 00926487.0
(22) Date of filing: 01.05.2000
(51) Int. Cl.: C07D 211/58, C07D 417/14, C07D 401/14, C07D 413/14, A61K 31/4468, A61K 31/4523, A61P 31/12, A61P 19/00

(54) **PIPERIDINE DERIVATIVES USEFUL AS CCR5 ANTAGONISTS**
PIPERIDINDERIVATE VERWENDBAR ALS CCR5 ANTAGONISTEN
DERIVES DE PIPERIDINE FAISANT OFFICE D'ANTAGONISTES CCR5

(30) Priority: 04.05.1999 US 305187
(43) Date of publication of application: 30.01.2002
(60) Divisional application: 05010590.7
(73) Proprietor: SCHERING CORPORATION, Kenilworth, New Jersey 07033-0530 (US)
(72) Inventor: BAROUDY, Bahige, M., Westfield, NJ 07090 (US); CLADER, John, W., Cranford, NJ 07016 (US); JOSIEN, Hubert, B., Hoboken, New Jersey 07030 (US); McCOMBIE, Stuart, W., Caldwell, NJ 07006 (US); McKITTRICK, Brian, A., Bloomfield, NJ 07003 (US); MILLER, Michael, W., Westfield, NJ 07090 (US); NEUSTADT, Bernard, R., West Orange, NJ 07052 (US); PALANI, Anandan, Bridgewater, New Jersey 08807 (US); STEENSMA, Ruo, Weehawken, NJ 07087 (US); TAGAT, Jayaram, R., Westfield, NJ 07090 (US); VICE, Susan, F., Mountainside, NJ 07092 (US); LAUGHLIN, Mark, A., Edison, NJ 08820 (US)
(74) Representative: Ritter, Stephen David
(86) International application number: PCT/US2000/011633
(87) International publication number: WO 2000/066559

(56) References cited:
- WO-A-98/01425
- WO-A-98/05292
- WO-A-98/06697
- WO-A-99/04794

## Description

### BACKGROUND

The present invention relates to piperidine derivatives useful as selective CCR5 antagonists, pharmaceutical compositions containing the compounds, and methods of treatment using the compounds. The invention also relates to the use of a combination of a CCR5 antagonist of this invention and one or more antiviral or other agents useful in the treatment of Human Immunodeficiency Virus (HIV). The invention further relates to the use of a CCR-5 antagonist of this invention, alone or in combination with another agent, in the treatment of solid organ transplant rejection, graft v. host disease, arthritis, rheumatoid arthritis, inflammatory bowel disease, atopic dermatitis, psoriasis, asthma, allergies or multiple sclerosis.

The global health crisis caused by HIV, the causative agent of Acquired Immunodeficiency Syndrome (AIDS), is unquestioned, and while recent advances in drug therapies have been successful in slowing the progression of AIDS, there is still a need to find a safer, more efficient, less expensive way to control the virus.

It has been reported that the CCR5 gene plays a role in resistance to HIV infection. HIV infection begins by attachment of the virus to a target cell membrane through interaction with the cellular receptor CD4 and a secondary chemokine co-receptor molecule, and proceeds by replication and dissemination of infected cells through the blood and other tissue. There are various chemokine receptors, but for macrophage-tropic HIV, believed to be the key pathogenic strain that replicates *in vivo* in the early stages of infection, the principal chemokine receptor required for the entry of HIV into the cell is CCR5. Therefore, interfering with the interaction between the viral receptor CCR5 and HIV can block HIV entry into the cell. The present invention relates to small molecules which are CCR5 antagonists.

CCR-5 receptors have been reported to mediate cell transfer in inflammatory diseases such as arthritis, rheumatoid arthritis, atopic dermatitis, psoriasis, asthma and allergies, and inhibitors of such receptors are expected to be useful in the treatment of such diseases, and in the treatment of other inflammatory diseases or conditions such as inflammatory bowel disease, multiple sclerosis, solid organ transplant rejection and graft v. host disease.

Related piperidine derivatives which are muscarinic antagonists useful in the treatment of cognitive disorders such as Alzheimer's disease are disclosed in US patents 5,883,096; 6,037,352; 5,889,006; 5,952,349; and 5,977,138.

A-M. Vandamme et al., Antiviral Chemistry & Chemotherapy, 9:187-203 (1998) disclose current clinical treatments of HIV-1 infections in man including at least triple drug combinations or so-called Highly Active Antiretroviral Therapy ("HAART"); HAART involves various combinations of nucleoside reverse transcriptase inhibitors ("NRTI"), non-nucleoside reverse transcriptase inhibitors ("NNRTI") and HIV protease inhibitors ("PI"). In compliant drug-naive patients, HAART is effective in reducing mortality and progression of HIV-1 to AIDS. However, these multidrug therapies do not eliminate HIV-1 and long-term treatment usually results in multidrug resistance. Development of new drug therapies to provide better HIV-1 treatment remains a priority.

### SUMMARY OF THE INVENTION

The present invention relates to the treatment of HIV comprising administering to a human in need of such treatment an effective amount of a CCR5 antagonist represented by the structural formula I: 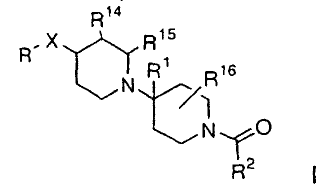 or a pharmaceutically acceptable salt thereof, wherein
X is -C(R¹³)₂-, -C(R¹³)(R¹⁹)-, -C(O)-, -O-, -NH-, -N((C₁-C₆)alkyl)-, 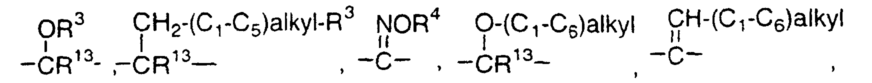 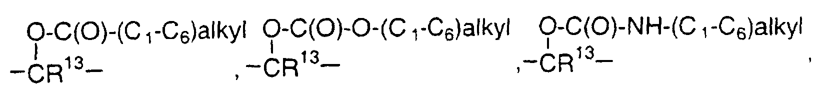 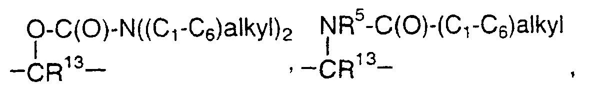 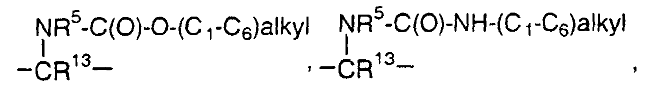 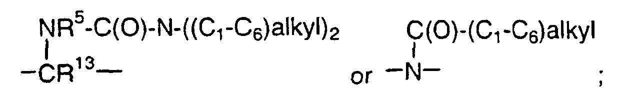 R is R⁶-phenyl, R⁶-pyridyl, R⁶-thiophenyl or R⁶-naphthyl;
R¹ is hydrogen, C₁-C₆ alkyl or C₂-C₆ alkenyl;
R² is R⁷, R⁸, R⁹-phenyl; R⁷, R⁸, R⁹-substituted 6-membered heteroaryl; R⁷, R⁸, R⁹-substituted 6-membered heteroaryl N-oxide;
R¹⁰, R¹¹-substituted 5-membered heteroaryl; naphthyl; fluorenyl; diphenylmethyl 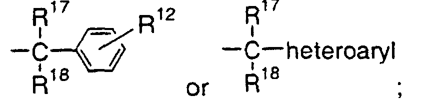 R³ is R⁶-phenyl, R⁶-heteroaryl or R⁶-naphthyl;
R⁴ is hydrogen, C₁-C₆ alkyl, fluoro-C₁-C₆ alkyl, cyclopropylmethyl, -CH₂CH₂OH, -CH₂CH₂-O-(C₁-C₆)alkyl, -CH₂C(O)-O-(C₁-C₆)alkyl, -CH₂C(O)NH₂, -CH₂C(O)-NH(C₁-C₆)alkyl or -CH₂C(O)-N((C₁-C₆)alkyl)₂;
R⁵ and R¹¹ are independently selected from the group consisting of hydrogen and (C₁-C₆)-alkyl;
R⁶ is 1 to 3 substituents independently selected from the group consisting of hydrogen, halogen, C₁-C₆ alkyl, C₁-C₆ alkoxy, -CF₃, CF₃O-, CH₃C(O)-, -CN, CH₃SO₂-, CF₃SO₂-, R¹⁴-phenyl, R¹⁴-benzyl, CH₃C(=NOCH₃)-, CH₃C(=NOCH₂CH₃)-, 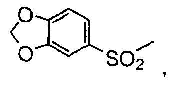 -NH₂, -NHCOCF₃, -NHCONH(C₁-C₆ alkyl), -NHCO(C₁-C₆ alkyl), -NHSO₂(C₁-C₆ alkyl), 5-membered heteroaryl and 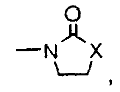 wherein X is -O-, -NH- or -N(CH₃)-;
R⁷ and R⁸ are independently selected from the group consisting of (C₁-C₆)alkyl, halogen, -NR²⁰R²¹, -OH, -CF₃, -OCH₃, -O-acyl, and -OCF₃;
R⁹ is R⁷, hydrogen, phenyl, -NO₂, -CN, -CH₂F, -CHF₂, -CHO, -CH=NOR²⁰, pyridyl, pyridyl N-oxide, pyrimidinyl, pyrazinyl, -N(R²⁰)CONR²¹R²², -NHCONH(chloro-(C₁-C₆)alkyl), -NHCONH((C₃-C₁₀)-cycloalkyl(C₁-C₆)alkyl), -NHCO(C₁-C₆)alkyl, -NHCOCF₃, -NHSO₂N((C₁-C₆)alkyl)₂, -NHSO₂(C₁-C₆)alkyl, -N(SO₂CF₃)₂, -NHCO₂(C₁-C₆)alkyl, C₃-C₁₀ cycloalkyl, -SR²³, -SOR²³, -SO₂R²³, -SO₂NH(C₁-C₆ alkyl), -OSO₂(C₁-C₆)alkyl, -OSO₂CF₃, hydroxy(C₁-C₆)alkyl, -CON R²⁰R²¹, -CON(CH₂CH₂-O-CH₃)₂,
-OCONH(C₁-C₆)alkyl, -CO₂R²⁰, -Si(CH₃)₃ or -B(OC(CH₃)₂)₂;
R¹⁰ is (C₁-C₆)alkyl, -NH₂ or R¹²-phenyl;
R¹² is 1 to 3 substituents independently selected from the group consisting of hydrogen, (C₁-C₆) alkyl, -CF₃, -CO₂R₂₀, -CN, (C₁-C₆)alkoxy and halogen;
R¹³, R¹⁴, R¹⁵ and R¹⁶ are independently selected from the group consisting of hydrogen and (C₁-C₆)alkyl;
R¹⁷ and R¹⁸ are independently selected from the group consisting of hydrogen and C₁-C₆ alkyl, or R¹⁷ and R¹⁸ together are a C₂-C₅ alkylene group and with the carbon to which they are attached form a spiro ring of 3 to 6 carbon atoms;
R¹⁹ is R⁶-phenyl, R⁶-heteroaryl, R⁶-naphthyl, C₃-C₁₀ cycloalkyl, (C₃-C₁₀)cycloalkyl(C₁-C₆)alkyl or (C₁-C₆)alkoxy(C₁-C₆)alkyl;
R²⁰, R²¹ and R²² are independently selected from the group consisting of H and C₁-C₆ alkyl; and
R²³ is C₁-C₆ alkyl or phenyl.

Preferred are compounds of formula I wherein R is R⁶-phenyl, especially wherein R⁶ is a single substituent, and especially wherein the R⁶ substituent is in the 4-position. Also preferred are compounds of formula I wherein R¹³, R¹⁴, R¹⁵ and R¹⁶ are each hydrogen or methyl, especially hydrogen. Also preferred are compounds of formula I wherein X is -CHOR³, -C(R¹³)(R¹⁹)- or -C(=NOR⁴)-; a preferred definition for R³ is pyridyl, especially 2-pyridyl, a preferred definition for R⁴ is (C₁-C₆)alkyl, especially methyl, ethyl or isopropyl, a preferred definition for R¹³ is hydrogen, and a preferred definition for R¹⁹ is R⁶-phenyl. For compounds of formula I, A¹ is preferably (C₁-C₆)alkyl, especially methyl.

In compounds of formula I, R² is preferably R⁷, R⁸, R⁹-phenyl, R⁷, R⁸, R⁹-pyridyl or an N-oxide thereof, or R⁷, R⁸, R⁹-pyrimidyl. When R² is pyridyl, it is preferably 3- or 4-pyridyl, and when pyrimidyl, it is preferably 5-pyrimidyl. The R⁷ and R⁸ substituents are preferably attached to carbon ring members adjacent to the carbon joining the ring to the rest of the molecule and the R⁹ substituent can be attached to any of the remaining unsubstituted carbon ring members, for example as shown in the following structures:

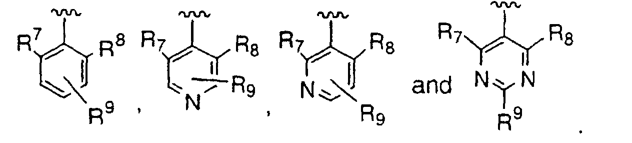

Preferred R⁷ and R⁸ substituents are: (C₁-C₆)alkyl, especially methyl; halogen, especially chloro; and -NH₂. A preferred R⁹ substituent is hydrogen.

Also claimed are novel CCR5 antagonist compounds represented by the structural formula II 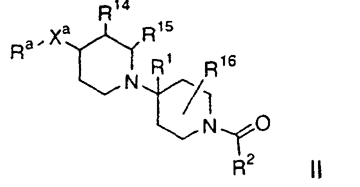 or a pharmaceutically acceptable salt thereof, wherein
(1) X^{a} is -C(R¹³)₂-, -C(R¹³)(R¹⁹)-, -C(O)-, -O-, -NH-, -N((C₁-C₆)alkyl)-, 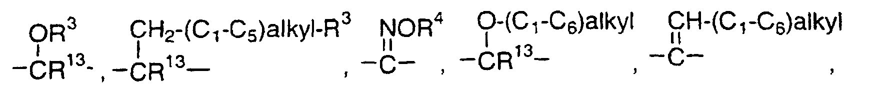 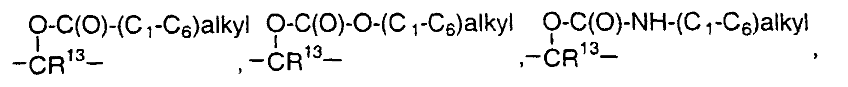 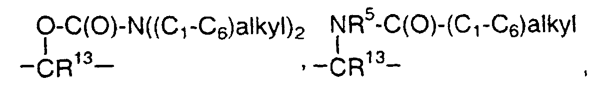 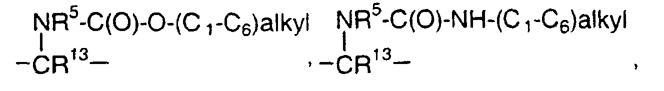 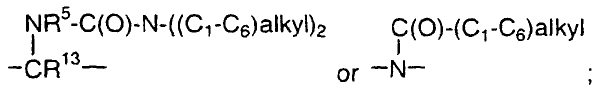 R^{a} is R^{6a}-phenyl, R^{6a}-pyridyl, R^{6a}-thiophenyl or R⁶-naphthyl;
   A¹ is hydrogen, C₁-C₆ alkyl or C₂-C₆ alkenyl;
   R² is R⁷, R⁸, R⁹-phenyl; R⁷, R⁸, R⁹-substituted 6-membered heteroaryl; R⁷, R⁸, R⁹-substituted 6-membered heteroaryl N-oxide;
   R¹⁰, R¹¹-substituted 5-membered heteroaryl; naphthyl; fluorenyl; diphenylmethyl 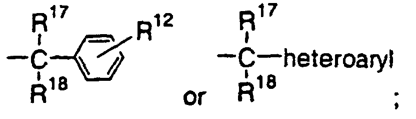 R³ is R¹⁰-phenyl, pyridyl, pyrimidyl, pyrazinyl or thiazolyl;
   R⁴ is hydrogen, C₁-C₆ alkyl, fluoro-C₁-C₆ alkyl, cyclopropylmethyl, -CH₂CH₂OH, -CH₂CH₂-O-(C₁-C₆)alkyl, -CH₂C(O)-O-(C₁-C₆)alkyl, -CH₂C(O)NH₂, -CH₂C(O)-NH(C₁-C₆)alkyl or -CH₂C(O)-N((C₁-C₆)alkyl)₂;
   R⁵ and R¹¹ are independently selected from the group consisting of hydrogen and (C₁-C₆)-alkyl;
   R^{6a} is 1 to 3 substituents independently selected from the group consisting of hydrogen, halogen, -CF₃, CF₃O-, -CN, -CF₃SO₂-, -NHCOCF₃, 5-membered heteroaryl and 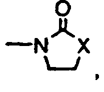 wherein X is -O-, -NH- or -N(CH₃)- ;
   R⁶ is independently selected from the group consisting of R^{6a} and CH₃SO₂-;
   R⁷ and R⁸ are independently selected from the group consisting of (C₁-C₆)alkyl, halogen, -NR²⁰R²¹, -OH, -CF₃, -OCH₃, -O-acyl, and -OCF₃;
   R⁹ is R⁷, hydrogen, phenyl, -NO₂, -CN, -CH₂F, -CHF₂, -CHO, -CH=NOR²⁰, pyridyl, pyridyl N-oxide, pyrimidinyl, pyrazinyl, -N(R²⁰)CONR²¹R²², -NHCONH(chloro-(C₁-C₆)alkyl), -NHCONH((C₃-C₁₀)-cycloalkyl(C₁-C₆)alkyl), -NHCO(C₁-C₆)alkyl, -NHCOCF₃, -NHSO₂N((C₁-C₆)alkyl)₂, -NHSO₂(C₁-C₆)alkyl, -N(SO₂CF₃)₂, -NHCO₂(C₁-C₆)alkyl, C₃-C₁₀ cycloalkyl, -SR²³, -SOR²³, -SO₂R²³, -SO₂NH(C₁-C₆ alkyl), -OSO₂(C₁-C₆)alkyl, -OSO₂CF₃, hydroxy(C₁-C₆)alkyl, -CON R²⁰R²¹, -CON(CH₂CH₂-O-CH₃)₂,
   -OCONH(C₁-C₆)alkyl, -CO₂R²⁰, -Si(CH₃)₃ or -B(OC(CH₃)₂)₂;
   R¹⁰ is (C₁-C₆)alkyl, -NH₂ or R¹²-phenyl;
   R¹² is1 to 3 substituents independently selected from the group consisting of hydrogen, (C₁-C₆) alkyl, -CF₃, -CO₂R₂₀, -CN, (C₁-C₆)alkoxy and halogen;
   R¹³, R¹⁴, R¹⁵ and R¹⁶ are independently selected from the group consisting of hydrogen and (C₁-C₆)alkyl;
   R¹⁷ and R¹⁸ are independently selected from the group consisting of hydrogen and C₁-C₆ alkyl, or R¹⁷ and R¹⁸ together are a C₂-C₅ alkylene group and with the carbon to which they are attached form a spiro ring of 3 to 6 carbon atoms;
   R¹⁹ is R⁶-phenyl, R⁶-heteroaryl, R⁶-naphthyl, C₃-C₁₀ cycloalkyl, (C₃-C₁₀)cycloalkyl(C₁-C₆)alkyl or (C₁-C₆)alkoxy(C₁-C₆)alkyl;
   R²⁰, R²¹ and R²² are independently selected from the group consisting of H and C₁-C₆ alkyl; and
   R²³ is C₁-C₆ alkyl or phenyl; or
(2):
   X^{a} is -C(R¹³)(R¹⁹)-, -C(O)-, -O-, -NH-, -N((C₁-C₆)alkyl)-, 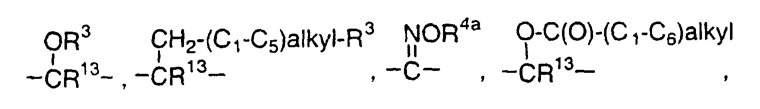 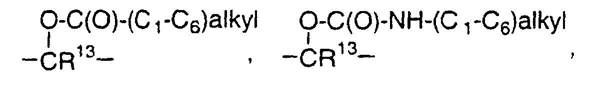 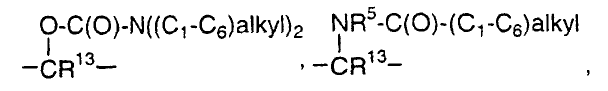 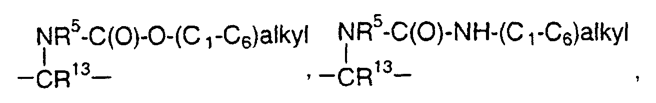 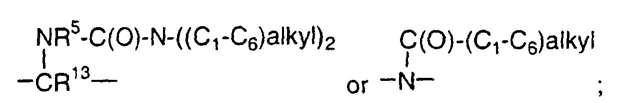 R^{a} is R^{6b}-phenyl, R^{6b}-pyridyl or R^{6b}-thiophenyl;
   R^{4a} is fluoro-C₁-C₆ alkyl, cyclopropylmethyl, -CH₂CH₂OH, -CH₂CH₂-O-(C₁-C₆)alkyl, -CH₂C(O)-O-(C₁-C₆)alkyl, -CH₂C(O)NH₂, - CH₂C(O)-NH-(C₁-C₆)alkyl or -CH₂C(O)-N((C₁-C₆)alkyl)₂;
   R^{6b} is CH₃SO₂-; and
   R¹, R², R³, R⁵, R¹⁴, R¹⁵, R¹⁶ and R¹⁹ are as defined in (1).

Preferred are compounds of formula II(1) wherein R^{a} is R^{6a}-phenyl, especially wherein R^{6a} is a single substituent, and especially wherein the R^{6a} substituent is in the 4-position. Also preferred are compounds of formula II(1) wherein X^{a} is -CHOR³, -C(R¹³)(R¹⁹)- or -C(=NOR⁴)-; a preferred definition for R³ is pyridyl, especially 2-pyridyl, a preferred definition for R⁴ is (C₁-C₆)alkyl, especially methyl, ethyl or isopropyl, a preferred definition for R¹³ is hydrogen, and a preferred definition for R¹⁹ is R⁶-phenyl. For compounds of formula II(1), R¹ is preferably (C₁-C₆)alkyl, especially methyl. Also for compounds of formula II(1), R¹⁴, R¹⁵ and R¹⁶ are preferably hydrogen.

Preferred are compounds of formula II(2) wherein R^{a} is R^{6b}-phenyl, especially wherein R^{6b} is a single substituent, and especially wherein the R^{6b} substituent is in the 4-position. Also preferred are compounds of formula II(2) wherein X^{a} is -CHOR³, -C(R¹³)(R¹⁹)- or -C(=NOR^{4a})-; a preferred definition for R³ is pyridyl, especially 2-pyridyl, preferred definitions for R^{4a} are cyclopropylmethyl and trifluoroethyl, a preferred definition for R¹³ is hydrogen, and a preferred definition for R¹⁹ is R⁶-phenyl. For compounds of formula II(2), R¹ is preferably (C₁-C₆)alkyl, especially methyl. Also for compounds of formula II(2), R¹⁴, R¹⁵ and R¹⁶ are preferably hydrogen.

In compounds of formula II(1) and (2), R² is preferably R⁷, R⁸, R⁹-phenyl; R⁷, R⁸, R⁹-pyridyl or an N-oxide thereof; or R⁷, R⁸, R⁹-pyrimidyl. When R² is pyridyl, it is preferably 3- or 4-pyridyl, and when pyrimidyl, it is preferably 5-pyrimidyl. The R⁷ and R⁸ substituents are preferably attached to carbon ring members adjacent to the carbon joining the ring to the rest of the molecule and the R⁹ substituent can be attached to any of the remaining unsubstituted carbon ring members as shown above for compounds of formula I. Preferred R⁷ and R⁸ substituents for compounds of formula II are: (C₁-C₆)alkyl, especially methyl; halogen, especially chloro; and -NH₂; a preferred R⁹ substituent is hydrogen.

Another aspect of the invention is a pharmaceutical composition for treatment of HIV comprising an effective amount of a CCR5 antagonist of formula II in combination with a pharmaceutically acceptable carrier. Another aspect of the invention is a pharmaceutical composition for treatment of solid organ transplant rejection, graft v. host disease, arthritis, rheumatoid arthritis, inflammatory bowel disease, atopic dermatitis, psoriasis, asthma, allergies or multiple sclerosis comprising an effective amount of a CCR5 antagonist of formula II in combination with a pharmaceutically acceptable carrier.

Yet another aspect of this invention is a method of treatment of HIV comprising administering to a human in need of such treatment an effective amount of a CCR5 antagonist compound of formula II. Another aspect of the invention is a method of treatment of solid organ transplant rejection, graft v. host disease, arthritis, rheumatoid arthritis, inflammatory bowel disease, atopic dermatitis, psoriasis, asthma, allergies or multiple sclerosis comprising administering to a human in need of such treatment an effective amount of a CCR5 antagonist compound of formula I or II.

Still another aspect of this invention is the use of a CCR5 antagonist of formula I or II of this invention in combination with one or more antiviral or other agents useful in the treatment of Human Immunodeficiency Virus for the treatment of AIDS. Still another aspect of this invention is the use of a CCR5 antagonist of formula I or II of this invention in combination with one or more other agents useful in the treatment of solid organ transplant rejection, graft v. host disease, inflammatory bowel disease, rheumatoid arthritis or multiple sclerosis. The CCR5 and antiviral or other agents which are components of the combination can be administered in a single dosage form or they can be administered separately; a kit comprising separate dosage forms of the actives is also contemplated.

### DETAILED DESCRIPTION OF THE INVENTION

As used herein, the following terms are used as defined below unless otherwise indicated.

Alkyl (including the alkyl portions of alkoxy, alkylamino and dialkylamino) represents straight and branched carbon chains and contains from one to six carbon atoms.

Alkenyl represents C₂-C₆ carbon chains having one or two unsaturated bonds, provided that two unsaturated bonds are not adjacent to each other.

Substituted phenyl means that the phenyl group can be substituted at any available position on the phenyl ring.

Acyl means a radical of a carboxylic acid having the formula alkyl-C(O)-, aryl-C(O)-, aralkyl-C(O)-, (C₃-C₇)cycloalkyl-C(O)-, (C₃-C₇)cycloalkyl-(C₁-C₆)alkyl-C(O)-, and heteroaryl-C(O)-, wherein alkyl and heteroaryl are as defined herein; aryl is R¹²-phenyl or R¹²-naphthyl; and aralkyl is aryl-(C₁-C₆)alkyl, wherein aryl is as defined above.

Heteroaryl represents cyclic aromatic groups of 5 or 6 atoms or bicyclic groups of 11 to 12 atoms having 1 or 2 heteroatoms independently selected from O, S or N, said heteroatom(s) interrupting a carbocyclic ring structure and having a sufficient number of delocalized pi electrons to provide aromatic character, provided that the rings do not contain adjacent oxygen and/or sulfur atoms. For 6-membered heteroaryl rings, carbon atoms can be substituted by R⁷, R⁸ or R⁹ groups. Nitrogen atoms can form an N-oxide. All regioisomers are contemplated, e.g., 2-pyridyl, 3-pyridyl and 4-pyridyl. Typical 6-membered heteroaryl groups are pyridyl, pyrimidinyl, pyrazinyl, pyridazinyl and the N-oxides thereof. For 5-membered heteroaryl rings, carbon atoms can be substituted by R¹⁰ or R¹¹ groups. Typical 5-membered heteroaryl rings are furyl, thienyl, pyrrolyl, thiazolyl, isothiazolyl, imidazolyl, pyrazolyl and isoxazolyl. 5-Membered rings having one heteroatom can be joined through the 2- or 3- position; 5-membered rings having two heteroatoms are preferably joined through the 4-position. Bicyclic groups typically are benzo-fused ring systems derived from the heteroaryl groups named above, e.g. quinolyl, phthalazinyl, quinazolinyl, benzofuranyl, benzothienyl and indolyl.

Preferred points of substitution for 6-membered heteroaryl rings at R² are described above. When R² is a 5-membered heteroaryl group, the R¹⁰ and R¹¹ substituents are preferably attached to carbon ring members adjacent to the carbon joining the ring to the rest of the molecule, and R¹¹ is preferably alkyl; however, if a heteroatom is adjacent to the carbon joining the ring to the rest of the molecule (i.e., as in 2-pyrrolyl), R¹⁰ is preferably attached to a carbon ring member adjacent to the carbon joining the ring to the rest of the molecule.

Halogen represents fluoro, chloro, bromo and iodo.

Fluoro(C₁-C₆)alkyl represents a straight or branched alkyl chain substituted by 1 to 5 fluoro atoms, which can be attached to the same or different carbon atoms, e.g., -CH₂F, -CHF₂, -CF₃, F₃CCH₂- and -CF₂CF₃.

A therapeutically effective amount of a CCR5 antagonist is an amount sufficient to lower HIV-1-RNA plasma levels.

One or more, preferaby one to four, antiviral agents useful in anti-HIV-1 therapy may be used in combination with a CCR5 antagonist of the present invention. The antiviral agent or agents may be combined with the CCR5 antagonist in a single dosage form, or the CCR5 antagonist and the antiviral agent or agents may be administered simultaneously or sequentially as separate dosage forms. The antiviral agents contemplated for use in combination with the compounds of the present invention comprise nucleoside and nucleotide reverse transcriptase inhibitors, non-nucleoside reverse transcriptase inhibitors, protease inhibitors and other antiviral drugs listed below not falling within these classifications. In particular, the combinations known as HAART are contemplated for use in combination with the CCR5 antagonists of this invention.

The term "nucleoside and nucleotide reverse transcriptase inhibitors" ("NRTI" s) as used herein means nucleosides and nucleotides and analogues thereof that inhibit the activity of HIV-1 reverse transcriptase, the enzyme which catalyzes the conversion of viral genomic HIV-1 RNA into proviral HIV-1 DNA.

Typical suitable NRTIs include zidovudine (AZT) available under the RETROVIR tradename from Glaxo-Wellcome Inc., Research Triangle, NC 27709; didanosine (ddl) available under the VIDEX tradename from Bristol-Myers Squibb Co., Princeton, NJ 08543; zalcitabine (ddC) available under the HIVID tradename from Roche Pharmaceuticals, Nutley, NJ 07110; stavudine (d4T) available under the ZERIT trademark from Bristol-Myers Squibb Co., Princeton, NJ 08543; lamivudine (3TC) available under the EPIVIR tradename from Glaxo-Wellcome Research Triangle, NC 27709; abacavir (1592U89) disclosed in WO96/30025 and available under the ZIAGEN trademark from Glaxo-Wellcome Research Triangle, NC 27709; adefovir dipivoxil [bis(POM)-PMEA] available under the PREVON tradename from Gilead Sciences, Foster City, CA 94404; lobucavir (BMS-180194), a nucleoside reverse transcriptase inhibitor disclosed in EP-0358154 and EP-0736533 and under development by Bristol-Myers Squibb, Princeton, NJ 08543; BCH-10652, a reverse transcriptase inhibitor (in the form of a racemic mixture of BCH-10618 and BCH-10619) under development by Biochem Pharma, Laval, Quebec H7V, 4A7, Canada; emitricitabine [(-)-FTC] licensed from Emory University under Emory Univ. U.S. Patent No. 5,814,639 and under development by Triangle Pharmaceuticals, Durham, NC 27707; beta-L-FD4 (also called beta-L-D4C and named beta-L-2', 3'-dicleoxy-5-fluoro-cytidene) licensed by Yale University to Vion Pharmaceuticals, New Haven CT 06511; DAPD, the purine nucleoside, (-)-beta-D-2,6,-diamino-purine dioxolane disclosed in EP 0656778 and licensed by Emory University and the University of Georgia to Triangle Pharmaceuticals, Durham, NC 27707; and lodenosine (FddA), 9-(2,3-dideoxy-2-fluoro-b-D-threo-pentofuranosyl)adenine, an acid stable purine-based reverse transcriptase inhibitor discovered by the NIH and under development by U.S. Bioscience Inc., West Conshohoken, PA 19428.

The term "non-nucleoside reverse transcriptase inhibitors" ("NNRTI"s) as used herein means non-nucleosides that inhibit the activity of HIV-1 reverse transcriptase.

Typical suitable NNRTIs include nevirapine (BI-RG-587) available under the VIRAMUNE tradename from Boehringer Ingelheim, the manufacturer for Roxane Laboratories, Columbus, OH 43216; delaviradine (BHAP, U-90152) available under the RESCRIPTOR tradename from Pharmacia & Upjohn Co., Bridgewater NJ 08807; efavirenz (DMP-266) a benzoxazin-2-one disclosed in WO94/03440 and available under the SUSTIVA tradename from DuPont Pharmaceutical Co., Wilmington, DE 19880-0723; PNU-142721, a furopyridine-thio-pyrimide under development by Pharmacia and Upjohn, Bridgewater NJ 08807; AG-1549 (formerly Shionogi # S-1153); 5-(3,5-dichlorophenyl)- thio-4-isopropyl-1-(4-pyridyl)methyl-IH-imidazol-2-ylmethyl carbonate disclosed in WO 96 /10019 and under clinical development by Agouron Pharmaceuticals, Inc., LaJolla CA 92037-1020; MKC-442 (1-(ethoxy-methyl)-5-(1-methylethyl)-6-(phenylmethyl)-(2,4(1H,3H)-pyrimidinedione) discovered by Mitsubishi Chemical Co. and under development by Triangle Pharmaceuticals, Durham, NC 27707; and (+)-calanolide A (NSC-675451) and B, coumarin derivatives disclosed in NIH U.S. Patent No. 5,489,697, licensed to Med Chem Research, which is co-developing (+) calanolide A with Vita-Invest as an orally administrable product.

The term "protease inhibitor" ("PI") as used herein means inhibitors of the HIV-1 protease, an enzyme required for the proteolytic cleavage of viral polyprotein precursors (e.g., viral GAG and GAG Pol polyproteins), into the individual functional proteins found in infectious HIV-1. HIV protease inhibitors include compounds having a peptidomimetic structure, high molecular weight (7600 daltons) and substantial peptide character, e.g. CRIXIVAN(available from Merck) as well as nonpeptide protease inhibitors e.g., VIRACEPT (available from Agouron).

Typical suitable PIs include saquinavir (Ro 31-8959) available in hard gel capsules under the INVIRASE tradename and as soft gel capsules under the FORTOVASE tradename from Roche Pharmaceuticals, Nutley, NJ 07110-1199; ritonavir (ABT-538) available under the NORVIR tradename from Abbott Laboratories, Abbott Park, IL 60064; indinavir (MK-639) available under the CRIXIVAN tradename from Merck & Co., Inc., West Point, PA 19486-0004; nelfnavir (AG-1343) available under the VIRACEPT tradename from Agouron Pharmaceuticals, Inc., LaJolla CA 92037-1020; amprenavir (141W94), tradename AGENERASE, a nonpeptide protease inhibitor under development by Vertex Pharmaceuticals, Inc., Cambridge, MA 02139-4211 and available from Glaxo-Wellcome, Research Triangle, NC under an expanded access program; lasinavir (BMS-234475) available from Bristol-Myers Squibb, Princeton, NJ 08543 (originally discovered by Novartis, Basel, Switzerland (CGP-61755); DMP-450, a cyclic urea discovered by Dupont and under development by Triangle Pharmaceuticals; BMS-2322623, an azapeptide under development by Bristol-Myers Squibb, Princeton, NJ 08543, as a 2nd-generation HIV-1 PI; ABT-378 under development by Abbott , Abbott Park, IL 60064; and AG-1549 an orally active imidazole carbamate discovered by Shionogi (Shionogi #S-1153) and under development by Agouron Pharmaceuticals, Inc., LaJolla CA 92037-1020.

Other antiviral agents include hydroxyurea, ribavirin, IL-2, IL-12, pentafuside and Yissum Project No. 11607. Hydroyurea (Droxia), a ribonucleoside triphosphate reductase inhibitor, the enzyme involved in the activation of T-cells, was discovered at the NCI and is under development by Bristol-Myers Squibb; in preclinical studies, it was shown to have a synergistic effect on the activity of didanosine and has been studied with stavudine. IL-2 is disclosed in Ajinomoto EP-0142268 , Takeda EP-0176299, and Chiron U. S. Patent Nos. RE 33653, 4530787, 4569790, 4604377, 4748234, 4752585, and 4949314, and is available under the PROLEUKIN (aldesleukin) tradename from Chiron Corp., Emeryville, CA 94608-2997 as a lyophilized powder for IV infusion or sc administration upon reconstitution and dilution with water; a dose of about 1 to about 20 million IU/day, sc is preferred; a dose of about 15 million IU/day, sc is more preferred. IL-12 is disclosed in WO96/25171 and is available from Roche Pharmaceuticals, Nutley, NJ 07110-1199 and American Home Prodocts, Madison, NJ 07940; a dose of about 0.5 microgram/kg/day to about 10 microgram/kg/day, sc is preferred. Pentafuside (DP-178, T-20) a 36-amino acid synthetic peptide,disclosed in U.S. Patent No.5,464,933 licensed from Duke University to Trimeris which is developing pentafuside in collaboration with Duke University; pentafuside acts by inhibiting fusion of HIV-1 to target membranes. Pentafuside (3-100 mg /day) is given as a continuous sc infusion or injection together with efavirenz and 2 PI's to HIV-1 positive patients refractory to a triple combination therapy; use of 100 mg/day is preferred. Yissum Project No. 11607, a synthetic protein based on the HIV -1 Vif protein, is under preclinical development by Yissum Research Development Co., Jerusalem 91042 , Israel. Ribavirin, 1-β-D-ribofuranosyl-1H-1,2,4-triazole-3-carboxamide, is available from ICN Pharmaceuticals, Inc., Costa Mesa, CA; its manufacture and formulation are described in U.S. Patent No. 4,211,771.

The term "anti-HIV-1 therapy" as used herein means any anti-HIV-1 drug found useful for treating HIV-1 infections in man alone, or as part of multidrug combination therapies, especially the HAART triple and quadruple combination therapies. Typical suitable known anti-HIV-1 therapies include, but are not limited to multidrug combination therapies such as (i) at least three anti-HIV-1 drugs selected from two NRTIS, one PI, a second PI, and one NNRTI; and (ii) at least two anti-HIV-1 drugs selected from NNRTIs and PIs. Typical suitable HAART - multidrug combination therapies include:
(a) triple combination therapies such as two NRTIs and one PI ; or (b) two NRTIs and one NNRTI ; and (c) quadruple combination therapies such as two NRTIs , one PI and a second PI or one NNRTI. In treatment of naive patients, it is preferred to start anti-HIV-1 treatment with the triple combination therapy; the use of two NRTIs and one PI is prefered unless there is intolerance to PIs. Drug compliance is essential. The CD4⁺ and HIV-1-RNA plasma levels should be monitored every 3-6 months. Should viral load plateau, a fourth drug,e.g., one PI or one NNRTI could be added. See the table below wherein typical therapies are further described:

| ANTI-HIV-1 MULTI DRUG COMBINATION THERAPIES | |
|---|---|
| A. Triple Combination Therapies | |
| 1. | Two NRTIs¹ + one PI² |
| 2. | Two NRTIs¹ + one NNRTI³ |
| | |

| B. Quadruple Combination Therapies⁴ | |
|---|---|
| | Two NRTIs + one PI + a second PI or one NNRTI |
| | |

| C. ALTERNATIVES:⁵ | |
|---|---|
| | Two NRTI¹ |
| | One NRTI⁵+ one PI² |
| | Two PIs⁶ ± one NRTI⁷or NNRTI³ |
| | One PI² + one NRTI⁷+ one NNRTI³ |

| | |
|---|---|
| FOOTNOTES TO TABLE 1. One of the following: zidovudine + lamivudine; zidovudine + didanosine; stavudine + lamivudine; stavudine + didanosine; zidovudine + zalcitabine | |
| 2. Indinavir, nelfinavir, ritonavir or saquinavir soft gel capsules. | |
| 3. Nevirapine or delavirdine. | |
| 4. See A-M. Vandamne et al Antiviral Chemistry & Chemotherapy 9:187 at p 193-197 and Figures 1 + 2. | |
| 5. Alternative regimens are for patients unable to take a recommended regimen because of compliance problems or toxicity, and for those who fail or relapse on a recommended regimen. Double nucleoside combinations may lead to HIV-resistance and clinical failure in many patients. | |
| 6. Most data obtained with saquinavir and ritonavir (each 400 mg bid). | |
| 7. Zidovudine, stavudine or didanosine. | |

Agents known in the treatment of rheumatoid arthritis, transplant and graft v. host disease, inflammatory bowel disease and multiple sclerosis which can be administered in combination with the CCR5 antagonists of the present invention are as follows:
solid organ transplant rejection and graft v. host disease: immune suppressants such as cyclosporine and Interleukin-10 (IL-10), tacrolimus, antilymphocyte globulin, OKT-3 antibody, and steroids;
inflammatory bowel disease: IL-10 (see US 5,368,854), steroids and azulfidine;
rheumatoid arthritis: methotrexate, azathioprine, cyclophosphamide, steroids and mycophenolate mofetil;
multiple sclerosis: interferon-beta, interferon-alpha, and steroids.

Certain CCR5 antagonist compounds of the invention may exist in different isomeric (e.g., enantiomers, diastereoisomers and atropisomers) forms. The invention contemplates all such isomers both in pure form and in admixture, including racemic mixtures.

Certain compounds will be acidic in nature, e.g. those compounds which possess a carboxyl or phenolic hydroxyl group. These compounds may form pharmaceutically acceptable salts. Examples of such salts may include sodium, potassium, calcium, aluminum, gold and silver salts. Also contemplated are salts formed with pharmaceutically acceptable amines such as ammonia, alkyl amines, hydroxyalkylamines, N-methylglucamine and the like.

Certain basic compounds also form pharmaceutically acceptable salts, e.g., acid addition salts. For example, the pyrido-nitrogen atoms may form salts with strong acid, while compounds having basic substituents such as amino groups also form salts with weaker acids. Examples of suitable acids for salt formation are hydrochloric, sulfuric, phosphoric, acetic, citric, oxalic, malonic, salicylic, malic, fumaric, succinic, ascorbic, maleic, methanesulfonic and other mineral and carboxylic acids well known to those in the art. The salts are prepared by contacting the free base form with a sufficient amount of the desired acid to produce a salt in the conventional manner. The free base forms may be regenerated by treating the salt with a suitable dilute aqueous base solution such as dilute aqueous NaOH, potassium carbonate, ammonia and sodium bicarbonate. The free base forms differ from their respective salt forms somewhat in certain physical properties, such as solubility in polar solvents, but the acid and base salts are otherwise equivalent to their respective free base forms for purposes of the invention.

All such acid and base salts are intended to be pharmaceutically acceptable salts within the scope of the invention and all acid and base salts are considered equivalent to the free forms of the corresponding compounds for purposes of the invention.

Compounds of the invention can be made by the procedures known in the art, for example by the procedures described in the following reaction schemes, by the methods described in the examples below, and by using the methods described in US patents 5,883,096; 6,037,352; 5,889,006; 5,952,349; and 5,977,138.

The following solvents and reagents may be referred to herein by the abbreviations indicated: tetrahydrofuran (THF); ethanol (EtOH); methanol (MeOH); acetic acid (HOAc or AcOH); ethyl acetate (EtOAc); N,N-dimethylformamide (DMF); trifluoroacetic acid (TFA); trifluoroacetic anhydride (TFAA); 1-hydroxy-benzotriazole (HOBT); m-chloroperbenzoic acid (MCPBA); triethylamine (Et₃N); diethyl ether (Et₂O); tert-butoxycarbonyl (BOC); 1,8-diazabicyclo[5.4.0]undec-7-ene (DBU); dimethylsulfoxide (DMSO); p-toluene sulfonic acid (p-TSA); potassium bis(trimethylsilyl)-amide (KHMDA); 4-dimethylaminopryidine (DMAP); N,N,N-diiospropylethylamine (Dipea); and 1-(3-dimethyl-aminopropyl)-3-ethyl carbodiimide hydrochloride (DEC). RT is room temperature.

Compounds of formula I and II wherein X is CHO(C=O)-(C₁-C₆)-alkyl, CHO(C=O)-(C₁-C₆)alkoxy, CHO(C=O)-NH-(C₁-C₆)alkyl, CHNR⁵(C=O)-(C₁-C₆)alkyl, CHNR⁵(C=O)-(C₁-C₆)alkoxy, CHNR⁵(C=O)-NH-(C₁-C₆)alkyl or -CHOR³ (and wherein R¹⁴, R¹⁵ and R¹⁶ are hydrogen) are prepared according to Schemes 1-4:

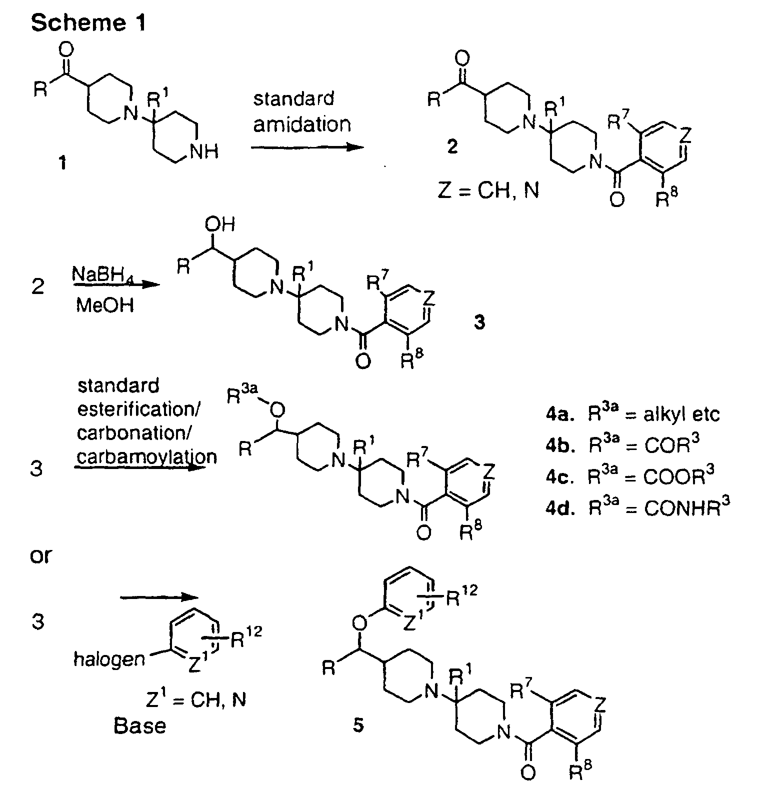

Compounds of formula **3,** wherein R, R⁷ and R⁸ are as defined for formula I, Z is CH or N, and R¹ is an alkyl group such as methyl were prepared as depicted in Scheme 1. Ketone **1,** the synthesis of which was described in WO98/05292, was subjected to standard amidation with ArCOOH, EDCI or DEC, and HOBT, or ArCOCl, wherein Ar is R⁷, R⁸-substituted phenyl or pyridyl, followed by reduction with NaBH₄ to obtain **3.** Derivatization of the free hydroxyl moiety with alkyl halides, acyl chlorides (R³COCl), alkyl chloroformates (ClCOOR³) and isocyanides (O=C=NR³) afforded ethers **4a,** esters **4b,** carbonates **4c,** and carbamates **4d,** respectively, wherein R³ is a lower alkyl group. The aryloxy compounds, **5,** were obtained after condensation of the hydroxyl **3** with phenyl or pyridyl halides in the presence of a base.

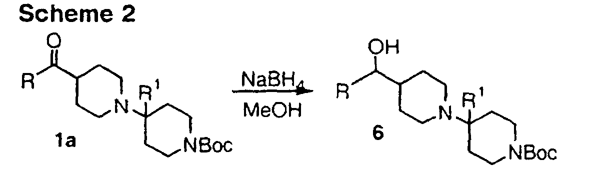

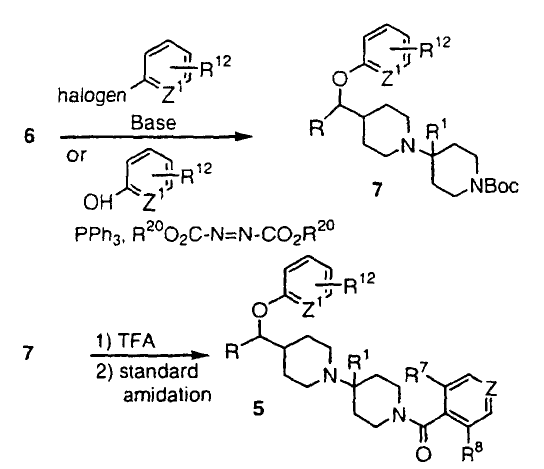

Alternatively, compounds of formula **5** can be prepared by reduction of the N-Boc ketone **1a** to the alcohol **6** first, followed by functionalization of the free hydroxyl group with a halogen-substituted aryl in the presence of a base as shown in Scheme 2, or by a hydroxy-substituted aryl or heteroaryl (wherein Z¹ is as defined in Scheme 1) in the presence of PPh₃ and an azodicarboxylate of the formula R¹⁹O₂C-N=N-CO₂R²⁰, wherein R²⁰ is C₁-C₆ lower alkyl. Removal of the Boc protecting group and conversion to the amide is performed as in Scheme 1. This route allows the introduction of various aryloxy and heteroaryloxy moieties at R³ through the use of nucleophilic displacement or Mitsunobu-type reaction on intermediate **6**.

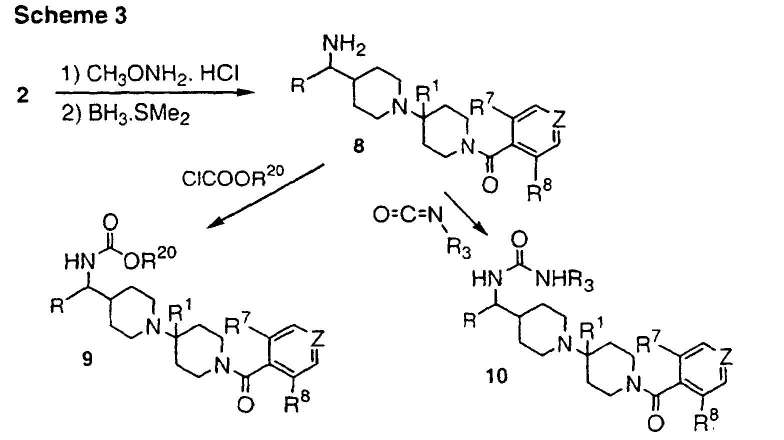

Compounds of formula **8,** wherein R, R¹, R⁷, R⁸ and Z are as described in Scheme 1, were prepared by conversion of the ketone **2** to an oxime group with CH₃ONH₂·HCl, and reduction with BH₃·S(CH₃)₂ to provide amine **8**. Derivatization of the free amine moiety with an alkyl chloroformate (CICOOR²⁰, wherein R²⁰ is C₁-C₆ alkyl) or an isocyanide (O=C=NR³) affords carbamate compounds **9** and urea compounds **10**, respectively.

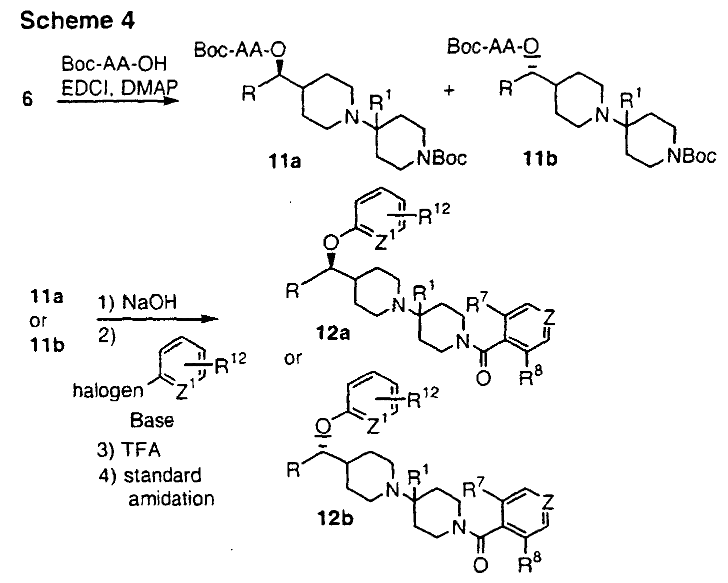

Preparation of chiral analogs was performed through chemical resolution. The alcohol 6 was coupled with a chiral Boc-protected amino acid to obtain diastereoisomers **11a** and **11b** which were separated by chromatography. The chiral auxialiary was then removed with NaOH for each diastereoisomer and the same sequence of reactions described in Scheme 2 was carried out on each individual enantiomer to obtain compounds **12a** and **12b.**

Oximes of formula I or II wherein X is C=NOR⁴ are prepared from the corresponding ketones from any of several methods known to those skilled in the art.

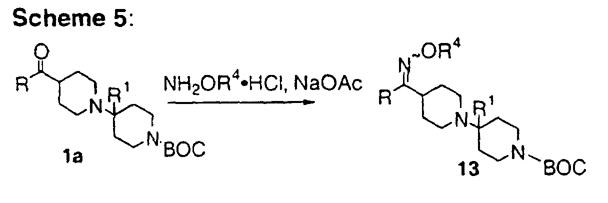

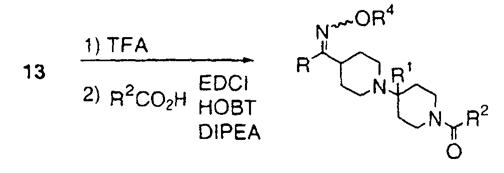

In Scheme 5, the ketone **1a,** wherein R and R¹ are as defined for formula I and II, is dissolved in a solvent such as CH₃OH or ethanol and treated with an R⁴-substituted hydroxylamine such as O-methylhydroxylamine hydrochloride in the presence of a base such as sodium acetate. The resulting mixture of Z- and E-O-substituted oximes **13** can be separated or the mixture carried through and separated at the end. The BOC protecting group is removed by treatment with an acid such as aqueous HCl or trifluoroacetic acid, and the resulting amine is coupled to an acid under standard conditions to obtain a compound of formula I or II.

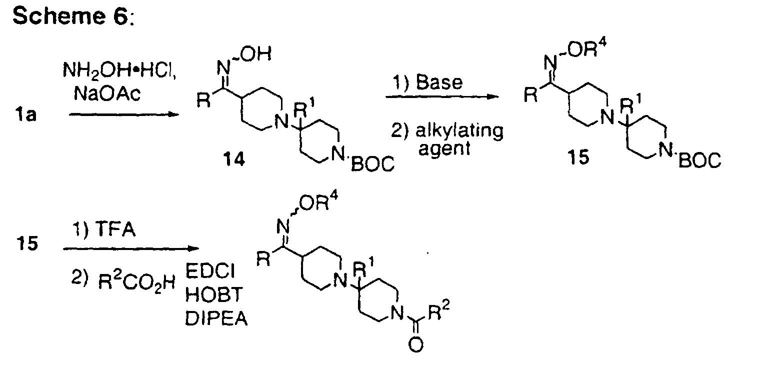

Alternatively, the ketone **1a** can be treated with HONH₂·HCl under similar conditions to yield, after separation, the E- and Z-oximes. Each oxime is then treated with a base such as potassium hexamethyldisilazide in a suitable solvent such as DMF followed by treatment with an alkylating agent, e.g., CH₃l, dimethylsulfate, CH₃CH₂l, trifluoroethyl triflate or similar electrophiles, to yield the desired O-substituted oxime.

The ketone starting material of formula **1a** can be prepared by known methods as shown in Schemes 7 and 8.

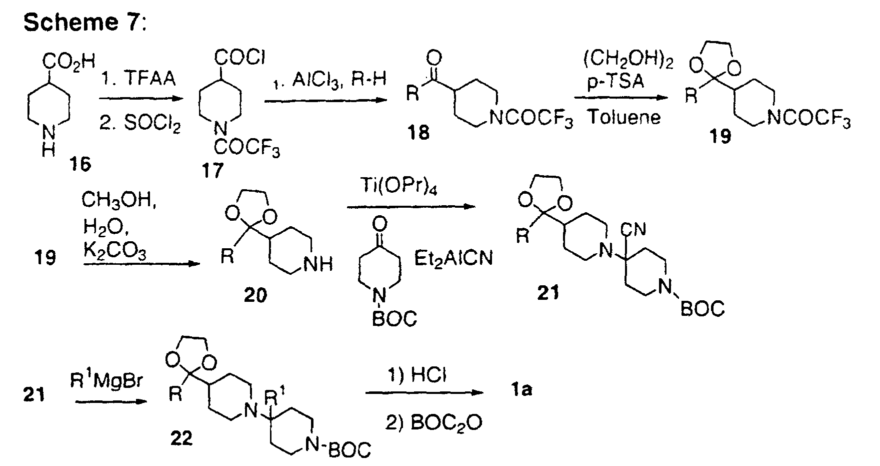

In Scheme 7, Friedel-Crafts condensation of N-trifluoroacetyl-isonipecotoyl chloride **17** and an aromatic group R-H in the presence of a suitable catalyst such as AlCl₃ and optionally in a solvent such as CH₂Cl₂ yields a ketone **18** which is converted to its ethylene ketal **19** under standard conditions. The N-trifluoroacetyl group is removed and the resulting free amine **20** is treated with N-BOC-piperidine-4-one in the presence of a dehydrating agent such as titanium isopropoxide followed by treatment with diethylaluminum cyanide to give an aminonitrile **21.** The aminonitrile is treated with a grignard reagent (R¹Mg-halide) such as CH₃MgBr or vinylmagnesium bromide to give the alkylated product **22.** The ketal is removed by treatment with aqueous acid followed by re-protection under standard conditions using BOC anhydride to give **1a.**

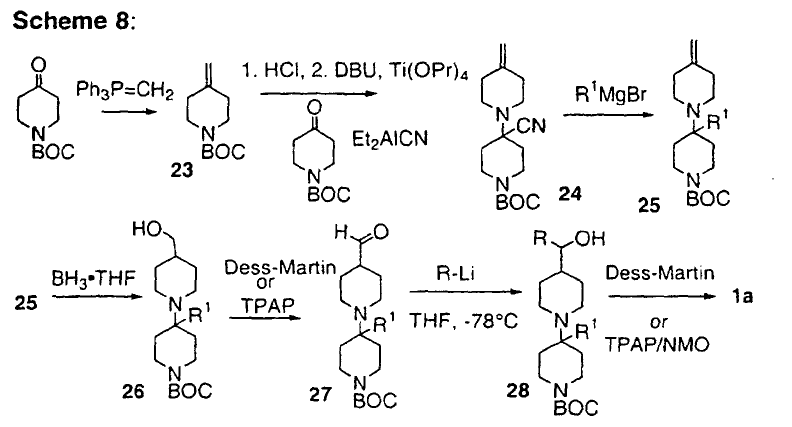

Alternatively, **23,** prepared via Wittig olefination of N-BOC-piperidone (Chen et al, Tetrahedron Lett., 37, 30 (1996), 5233-5234), is transformed to intermediate **25** by analogy to the procedure described in Scheme 7. **25** is converted to alcohol **26** by hydroboration/oxidation. Alcohol **26** is treated with a suitable oxidant such as a mixture tetrapropylammonium perruthenate (TPAP) and N-methylmorpholine N-oxide (NMO) to give aldehyde **27.** The aldehyde is treated with an aryllithium reagent in a suitable solvent such as ether or THF and the resulting alcohol **28** is treated with an oxidizing agent such as Dess-Martin periodinane or TPAP/NMO to give the desired ketone.

Compounds of formula I or II wherein X is -C(R¹³)(R¹⁹)-, wherein R and R¹⁹ are the same, or wherein R and R¹⁹ are different are prepared according to schemes 9 and 10, respectively. The schemes are exemplified by processes wherein R and R¹⁹ are each phenyl and wherein R is phenyl and R¹⁹ is CF₃-phenyl, respectively, but the general procedures apply to other R and R¹⁹ groups.

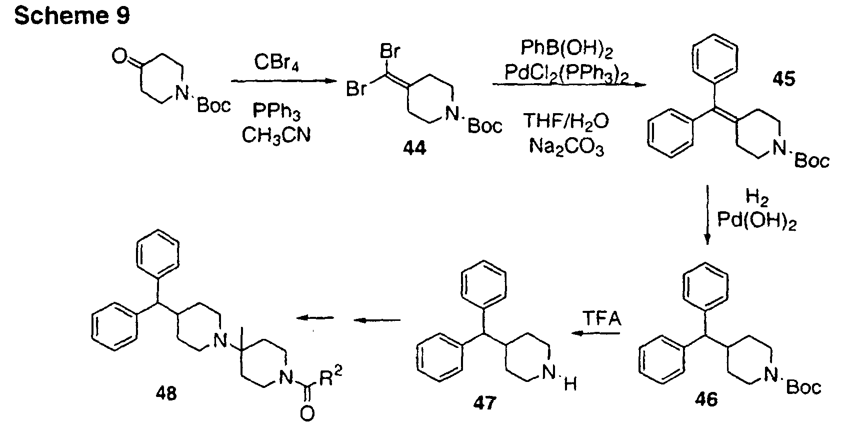

N-BOC-4-piperidone is treated with CBr₄ to obtain the di-bromo compound of formula **44,** which is then treated with phenylboronic acid to obtain the BOC-protected diphenylmethylene-piperidine of formula **45.** The methylene bond is reduced using standard conditions to obtain the BOC-protected diphenylmethyl-piperidine of formula **46,** the BOC group is removed and the amine of formula **47** is treated as described for compounds **20-22** of Scheme 7, the BOC group is removed by treatment with TFA, and the resultant amine subjected to a standard amidation procedure, e.g., treatment with a reagent R²COOH and coupling agents such as EDCI, HOBT and a base, to obtain the compounds of formula **48**.

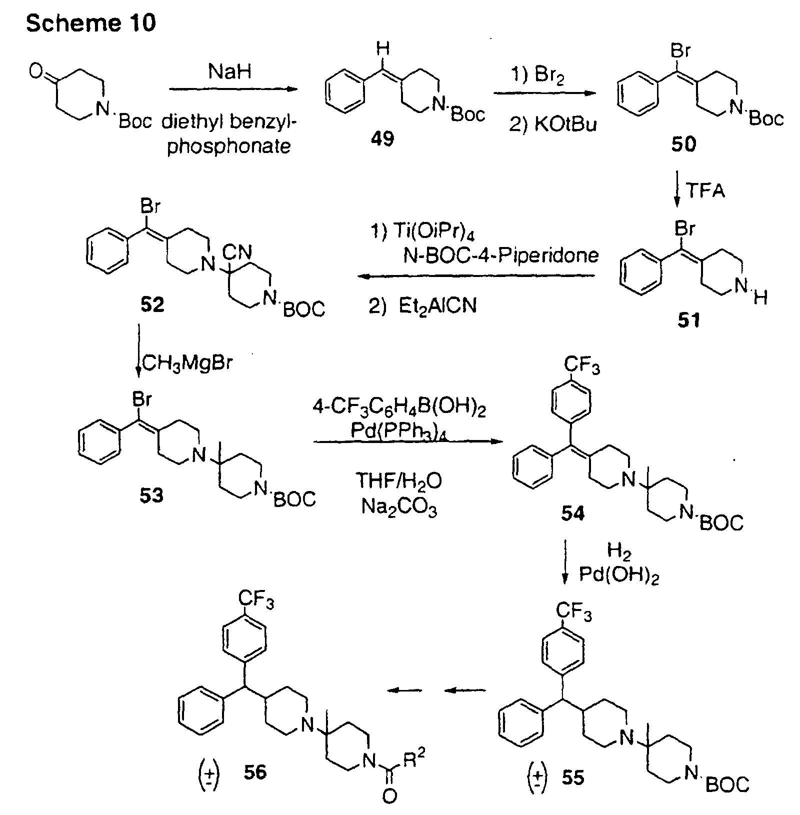

N-BOC-4-piperidone is treated with a reagent such as diethyl benzylphosphonate to obtain the phenylmethylene-piperidine of formula **49**, which is then brominated to obtain the bromophenylmethylene-piperidine of fomula **50.** The BOC protecting group is removed using standard conditions, e.g., treatment with TFA, to obtain amine **51,** and the amine **51** is treated as described for compounds **20-22** of Scheme 7 to obtain the aminonitrile **52,** then the protected amine **53.** The amine **53** is treated with a reagent such as 4-CF₃-phenylboronic acid to obtain compound **54** and the methylene bond is reduced using standard conditions to obtain racemic 55. The BOC group is removed by treatment with TFA, and the resultant amine subjected to a standard amidation procedure, e.g., treatment with a reagent R²COOH and coupling agents such as EDCI, HOBT and a base, to obtain the racemic compounds of formula **56.**

Compounds useful in this invention are exemplified by the following preparative examples, which should not be construed to limit the scope of the disclosure. Alternative mechanistic pathways and analogous structures within the scope of the invention may be apparent to those skilled in the art.

### Example 1

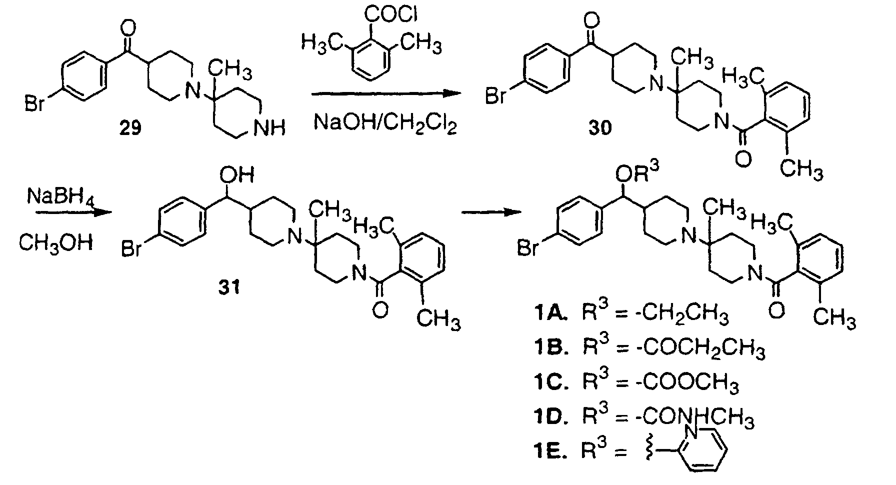

A solution of free amine **29** (1.45 g, 3.97 mmol) and 2,6-dimethylbenzoyl chloride (840 mg, 5.0 mmol) in aqueous 1 N NaOH (20 ml) and CH₂Cl₂ (20 ml) was stirred overnight at RT. The reaction mixture was extracted with CH₂Cl₂, dried over Na₂SO₄ and concentrated under high vacuum to provide **30** (1.97 g, 97%), as a slightly yellow foam.

To a solution of ketone **30** (550 mg, 1.11 mmol) in CH₃OH (6 ml) was added NaBH₄ (60 mg, 1.59 mmol) and the solution was stirred overnight at RT. The reaction mixture was then poured into 0.1 N NaOH, extracted with CH₂Cl₂, dried over Na₂SO₄, and concentrated to give **31** (543 mg, 98%), as a slightly yellow foam.

### Example 1A:

To a solution of alcohol **31** (50 mg, 0.10 mmol) in anhydrous DMF (0.5 ml) was added NaH (6.0 mg, 0.25 mmol) followed by ethyl iodide (12 µl, 0.15 mmol) and the reaction was stirred 4 h at 40 °C. The reaction mixture was poured into aqueous 0.1 N NaOH, extracted with CH₂Cl₂, dried over Na₂SO₄, and concentrated. Purification by preparative chromatography (eluting with CH₂Cl₂/CH₃OH, 9:1) yielded **1A** (31 mg, 59%) as a colorless oil: ¹H -NMR (300 MHz, CDCl₃) δ 7.39 (br d, *J* = 8.4 Hz, 2H), 7.02-7.12 (m, 3H), 6.95 (m, 2H), 3.94 (m, 1H), 3.79 (d, *J* = 7.2 Hz, 1H), 3.10-3.35 (m, 4H), 2.60-3.00 (m, 3H), 2.19 (br s, 6H), 1.60-2.10 (m, 5H), 1.05-1.50 (m, 5H), 1.08 (br t, 3H), 0.94 (s, 3H); HRMS (MH⁺) 527.2271.

### Example 1B:

To a solution of alcohol **31** (50 mg, 0.10 mmol) and pyridine (16.2 µl, 0.20 mmol) in anhydrous CH₂Cl₂ (0.5 mL) was added propionyl chloride (30 µl, 0.30 mmol) and the solution was stirred overnight at RT. The reaction mixture was treated as for **1A** to give, after preparative chromatography (eluting with CH₂Cl₂/CH₃OH, 9:1), **1B** (44.7 mg, 81%) as a colorless oil: ¹H -NMR (300 MHz, CDCl₃) δ 7.42 (br d, *J* = 8.2 Hz, 2H), 7.05-7.15 (m, 3H), 6.97 (m, 2H), 5.40 (d, *J* = 7.8 Hz, 1H), 4.09 (m, 1H), 3.43 (m, 1H), 3.23 (m, 1H), 2.96 (m, 1H), 2.82 (m, 1H), 2.70 (m, 1H), 2.21 (d, 3H), 1.60-2.10 (m, 5H), 1.05-1.45 (m, 5H), 1.08 (m, 3H), 0.95 (s, 3H); HRMS (MH⁺) 555.2230.

### Example 1C :

To a solution of alcohol **31** (29.4 mg, 0.059 mmol) and pyridine (9.5 µl, 0.118 mmol) in anhydrous CH₂Cl₂ (0.3 mL) was added methylchloro-formate (13.8 µl, 0.18 mmol) and the solution was stirred overnight at RT. The reaction mixture was treated as for **1A** to give, after preparative chromatography (eluting with CH₂Cl₂/CH₃OH, 9:1), **1C** (15 mg, 46%) as a colorless oil: ¹H -NMR (300 MHz, CDCl₃) δ 7.46 (br d, *J* = 8.4 Hz, 2H), 7.14 (d, *J* = 8.4 Hz, 2H), 7.09 (m, 1H), 6.98 (m, 2H), 5.21 (d, *J* = 7.2 Hz, 1H), 4.09 (m, 1H), 3.71 (m, 3H), 3.45 (m, 1H), 3.24 (m, 1H), 2.97 (m, 1H), 2.82 (m, 1H), 2.70 (m, 1H), 2.22 (br s, 3H), 1.60-2.10 (m, 5H), 1.10-1.50
(m, 5H), 0.95 (s, 3H); HRMS (MH⁺) 557.2017.

### Example 1D:

A solution of alcohol **31** (30 mg, 0.060 mmol), pyridine (9.7 µl, 0.12 mmol) and methylisocyanate (40 µl, 0.68 mmol) in anhydrous THF (0.3 ml) was stirred 5 h at 45 °C. The reaction mixture was treated as for **1A** to give, after preparative chromatography (eluting with CH₂Cl₂/CH₃OH, 9:1), **1D** (25 mg, 75%) as a colorless oil: ¹H -NMR (300 MHz, CDCl₃) δ 7.42 (br d, *J* = 8.2 Hz, 2H), 7.05-7.15 (m, 3H), 6.98 (m, 2H), 5.34 (m, 1H), 4.08 (m, 1H), 3.44 (m, 1H), 3.24 (m, 1H), 3.19 (s, 3H), 2.96 (m, 1H), 2.65-2.85 (m, 2H), 2.20 (br s, 3H), 1.55-2.10 (m, 5H), 1.10-1.50 (m, 5H), 0.95 (s, 3H);
HRMS (MH⁺) 556.2169.

### Example 1E:

A solution of alcohol **31** (50 mg, 0.10 mmol), NaH 60% in mineral oil (6 mg, 0.15 mmol), and 2-chloropyridine (28.2 µl, 0.30 mmol) in anhydrous DMF (0.5 ml) was stirred 16 h at 90 °C. The reaction mixture was treated as for **1A** to give, after preparative chromatography (eluting with CH₂Cl₂/CH₃OH, 9:1), **1E** (50 mg, 86%) as a colorless oil: ¹H -NMR (300 MHz, CDCl₃) δ 7.98 (m, 1H), 7.47 (br t, *J* = 7.2 Hz, 1H), 7.38 (d, *J* = 8.0 Hz, 2H), 7.21 (d, *J* = 8.0 Hz, 2H), 6.95-7.15 (m, 3H), 6.65-6.80 (m, 2H), 5.74 (br d, *J* = 7.0 Hz, 1H), 4.09 (m, 1H), 3.44 (m, 1H), 3.24 (m, 1H), 2.65-3.05 (m, 3H), 2.22 and 2.23 (s, 3H), 1.60-2.15 (m, 5H), 1.10-1.50 (m, 5H), 0.87 (s, 3H); HRMS (MH⁺) 576.2230.

Using similar procedures, compounds of the following structure were prepared 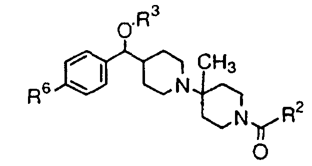 wherein R³, R⁶ and R² are as defined in the table:

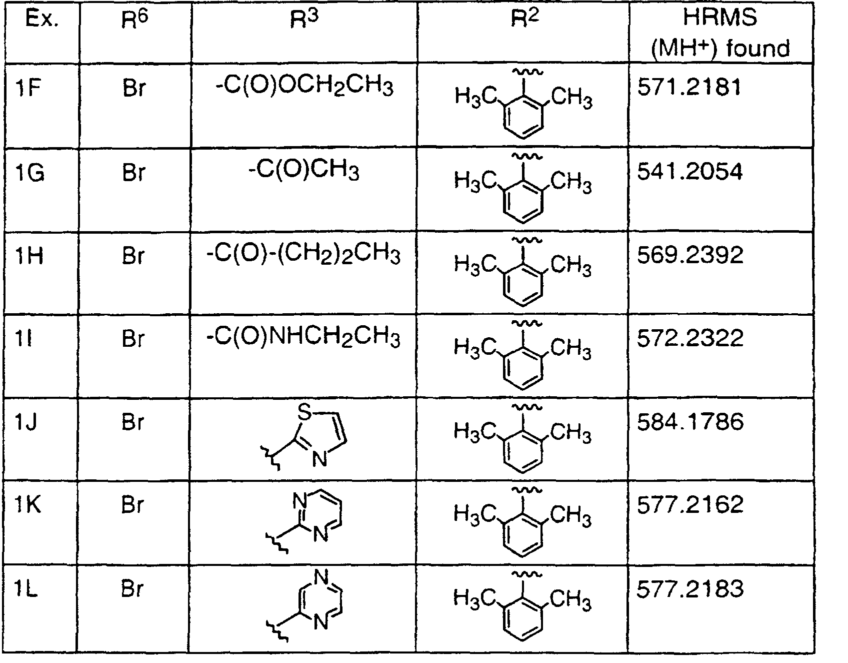

Additional data for compounds of Example 1:

| Ex. | ¹H-NMR (300 MHz ¹H NMR (CDCl₃)) |
|---|---|
| 1J | 7.49 (d, *J* = 8.4 Hz, 2H), 7.20-7.35 (m, 3H), 7.15 (m, 1H), 7.04 (m, 2H), 6.64 (d, *J* = 4.5 Hz, 1H), 5.58 and 5.60 (d, *J* = 7.2 Hz, 1H), 4.13 (m, 1H), 3.25-3.60 (m, 2H), 2.70-3.10 (m, 3H), 2.28 and 2.29 (s, 3H), 1.65-2.20 (m, 5H), 1.20-1.55 (m, 5H), 0.92 (br s, 3H) |
| 1K | 8.39 (d, *J* = 5.6 Hz, 1H), 7.42 (d, *J* = 8.4 Hz, 2H), 7.27 (d, *J* = 8.4 Hz, 2H), 7.05-7.20 (m, 2H), 6.99 (m, 2H), 6.84 (m, 1H), 5.70 (d, *J* = 7.8Hz, 1H), 4.11 (m, 1H), 3.43 (m, 1H), 3.25 (m, 1H), 2.65-3.05 (m, 3H), 2.23 and 2.25 (s, 3H), 1.55-2.10 (m, 5H), 1.10-1.50(m, 5H), 0.88 (br s, 3H) |

### Example 2

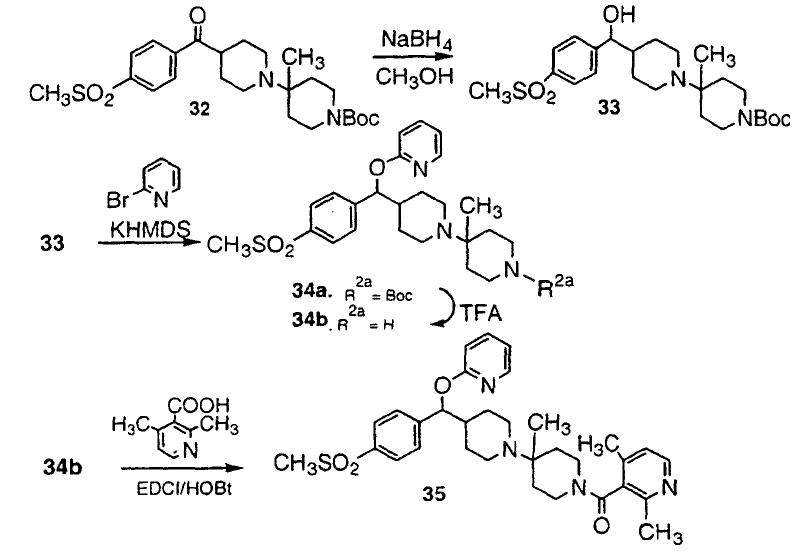

A solution of ketone **32** (0.60 g, 1.29 mmol) and NaBH₄ (60 mg, 1.59 mmol) in CH₃OH (5 ml) was stirred overnight at RT. The reaction mixture was poured into 0.1 N NaOH, extracted with CH₂Cl₂, dried over Na₂SO₄, and concentrated to give **33** (0.60 g, 100%), as a white foam.

To a solution of alcohol 33 (543 mg, 1.2 mmol) in anhydrous toluene (4 ml) was added KHMDA, 0.5 N in toluene (2.6 ml, 1.30 mmol) followed, 15 min. later, by 2-bromopyridine (125 µl, 1.30 mmol). The reaction was heated 5 h at 60 °C, cooled to RT and poured into 5% aqueous NaHCO₃ (25 ml). Extraction with CH₂Cl₂, drying over Na₂SO₄ and concentration afforded an oil which was purified by flash chromatography over silica gel (eluting with CH₂Cl₂/AcOEt/Et₃N 50:50:1 to 40:60:1) to yield **34a** (310 mg, 49%), as a yellow foam.

A solution of **34a** (310 mg, 0.57 mmol) in anhydrous CH₂Cl₂ (2 ml) and TFA (2 ml) was stirred 30 min. at RT. After concentration, the residue was taken up in aqueous 1 N NaOH, extracted with CH₂Cl₂, dried over Na₂SO₄ and concentrated to give **34b** (220 mg, 87%), as a white foam.

A solution of free amine **34b** (85 mg, 0.19 mmol), 2,4-dimethylnicotinic acid (50 mg, 1.45 mmol), DEC (60 mg, 0.31 mmol), HOBT (50 mg. 0.37 mmol) and *N*-methylmorpholine (80 ml, 0.72 mmol) in anhydrous DMF (1 ml) was stirred overnight at 40 °C. After concentration, the residue was taken up in aqueous 0.1 N NaOH, extracted with CH₂Cl₂, and dried over Na₂SO₄. The residue obtained after concentration of the solvent was purified by preparative chromatography over silica gel (eluting with CH₂Cl₂/CH₃OH/NH₄OH, 96:4:1) to afford **35** (95 mg, 85%), as a colorless oil: ¹H -NMR (300 MHz, CDCl₃) δ 8.33 (d, *J* = 5.1 Hz, 1H), 7.99 (dd, *J* = 4.8 and 1.8 Hz, 1H), 7.86 (d, *J* = 8.4 Hz, 2H), 7.56 (d, *J* = 8.4 Hz, 2H), 7.53 (m, 1H), 6.96 (d, *J* = 5.1 Hz, 1H), 6.75-6.85 (m, 2H), 4.15 (m, 1H), 3.45 (m, 1H), 3.30 (m, 1H), 3.02 (s, 3H), 2.99 (m, 2H), 2.79 (m, 1H), 2.47 and 2.48 (s, 3H), 2.45 (m, 1H), 2.25 and 2.26 (s, 3H), 1.65-2.15 (m, 5H), 1.15-1.55 (m, 5H), 0.90 (s, 3H); HRMS (MH⁺) 577.2858.

Using similar procedures, compounds of the following structure were prepared 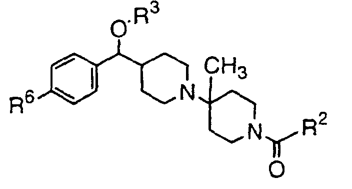 wherein R³, R⁶ and R² are as defined in the table:

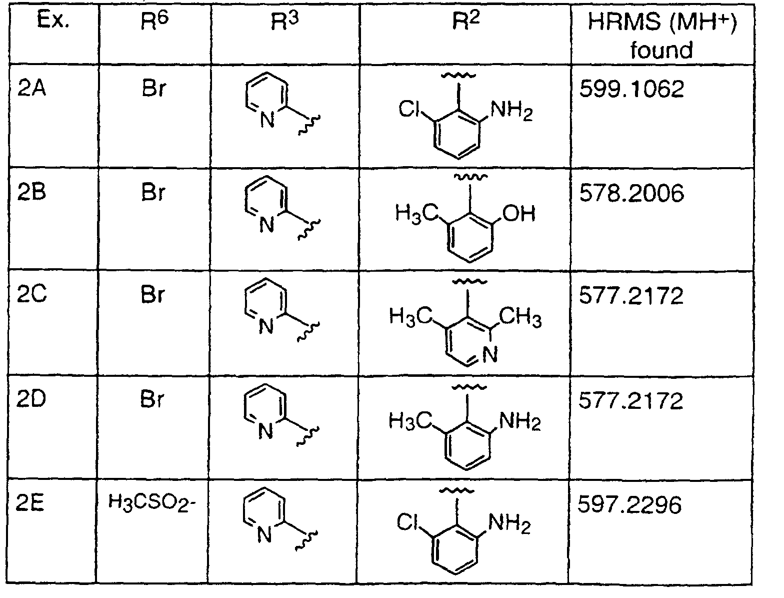

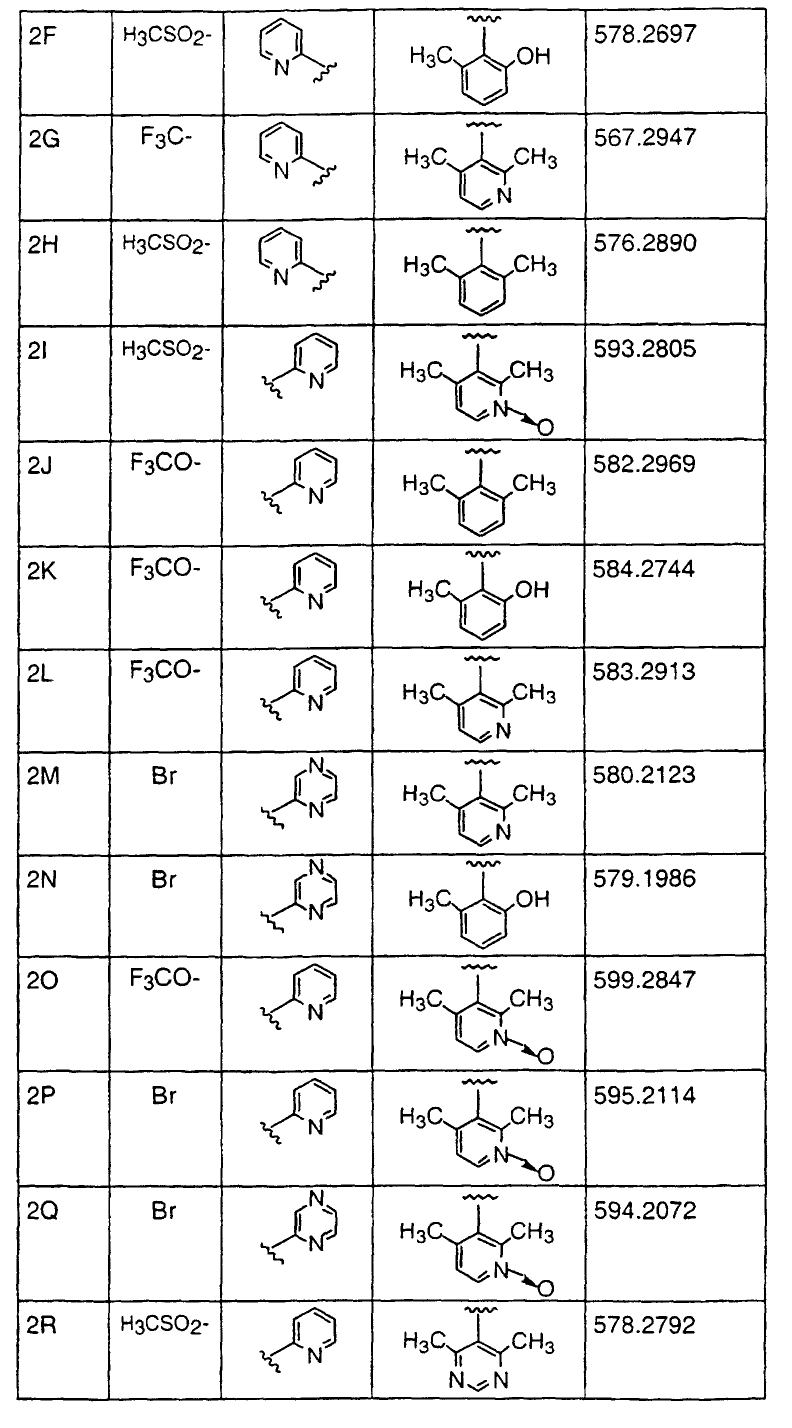

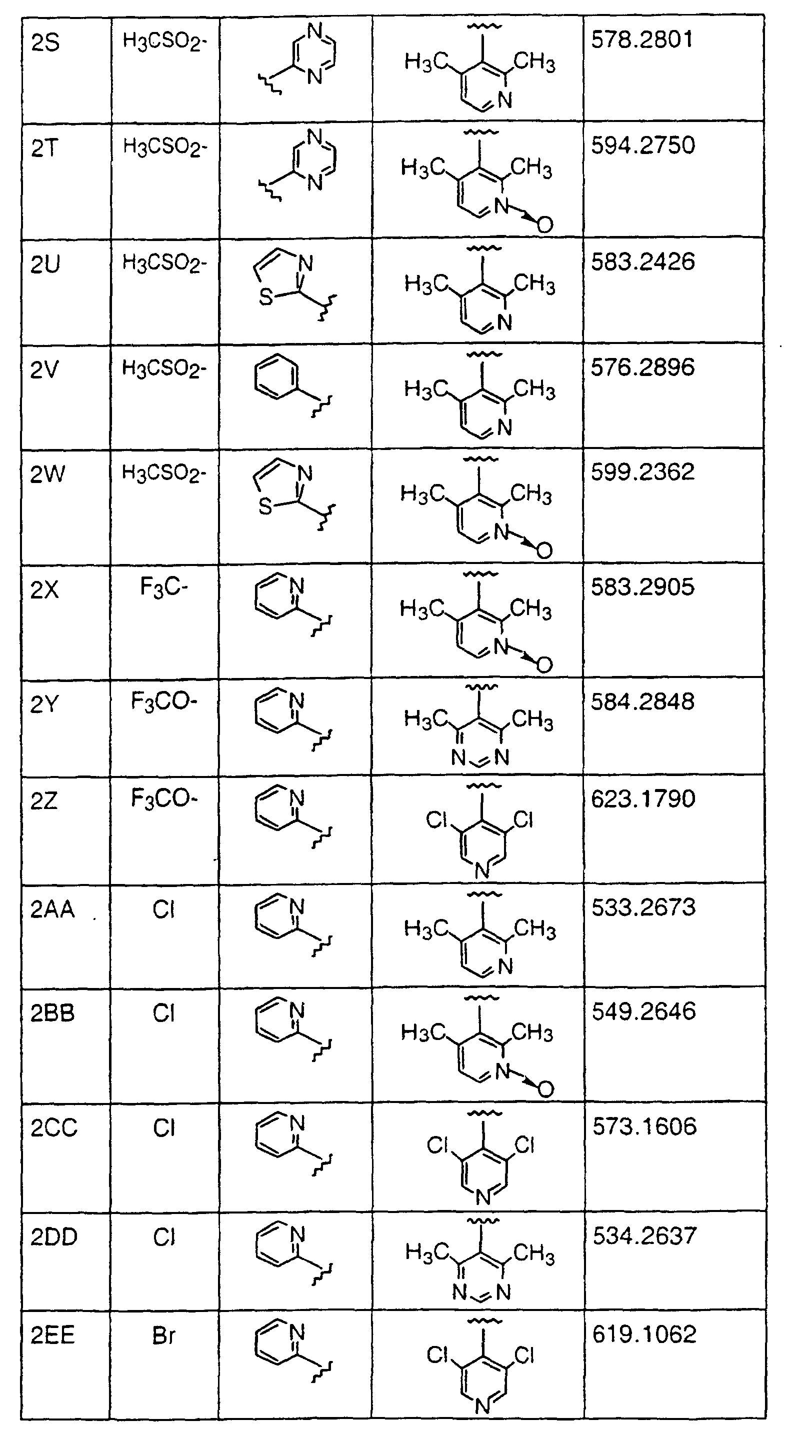

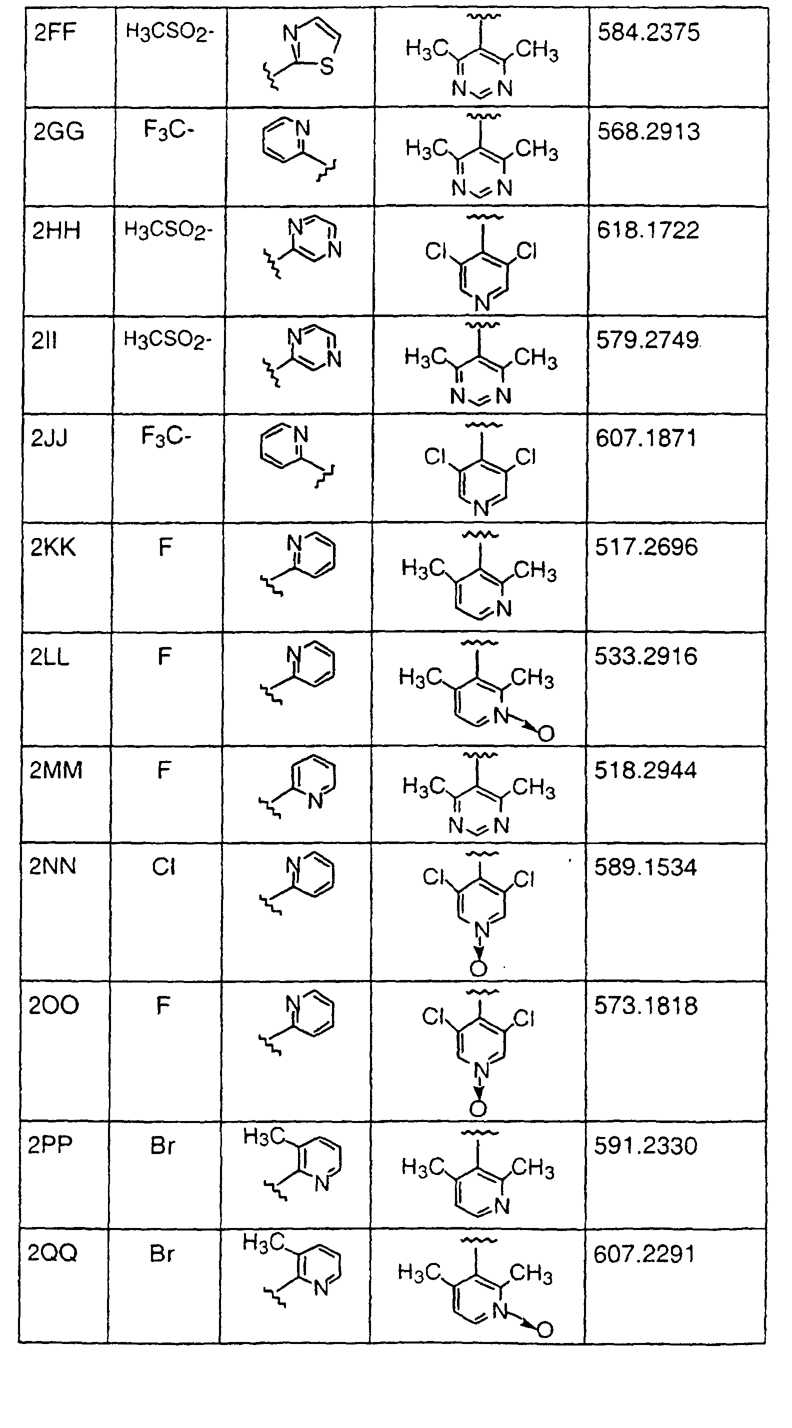

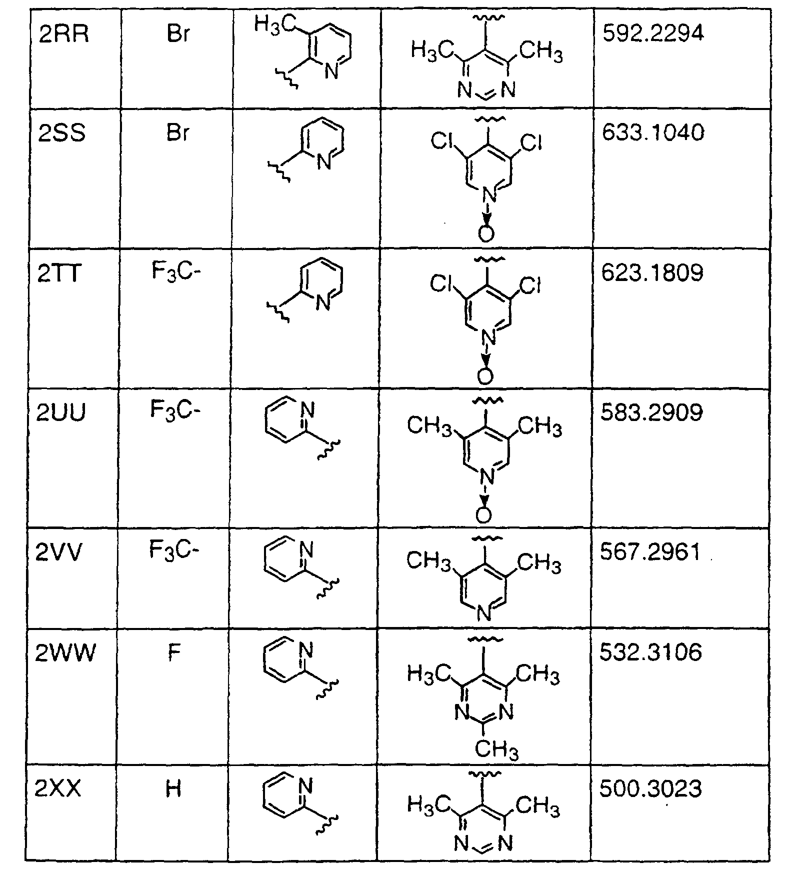

Additional data for compounds of Example 2:

| Ex. | ¹H-NMR (300 MHz ¹H NMR (CDCl₃)) |
|---|---|
| 2A | 7.98 (m, 1H), 7.49 (br t, *J* = 7.1 Hz, 1H), 7.39 (d, *J* = 8.1 Hz, 2H), 7.12 (d, *J* = 8.1 Hz, 2H), 7.01 (t, *J* = 8.4 Hz, 1H), 6.65-6.80 (m, 3H), 6.56 (d, *J* = 8.4 Hz, 1H), 5.76 (d, *J* = 7.2 Hz, 1H), 3.95-4.20 (m, 1H), 3.89 and 3.92 (s, 2H), 3.30-3.55 (m, 2H), 3.12 (m, 1H), 2.70-3.00 (m, 2H), 1.65-2.10 (m, 5H), 1.20-1.60 (m, 5H), 0.95 and 0.99 (s, 3H) |
| 2G | 8.31 (d, 1H), 8.01 (d, 1H), 7.50 (m, 4H), 6.95 (d, 1H), 6.80 (m, 2H), 5.90 (d, 1H), 4.15 (d, 1H), 3.25-3.55 (m, 2H), 2.80-3.15 (m, 3H), 2.50 (d, 3H), 2.30 (d, 3H), 1.80-2.15 (m, 7H), 1.20-1.60 (m, 5H), 0.92 (s, 3H) |
| 2K | 7.97 (m, 1H), 7.45 (m, 1H), 7.32 (t, *J* = 8.4 Hz, 2H), 7.06 (m, 2 H), 7.01 (m, 1H), 6.60-6.75 (m, 4H), 5.77 and 5.79 (d, *J* = 5.6 Hz, 1H), 3.55 (m, 1H), 3.32 (m, 1H), 2.70-2.95 (m, 2H), 2.18 (s, 3H), 1.65-2.10 (m, 5H), 1.15-1.55 (m, 5H), 0.78 and 0.91 (s, 3H) |
| 2M | 8.29 (d, *J* = 5.2 Hz, 1 H), 8.18 (m, 1H), 7.98 (br s, 1H), 7.89 (br s, 1H), 7.38 (d, *J* = 8.4 Hz, 2H), 7.18 (d, *J* = 8.4 Hz, 2H), 6.92 (d, *J* = 5.2 Hz, 1H), 5.67 (d, *J* = 7.2 Hz, 1H), 4.07 (m, 1H), 3.43 (m, 1H), 3.26 (m, 1H), 2.65-3.05 (m, 3H), 2.41 and 2.42 (s, 3H), 2.20 (br s, 3H), 1.60-2.20 (m, 5H), 1.05-1.50 (m, 5H), 0.85 (br s, 3H) |
| 2P | 8.14 (d, *J* = 6.8 Hz, 1H), 8.02 (m, 1H), 7.51 (m, 1H), 7.41 (d, *J* = 8.0 Hz, 2H), 7.25 (d, *J* = 8.0 Hz, 2H), 6.98 (d, *J* = 6.4 Hz, 1H), 6.78 (m, 1H), 6.73 (m, 1H), 5.78 (d, *J* = 6.8 Hz, 1H), 4.17 (m, 1H), 3.43 (m, 1H), 3.32 (m, 1H), 2.95 (m, 1H), 2.86 (m, 1H), 2.75 (m, 1H), 2.44 and 2.46 (s, 3H), 2.23 and 2.25 (s, 3H), 1.65-2.10 (m, 5H), 1.15-1.50 (m, 5H), 0.90 (s, 3H) |
| 2HH | 8.49 (s, 2H), 8.26 (br s, 1H), 8.04 (br s, 1H), 7.80-7.95 (m, 3H), 7.53 (d, *J* = 8.4 Hz, 2H), 5.81 (d, *J* = 6.8 Hz, 1H), 4.16 (m, 1H), 3.30-3.50 (m, 2H), 2.94 (m, 2H), 2.80 (m, 1H), 1.75-2.15 (m, 5H), 1.25-1.50 (m, 5H), 0.89 (s, 3H) |
| 2MM | 8.93 (s, 1H), 8.04 (br d, *J* = 4.8 Hz, 1H), 7.50 (m, 1H), 7.32 (m, 2H), 6.97 (m, 2H), 6.78 (m, 1H), 6.72 (m, 1H), 5.82 (m, 1H), 4.21 (m, 1H), 3.25-3.50 (m, 2H), 2.93 (m, 2H), 2.78(m, 1H), 2.44 and 2.46 (s, 3H), 1.90-2.15 (m, 3H), 1.70-1.90 (m, 2H), 1.15-1.50(m, 5H), 0.90(s, 3H) |
| 2NN | 8.17 (s, 1H), 8.01 (br d, *J* = 4.0 Hz, 1H), 7.50 (br t, *J* = 8.0 Hz, 1H), 7.20-7.35 (m, 4H), 6.78 (t, *J* = 6.8 Hz, 1H), 6.71 (m, 1H), 5.80 (d, *J* = 6.8 Hz, 1H), 4.18 (m, 1H), 3.44 (m, 1H), 3.39 (m, 1H), 3.00 (m, 2H), 2.80 (m, 1H), 1.70-2.15 (m, 5H), 1.10-1.50 (m, 5H), 0.90 (s, 3H) |
| 2PP | 8.37 (d, *J* = 6.0 Hz, 1H), 7.83 (br d, *J* = 4.6 Hz, 1H), 7.41 (d, *J* = 8.4 Hz, 2H), 7.34 (d, *J* = 6.0 Hz, 1H), 7.22 (d, *J* = 8.4 Hz, 2H), 6.97 (d, *J* = 4.6 Hz, 1H), 6.68 (br t, *J* = 6.0 Hz 1H), 5.89 (br d, *J* = 6.8 Hz, 1H), 4.20 (m, 1H), 3.20-3.50 (m, 2H), 2.97 (m, 2H), 2.78 (m, 1H), 2.47 and 2.49 (s, 3H), 2.23 and 2.26 (s, 3H), 2.23 (s, 3H), 1.65-2.15 (m, 5H), 1.15-1.55 (m, 5H), 0.90 (s, 3H) |

### Example 3

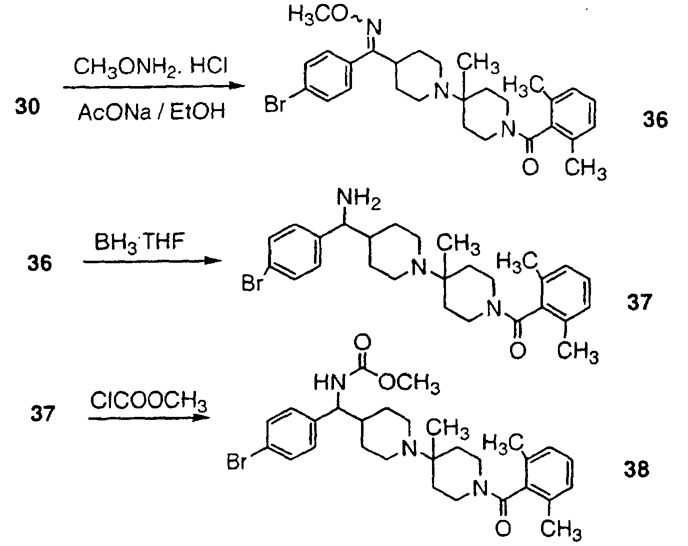

To a solution of ketone **30** (1.5 g, 3.22 mmol) in CH₃OH (50 ml) was added sodium acetate (5.0 g, 47 mmol) and *O*-Methyl hydroxylamine hydrochloride (3.26 g, 47 mmol), and the solution was stirred at RT for 24 h. The resulting mixture was then poured into aqueous NaOH and extracted with CH₂Cl₂. The combined extracts were dried, concentrated and chromatographed to yield 1.50 g (94%) of oxime **36,** as a mixture of E and Z isomers.

To a stirred solution of oxime **36** (0.200 g, 0.380 mmol) in THF (5 ml) was added BH₃•THF (1.0 M solution in THF) at 0 °C and the solution was then warmed to RT and stirred for 1 h. The reaction mixture was then cooled to 0°C and a solution of 1 N KOH in CH₃OH (5 ml) was added. The reaction was warmed slowly to 60°C for 2 h, cooled to RT, quenched with water and extracted with CH₂Cl₂. Combined organic layers were concentrated and chromatographed over silica gel (eluting with 20% EtOH/EtOAc) to afford 0.100 g (50%) of amine **37**.

To a stirred solution of amine **37** (0.015 g, 0.030 mmol) was added pyridine (0.5 ml) and ClCOOCH₃ (0.25 ml), and the solution was stirred overnight. It was then poured into water, extracted with EtOAc, dried, concentrated and purified by preparative chromatography to give 0.010 g of desired product **38:** ¹H-NMR (300 MHz, CDCl₃) δ 7.45 (d, 2H), 7.05-7.12 (m, 3H), 6.95 (d, 2H), 4.95 (m, 1H), 4.45 (m, 1H), 4.15 (m, 1H), 3.62 (s, 3H), 3.47 (m, 1H), 3.25 (m, 1H), 2.88-3.10 (m, 3H), 2.25 (s, 6H), 1.20-2.10 (m, 12H), 0.90 (s, 3H); HRMS (MH⁺) 558.3013.

### Example 4

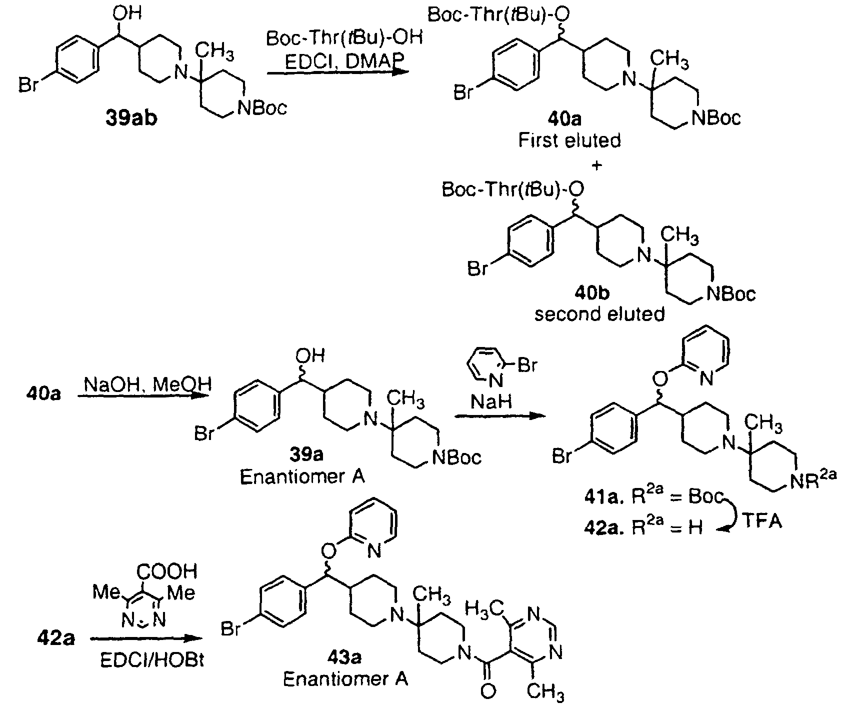

A solution of alcohol **39ab** (660 mg, 1.41 mmol), Boc-Thr(*t*-Bu)-OH (413 mg, 1.50 mmol), DEC (290 mg, 1.50 mmol) and DMAP (190 mg, 1.55 mmol) in anhydrous CH₂Cl₂ (5 ml) was stirred overnight at RT. The reaction mixture was poured into aqueous saturated NaHCO₃, extracted with CH₂Cl₂, and dried over Na₂SO₄. The residue obtained after concentration of the solvent was subjected to flash chromatography over silica gel (eluting with CH₂Cl₂/acetone, 9:1) to afford, in order of elution: (i) first **40a** (391 mg, 38%), as a white foam; (ii) second **40b** (391 mg, 38%), as a white foam.

To a solution of diastereoisomer **40a** (391 mg, 0.54 mmol) in CH₃OH (3 ml) was added NaOH (110 mg, 2.75 mmol; 5 equiv.) and the solution was stirred at 65 °C for 3 h. The final mixture was then poured into aqueous 0.1 N NaOH and extracted with CH₂Cl₂ to yield **39a** (Enantiomer A) (246 mg, 98%) as a white foam. (Following the same procedure, **40b** gave **39b** (Enantiomer B). **40a** gives **43a** (Enantiomer A) and **40b** gives **43b** (Enantiomer B.)).

A solution of alcohol **39a** (210 mg, 0.45 mmol), NaH 60% in mineral oil (23 mg, 0.96 mmol), and 2-bromopyridine (60 µl; 0.62 mmol) in anhydrous DMF (1.5 ml) was stirred 2 h at 75 °C. The reaction mixture was poured into aqueous sat'd NaHCO₃, extracted with CH₂Cl₂, dried over Na₂SO₄ and purified by flash chromatography over silica gel (eluting with CH₂Cl₂/AcOEt/Et₃N, 60:40:0.5 to 40:60:0.5) to afford **41a** (143 mg, 59%).

Removal of the Boc-protecting group in **41a** (93 mg, 0.17 mmol) proceeded as for **34b** to provide **42a** (68 mg, 91%), as a white foam.

The amine **42a** (50 mg, 0.11 mmol) was coupled with 4,6-dimethylpyrimidine-5-carboxylic acid following the conditions described for the synthesis of **35** to yield **43a** (28 mg, 44%). ¹H-NMR (300 MHz, CDCl₃) δ 8.92 (s, 1H), 8.02 (m, 1H), 7.51 (m, 1H), 7.51 (br t, *J* = 8.4 Hz, 1H), 7.41 (d, *J* = 8.4 Hz, 2H), 7.24 (d, *J* = 8.4 Hz, 2H), 6.78 (m, 1H), 6.73 (m, 1H), 5.78 (m, 1H), 4.19 (m, 1H), 3.41 (m, 1H), 3.36 (m, 1H), 2.94 (m, 1H), 2.78 (m, 1H), 2.44 and 2.46 (s, 3H), 1.65-2.15 (m, 5H), 1.15-1.50 (m, 5H), 0.90 (s, 3H)); HRMS(MH⁺) 578.2140.

The following compounds were prepared via similar methods: 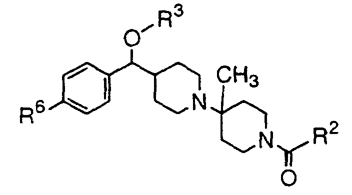 wherein R³, R⁶ and R² are as defined in the table:

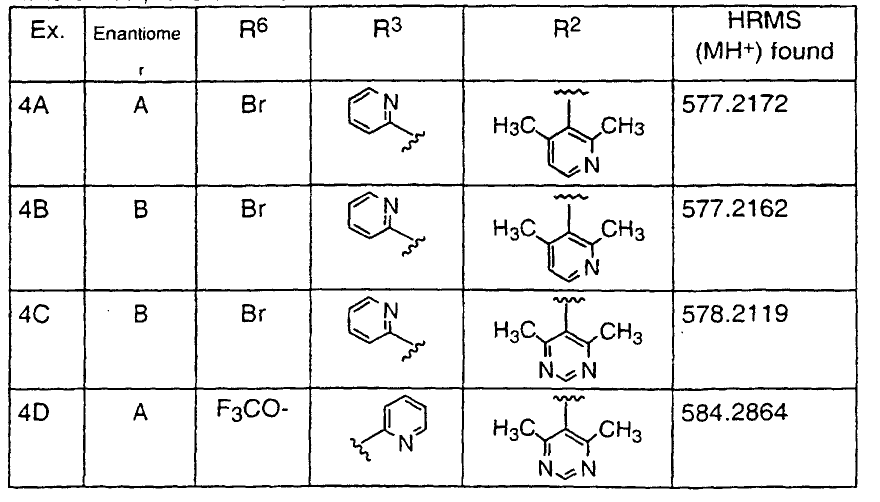

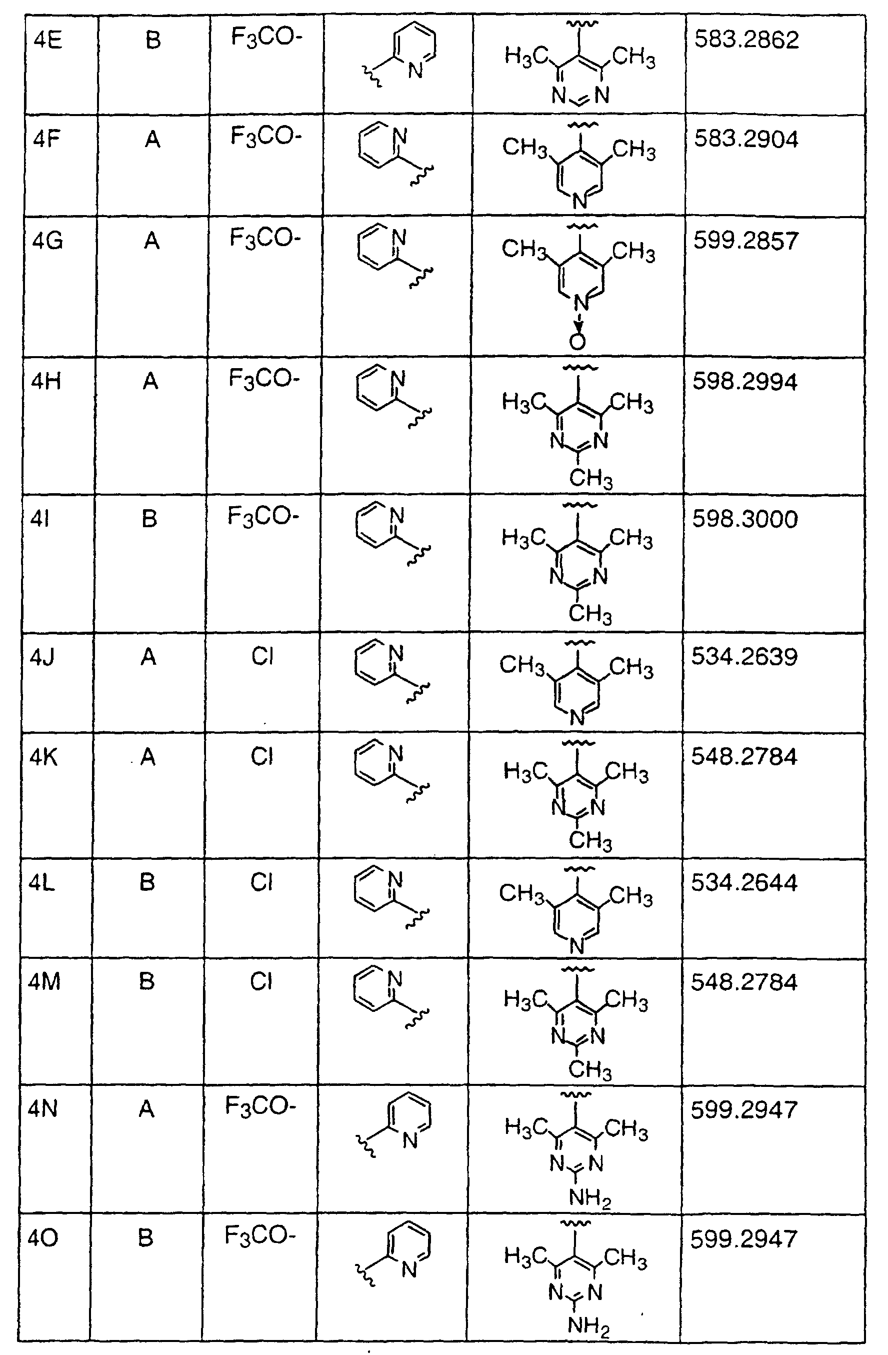

Additional data for compounds of Example 4:

| Ex. | ¹H-NMR (300 MHz ¹H NMR (CDCl₃)) |
|---|---|
| 4G | 8.05 (m, 1H), 7.97 (s, 2H), 7.53 (t, *J* = 7.5 Hz, 1H), 7.41 (d, *J* = 8.4 Hz, 2H), 7.16 (d, *J* = 8.4 Hz, 2H), 6.81 (t, *J* = 6.4 Hz, 1H), 6.76 (m, 1H), 5.87 (m, 1H), 4.19 (m, 1H), 3.30-3.50 (m, 2H), 2.99 (m, 2H), 2.79 (m, 1H), 2.20 and 2.22 (s, 3H), 1.70-2.15 (m, 5H), 1.15-1.50 (m, 5H), 0.91 (s, 3H) |
| 4I | 8.03 (m, 1H), 7.53 (m, 1H), 7.39 (d, *J* = 8.4 Hz, 2H), 7.14 (d, *J* = 8.4 Hz, 2H), 6.79 (t, *J* = 6.8 Hz, 1H), 6.73 (m, 1H), 5.87 (m, 1H), 4.19 (m, 1H), 3.42 (m, 1H), 3.37 (m, 1H), 2.98 (m, 2H), 2.80 (m, 1H), 2.41 and 2.43 (s, 3H), 1.90-2.15 (m, 3H), 1.70-1.90 (m, 2H), 1.20-1.50 (m, 5H), 0.91 (s, 3H) |

### Example 5

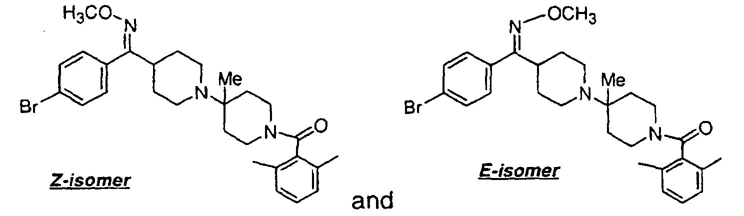

1) Trifluroacetic anhydride (TFAA) (300 ml) is added to isonipecotic acid (96 g) at 0°C and the reaction mixture is heated at reflux for 4h. Excess TFAA is removed under vacuo, the reaction mixture is taken up in EtOAc, washed with water and concentrated to give 160 g of the amide. 50 g of this amide is treated with SOCl₂ (300 ml) and the reaction mixture heated at reflux overnight. Excess thionyl chloride is then removed under vacuo to give 54 g of the acid chloride.
2) AlCl₃ (11g) is added slowly to a solution of the product of step 1 (10 g) in bromobenzene (40 ml) at ambient temperature and the reaction mixture is heated at reflux for 4 h. It is then cooled and poured into a mixture of conc. HCl and ice, and the product is extracted with EtOAc. The organic layer is separated and washed with water, half saturated NaHCO₃ solution and concentrated to give 16.21 g of the desired ketone.
3) The product of step 2 (16.21 g) is dissolved in toluene (200 ml) containing ethylene glycol (25 ml) and p-toluenesulfonic acid (0.5 g). The reaction mixture is heated at reflux with azeotropic removal of water until no further water is collected. The reaction mixture is concentrated to give 17.4 g of the desired ketal.
4) The crude product of step 3 (17.4 g) is dissolved in CH₃OH (100ml) and to this is added water (25 ml) and K₂CO₃ (12 g) and the reaction mixture is stirred at ambient temperature overnight. The reaction mixture is diluted with water and extracted with EtOAc. The organic layer is separated, washed with water and brine, and concentrated to give 12.55 g of the desired amine.
5) To a stirred solution of the product of step 4 (7.2 g, 23 mmol) and N-BOC-piperidine-4-one (4.8 g, 24 mmol) in 1,2-dichloroethane (20 ml) is added titanium isopropoxide (6.7 ml, 32.3 mmol) and the mixture is stirred for 12 h at RT. The reaction mixture is concentrated and a 1.0 M solution of diethyl aluminium cyanide (35 ml) is added at RT and stirred for 3 h. The reaction mixture is then diluted with EtOAc, quenched with water (5 ml) and stirred for 2 h. The mixture is then filtered through celite and the resulting filtrate is concentrated and chromatographed with 30 % EtOAc/hexanes to afford 7.3 g (63%) of the desired cyanide.
6) To a stirred solution of the product of step 5 (7.3 g, 14.03 mmol) in THF (100 ml) is added a 3.0M solution CH₃MgBr in Et₂O (14.0 ml, 42 mmol) at RT and the mixture is stirred for 2 h. The reaction mixture is then quenched with saturated aqueous NH₄Cl and extracted with CH₂Cl₂. The extracts are concentrated to afford 7.0 g of desired methylated compound.
7) The crude ketal of step 6 is dissolved in EtOAc (100 ml) and 6 N HCl (40 ml) and conc. HCl (10 ml) is added and the mixture stirred at RT for 24 h. The reaction mixture is then neutralised with 20%NaOH and extracted with EtOAc, dried and concentrated to yield 5.0 g (98%) of amine.
8) To a stirred solution of the product of step 7 (5.0 g, 13.6 mmol) in Et₂O (200 ml) is added 10% NaOH (50 ml) and BOC₂O, and the mixture is stirred at RT overnight. The layers are separated and the organic layer is washed with brine, dried, concentrated and chromatographed with 20% EtOAc/hexanes to yield 5.1 g (79%) of the desired product.
9) To a stirred solution of the product of step 8 (1.5 g, 3.22 mmol) in CH₃OH (50 ml) is added sodium acetate (5.0 g, 47 mmol) and O-Methyl hydroxylamine hydrochloride and the mixture is stirred at RT for 24 h. The resulting mixture is then poured into aqueous NaOH and extracted with CH₂Cl₂. The combined extracts are dried, concentrated and chromatographed to yield 1.5 g (94%) of oxime as a mixture of E and Z isomers.
10) To a stirred solution of the product of step 9 (1.5 g, 3.0 mmol) in CH₂Cl₂ (10 ml) is added TFA (3 mL) and the mixture is stirred at RT for 2 h. The reaction mixture is concentrated and poured into 10% NaOH and extracted with CH₂Cl₂. The combined extracts are dried concentrated to afford 1.2 g (100%) of amine.
11) To stirred solution of the product of step 10 (1.3 g, 3.2 mmol) in CH₂Cl₂ is added 2,6-dimethylbenzoic acid (0.74 g, 4.96 mmol), EDCI (0.94 g, 4.94 mmol), DIPEA (0.84 g, 6.58 mmol) and HOBT (0.66g, 4.94 mmol) and the mixture is stirred for 12 h at RT. The reaction mixture is quenched with NaHCO₃ and extracted with CH₂Cl₂. The combined extracts are dried and concentrated to yield 1.6 g of oxime as a mixture of E and Z isomers. The isomers are separated by chromatography by eluting withCH₂Cl₂:Et₂O (4:1) to afford 0.77 g of E isomer and 0.49 g of Z isomer.
   E isomer: 300 MHz-¹H NMR (CDCl₃) δ 7.5 (d, 2H), 7.23 (m, 2H), 7.10 (m, 1H), 6.90 (d, 2H), 4.03 (m, 1H), 3.90 (s, 3H), 3.55 (m, 1H), 3.20 (m, 3H), 3.00 (m, 3H), 2.82 (m, 1H), 2.24 (s, 3H), 2.23 (s, 3H), 2.15 (m, 3H), 1.80-1.20 (m, 5H), 0.92 (s, 3H); MS FAB+ observed= 526.2070; estimated = 526.2069
   Z isomer: 300 Mhz -¹H NMR (CDCl₃) δ 7.50 (d, 2H), 7.15 -6.95 (m, 5H), 4.15 (m, 1H), 3.80 (s, 3H), 3.45 (s, 3), 3.25 (s, 3H), 3.00 (m, 2H), 2.24 (s, 3H), 2.25 (s, 3H), 2.10 (m, 2H), 1.80- 1.50 (m, 7H), 0.92 (s, 3H);
   MS FAB+ observed= 526.2072; estimated = 526.2069.

The following compounds were prepared via similar methods: 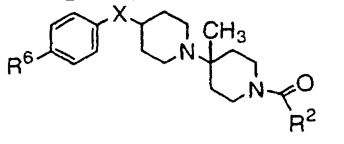 wherein X, R⁶ and R² are as defined in the table:

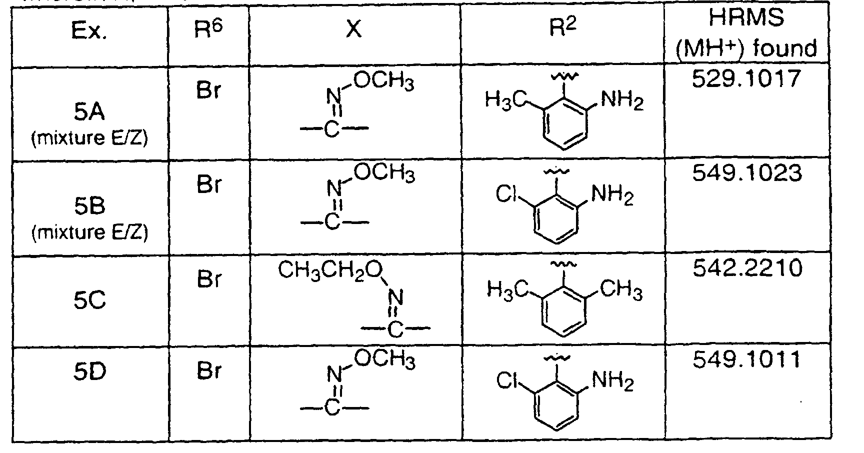

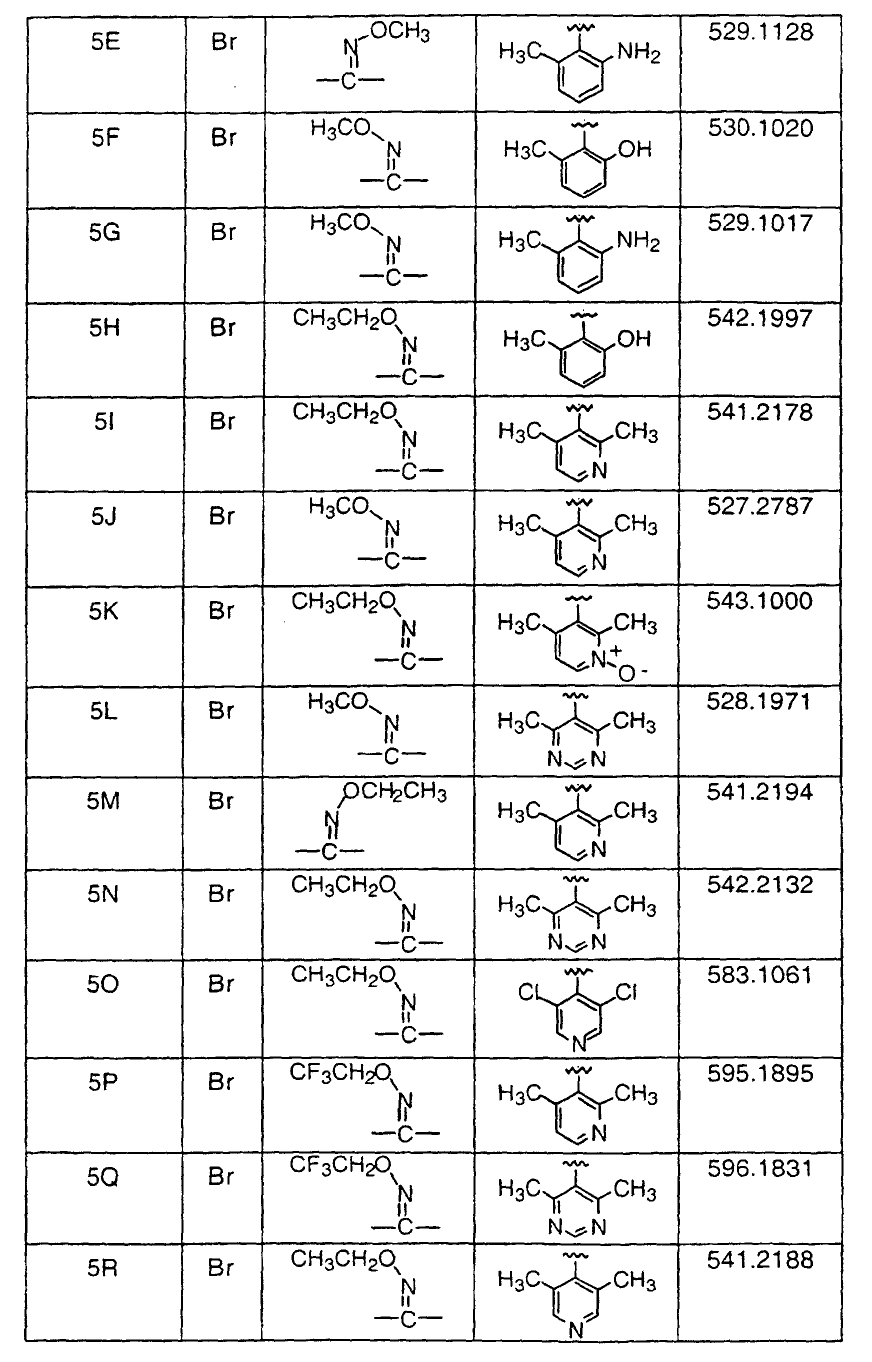

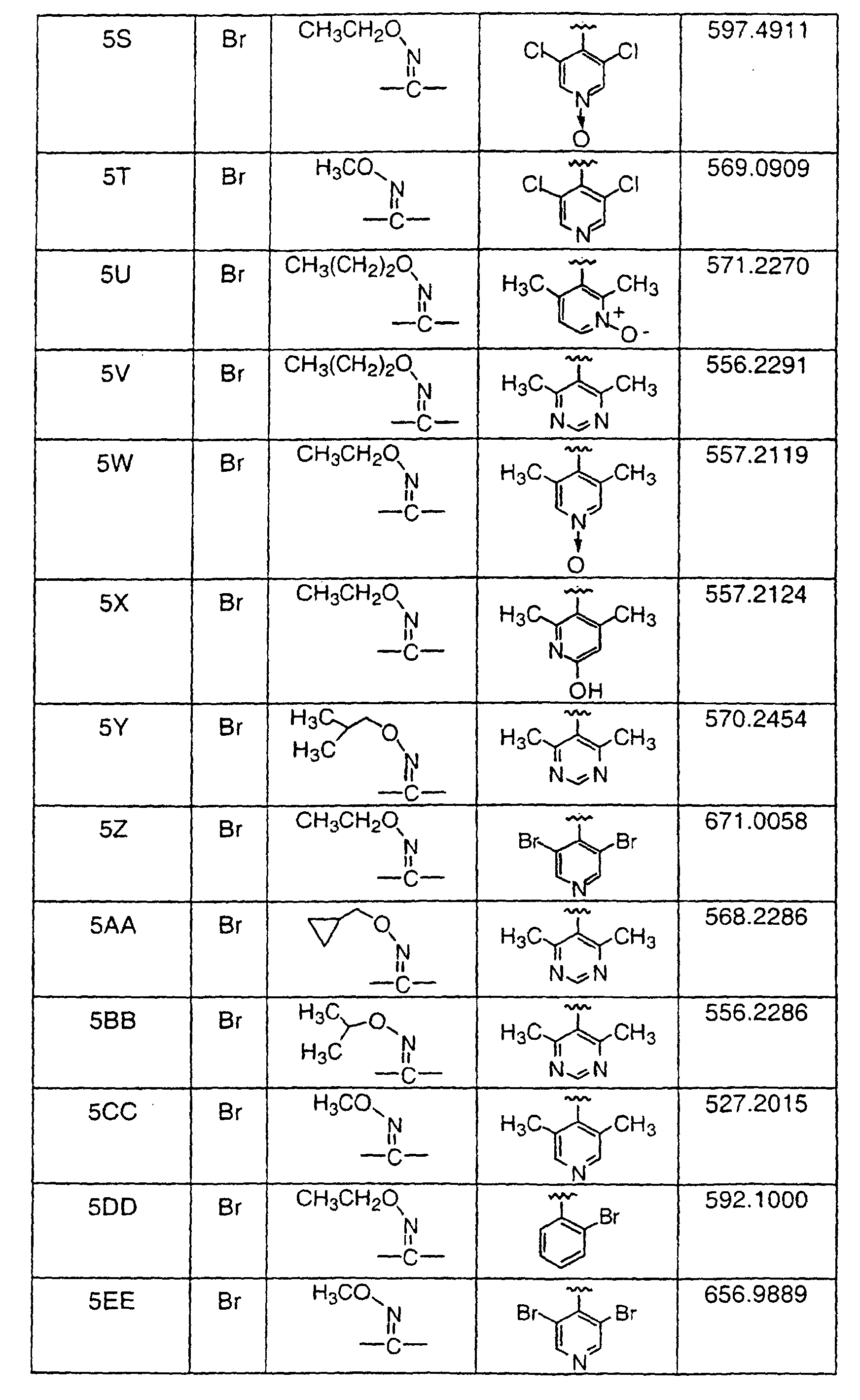

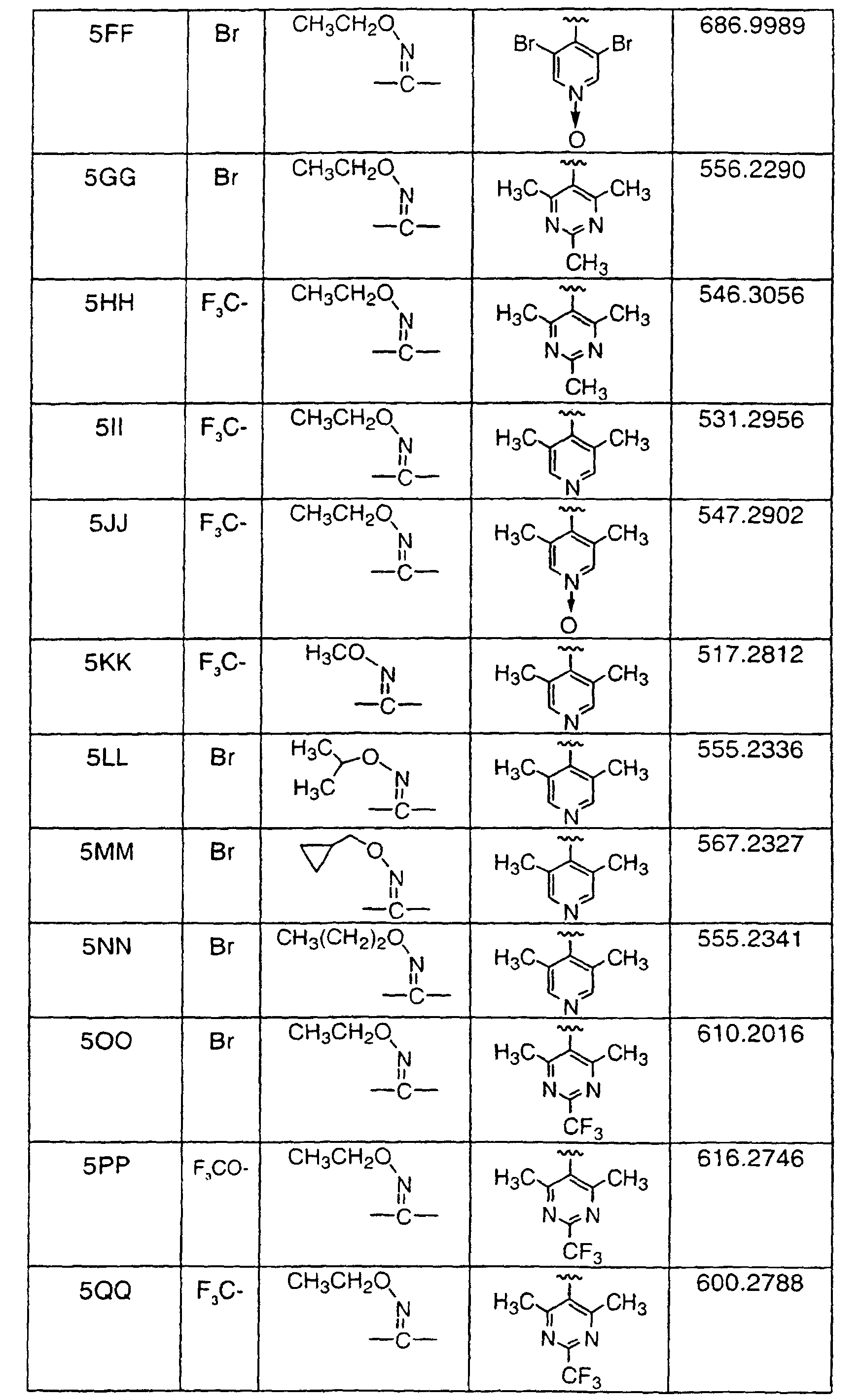

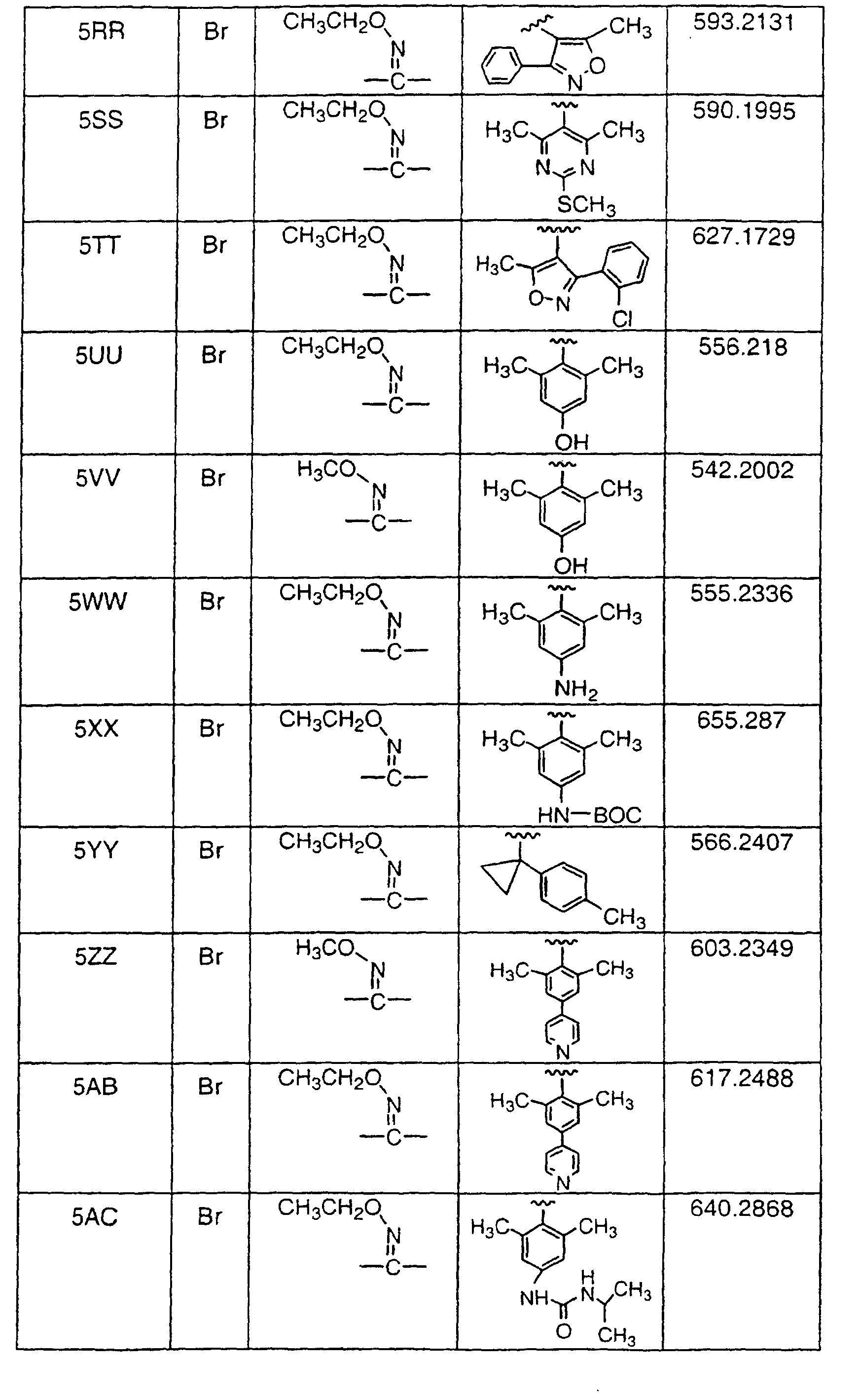

Additional data for compounds of Example 5:

| Ex. | ¹H-NMR (300 MHz ¹H NMR (CDCl₃)) |
|---|---|
| 5J | 7.50 (d, 2H), 7.15 -6.95 (m, 5H), 4.15 (m, 1H) 3.80 (s, 3H), 3.45 (s, 3H), 3.25 (s, 3H), 3.00 (m, 2H), 2.24 (s, 3H), 2.25 (s, 3H), 2.10 (m, 2H), 1.80- 1.50 (m, 7H), 0.92 (s, 3H) |
| 5L | 8.95 (s, 1H), 7.53 (d, *J* = 8.4 Hz, 2H), 7.10 (d, *J* = 8.4 Hz, 2H), 4.24 (m, 1H), 3.81 (s, 3H), 3.98 (m, 2H), 2.75-3.00 (m, 3H), 2.48 (s, 3H), 2.45 (s, 3H), 1.99 -2.20 (m, 4H), 1.73 (m, 3H), 1.20-1.62 (m, 4H), 0.94 (s, 3H) |
| 5N | 8.92 (s, 1H), 7.45 (d, *J* = 9.0 Hz, 2H), 7.10 (d, *J* = 8.7 Hz, 2H), 4.21 (m, 1H), 4.02 (q, *J* = 6.9 Hz, 2H), 3.98 (m, 2H), 2.75-2.92 (m, 3H), 2.46 (s, 3H), 2.41 (s, 3H), 1.90 -2.20 (m, 4H), 1.73 (m, 3H), 1.27-1.62 (m, 4H), 1.15 (t, *J* = 8.1 Hz, 3H), 0.93 (s, 3H) |

### Example 6

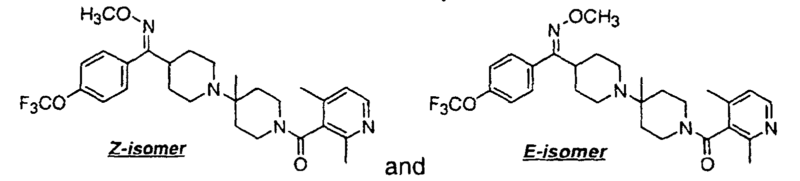

A) Preparation of intermediate **27** (Scheme 8 (R¹ = CH₃)).
   1) **23** (40.0 g, 0.203 mol) is vigorously stirred in EtOAc (200 ml) and concentrated aqueous HCl (80 ml) for 1.5 h. The solution is concentrated, diluted with Et₂O (300 ml) and H₂O (150 ml), the aqueous layer is separated and the organic layer is extracted once with H₂O (20 ml). Combined aqueous layers are concentrated and the residue is dried 24 h under high vaccum to provide 26.7 g (84%) of a white solid. To this hydrochloride and N-*tert*-butoxycarbonyl-4-piperidone (43.8 g, 0.22 mol) in anhydrous ClCH₂CH₂Cl (80 mL) with 4 Å molecular sieves, are successively added DBU (33.2 ml, 0.22 mol) and titanium(IV) isopropoxide (65.5 ml, 0.22 mol) at 0° C, the reaction mixture is allowed to warm to RT and is stirred overnight at RT. The mixture is then cooled to 0 °C and diethylaluminum cyanide, 1 N in toluene (260 ml, 0.26 mol) is added with vigorous stirring. The reaction is allowed to warm to RT and stirred an additional 3 h, after which are added CH₂Cl₂ (300 ml), EtOAc (300 ml), and Celite (50 g). The reaction mixture is cooled to 0 °C, water (40 ml) is added slowly with vigorous stirring and, after an additional 5 min. stirring at RT, the excess of water is quenched with Na₂SO₄. The final mixture is then filtered over Celite, evaporated and subjected to flash chromatography over silica gel (eluting with Hexanes/EtOAc, 8:2), to provide 50.3 g (83%) of **24** as a colorless oil which solidifies upon standing.
   2) To a solution of **24** (27.7 g, 90.6 mmol) in anhydrous THF (200 mL) at 0 °C is slowly added CH₃MgBr 3 M in Et₂O (91 ml, 3 equiv.) with vigorous stirring. After the addition, the reaction is allowed to warm to RT and stirred 3 h. The reaction is then poured into aqueous saturated NH₄Cl, extracted with Et₂O (4 times), washed with brine, dried over Na₂SO₄, and concentrated to give 27.1 g (100%) of **25** as a colorless oil.
   3) To a solution of **25** (11.6 g, 39.3 mmol) in anhydrous THF (50 ml) at 0 °C is slowly added BH₃·S(CH₃)₂ 2 N in THF (14 ml, 28 mmol) and the solution is stirred 2 days at RT. The final mixture is concentrated to ca. 50 ml and slowly poured into ice-cooled EtOH/THF 1:1 (50 ml). After 15 min. at 0 °C, 50 ml of a pH 7 buffer solution are added, followed slowly by 30% H₂O₂ aqueous solution (50 ml). The reaction mixture is stirred overnight at RT, diluted with 1 N NaOH and extracted with CH₂Cl₂. Combined organic layers are dried over Na₂SO₄, concentrated, then subjected to flash chromatography over silica gel (eluting with EtOAc/EtOH, 8:2) to yield 9.69 g (79%) of **26** as a colorless oil.
   4) A solution of **26** (11.2 g, 35.8 mmol) and *N*-methylmorpholine *N*-oxide (4.67 g, 39.4 mmol) in anhydrous CH₂Cl₂ (100 ml) is stirred 1 h at RT, cooled to 0 °C, and TPAP (885 mg) is added portionwise. The reaction is allowed to warm to RT and stirred 1 h. Additional *N*-methyl-morpholine *N*-oxide (1.30 g, 11 mmol) and TPAP (300 mg) are then added to drive the reaction to completion after 1 h. The reaction mixture is filtered over Celite, concentrated, then subjected to flash chromatography over silica gel (eluting with CH₂Cl₂/acetone, 8:2 to 7:3) to provide 5.91 g (53%) of **27** as a yellow oil.
B) Preparation of title compounds of Example 6.
   1) A solution of 1-bromo-4-(trifluoromethoxy)-benzene (4.20 ml, 28.0 mmol) in anhydrous THF (100 mL) is cooled to -78 °C and n-BuLi 2.5 N in hexanes (11.2 ml, 28.0 mmol) is added via syringe. The reaction mixture is allowed to warm to -50 °C for 10 min, cooled to -78 °C, and a solution of aldehyde **27** (6.20 g, 20.0 mmol) in anhydrous THF (15 ml) is added dropwise. After stirring 30 min at -78 °C, then 30 min at -20 °C, the solution is poured into half-brine and extracted with CH₂Cl₂ (3 x 100 ml). Combined organic layers are dried over Na₂SO₄, and concentrated to give 8.85 g (94%) of an alcohol as a yellow oil.
   2) To a solution of the product of step 1 (8.85 g, 39.3 mmol) in CH₂Cl₂ (100 ml) at 0 °C is added Dess-Martin periodinane (19.70 g, 2.5 equiv.) and the reaction mixture is stirred 2 h at RT. An additional 8.0 g of Dess-Martin periodinane is added and the reaction is stirred for an additional 4 h. The solution is poured into a 1:1 mixture of aqueous saturated NaHCO₃ and aqueous saturated Na₂S₂O₃ (200 ml), stirred 10 min, extracted with CH₂Cl₂, and dried over Na₂SO₄. The residue obtained after concentration of the solvents is purified by flash chromatography over silica gel (eluting with hexanes/EtOAc, 7:3) to yield 5.48 g (63%) of the ketone as a yellow oil.
   3) A solution of the product of step 2 (2.85 g, 6.05 mmol), HONH₂·HCl (2.08 g, 30 mmol), and AcONa (2.46 g, 30 mmol) in EtOH (50 mL) is heated at reflux under N₂ for 4 h. After evaporation of the solvent, the residue is taken up in aqueous 0.1 N NaOH and extracted with CH₂Cl₂. The residue obtained after evaporation of the solvents is subjected to flash chromatography over silica gel, to afford first the E-hydroxime (eluting with CH₂Cl₂/EtOAc, 7:3; 0.84 g; 29%), then the Z-hydroxime (eluting with CH₂Cl₂/EtOAc 1:1; 1.10 g; 37%), both products as white solids.
   4) To a suspension of Z-hydroxime (0.89 g, 1.84 mmol) in anhydrous DMF (5 ml) is slowly added KHMDA 0.5 N in toluene (4.0 ml, 2.02 mmol) at 0 °C, leading to the appearance of a yellow solution. After 2 min. at this temperature, dimethylsulfate (350 µl, 3.7 mmol) is slowly added and the solution is allowed to warm to RT and stirred 1 h. The mixture is poured into aqueous 0.1 N NaOH, extracted with CH₂Cl₂, and dried over Na₂SO₄. The residue obtained after concentration of the solvents is purified by flash chromatography over silica gel (eluting with hexanes/EtOAc, 75:25) to afford 0.55 g (62%) of the Z-methoxime as a slighly yellow oil.
   5) A solution of Z-methoxime (0.59 g, 1.18 mmol) in anhydrous CH₂Cl₂ (6 ml) and TFA (3 ml) is stirred 1 h at RT. After concentration, the residue is taken up in aqueous 1 N NaOH, extracted with CH₂Cl₂, dried over Na₂SO₄ and concentrated to give 0.47 g (100%) of the free amine as a white foam.
   6) A solution of the product of step 5 (470 mg, 1.18 mmol), 2,4-dimethylnicotinic acid (220 mg, 1.45 mmol), DEC (280 mg, 1.45 mmol), HOBT (243 mg, 1.80 mmol) and *N*-methylmorpholine (0.33 ml, 3.0 mmol) in anhydrous DMF is stirred 14 h. After concentration, the residue is taken up in aqueous 0.1 N NaOH, extracted with CH₂Cl₂, and dried over Na₂SO₄. The residue obtained after concentration of the solvent is purified by flash chromatography over silica gel (eluting with CH₂Cl₂/acetone, 7:3 to 1:1) to afford 640 mg (100%) of a colorless oil.
      ¹H-NMR (400 MHz, CDCl₃) δ 8.35 (d, *J* = 7.8 Hz, 1H), 7.25 (AB system, 4H), 6.98 (d, *J* = 7.8 Hz, 1H), 4.22 (m, 1H), 3.82 (s, 3H), 3.43 (m, 1H), 3.33 (m, 1H), 2.99 (m, 2H), 2.85 (m, 1H), 2.49 (s, 3H, atropisomer a) and 2.51 (s,3H, atropisomer b), 2.26 (s, 3H, atropisomer a) and 2/28 (s, 3H, atropisomer b), 1.95-2.21 (m, 3H), 1.20-1.90 (m, 7H), 0.92 (s, 3H).
      HRMS (M+H⁺) 533.2747.

Following steps B-4, B-5, and B-6 using the E-oxime yields the corresponding E-methoxime product.

The following compounds are prepared via similar procedures: 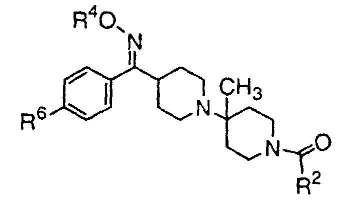 wherein R⁴, R⁶ and R² are as defined in the table:

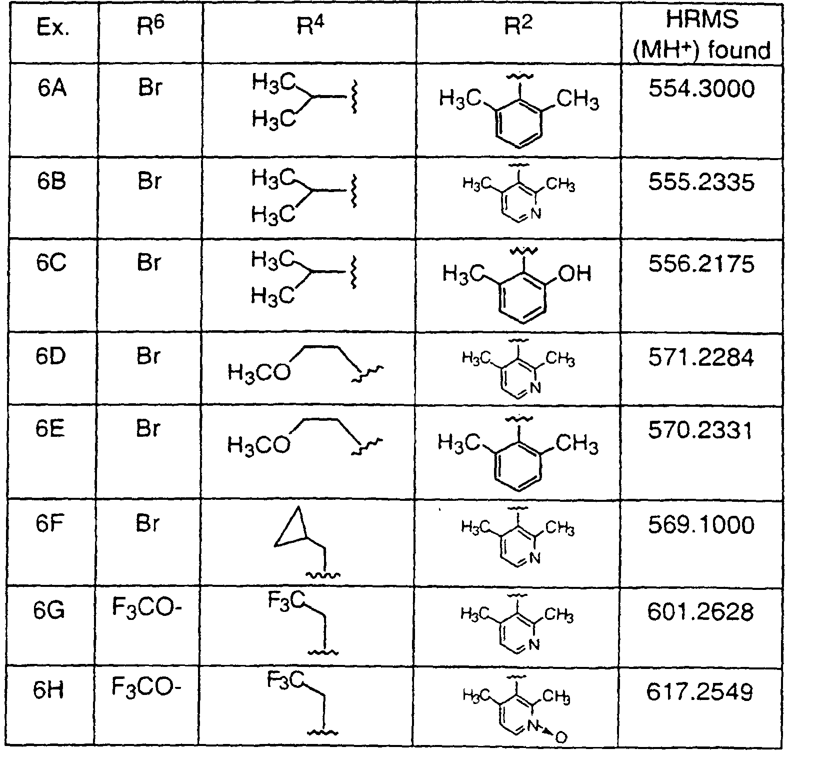

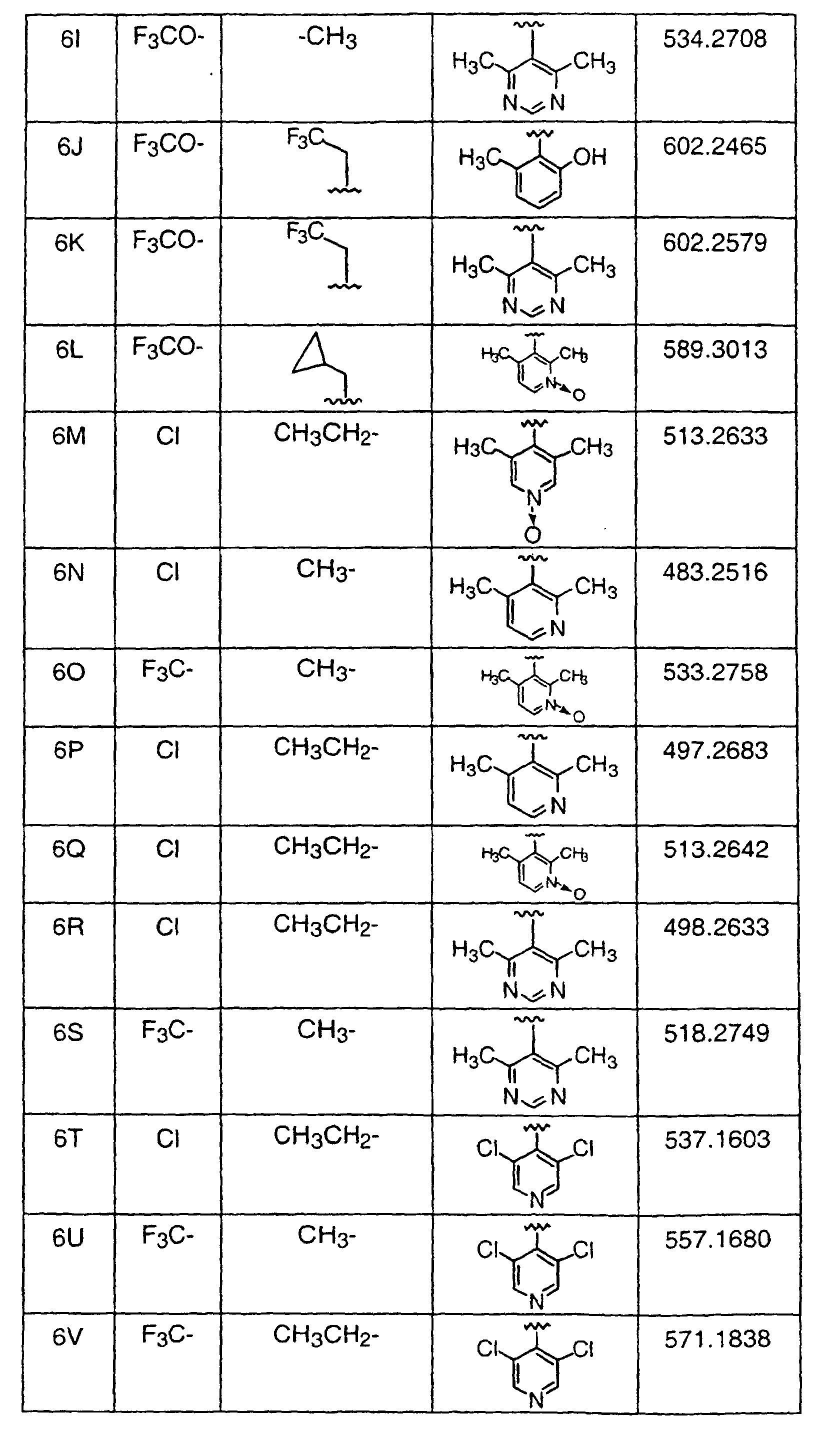

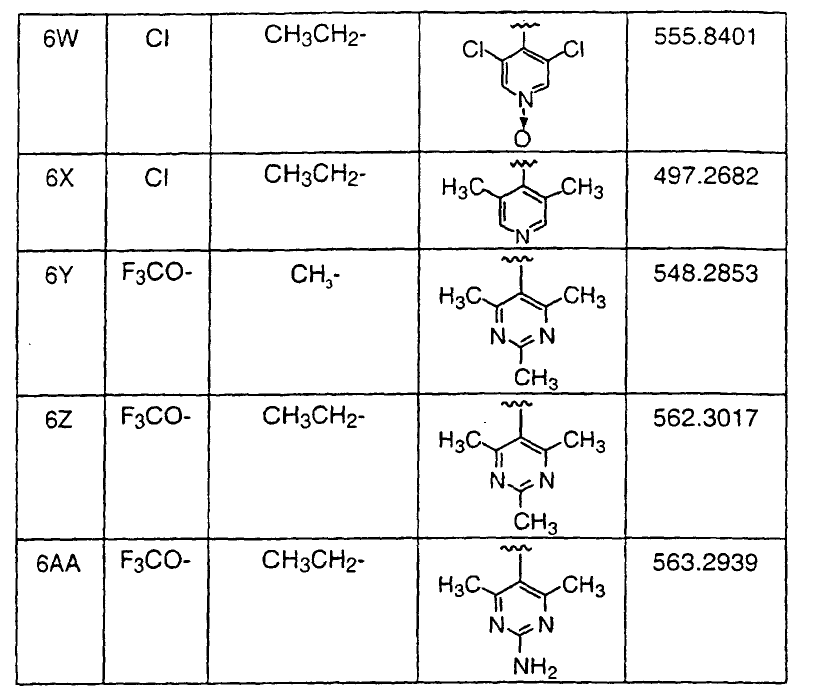

Additional data for compounds of Example 6:

| Ex. | ¹H-NMR (300 MHz ¹H NMR (CDCl₃)) |
|---|---|
| 6F | 8.31 (d, 1H), 7.51 (d, 2H), 7.10 (d, 2H), 6.95 (d, 2H), 4.20 (m, 2H), 3.40 (d, 2H), 3.30 (m, 2H), 3.35 (m, 3H), 2.80-3.05 (m, 5H), 2.45 (d, 3H), 2.25 (d, 3H), 1.25-2.20 (m, 10H), 0.50 (m, 2 H), 0.22 (m, 2H), 0.90 (s, 3H) |
| 6G | 8.34 (d, *J* = 5.1 Hz, 1H), 7.24 (br s, 4H), 6.96 (d, *J* = 5.1 Hz, 1H), 4.33 (q, *J* = 8.6 Hz, 2H), 4.13 (m, 1H), 3.45 (m, 1H), 3.30 (m, 1H), 2.98 (m, 2H), 2.82 (m, 1H), 2.46 and 2.49 (s, 3H), 2.41 (m, 1H), 2.24 and 2.27 (s, 3H), 2.10 (m, 2H), 1.96 (m, 1H), 1.15-1.90 (m, 7H), 0.92 (s, 3H) |
| 6I | 8.92 (s, 1H), 7.23 (br s, 4H), 4.11 (m, 1H), 3.79 (s, 3H), 3.30-3.45 (m, 2H), 2.97 (m, 2H), 2.81 (m, 1H), 2.45 and 2.42 (s, 6H), 2.40 (m, 1H), 1.90-2.20 (m, 3H), 1.15-1.90 (m, 7H), 0.92 (s, 3H) |

### Example 7

Alternate synthesis of the compounds of Example 6.
1) The product of Example 6, step B-2 (566 mg, 1.20 mmol) is treated with H₃CONH₂·HCl using conditions similar to those shown in Example 6, step B-3. The resulting crude mixture of Z- and E-methoximes is separated on a preparative silica gel TLC plate (eluting with hexanes/ EtOAc, 80:20) to afford, in order of elution, first the E-methoxime (175 mg; 29%), then the Z-methoxime (175 mg; 29%), both products as oils.
2) The Z-methoxime (75 mg; 0.15 mmol) of step 1 is deprotected following conditions similar to those shown in Example 6, step B-5 and the resulting free amine (46 mg) is directly subjected to amidation with 2,4-dimethylnicotinic acid using conditions similar to those shown in Example 6, step B-6 to yield 50 mg (82%) of a colorless oil.

The following compounds are prepared via similar procedures: 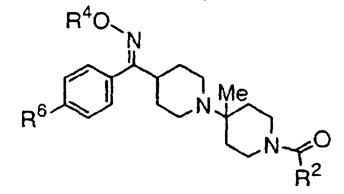 wherein R⁴, R⁶ and R² are as defined in the table:

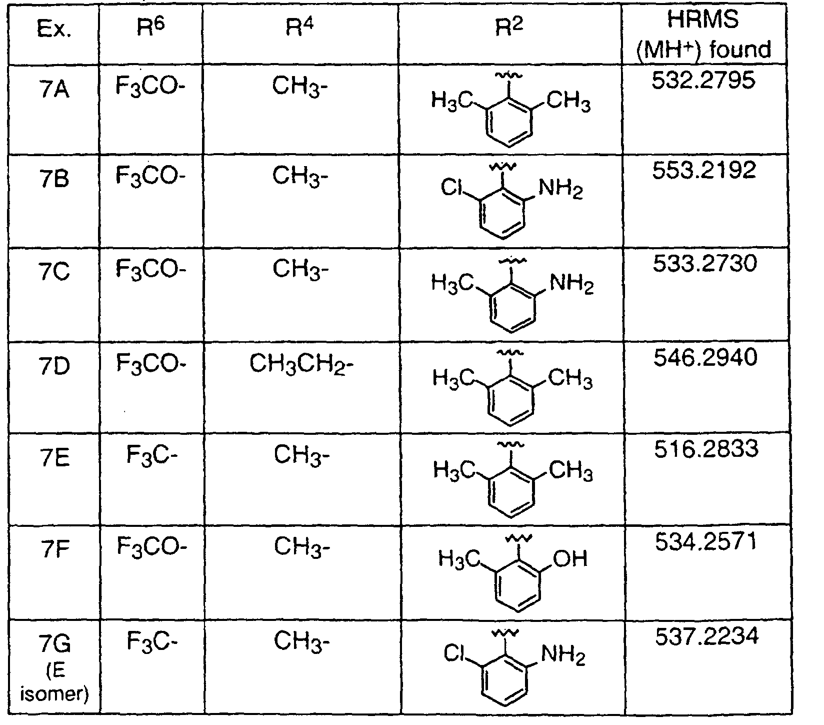

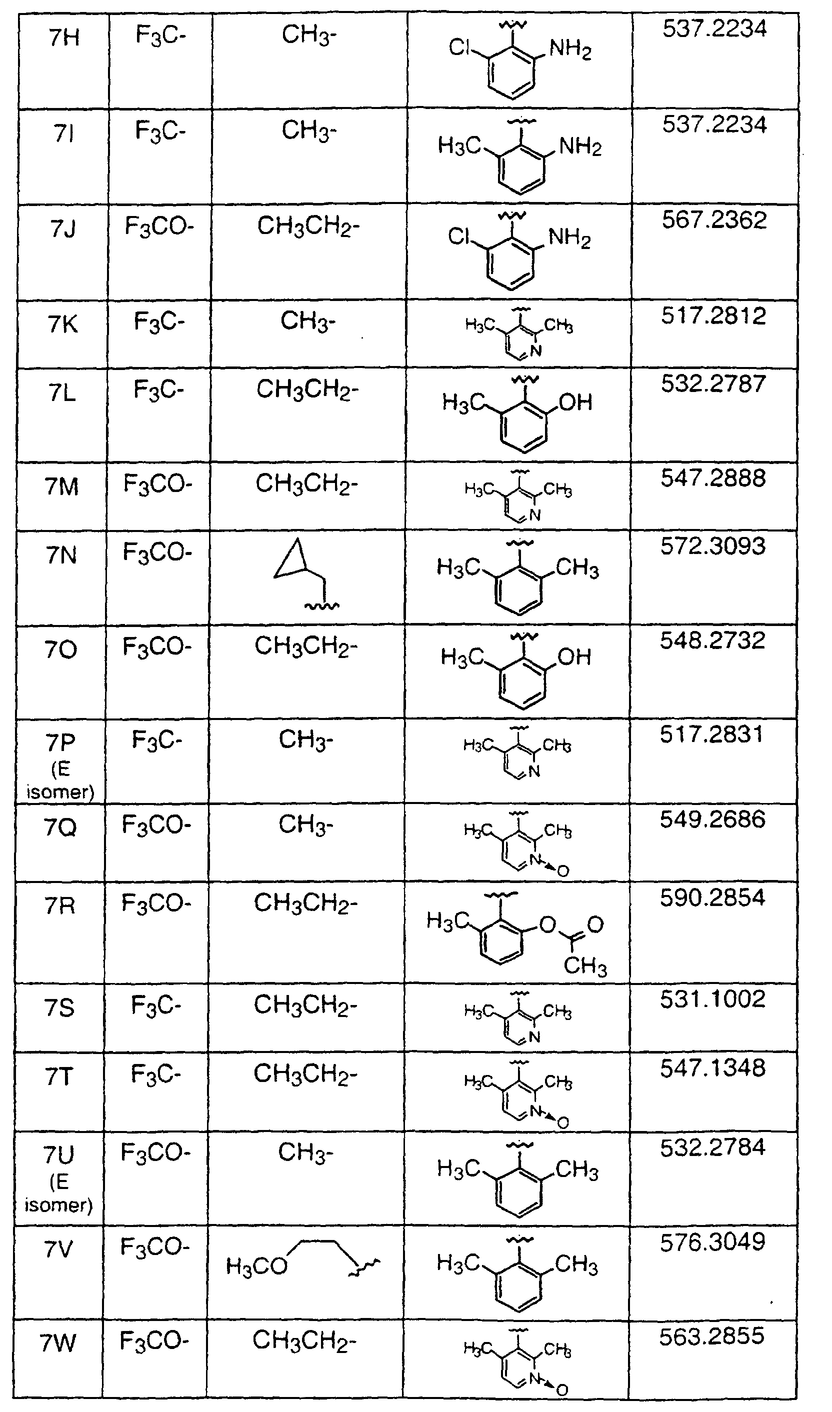

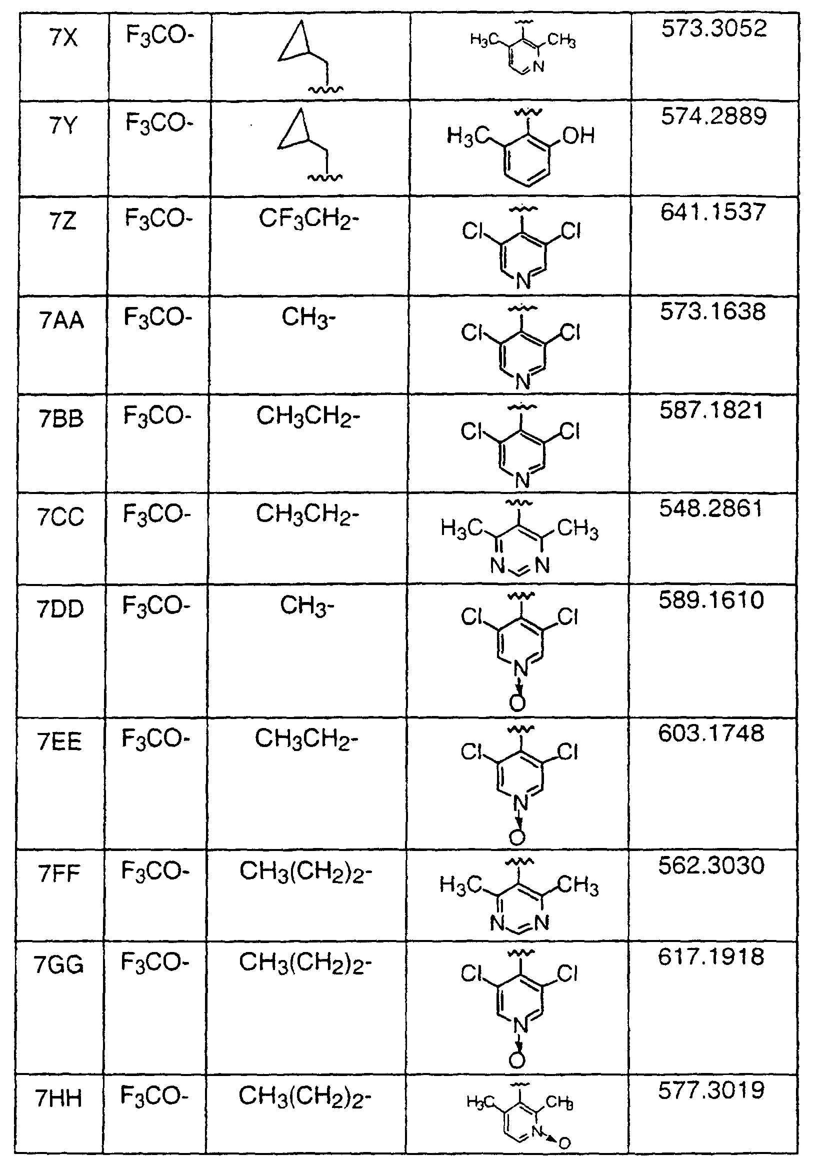

Additional data for compounds of Example 7:

| Ex. | ¹H-NMR (300 MHz ¹H NMR (CDCl₃)) |
|---|---|
| 7H | 7.55 (d, 2H), 7.30 (d, 2H), 7.15 (t, 1H), 6.75 (d, 1H), 6.60 (d, 1H), 4.25 (m, 2H), 3.80 (s, 3H), 3.40 (m, 2H), 2.80-3.20 (m, 3H), 2.40 (m, 1H), 1.40-2.20 (m, 13H), 0.90 (s, 3H) |
| 7K | 8.31 (d, 1H), 7.61 (d, 2H), 7.31 (d, 2H), 6.95 (d, 2H), 4.30 (m, 2H), 3.80 (3, 2H), 3.20-3.50 (m, 2H), 2.75-3.05 (m, 3H), 2.45 (d, 3H), 2.25 (d, 3H), 1.45-2.20 (m, 11H), 0.92 (s, 3H) |
| 7Q | 8.11 (d, *J* = 6.8 Hz, 1H), 7.25 (br s, 4H), 6.94 (d, *J* = 6.8 Hz, 1H), 4.16 (m, 1H), 3.75 (s, 3H), 3.20-3.45 (m, 2H), 2.85-3.00 (m, 3H), 2.41 (d, *J* = 11.6 Hz, 3H), 2.45 (m, 1H), 2.20 (d, *J* = 11.6 Hz, 3H), 1.85-2.20 (m, 3H), 1.15-1.85 (m, 7H), 0.88 (s, 3H) |
| 7R | 7.13-7.30 (m, 5H), 7.14 (m, 1H), 6.95 (m, 1H), 4.13 (m, 1H), 4.03 (q, *J* = 7.1 Hz, 2H), 3.15-3.50 (m, 2H), 2.86-3.10 (m, 2H), 2.80 (m, 1H), 2.39 (m, 1H), 2.15-2.30 (m, 6H), 1.85-2.15 (m, 3H), 1.10-1.85 (m, 7H), 1.28 (t, *J* = 7.1 Hz, 3H), 0.88 (br s, 3H) |
| 7S | 8.31 (d, 1H), 7.61 (d, 2H), 7.32 (d, 2H), 6.95 (d, 2H), 4.25 (m, 2H), 4.05 (q, 2H), 3.20-3.50 (m, 2H), 2.80-3.15 (m, 3H), 2.45 (d, 3H), 2.25 (d, 3H), 1.45-2.20 (m, 9H), 1.20 (t, 3H), 0.90 (s, 3H) |

### Example 8

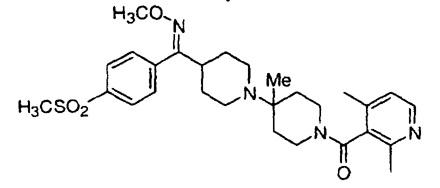

1) To a stirred solution of the product of Example 5, step 8 (0.500 g, 1.07 mmol) in DMF (25 ml) is added sodium methylmercaptide (0.113 g, 1.62 mmol) and the mixture is heated to 70° C for 12 h. The reaction mixture is then cooled to RT, diluted with Et₂O, washed with brine, dried and concentrated to yield 0.437 g (97%) of sulfide.
2) A solution of the product of step 1 (1.00 g; 2.31 mmol), H₃CONH₂·HCl (3.80 g, 46.2 mmol), and AcONa (3.79 g, 46.2 mmol) in EtOH (30 ml) is heated at reflux under N₂ for 4 h. After evaporation of the solvent, the residue is taken up in aqueous 0.1 N NaOH and extracted with CH₂Cl₂. The residue obtained after evaporation of the solvents is subjected to flash chromatography over silica gel, to afford first the E-oxime (eluting Et₂O/CH₂Cl₂, 1:4; 0.45 g; 24%), then the Z-oxime (0.25 g, 15%).
3) To a solution of Z-oxime (0.250 g, 0.543 mmol) of step 2 in CH₃OH (5 ml) is at 0° C is added oxone (1.00 g, 1.627 mmol in 5 ml of CH₃OH) and the mixture is stirred at 0°C for 4 h. The reaction is then quenched with 10% NaOH, concentrated, poured into water (10 ml) and extracted with CH₂Cl₂, dried and concentrated to yield 0.220 g (82%) of sulfone.
4) To a stirred solution of the product of step 3 (0.300 g, 0.608 mmol) in CH₂Cl₂ (5 ml) is added TFA(1 ml) and the mixture is stirred at RT for 2 h. The reaction mixture is concentrated, poured into 10% NaOH and extracted with CH₂Cl₂. The combined extracts are dried and concentrated to afford 0.240 g (100%) of amine.
5) To stirred solution of the product of step 4 (0.45 g, 0.114 mmol) in CH₂Cl₂ is added 2,6-dimethylnicotinic acid (0.26 g, 0.172 mmol), DEC (0.33 g, 0.172 mmol), N,N,N-diisopropylethylamine (DIPEA) (0.2 ml) and HOBT (0.24g, 0.172 mmol) and the mixture is stirred for 12 h at RT The reaction mixture is quenched with NaHCO₃, extracted with CH₂Cl₂, dried, concentrated and purified by preparative chromatography (20% EtOH/EtOAc) to afford 0.046 g (76%) of Z-oxime amide.
   300 MHz -¹H NMR (CDCl₃) δ 8.32 (d, 1H), 7.95 (d, 2H), 7.40 (d, 2H), 6.95 (d, 1H), 4.20 (m, 1H), 3.82 (s, 3H), 3.30-3.45 (m, 3H), 3.10 (s, 3H), 2.80-3.00 (m, 3H), 2.50 (d, 2H), 2.25 (d, 2H), 1.30- 2.20 (m, 12H), 0.92 (s, 3H).

The following compounds were prepared in a similar manner: 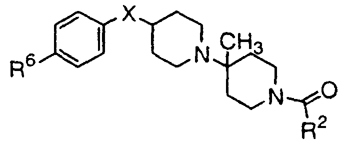 wherein X, R⁶ and R² are as defined in the table:

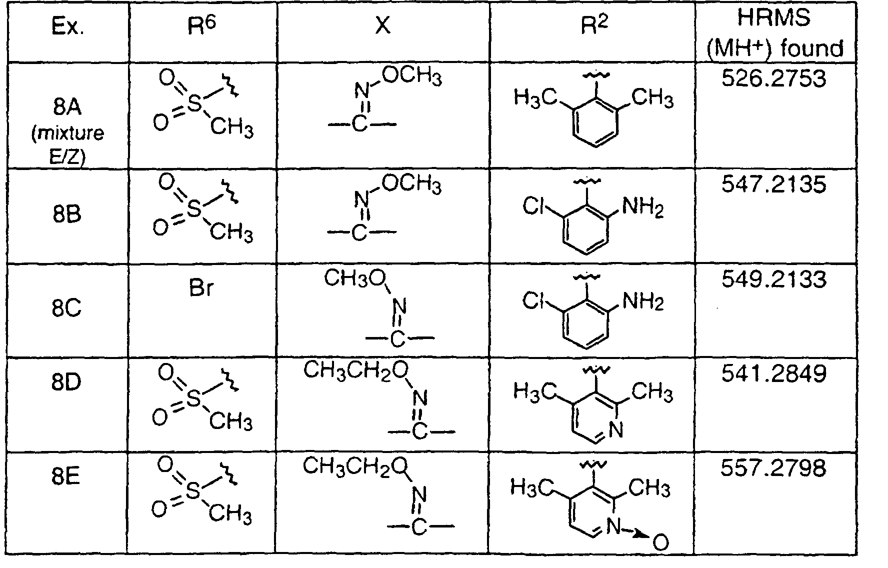

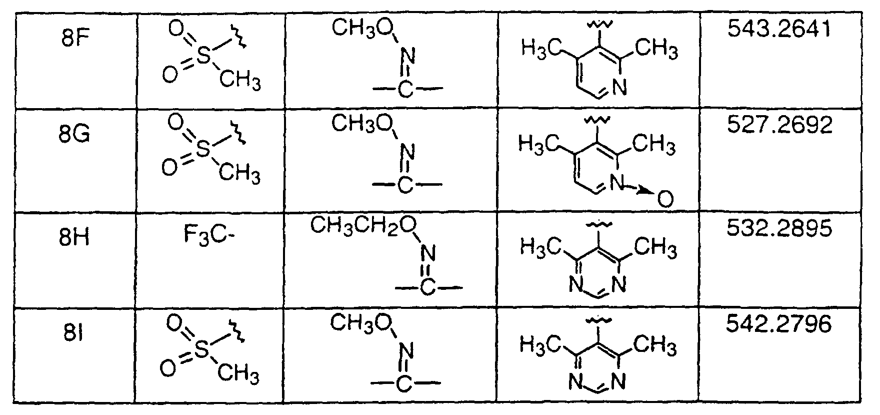

### Example 9

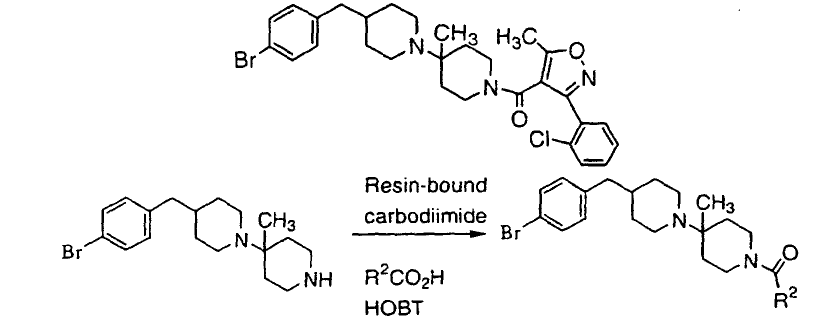

Dissolve the starting amine (2.0 g , 5.7 mmol) in CHCl₃ (57 ml; = Stock solution A ~ 0.1 M). Add 430 µl of stock solution A (0.043 mmol) to a slurry of 0.25 g (~ 0.22 mmol) of resin bound cardodiimide (prepared by reacting Argopore-Cl resin with 1-(3-dimethylaminopropyl)3-ethyl carbodiimide in DMF at 100 C) in DMF (2 ml) in a polyethylene SPE cartridge. To this mixture add 0.12 ml of a 1 M solution of 5-methyl-3-[2-chlorophenyl]isoxazole-4-carboxylic acid in DMF (0.12 mmol), HOBT (86 µl of a 0.5M solution in DMF) and DMAP (25 µl of a 0.05M solution in DMF). Shake this mixture for 14 h, filter and add 0.3 g of Amberlyst-15 resin (~ 1.5 mmol) to the filtrate. Shake for 1 to 2 h, filter and wash the resin twice with each of the following solvents: THF, CH₂Cl₂ and CH₃OH, then wash with THF and CH₂Cl₂. Treat the resin with 2M NH₃ in CH₃OH (1 time for 30 min, and 1 time for 5 min.). Combine and concentrate the filtrates under reduced pressure to afford the title compound. LCMS found MH⁺= 570, 572 (calculated MW 571); TLC R_{f} = 0.45 (CH₂Cl₂/CH₃OH/ NH₄OH (95/5/0.5)).

Using a similar procedure, the following compounds were prepared 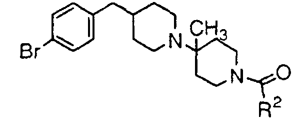 wherein R² is as defined in the table:

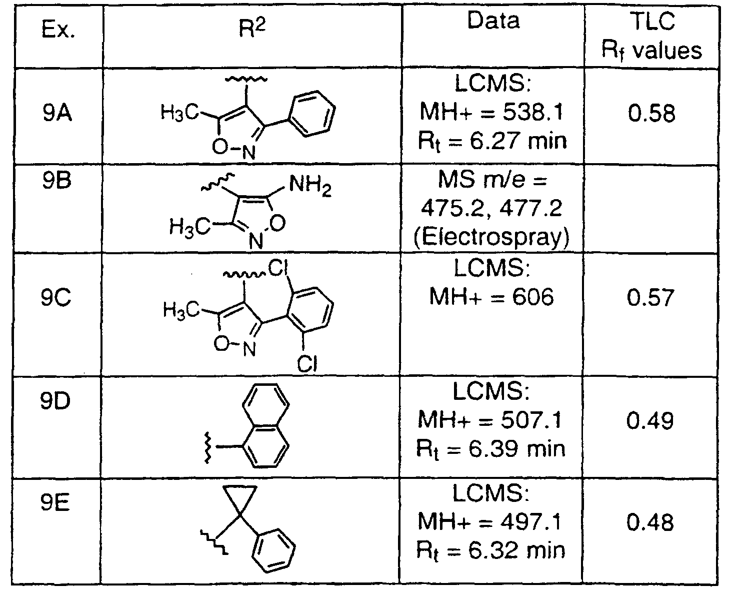

### Example 10

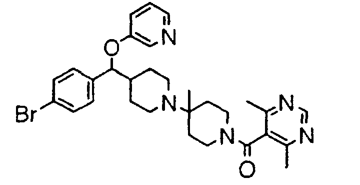

Step 1: To a solution of alcohol **39ab** (406 mg; 0.87 mmol), 3-hydroxypyridine (95.1 mg; 1 mmol) and PPh₃ (262 mg; 1 mmol) in anhydrous THF (2 ml) at 0 °C was added diethylazodicarboxylate (160 ml; 1 mmol) and the mixture was allowed to warm to RT overnight. The reaction was poured into 5% aqueous NaHCO₃, extracted with CH₂Cl₂, and dried over Na₂SO₄. After concentration of the solvents, the resulting oil was purified by flash chromatography over silica gel (eluting CH₂Cl₂/CH₃OH 97:3 to 95:5) to afford the desired compound (290 mg; 61%), as an oil.
Step 2: Removal of the Boc-protecting group of the product of step 1 (290 mg; 0.53 mmol) proceeded as in Example 2 to obtain the desired amine (210 mg; 89%), as a white foam.
Step 3: The amine of step 2 (50 mg; 0.11 mmol) was coupled with 4,6-dimethylpyrimidine-5-carboxylic acid following the conditions described in Example 2 to obtain the title compound (32 mg; 49%) as a colorless oil: ¹H-NMR (300 MHz, CDCl₃) δ 8.91 (s, 1H), 8.20 (br s, 1H), 8.10 (d, *J* = 4.5 Hz, 1H), 7.43 (br d, *J* = 8.4 Hz, 2H), 7.14 (br d, *J* = 8.4 Hz, 2H), 6.95-7.10 (m, 2H), 4.75 (br d, *J* = 6.8 Hz, 1H), 4.15 (m, 1H), 3.44 (m, 1H), 3.33 (m, 1H), 2.95 (m, 2H), 2.79 (m, 1H), 2.42 and 2.44 (s, 3H), 1.85-2.15 (m, 3H), 1.65-1.85 (m, 2H), 1.15-1.50 (m, 5H), 0.90 (s, 3H); HRMS (MH⁺) 578.2115.

Using similar procedures, compounds of the following structure were prepared 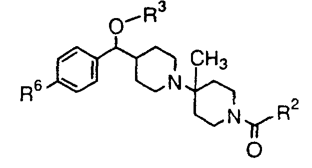 wherein R³, R⁶ and R² are as defined in the table:

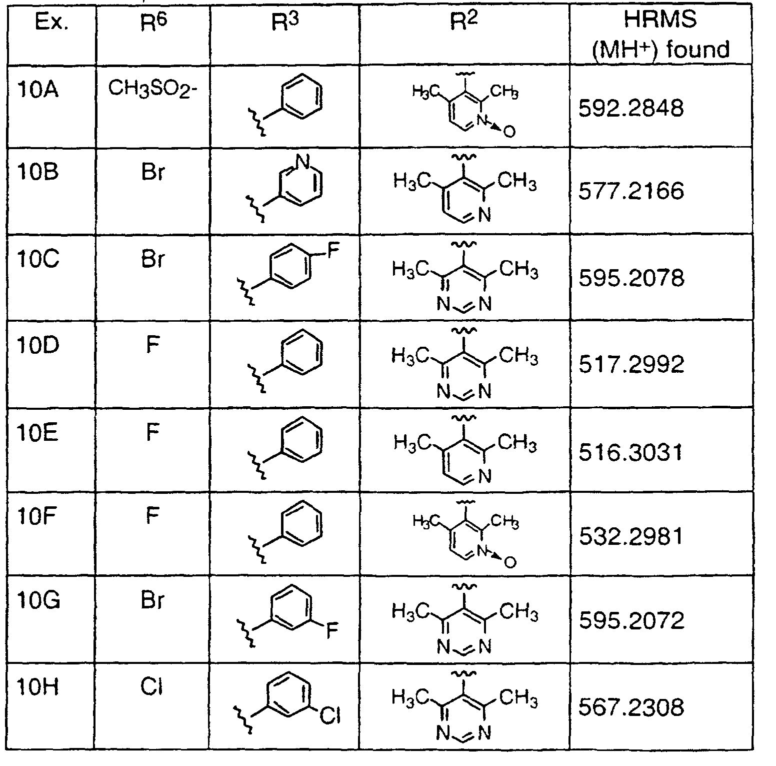

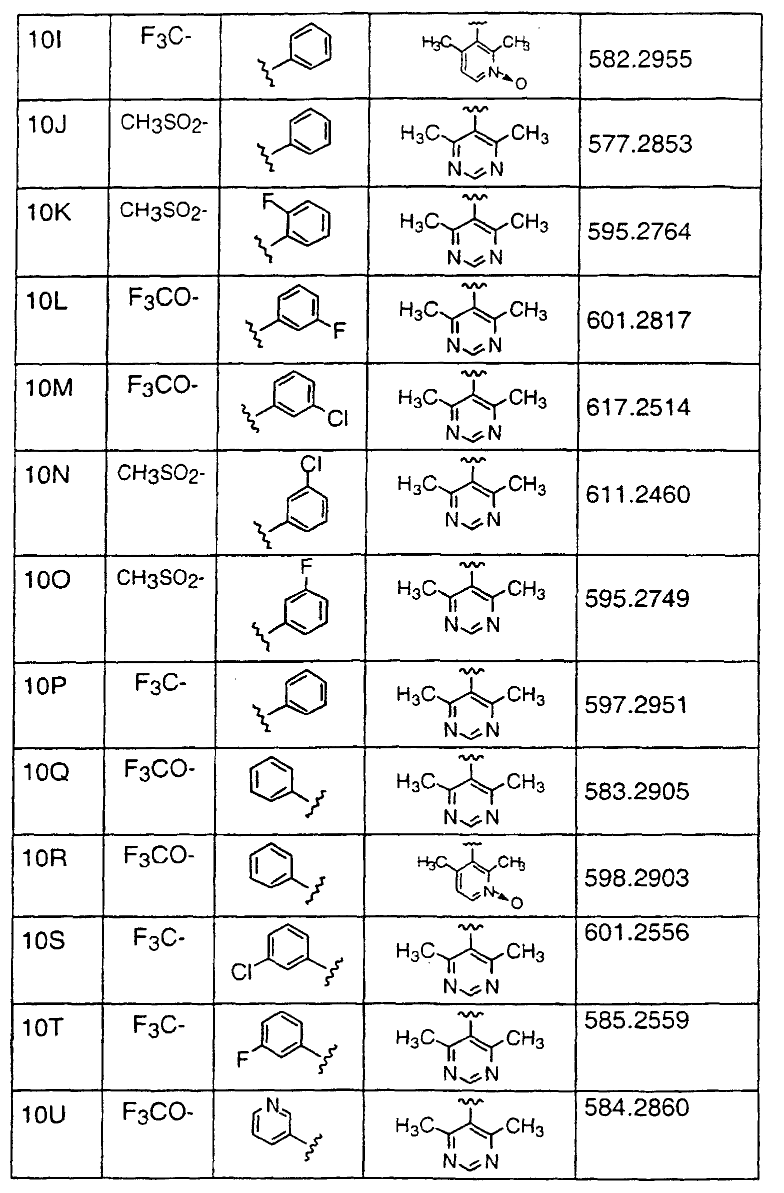

Additional data for compounds of Example 10:

| Ex. | ¹H-NMR (300 MHz ¹H NMR (CDCl₃)) |
|---|---|
| 10C | 8.95 (s, 1H), 7.46 (br d, *J* = 8.4 Hz, 2H), 7.17 (br d, *J* = 8.4 Hz, 2H), 6.86 (t, *J* = 9 Hz, 2H), 6.70-6.72 (m, 2H), 4.69 (br d, *J* = 6.4 Hz, 1H), 4.19 (m, 1H), 3.47 (m, 1H), 3.37 (m, 1H), 2.99 (m, 2H), 2.82 (m, 1H), 2.47 and 2.50 (s, 3H), 1.90-2.15 (m, 3H), 1.65-1.90 (m, 2H), 1.20-1.50 (m, 5H), 0.93 (s, 3H) |
| 10F | 8.17 (d, *J* = 6.8 Hz, 1H), 7.28 (m, 2H), 7.18 (t, *J* = 7.5 Hz, 1H), 6.95-7.10 (m, 3H), 6.87 (t, *J* = 7.5 Hz, 1H), 6.80 (d, *J* = 7.5 Hz, 2H), 4.80 (d, *J* = 6.8 Hz, 1H), 4.17 (m, 1H), 3.25-3.50 (m, 2H), 2.99 (m, 2H), 2.80 (m, 1H), 2.43 (br s, 3H), 2.24 (br s, 3H), 1.65-2.20 (m, 5H), 1.15-1.50 (m, 5H), 0.90 (s, 3H) |
| 10H | 8.95 (s, 1H), 7.32 (br d, *J* = 8.4 Hz, 2H), 7.23 (br d, *J* = 8.4 Hz, 2H), 7.08 (t, *J* = 8.1 Hz, 1H), 6.80-6.90 (m, 2H), 6.68 (m, 1H), 4.77 (br d, *J* = 6.8 Hz, 1H), 4.19 (m, 1H), 3.46 (m, 1H), 3.37 (m, 1H), 3.00 (m, 2H), 2.81 (m, 1H), 2.47 and 2.49 (s, 3H), 1.90-2.15 (m, 3H), 1.65-1.90 (m, 2H), 1.20-1.50 (m, 5H), 0.93 (s, 3H) |
| 10K | 8.81 (s, 1H), 7.78 (d, *J* = 8.4 Hz, 2H), 7.53 (m, 1H), 7.47 (d, *J* = 8.4 Hz, 2H), 6.90 (m, 1H), 6.74 (m, 1H), 6.59 (m, 1H), 4.83 (d, *J* = 6.8 Hz, 1H), 4.08 (m, 1H), 3.20-3.40 (m, 2H), 2.70-3.00 (m, 3H), 2.35 (br s, 3H), 1.65-2.15 (m, 5H), 1.1.5-1.50 (m, 5H), 0.87 (s, 3H) |
| 10L | 8.33 (d, *J* = 5.1 Hz, 1H), 7.99 (dd, *J* = 4.8 and 1.8 Hz, 1H), 7.86 (d, *J* = 8.4 Hz, 2H), 7.56 (d, *J* = 8.4 Hz, 2H), 7.53 (m, 1H), 6.96 (d, *J* = 6.4 Hz, 1H), 6.75-6.85 (m, 2H), 4.15 (m, 1H), 3.45 (m, 1H), 3.30 (m, 1H), 3.02 (s, 3H), 2.99 (m, 2H), 2.79 (m, 1H), 2.47 and 2.48 (s, 3H), 2.45 (m, 1H), 2.25 and 2.26 (s, 3H), 1.65-2.15 (m, 5H), 1.15-1.55 (m, 5H), 0.90 (s, 3H) |

### Example 11

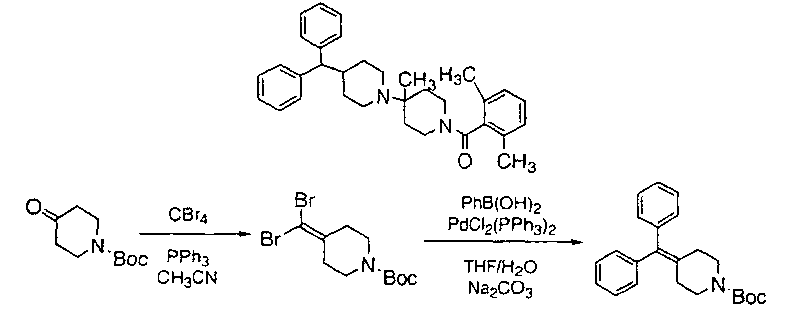

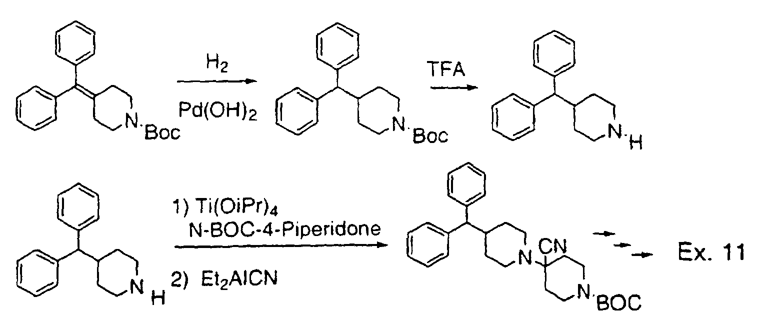

1) N-Boc-4-piperidone (10 g, 50 mmol) and PPh₃ (53 g, 200 mmol) were taken up in CH₃CN (100 ml). The solution was cooled to 0 °C and CBr₄ (33 g, 100 mmol) was added to the solution at 0 °C. The solution was stirred at 0 °C for 15 min. and at 25 °C for 2 h. Et₂O (200 ml) was added, and the resulting mixture was filtered through a plug of SiO₂.
   Concentration gave a yellow solid. Purification via flash chromatography (9/1 hexanes/Et₂O, SiO₂) gave 10 g (56 %) of the di-bromo product as a white solid.
2) A solution of the product of step 1 (1 g, 2.8 mmol), PhB(OH)₂ (1.2 g, 9.9 mmol), PdCl₂(PPh₃)₂ (197 mg, 0.28 mmol), and Na₂CO₃ (897 mg, 8.5 mmol) were taken up in THF/H₂O (4/1, 20 ml) and stirred at 65 °C under N₂ for 24 h. The solution was partitioned between EtOAc and H₂O, the aqueous layer was extracted with EtOAc and the combined organic layers were washed with brine and dried over Na₂SO₄. Filtration and concentration gave a dark brown oil. Purification via flash chromatography (9/1 hexanes/Et₂O, SiO₂) gave 941 mg (96 %) of the desired product as a white solid, m.p. = 152-153 °C.
3) A solution of the product of step 2 (500 mg, 1.4 mmol) and Pd(OH)₂ on carbon (100 mg, 20 wt % Pd (dry basis), 50 wt % H₂O) were taken up in CH₃OH (20 ml) and shaken in a Parr apparatus under H₂ (50 psi) for 15 h. The mixture was filtered and concentrated to give 501 mg (99 %) of the diphenylmethyl piperidine as a colorless oil.
4) TFA (1.4 ml) was added to a solution of the product of step 3 (500 mg, 1.4 mmol) in CH₂Cl₂ (15 ml). The solution was stirred at 25 °C for 23 h. The solution was concentrated and the residue partitioned between CH₂Cl₂ and 1 N NaOH. The aqueous layer was extracted with CH₂Cl₂, the combined organic layers were dried over Na₂SO₄, filtered and concentrated to obtain 349 mg (99 %) of the free amine as a yellow oil, m.p. (HCl) = decomp. above 220-230 °C. HRMS calc'd for C₁₈H₂₂N (MH⁺): 252.1752, Found: 252.1751.
5) A solution of the product of step 4 (349 mg, 1.4 mmol), N-Boc-4-piperidone 280 mg, 1.4 mmol), and Ti(OiPr)₄ (0.42 ml, 1.4 mmol) were taken up CH₂Cl₂ (15 ml) under N₂. After stirring at 25 °C for 17 h, Et₂AlCN (2.8 mmol, 2.8 ml of 1.0 M in toluene) was added and the solution was stirred an additional 18 h at 25 °C. The solution was quenched with sat. NaHCO₃, diluted with EtOAc and filtered through Celite. The aqueous layer was extracted with EtOAc and the combined EtOAc layers were dried over Na₂SO₄. Filtration and concentration gave a yellow oil. Purification via preparative layer chromatography (3/1 hexanes/EtOAc, SiO₂) gave 430 mg (67 %) of the desired product as an oil.
6) A solution of the product of step 5 (430 mg, 0.94 mmol) in THF (20 ml) was cooled to 0 °C under N₂. CH₃MgBr (1.6 ml of 3.0 M in Et₂O, 4.7 mmol) was added at 0 °C and the solution stirred at 25 °C for 19 h. The reaction mixture was quenched with sat. NH₄Cl, diluted with CH₂Cl₂ and 1 N NaOH (check aqueous layer with pH paper, pH = 8-10). The layers were separated and the aqueous layer extracted with CH₂Cl₂. The combined organic layers were dried over Na₂SO₄, filtered and concentrated to obtain a yellow oil. Purification via flash chromatography (3/1 hexanes/EtOAc, SiO₂) gave 275 mg (65 %) of the product as a yellow oil.
7) TFA (0.60 ml) was added to a solution of the product of step 6 (275 mg, 0.61 mmol) in CH₂Cl₂ (15 ml) and the solution was stirred at 25 °C for 18 h. The solution was concentrated and the residue was partitioned between CH₂Cl₂ and 1 N NaOH. The aqueous layer was extracted with CH₂Cl₂, the combined organic layers were dried over Na₂SO₄, filtered and concentrated to obtain 209 mg (99 %) of thje amine as a yellow oil. HRMS calc'd for C₂₄H₃₃N₂ (MH⁺): 349.2644, Found: 349.2638.
8) A solution of the product of step 7 (50 mg, 0.14 mmol), 2.6-dimethylbenzoic acid (63 mg, 0.42 mmol), EDCI (54 mg, 0.28 mmol), HOBT (38 mg, 0.28 mmol), and iPr₂NEt (0.10 ml) were taken up in CH₂Cl₂ (3 ml). The solution was stirred at 25 °C for 18 h, then diluted with CH₂Cl₂ and washed with 1 N NaOH. The aqueous layer was extracted with CH₂Cl₂, the combined organic layers were dried over Na₂SO₄, and filtered and concentrated to give a yellow oil. Purification via preparative thin-layer chromatography (3/1 hexanes/EtOAc SiO₂) gave 47 mg (70 %) of the title compound as a colorless oil, m.p. (HCl salt) = 195-201 °C. HRMS calc'd for C₃₃H₄₁N₂O (MH⁺): 481.3219, Found: 481.3225.

Using similar procedures, compounds of the following structure were prepared 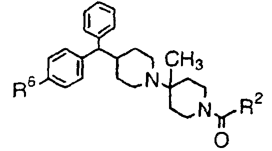 wherein R⁶ and R² are as defined in the table:

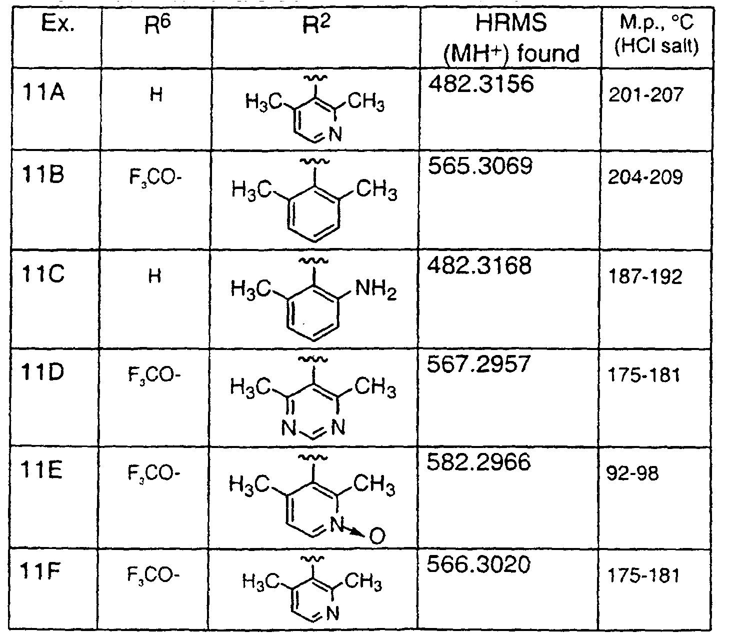

### Example 12

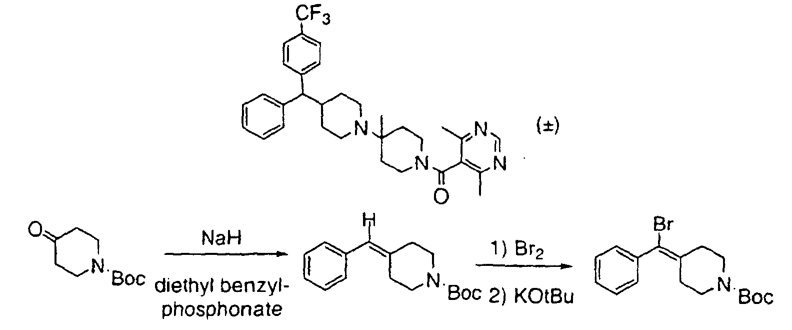

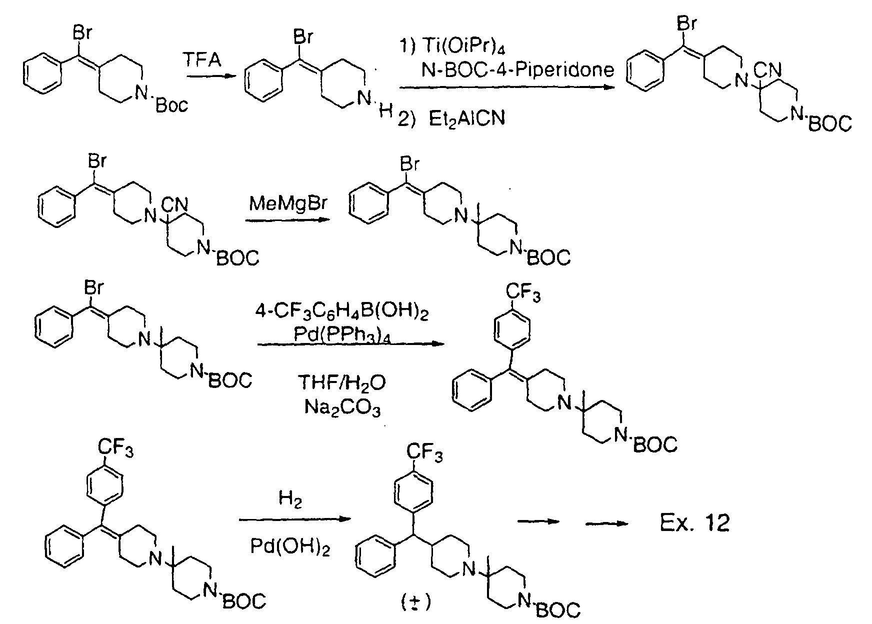

1) N-Boc-4-piperidone (10 g, 50 mmol) and diethyl benzylphosponate (12.6g, 55 mmol) were taken up in dry THF (50 ml) under N₂. NaH (2.4 g, 60 mmol, 60 wt % in oil dispersion) was added to the solution at 25 °C.
   The resulting mixture was heated at reflux for 3.5 h. The solution was partitioned between EtOAc and saturated NH₄Cl, the aqueous layer was extracted with EtOAc and the combined EtOAc layers were washed with brine and dried over MgSO₄. Filtration and concentration afforded a yellow oil. Purification via flash chromatography (10/1 hexanes/Et₂O, SiO₂) gave 9.85 g (72 %) of the desired compound as a solid, m.p. = 63-65 °C.
2) Bromine (1 ml, 20 mmol; dissolved in 10 ml CH₂Cl₂) was added dropwise to a CH₂Cl₂ (100 ml) solution of the product of step 1 (5.0 g, 18 mmol) at 0 °C. The solution was stirred at 0 °C for 15 min, then concentrated under reduced pressure. The crude product was taken up in tert-butanol/THF (4/1, 100 ml), and KOtBu (4.1 g, 36 mmol) was added to the solution in portions. The yellow mixture was stirred at 25 °C for 5h, then concentrated under reduced pressure. The residue was partitioned between EtOAc and saturated NH₄Cl, the aqueous layer was extracted with EtOAc, and the combined EtOAc layers were washed with brine and dried over MgSO₄. Filtration and concentration gave a yellow solid. Purification via flash chromatography (7/1 hexanes/Et₂O, SiO₂) gave 5.2 g (81 %) of the desired product as a yellow solid. m.p. = 80-83 °C.
3) TFA (5.9 ml) was added to a solution of the product of step 2 (2.1 g, 5.9 mmol) in CH₂Cl₂ (25 ml). The solution was stirred at 25 °C for 5 h, concentrated and the residue was partitioned between CH₂Cl₂ and 1 N NaOH. The aqueous layer was extracted with CH₂Cl₂ and the combined organic layers were dried over Na₂SO₄, filtered and concentrated to obtain 1.46 g (98 %) of the amine as an orange oil, m.p. (HCl salt) = decomp. above 185-195 °C. HRMS calc'd for C₁₂H₁₅BrN (MH⁺): 254.0367, Found: 254.0374.
4) A solution of the product of step 3 (1.4 g, 5.6 mmol), N-Boc-4-piperidone (1.1 g, 5.6 mmol), and Ti(OiPr)₄ (1.7 ml, 5.6 mmol) were taken up in CH₂Cl₂ (30 ml) under N₂. After stirring at 25 °C for 18 h, Et₂ALCN (6.7 mmol, 6.7 ml, 1.0 M in toluene) was added to the solution and the solution was stirred an additional 18 h at 25 °C. The solution was quenched with sat. NaHCO₃, diluted with EtOAc and filtered through Celite. The aqueous layer was extracted with EtOAc and the combined EtOAc layers were dried over Na₂SO₄. Filtration and concentration gave a yellow oil. Purification via flash chromatography (3/1 hexanes/EtOAc, SiO₂) gave 2.0 g (78 %) of the desired product as an off-white solid.
5) A solution of the product of step 4 (2.0 g, 4.3 mmol) in THF (30 ml) was cooled to 0 °C under N₂. CH₃MgBr (7.2 ml of 3.0 M in Et₂O, 21 mmol) was added to the solution at 0 °C. The solution was warmed to 25 °C and stirred at that temperature for 16 h. The reaction mixture was quenched with sat. NH₄Cl and diluted with CH₂Cl₂ and 1 N NaOH (check aqueous layer with pH paper, pH = 8-10). The layers were separated, the aqueous layer was extracted with CH₂Cl₂ and the combined organic layers were dried over Na₂SO₄. Filtration and concentration gave a yellow oil. Purification via flash chromatography (3/1 hexanes/EtOAc, SiO₂) gave 1.56 g (82 %) of the desired product as a yellow oil.
6) A solution of the product of step 5 (300 mg, 0.67 mmol), 4-CF₃C₆H₄B(OH)₂ (380 mg, 2 mmol), PdCl₂(PPh₃)₂ (50 mg, 0.067 mmol), and Na₂CO₃ (210 mg, 2 mmol) were taken up THF/H₂O (4/1, 15 ml) and stirred at 65 °C under N₂ for 18 h. The solution was partitioned between EtOAc and H₂O and the aqueous layer was extracted with EtOAc. The combined organic layers were washed with brine and dried over Na₂SO₄. Filtration and concentration gave a dark brown oil. Purification via flash chromatography (4/1 hexanes/EtOAc, SiO₂) gave 229 mg (67 %) of the desired product as a colorless oil.
7) A solution of the product of step 6 (229 mg, 0.45 mmol) and Pd(OH)₂ on carbon (200 mg, 20 wt % Pd (dry basis), 50 wt % H₂O) were taken up in CH₃OH (35 ml) and shaken in a Parr apparatus under H₂ (50 psi) for 20 h. The mixture was filtered and concentrated to obtain 232 mg (100 %) of the (±)-product as a colorless foam. HRMS calc'd for C₃₀H₄₀O₂N₃ (MH⁺): 517.3042, Found: 517.3050.
8) TFA (0.45 ml) was added to a solution of the product of step 7 (235 mg, 0.45 mmol) in CH₂Cl₂ (15 ml). The solution was stirred at 25 °C for 24 h, then concentrated and the residue was partitioned between CH₂Cl₂ and 1 N NaOH. The aqueous layer was extracted with CH₂Cl₂, the combined organic layers were dried over Na₂SO₄, filtered and concentrated to obtain 146 mg (78 %) of the (±)-amine as a yellow oil.
9) A solution of the product of step 8 (102 mg, 0.25 mmol), 4,6-dimethylpyrimidine-5-carboxylic acid (110 mg, 0.75 mmol), EDCI (96 mg, 0.50 mmol), HOBT (70 mg, 0.50 mmol), and iPr₂NEt (0.17 ml) was taken up in CH₂Cl₂ (3 ml). The solution was stirred at 25 °C for 18 h, then diluted with CH₂Cl₂ and washed with 1 N NaOH. The aqueous layer was extracted with CH₂Cl₂, the combined organic layers were dried over Na₂SO₄, filtered and concentrated to obtain a yellow oil. Purification via preparative thin-layer chromatography (1/1 acetone/hexanes SiO₂) gave 121 mg (88 %) of the title compound as a colorless oil, m.p. (HCl salt) = 186-191 °C. HRMS calc'd for C₃₂H₃₈N₄OF₃ (MH⁺): 551.2998, Found: 551.3012.

The 4,6-dimethylpyrimidine-5-carboxylic acid used in step 9 was made by the following process: 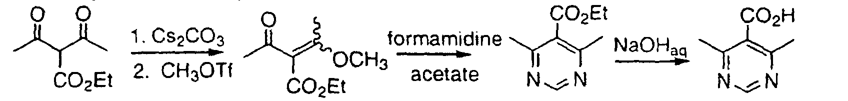
Step 1: Ethyl diacetoacetate (93.4 g), Cs₂CO₃ (185 g) and CH₃CN (550 ml) were mixed together, using an overhead mechanical stirrer. CH₃CN (50 ml) was added and the resulting mixture was cooled to 0°C. Methyl trifluoromethane sulfonate (88.6 g) was added dropwise and after addition, the cooling bath was removed. The mixture was stirred for 1 h at RT, filtered, and the salts were washed with Et₂O (2 X 50 ml). The organic extracts were combined and Et₂O (300 ml) was added. The resulting mixture was filtered, the filter cake was washed with Et₂O (2 X 100 ml), the Et₂O extracts were combined and evaporated to half volume. The solution was cooled in an ice bath and washed once with cooled (0°C) 2 N NaOH (pH = 11). The Et₂O layer was dried over MgSO₄, filtered and evaporated to give the desired product as a yellow liquid (64.7 g) in 65% yield, which was used directly in the next step.
Step 2: The product of step 1 (64.2 g), sodium ethoxide in ethanol (commercial solution; 21 wt%; 113 g) and formamidine acetate (36.2 g) were mixed together at RT. After refluxing for 4 h, the mixture was cooled to RT, the resulting precipitate was filtered off and the ethanol was removed under vacuum. The resulting liquid was partitioned between water and CH₂Cl₂ and the aqueous layer was extracted with CH₂Cl₂ (3 x 150 ml). The CH₂Cl₂ extracts were dried over MgSO₄, filtered and evaporated to give a dark crude liquid (50.7 g) which was purified by silica gel chromatography (980 g; 4:1 hexanes:EtOAc as eluant). After evaporation of the appropriate fractions, the desired product (28.5 g) was isolated in 46% yield and used directly in the next step.
Step 3: The product of step 2 (28.1 g), NaOH (6.72 g), water (65 ml) and EtOH (130 ml) were mixed together at RT and heated at reflux for 1h. The resulting solution was cooled to RT and the volatile materials were removed in vacuo until a thick paste resulted. Water (20 ml) was added, the mixture was cooled to 0°C and conc. HCl (14.3 ml) was added dropwise with stirring. The resulting white precipitate was collected by filtration, washed with ice water (2 X 10 ml) and air dried with suction for 30 min. The resulting white solid was treated with toluene (2 x 20 ml), the solvent was removed in vacuo at 50°C and then dried under vacuum (1 mm Hg) for 18 h. The desired product (14.9 g) was isolated as a white solid in 63% yield, mp: 176-178°C. Elemental analysis of C₇H₈N₂O₂: calc'd C 55.26%, H 5.30%, N 18.41%; found: C 55.13%, H 5.44%, N 18.18%.

A second crop of product was isolated by evaporation of the aqueous filtrate (from above) to dryness and addition of water (20 ml). The resulting mixture was stirred at RT for 5 min, cooled in an ice bath and the precipitate formed was collected by filtration. The resulting solid was washed with ice water (2 X 5 ml) and dried as described above to give the product (4.68 g) as a cream colored solid to give a combined yield of 83%.

### Example 13

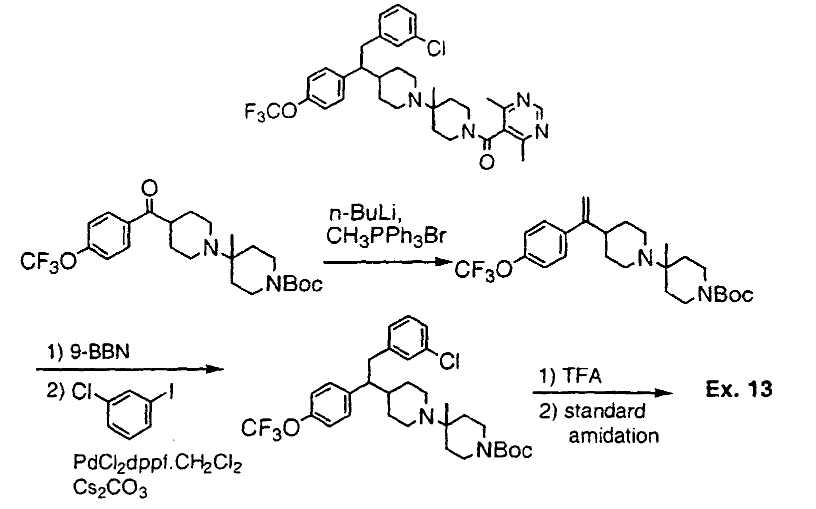

Step1: To a suspension of methyltriphenylphosphonium bromide (1.89 g; 4.80 mmol) in anhydrous THF (15 ml) at -40 °C is added n-BuLi 2.5 N in hexanes (2.12 ml; 5.3 mmol) via syringe. The reaction is allowed to warm to 0 °C, stirred 30 min at this temperature, and a solution of the product of Example 6, step B-2 (2.24 g; 4.8 mmol) is added. The solution is then allowed to warm to RT overnight, poured into CH₂Cl₂, and washed with saturated NaHCO₃ then brine. The residue obtained after concentration of the organic layer is purified by flash chromatography over silica gel (eluting with CH₂Cl₂/EtOAc, 9:1) to afford 0.56 g (25%) of an oil.
Step 2: A solution of the product of step 1 (0.56 g; 1.2 mmol) and 9-BBN 0.5 N in THF (3 ml; 1.5 mmol) is refluxed 2 h under inert atmosphere. Part of this solution (1.5 ml; 0.59 mmol of theoretical intermediate) is added to a mixture of 1-chloro-3-iodobenzene (88 µl; 0.71 mmol), PdCl₂dppf.CH₂Cl₂ (19.8 mg), triphenylarsine (24.1 mg) and Cs₂CO₃ (250 mg) in DMF (0.40 ml) and water (80 µl). The reaction is stirred 2 h at 60 °C and overnight at RT, poured into 5% aqueous NaHCO₃, and extracted with CH₂Cl₂. Combined organic layers are dried over Na₂SO₄, concentrated, and purified by chromatography over silica gel (eluting with EtOAc/hexanes, 8:2) to provide 100 mg (29%) of an oil.
Step 3: The Boc-protecting group of the product of step 2 (100 mg; 0.17 mmol) was removed as in Example 2 to obtain the desired amine (70 mg; 86%). This amine (45 mg; 0.09 mmol) was coupled with 4,6-dimethylpyrimidine-5-carboxylic acid following the conditions described in Example 2 to obtain the title compound as a colorless oil (32 mg). ¹H-NMR (300 MHz, CDCl₃) δ 8.93 (d, *J* = 3.8 Hz, 1H), 6.90-7.10 (m, 5H), 6.88 (br s, 1H), 6.71 (d, *J* = 7 Hz, 1H), 4.20 (m, 1H), 3.25-3.55 (m, 2H), 3.19 (m, 2H), 2.50-3.10 (m, 5H), 2.47 and 2.48 (s, 3H), 2.42 and 2.43 (s, 3H), 1.70-2.20 (m, 5H), 1.20-1.65 (m, 5H), 0.92 (s, 3H); HRMS (MH+) 615.2722.

Using a similar procedure, the following compound was also prepared:

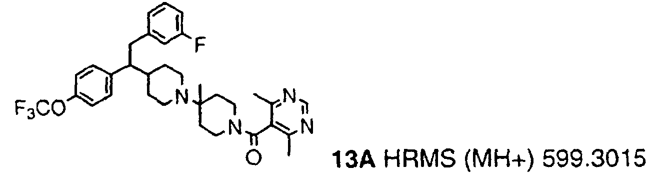

### Example 14

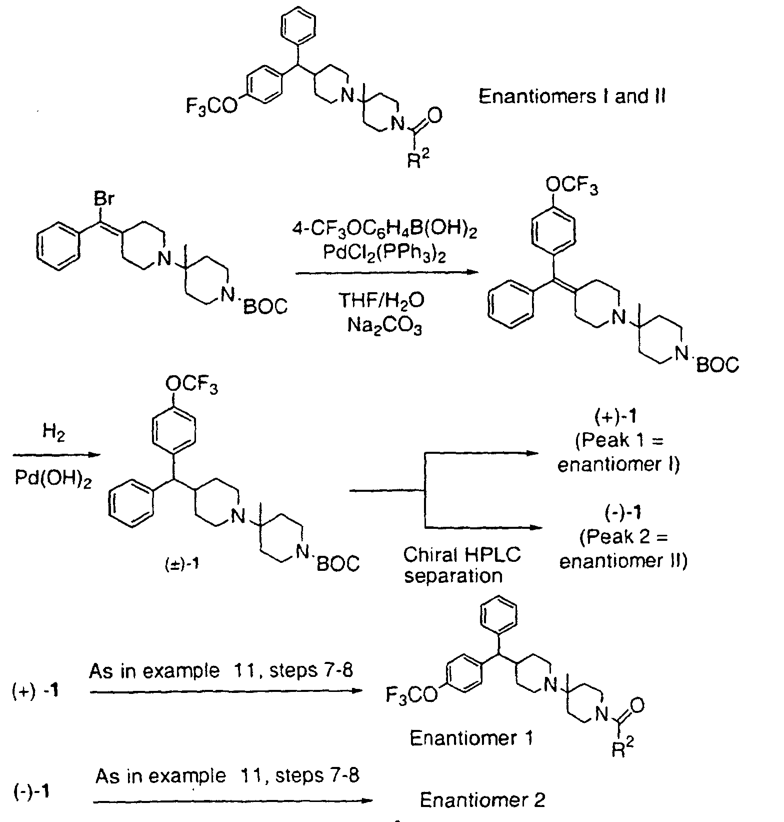

To prepare a compound wherein R² is 2,6-dimethylphenyl:
1) A solution of the product of step 5 in example 12 (300 mg, 0.67 mmol), 4-CF₃OC₆H₄B(OH)₂ (410 mg, 2 mmol), PdCl₂(PPh₃)₂ (50 mg, 0.067 mmol), and Na₂CO₃ (210 mg, 2 mmol) were taken up in THF/H₂O (4/1, 15 ml) and stirred at 65 °C under N₂ for 19 h. The solution was partitioned between EtOAc and H₂O, and the aqueous layer was extracted with EtOAc. The combined organic layers were washed with brine and dried over Na₂SO₄. Filtration and concentration gave a dark brown oil. Purification via flash chromatography (4/1 hexanes/Et₂O, SiO₂) gave 356 mg (100 %) of the desired product as a yellow oil.
2) A solution of the product in step 1 (340 mg, 0.64 mmol) and Pd(OH)₂ on carbon (300 mg, 20 wt % Pd (dry basis), 50 wt % H₂O) were taken up in CH₃OH (35 ml) and shaken in a Parr apparatus under H₂ (50 psi) for 18 h. The mixture was filtered and concentrated to obtain 341 mg (100 %) of the product, (±)-**1**, as a colorless foam.
3) The amine (±)-**1** was resolved via chiral HPLC separation. The conditions are as follows: CHIRALCEL® OD™ (5 cm x 30 cm); Hexane/isopropyl alcohol/diethylamine 75/25/0.05) at 25° C; 254 nm detection. The retention times for peak 1, (+)-enantiomer, and peak 2, (-)-enantiomer were 3.8 and 4.9 minutes, respectively [CHIRALCEL® OD™ (hexane/ethanol/diethylamine 90/10/0.1) 25° C at 254 nm]. Peak 1 and peak 2 are the first and second eluting peaks from the column, respectively. The enantiomers (I and II) were deprotected (CH₂Cl₂/TFA), and the free amine was coupled to the 2,6-dimethylbenzoic acid using the conditions described in example 11, steps 7 and 8. The hydrochloride salts were obtained by taking the free base up in EtOAc and triturating with 1 M HCl in Et₂O.

Data for the above compounds, 14A and 14B, and for additional compounds made in a similar manner, are given in the following table. In each case, the enantiomer designator I is derived from (+)-1 and the enantiomer designated II is derived from (-)-1.

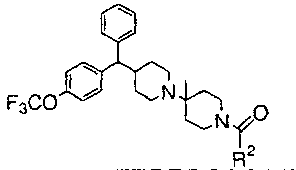

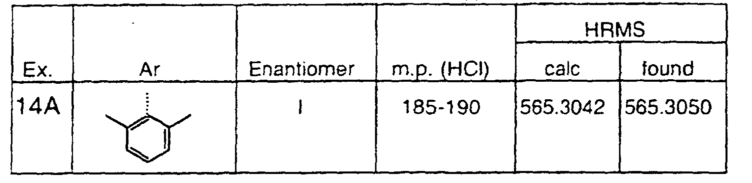

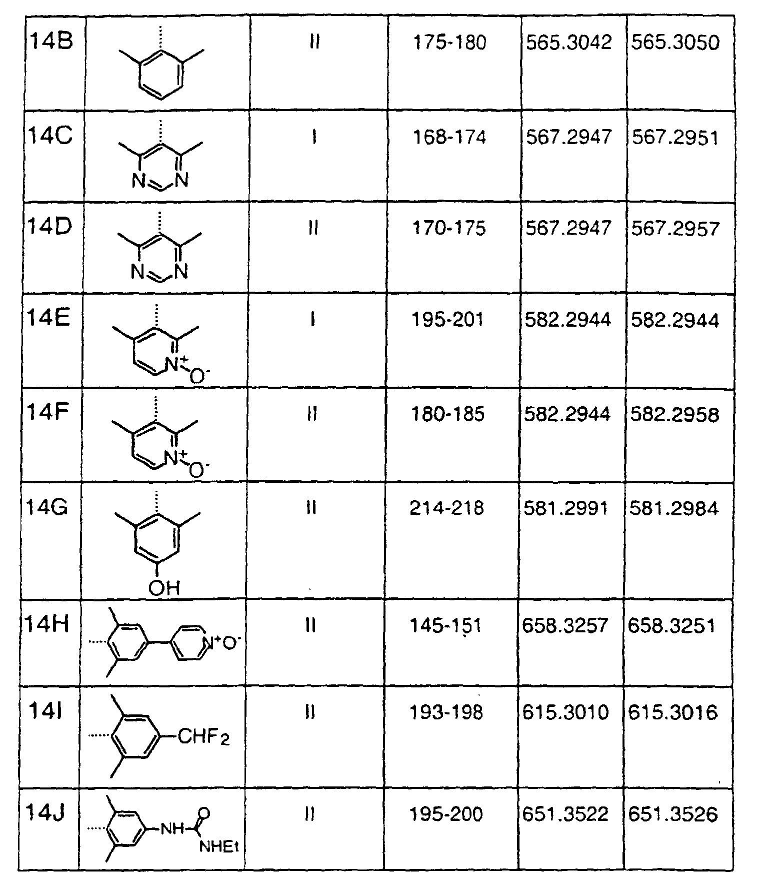

### Example 15

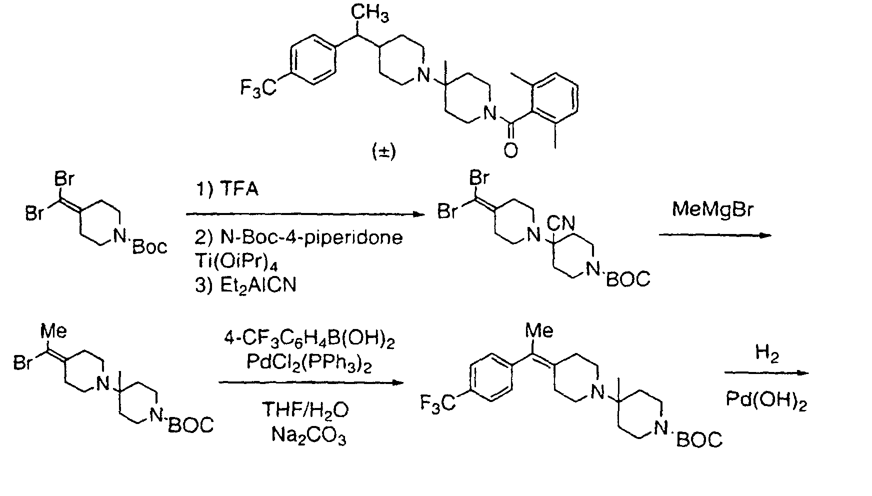

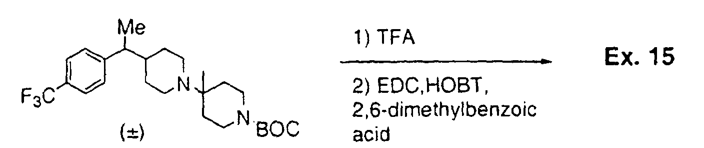

1) The dibromo-olefin (3.55 g, 10 mmol) and TFA (10 ml) were taken up in CH₂Cl₂ and stirred at 25 °C for 20 h. The solution was concentrated. The residue was partitioned between CH₂Cl₂ and 1 N NaOH. The aqueous layer was extracted with CH₂Cl₂. The combined organic layers were dried (Na₂SO₄). Filtration and concentration gave the 2.4 g (94 %) of the free piperdine as a colorless oil. The free piperdine (2.41 g, 9.45 mmol) was treated sequentially with (a) N-Boc-4-piperidone/Ti(OiPr)₄, and (b) Et₂AlCN, to give the cyano-amine as described in Step 5 of Example 11.
2) The product of Step 1 and MeMgBr (16 ml, 3.0 M in Et₂O) were taken up in THF (30 ml) and stirred at 25 °C for19 h. The solution was quenched with 1 N NaOH and EtOAc. The mixture was filtered (Celite). The aqueous layer was extracted with EtOAc, the combined EtOAc layers were washed with brine and dried (Na₂SO₄). Filtration and concentration gave a yellow oil. Purification via flash chromatography (6/1 hexanes/EtOAc, SiO₂) gave 2.54 g (69 % from the free piperidine) of the vinyl bromide as a solid. m.p. (free base) 85-90 °C. HRMS (MH⁺) calcd. for C₁₈H₃₂O₂N₂Br, 387.1647; Found, 387.1638.
3) The product of Step 2 (200 mg, 0.52 mmol), 4-CF₃C₆H₄B(OH)₂ (344 mg, 1.8 mmol), PdCl₂(PPh₃)₂ (36 mg, 0.052 mmol), and Na₂CO₃ (165 mg, 1.56 mmol) were taken up in THF/H₂O (4/1, 10 ml) and heated at 75 °C (oil bath) for 21 hours. The solution was partitioned between EtOAc and H₂O. The aqueous layer was extracted with EtOAc, the combined EtOAc layers were washed with brine and dried (Na₂SO₄). Filtration and concentration gave a yellow oil. Purification via flash chromatography (3/1 to 1/1 hexanes/EtOAc, SiO₂) gave 210 mg (89 %) of the phenyl substitued olefin as an oil. HRMS (MH⁺) calcd. for C₂₅H₃₆O₂N₂F₃ , 453.2729; Found, 453.2728.
4) The product of Step 3 was hydrogenated as described in Step 3 of Example 11. The reduced product was deprotected and coupled to 2,6-dimethyl benzoic acid as described in Example 11, steps 7- 8 to give the title compound as a yellow oil (37 mg, 55%). m.p. (HCl salt) 130-140 °C. HRMS (MH⁺) calcd. for C₂₉H₃₈ON₂F₃ , 487.2936; Found, 487.2928.

Using a similar procedure, the following compound was prepared: 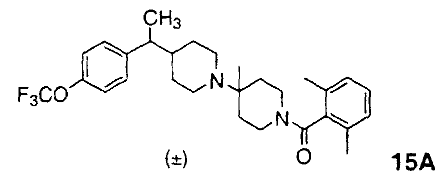 m.p. (HCl salt) 135-145°C. HRMS (MH⁺) calcd. for C₂₉H₃₈O₂N₂F₃, 503.2885; Found, 503.2896.

The following assays can be used to determine the CCR5 inhibitory and antagonistic activity of the compounds of the invention.

### CCR5 Membrane Binding Assay:

A high throughput screen utilizing a CCR5 membrane binding assay identifies inhibitors of RANTES binding. This assay utilizes membranes prepared from NIH 3T3 cells expressing the human CCR5 chemokine receptor which have the ability to bind to RANTES, a natural ligand for the receptor. Using a 96-well plate format, membrane preparations are incubated with ¹²⁵I-RANTES in the presence or absence of compound for one hour. Compounds are serially diluted over a wide range of 0.001 ug/ml to 1 ug/ml and tested in triplicates. Reaction cocktails are harvested through glass fiber filters, and washed thoroughly. Total counts for replicates are averaged and data reported as the concentration required to inhibit 50 percent of total ¹²⁵I-RANTES binding. Compounds with potent activity in the membrane binding assay are further characterized in seconday cell-based HIV-1 entry and replication assays.

### HIV-1 Entry Assay:

Replication defective HIV-1 reporter virions are generated by cotransfection of a plasmid encoding the NL4-3 strain of HIV-1 (which has been modified by mutation of the envelope gene and introduction of a luciferase reporter plasmid) along with a plasmid encoding one of several HIV-1 envelope genes as described by Connor et al, Virology, 206 (1995), p. 935-944. Following transfection of the two plasmids by calcium phosphate precipitation, the viral supernatants are harvested on day 3 and a functional viral titer determined. These stocks are then used to infect U87 cells stably expressing CD4 and the chemokine receptor CCR5 which have been preincubated with or without test compound. Infections are carried out for 2 hours at 37 °C, the cells washed and media replaced with fresh media containing compound. The cells are incubated for 3 days, lysed and luciferase activity determined. Results are reported as the concentration of compound required to inhibit 50% of the luciferase activity in the control cultures.

### HIV-1 Replication Assay:

This assay uses primary peripheral blood mononuclear cells or the stable U87-CCR5 cell line to determine the effect of anti-CCR5 compounds to block infection of primary HIV-1 strains. The primary lymphocytes are purified from normal healthy donors and stimulated *in vitro* with PHA and IL-2 three days prior to infection. Using a 96-well plate format, cells are pretreated with drug for 1 hour at 37 °C and subsequently infected with an M-tropic HIV-1 isolates. Following infection, the cells are washed to remove residual inoculum and cultured in the presence of compound for 4 days. Culture supernatants are harvested and viral replication measured by determination of viral p24 antigen concentration.

### Calcium Flux Assay:

Cells expressing the HIV coreceptor CCR5 are loaded with calcium sensitive dyes prior to addition of compound or the natural CCR5 ligand. Compounds with agonist properties will induce a calcium flux signal in the cell, while CCR5 antagonists are identified as compounds which do not induce signaling by themselves but are capable of blocking signaling by the natural ligand RANTES.

### GTPγS Binding Assay (secondary membrane binding assay):

A GTPγS binding assay measures receptor activation by CCR5 ligands. This assay measures the binding of ³⁵S labeled-GTP to receptor coupled G-proteins that occurs as a result of receptor activation by an appropriate ligand. In this assay, the CCR5 ligand, RANTES, is incubated with membranes from CCR5 expressing cells and binding to the receptor activation (or binding) is determined by assaying for bound ³⁵S label. The assay quantitatively determines if compounds exhibit agonist characteristics by inducing activation of the receptor or alternatively antagonist properties by measuring inhibition of RANTES binding in a competitive or non-competitive fashion.

### Chemotaxis Assay:

The chemotaxis assay is a functional assay which characterizes the agonist vs. antagonist properties of the test compounds. The assay measures the ability of a non-adherent murine cell line expressing human CCR5 (BaF-550) to migrate across a membrane in response to either test compounds or natural ligands (i.e., RANTES, MIP-1β). Cells migrate across the permeable membrane towards compounds with agonist activity. Compounds that are antagonists not only fail to induce chemotaxis, but are also capable of inhibiting cell migration in response to known CCR5 ligands.

The role of CC chemokine receptors such as CCR-5 receptors in inflammatory conditions has been reported in such publications as Immunology Letters, 57, (1997), 117-120 (arthritis); Clinical & Experimental Rheumatology, 17 (4) (1999), p. 419-425 (rheumatoid arthritis); Clinical & Experimental Immunology, 117 (2) (1999), p.237-243 (atopic dermatitis); International Journal of Immunopharmacology, 20 (11) (1998), p. 661-7 (psoriasis); Journal of Allergy & Clinical Immunology, 100 (6, Pt 2) (1997), p. S52-5 (asthma); and Journal of Immunology, 159 (6) (1997), p. 2962-72 (allergies).

In the assay to determine inhibition of RANTES binding, compounds of the invention range in activity from a Ki of 0.1 to 2000 nM, with preferred compounds having a range of activity from 0.1 to 1000 nM, more preferably 0.1 to 500 nM, and most preferably 0.1 to 100 nM. The results for preferred and representative compounds of formulas I and II in the test to determine inhibition of RANTES binding are given in the table below. In the table, "Ex. No." stands for "Example Number" and "nM" stands for "nanomolar."

| Ex. No. | Ki (nM) Inhibition of RANTES binding |
|---|---|
| 1B | 14 |
| 1J | 1 |
| 2 | 9.6 |
| 2G | 1.8 |
| 2S | 17.9 |
| 2JJ | 0.58 |
| 4B | 0.5 |
| 4C | 0.5 |
| 5L | 7.9 |
| 5N | 1.7 |
| 5O | 0.4 |
| 5Z | 0.3 |
| 5AB | 0.1 |
| 6V | 0.8 |
| 7U | 62.5 |
| 9D | 588 |

For preparing pharmaceutical compositions from the CCR5 antagonist compounds described by this invention, inert, pharmaceutically acceptable carriers can be either solid or liquid. Solid form preparations include powders, tablets, dispersible granules, capsules, cachets and suppositories. The powders and tablets may be comprised of from about 5 to about 95 percent active ingredient. Suitable solid carriers are known in the art, e.g. magnesium carbonate, magnesium stearate, talc, sugar or lactose. Tablets, powders, cachets and capsules can be used as solid dosage forms suitable for oral administration. Examples of pharmaceutically acceptable carriers and methods of manufacture for various compositions may be found in A. Gennaro (ed.), Remington's Pharmaceutical Sciences, 18th Edition, (1990), Mack Publishing Co., Easton, Pennsylvania.

Liquid form preparations include solutions, suspensions and emulsions. As an example may be mentioned water or water-propylene glycol solutions for parenteral injection or addition of sweeteners and opacifiers for oral solutions, suspensions and emulsions. Liquid form preparations may also include solutions for intranasal administration.

Aerosol preparations suitable for inhalation may include solutions and solids in powder form, which may be in combination with a pharmaceutically acceptable carrier, such as an inert compressed gas, e.g. nitrogen.

Also included are solid form preparations which are intended to be converted, shortly before use, to liquid form preparations for either oral or parenteral administration. Such liquid forms include solutions, suspensions and emulsions.

The compounds of the invention may also be deliverable transdermally. The transdermal compositions can take the form of creams, lotions, aerosols and/or emulsions and can be included in a transdermal patch of the matrix or reservoir type as are conventional in the art for this purpose.

Preferably the compound is administered orally.

Preferably, the pharmaceutical preparation is in a unit dosage form. In such form, the preparation is subdivided into suitably sized unit doses containing appropriate quantities of the active component, e.g., an effective amount to achieve the desired purpose.

The quantity of active compound in a unit dose of preparation may be varied or adjusted from about 10 mg to about 500 mg, preferably from about 25 mg to about 300 mg, more preferably from about 50 mg to about 250 mg, and most preferably from about 55 mg to about 200 mg, according to the particular application.

The actual dosage of CCR5 compound employed may be varied depending upon the requirements of the patient and the severity of the condition being treated. Determination of the proper dosage regimen for a particular situation is within the skill of the art. For convenience, the total daily dosage may be divided and administered in portions during the day as required.

The amount and frequency of administration of the CCR5 compounds of the invention and/or the pharmaceutically acceptable salts thereof will be regulated according to the judgment of the attending clinician considering such factors as age, condition and size of the patient as well as severity of the symptoms being treated. A typical recommended daily dosage regimen for oral administration can range from about 100 mg/day to about 300 mg/day, preferably 150 mg/day to 250 mg/day, more preferably about 200 mg/day, in two to four divided doses.

The doses and dosage regimens of the NRTIs, NNRTIs, PIs and other agents used in combination with the CCR5 antagonists will be determined by the attending clinician inview of the approved doses and dosage regimens in the package inserts or as set forth in the protocols, taking into consideration the age, sex and condition of the patient and the severity of the condition treated.

The goal of the HIV-1 therapy of the present invention is to reduce the HIV-1-RNA viral load below the detectable limit. The "detectable limit of HIV-1-RNA" in the context of the present invention means that there are fewer than about 200 to fewer than about 50 copies of HIV-1-RNA per ml of plasma of the patient as measured by quantitative, multi-cycle reverse transcriptase PCR methodology. HIV-1-RNA is preferably measured in the present invention by the methodology of Amplicor -1 Monitor 1.5 (available from Roche Diagnsotics) or of Nuclisens HIV-1 QT -1.

## Claims

1. A compound represented by the structure formula II 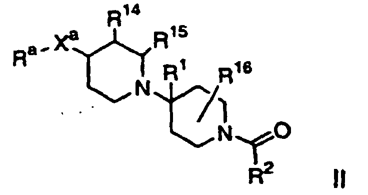 or a pharmaceutically acceptable salt thereof, wherein
(1) X^{a} is -C(R¹³)₂-, -C(R¹³)(R¹⁹)-, -C(O)-, -O-, -NH-, -N((C₁-C₆)alkyl)-, 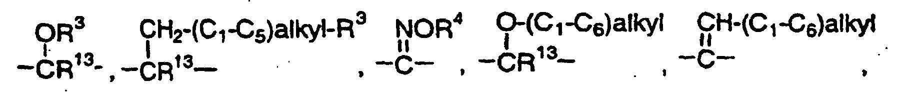 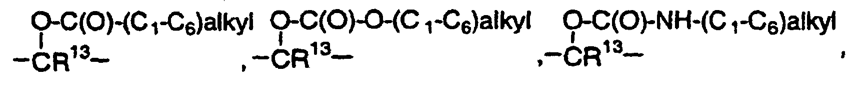 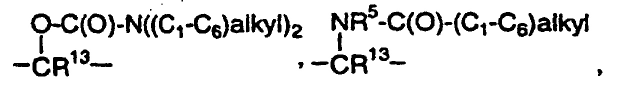 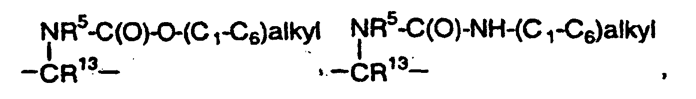 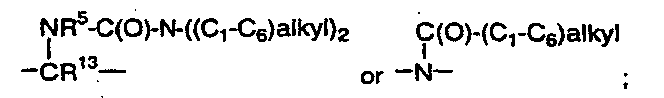 R^{a} is R^{6a}-phenyl, R^{6a}-pyridyl, R^{6a}-thiophenyl or R⁶-naphthyl;
R¹ is hydrogen, C₁-C₆ alkyl or C₂-C₆ alkenyl;
R² is 6-membered heteroaryl substituted by R⁷, R⁸ and R⁹; 6-membered heteroaryl N-oxide substituted by R⁷, R⁸ and R⁹; 5-membered heteroaryl substituted by R¹⁰ and R¹¹; naphthyl; fluorenyl; diphenylmethyl; 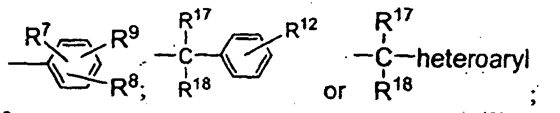 R³ is R¹⁰-phenyl, pyridyl, pyrimidyl, pyrazinyl or thiazolyl;
R⁴ is hydrogen, C₁-C₆ alkyl, fluoro-C₁-C₆ alkyl, cyclopropylmethyl, -CH₂CH₂OH, -CH₂CH₂-O-(C₁-C₆)alkyl, -CH₂C(O)-O-(C₁-C₆)alkyl, -CH₂C(O)NH₂, -CH₂C(O)-NH(C₁-C₆)alkyl or -CH₂C(O)-N((C₁-C₆)alkyl)₂;
R⁵ and R¹¹ are independently selected from the group consisting of hydrogen and (C₁-C₆)-alkyl;
R^{6a} is 1 to 3 substituents independently selected from the group consisting of hydrogen, halogen, -CF₃, CF₃O-, -CN, -CF₃SO₂-, -NHCOCF₃, 5-membered heteroaryl and 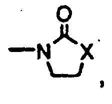 wherein X is -O-, -NH- or -N(CH₃)- ;
R⁶ is independently selected from the group consisting of R^{6a} and CH₃SO₂-;
R⁷ and R⁸ are independently selected from the group consisting of (C₁-C₆)alkyl, halogen, -NR²⁰R²¹, -OH, -CF₃, -OCH₃, -O-acyl, and -OCF₃;
R⁹ is R⁷, hydrogen, phenyl, -NO₂, -CN, -CH₂F, -CHF₂, -CHO, -CH=NOR²⁰, pyridyl, pyridyl N-oxide, pyrimidinyl, pyrazinyl, -N(R²⁰)CONR²¹R²², -NHCONH(chloro-(C₁-C₆)alkyl), -NHCONH((C₃-C₁₀)-cycloalkyl(C₁-C₆)alkyl), -NHCO(C₁-C₆)alkyl, -NHCOCF₃, -NHSO₂N((C₁-C₆)alkyl)₂, -NHSO₂(C₁-C₆)alkyl, -N(SO₂CF₃)₂, -NHCO₂(C₁-C₆)alkyl, C₃-C₁₀ cycloalkyl, -SR²³, -SOR²³, -SO₂R²³, -SO₂NH(C₁-C₆ alkyl), -OSO₂(C₁-C₆)alkyl, -OSO₂CF₃, hydroxy(C₁-C₆)alkyl, -CON R²⁰R²¹, -CON(CH₂CH₂-O-CH₃)₂,
-OCONH(C₁-C₆)alkyl, -CO₂R²⁰, -Si(CH₃)₃ or -B(OC(CH₃)₂)₂;
R¹⁰ is (C₁-C₆)alkyl, -NH₂ or R¹²-phenyl;
R¹² is1 to 3 substituents independently selected from the group consisting of hydrogen, (C₁-C₆) alkyl, -CF₃, -CO₂R₂₀, -CN, (C₁-C₆)alkoxy and halogen;
R¹³, R¹⁴, R¹⁵ and R¹⁶ are independently selected from the group consisting of hydrogen and (C₁-C₆)alkyl;
R¹⁷ and R¹⁸ are independently selected from the group consisting of hydrogen and C₁-C₆ alkyl, or R¹⁷ and R¹⁸ together are a C₂-C₅ alkylene group and with the carbon to which they are attached form a spiro ring of 3 to 6 carbon atoms;
R¹⁹ is R⁶-phenyl, R⁶-heteroaryl, R⁶-naphthyl, C₃-C₁₀ cycloalkyl, (C₃-C₁₀)cycloalkyl(C₁-C₆)alkyl or (C₁-C₆)alkoxy(C₁-C₆)alkyl;
R²⁰, R²¹ and R²² are independently selected from the group consisting of H and C₁-C₆ alkyl; and
R²³ is C₁-C₆ alkyl or phenyl; or
(2):
X^{a} is -C(R¹³)(R¹⁹)-, -C(O)-, -O-, -NH-, -N((C₁-C₆)alkyl)-, 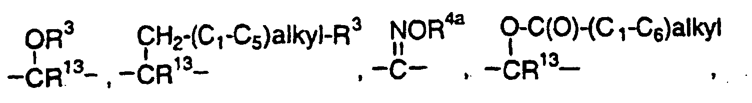 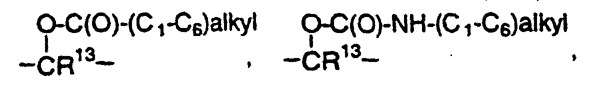 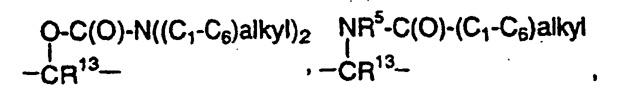 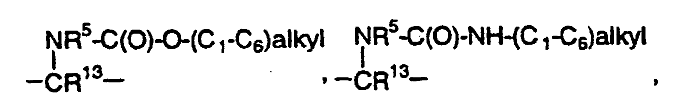 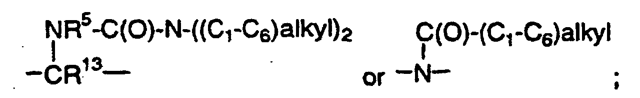 R^{a} is R^{6b}-phenyl, R^{6b}-pyridyl or R^{6b}-thiophenyl;
R^{4a} is fluoro-C₁-C₆ alkyl, cyclopropylmethyl, -CH₂CH₂OH, -CH₂CH₂-O-(C₁-C₆)alkyl, -CH₂C(O)-O-(C₁-C₆)alkyl, -CH₂C(O)NH₂, -CH₂C(O)-NH-(C₁-C₆)alkyl or -CH₂C(O)-N((C₁-C₆)alkyl)₂;
R^{6b} is CH₃SO₂-; and
R¹, R², R³, R⁵, R¹⁴, R¹⁵, R¹⁶ and R¹⁹ are as defined in (1).

2. The compound of claim 1 wherein R^{a} is

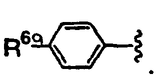

3. The compound of claim 1, formula II(1), wherein X^{a} is -CHOR³, -C(R¹³)(R¹⁹)- or -C(=NOR⁴)-.

4. The compound of claim 3 wherein R³ is pyridyl, R⁴ is (C₁-C₆)alkyl, or R¹³ is hydrogen and R¹⁹ is R⁶-phenyl.

5. The compound of claim 1, formula II(2), wherein X^{a} is -CHOR³, -C(R¹³)(R¹⁹)- or -C(=NOR^{4a})-.

6. The compound of claim 5 wherein R³ is pyridyl, R^{4a} is cyclopropylmethyl or trifluoroethyl, or R¹³ is hydrogen and R¹⁹ is R⁶-phenyl.

7. The compound of Claim 1 wherein R² is

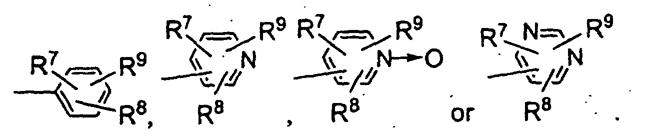

8. The compound of claim 7 wherein R² is selected from the group consisting of 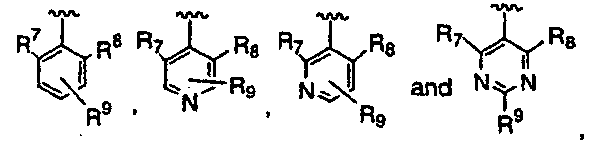 wherein R⁷ and R⁸ are selected from the group consisting of (C₁-C₆)alkyl, halogen, and -NH₂, and R⁹ is hydrogen.

9. A compound of selected from the group consisting of those represented by the formula 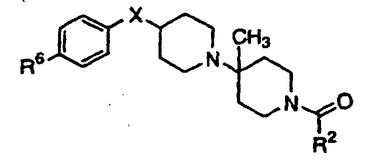 wherein R⁶, X and R² are as defined in the following table:

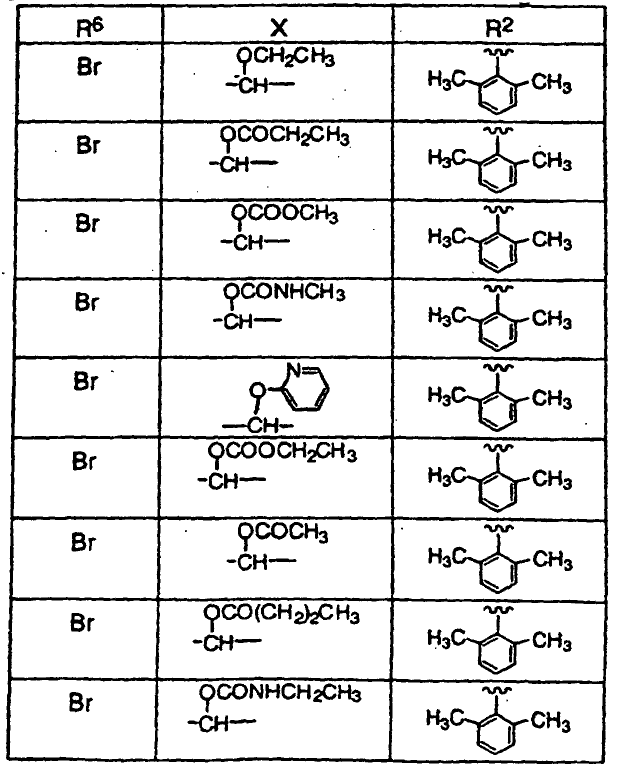

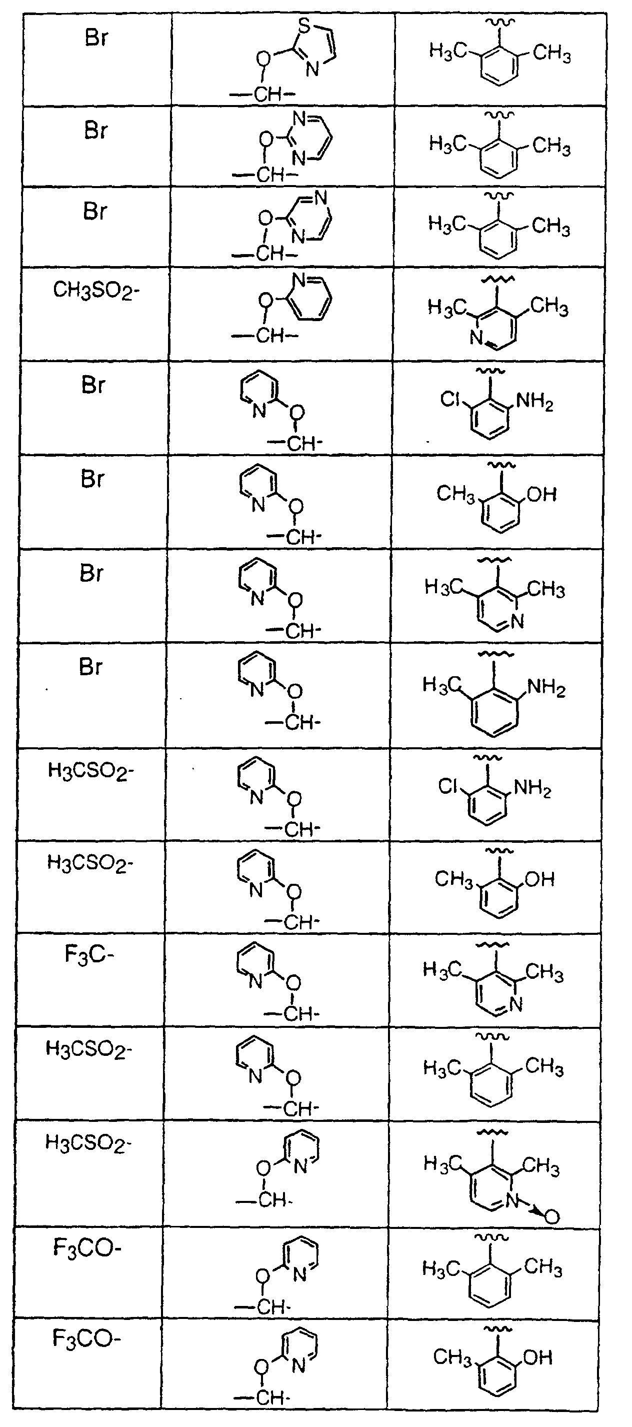

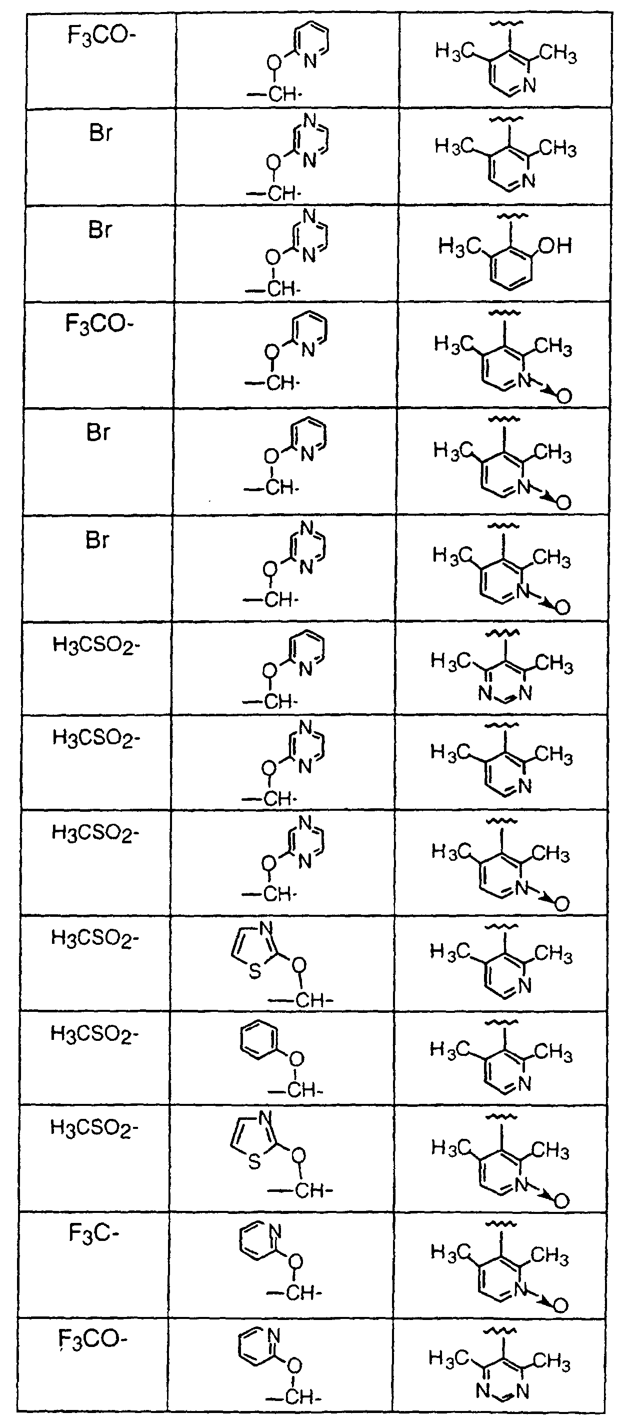

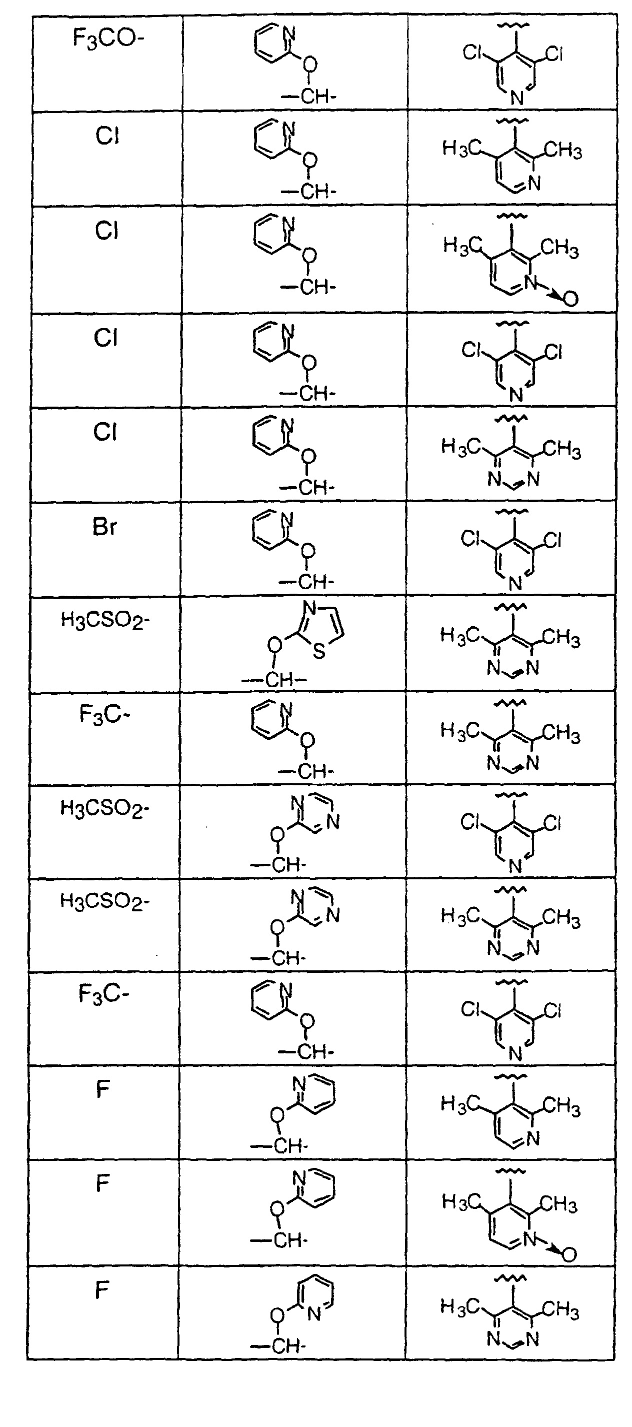

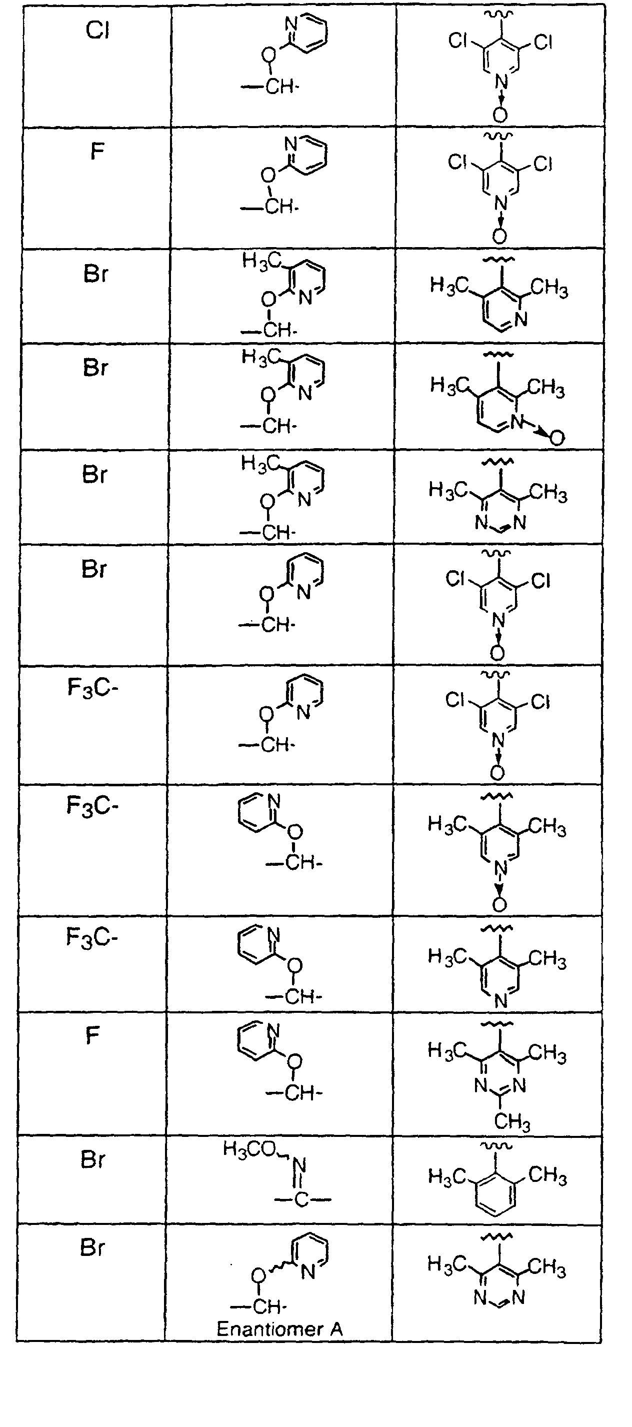

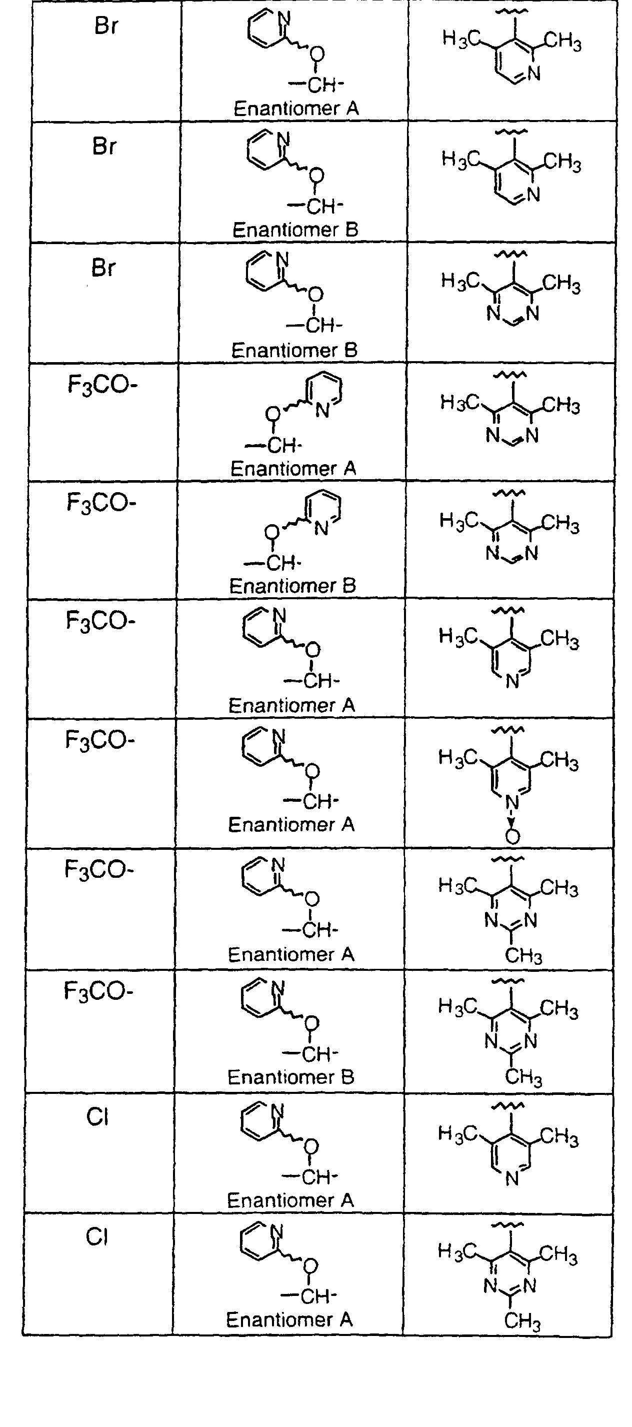

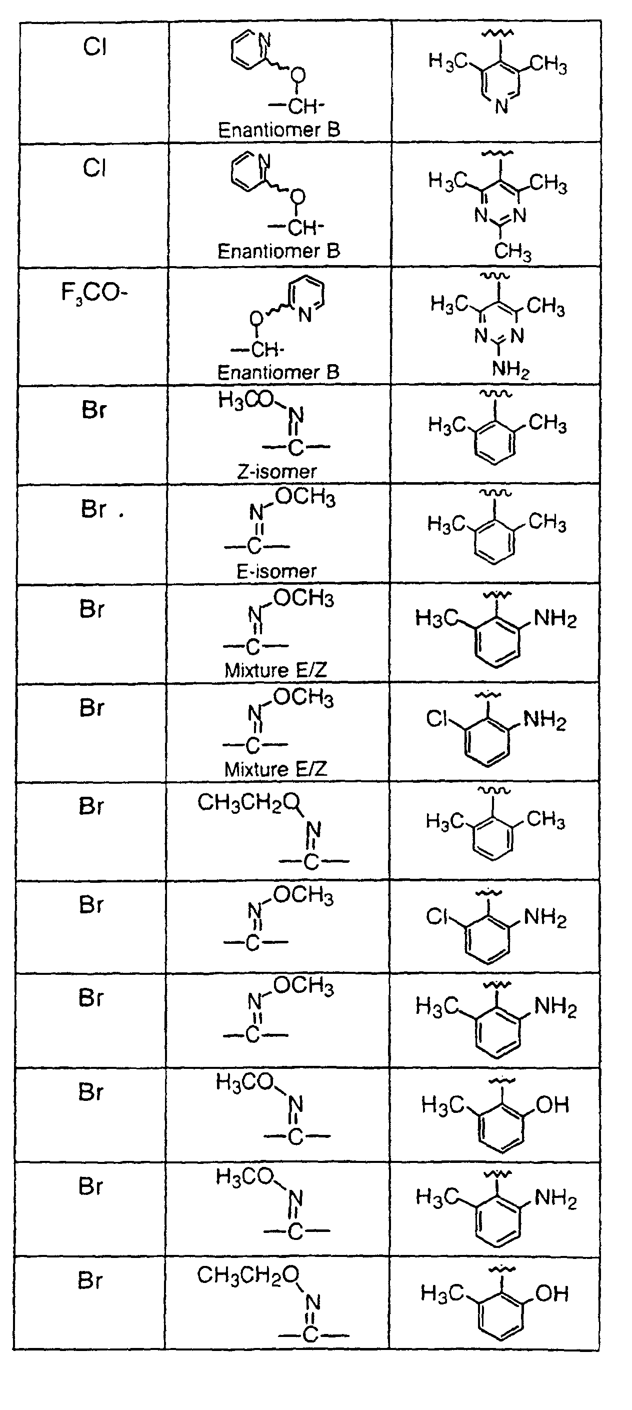

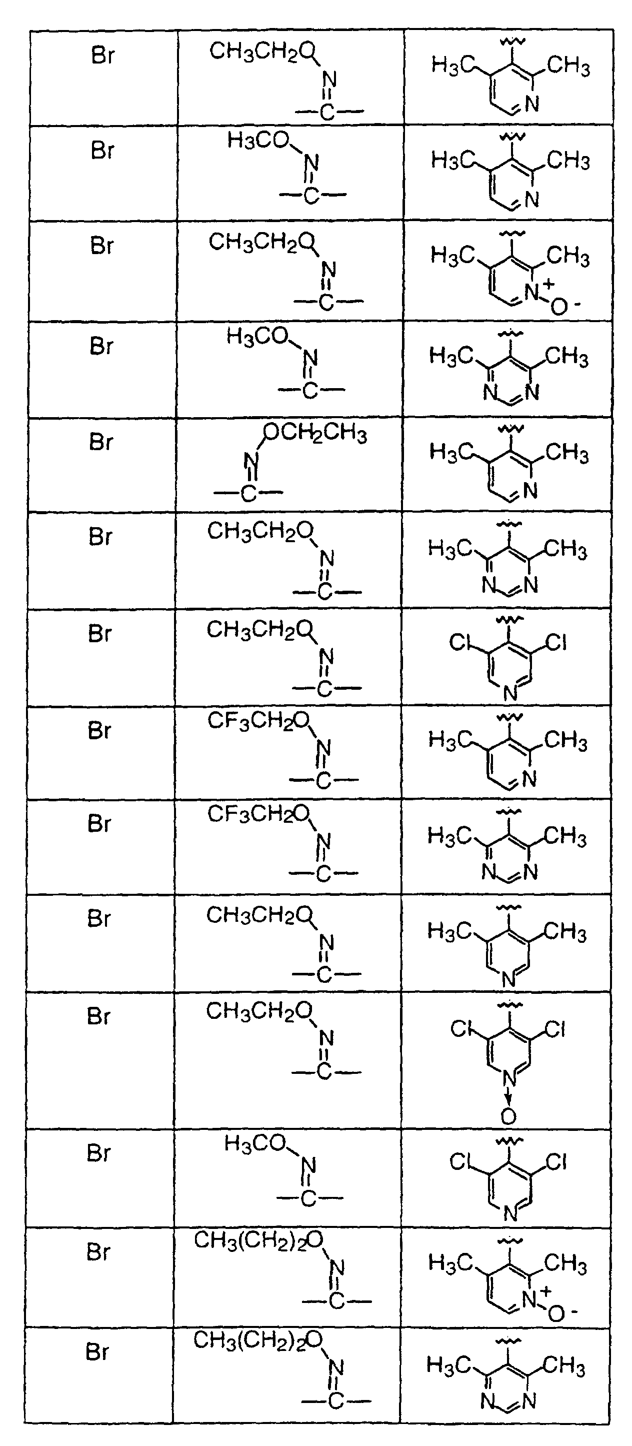

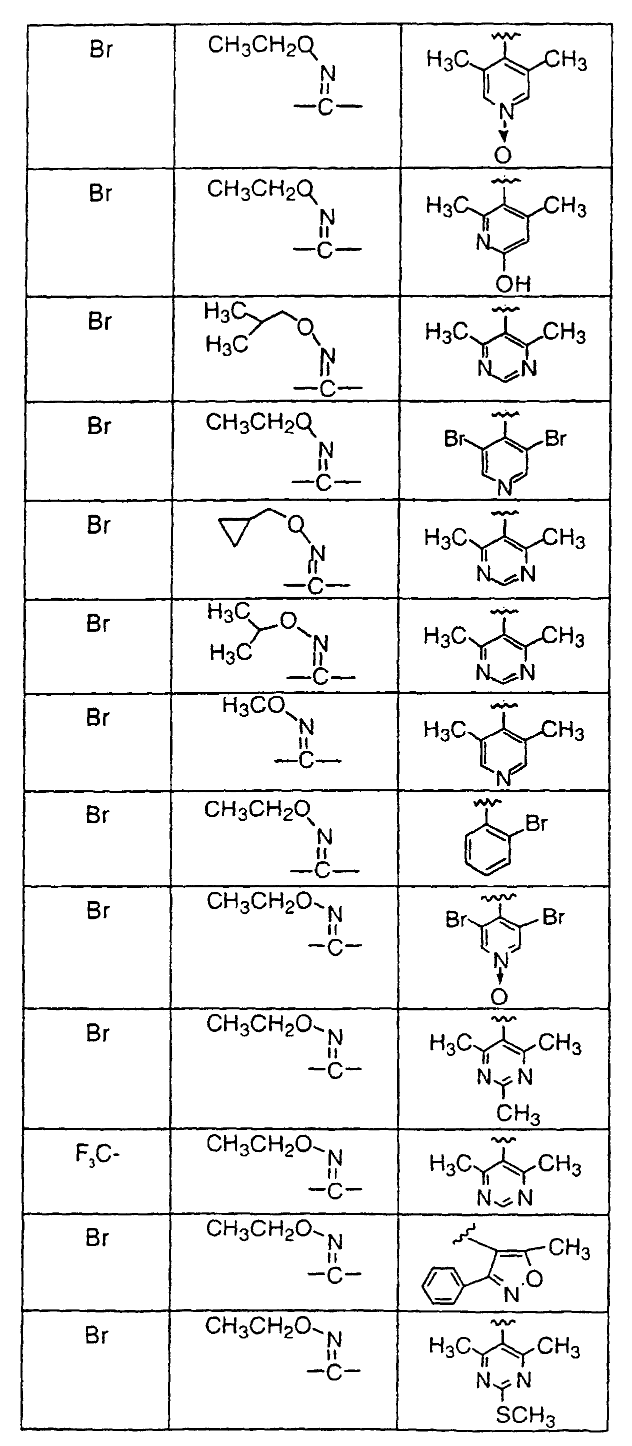

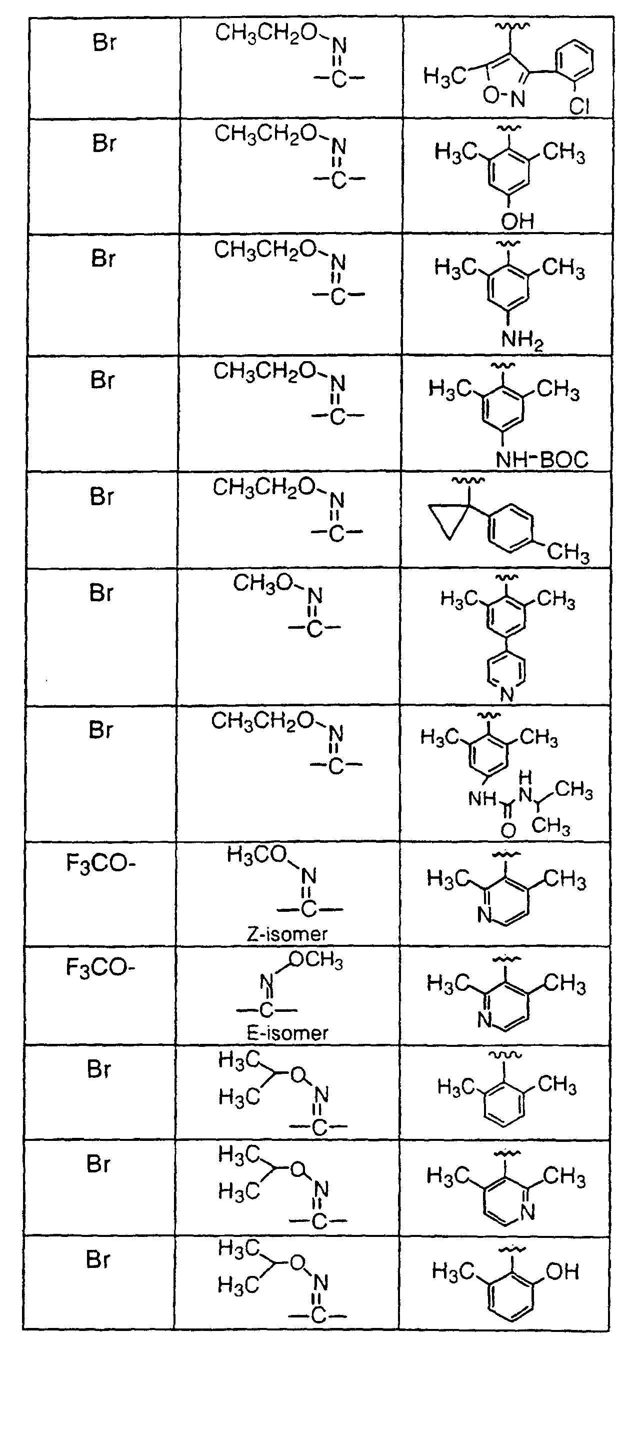

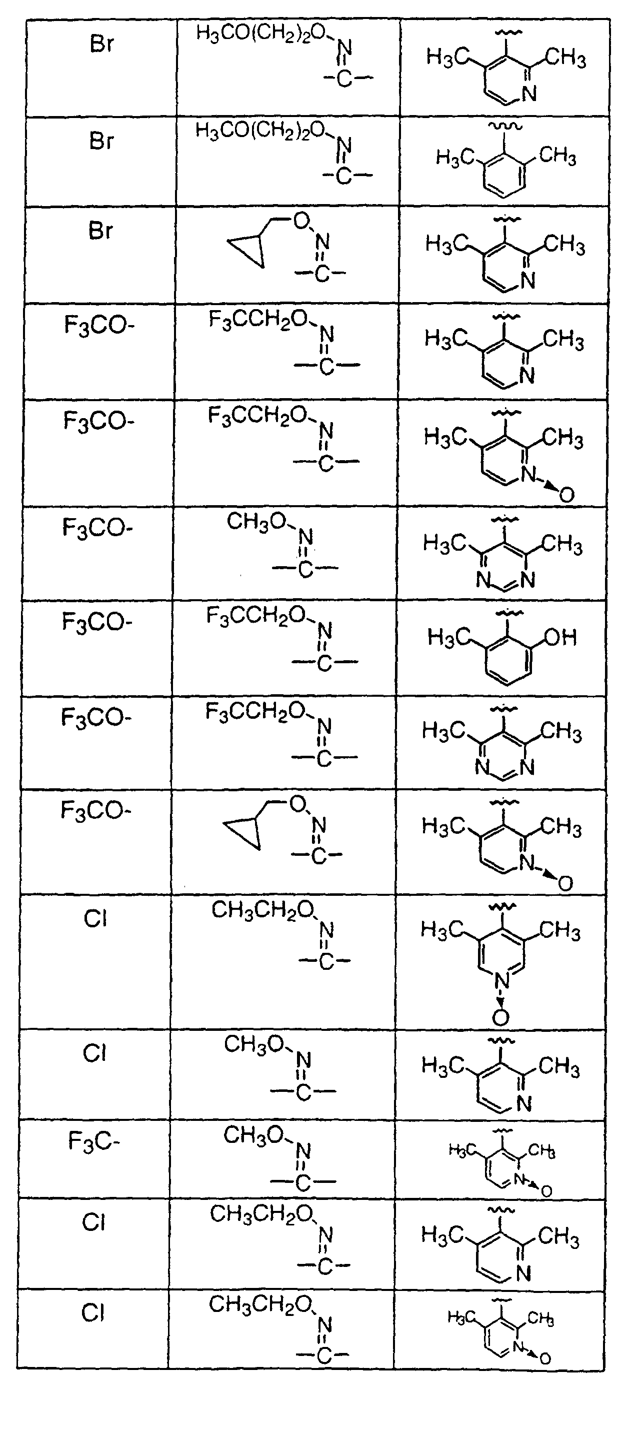

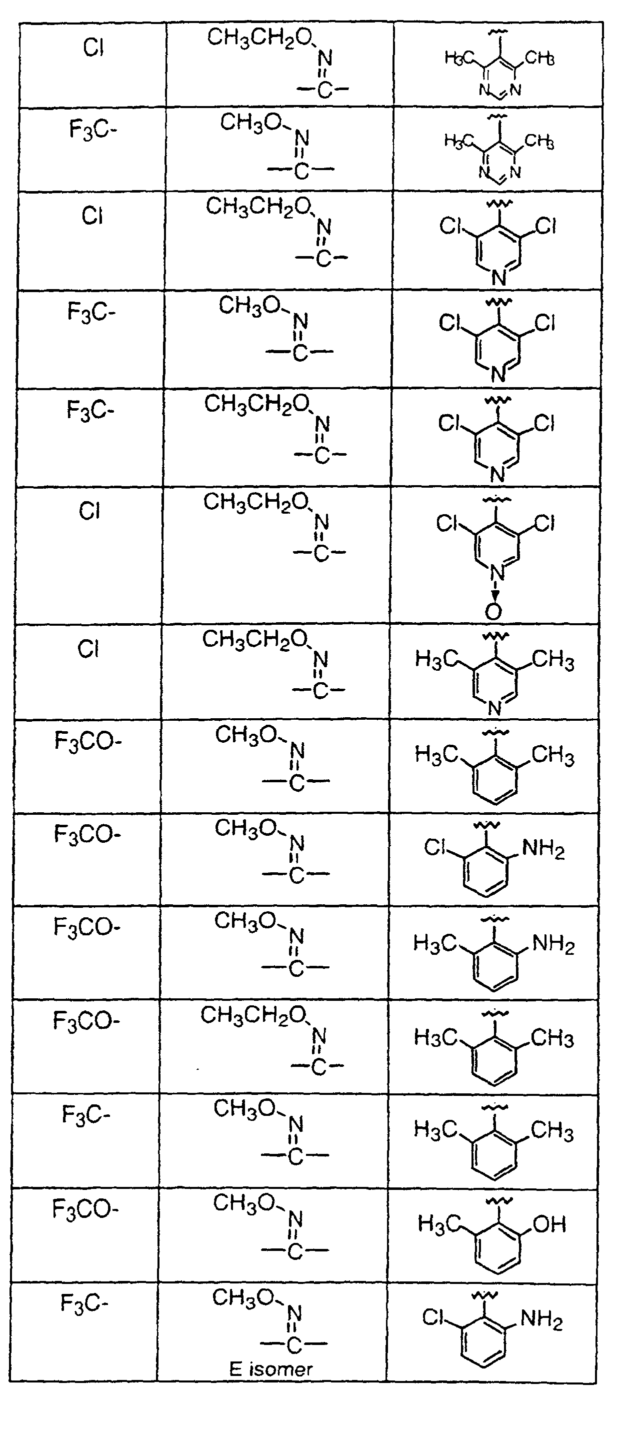

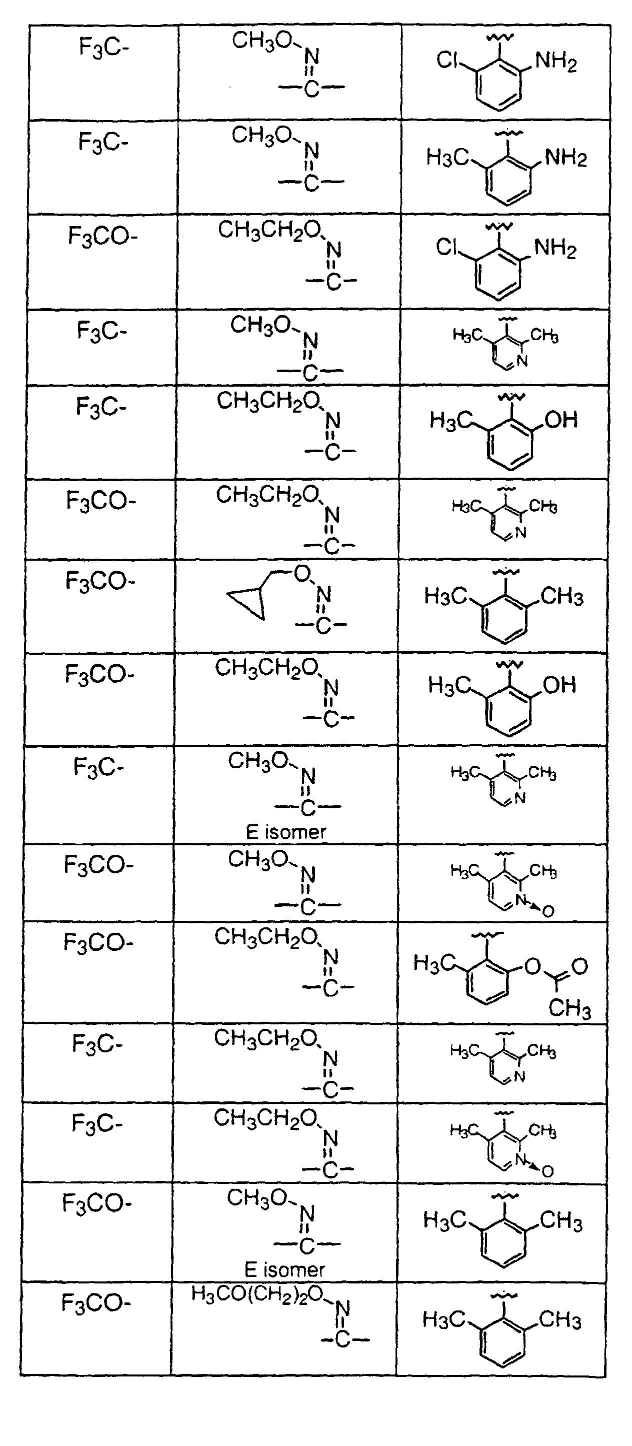

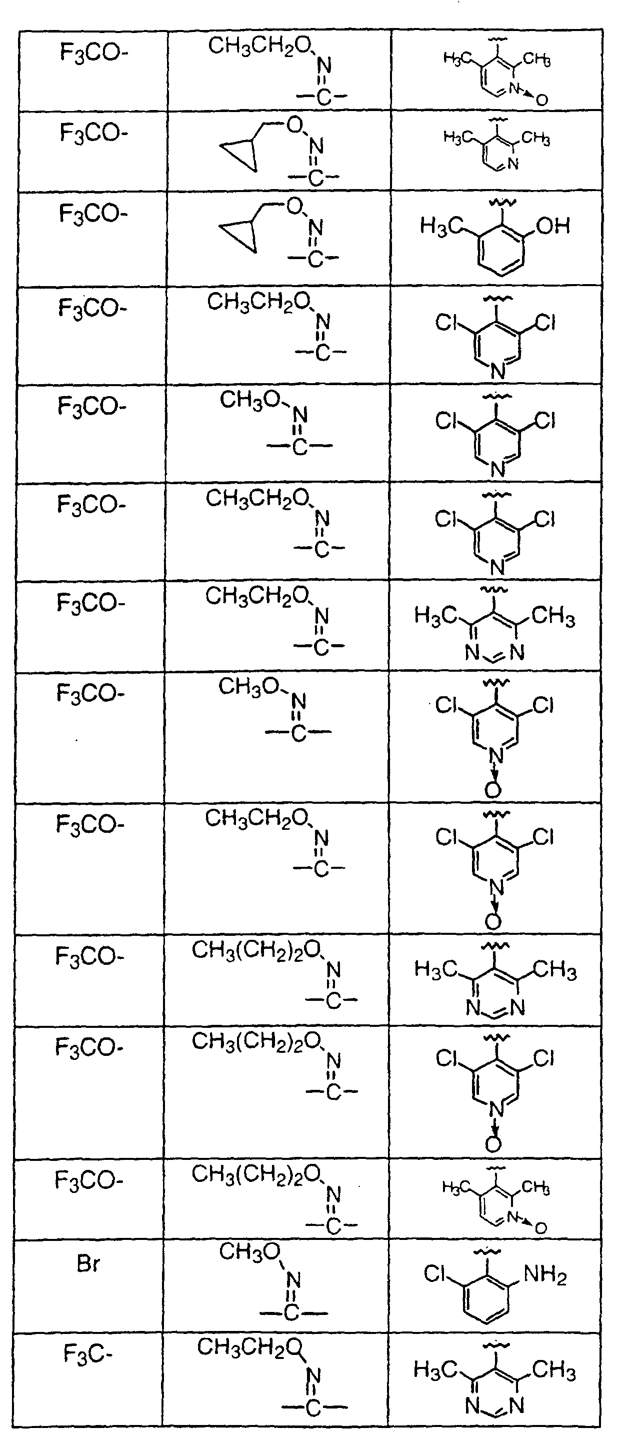

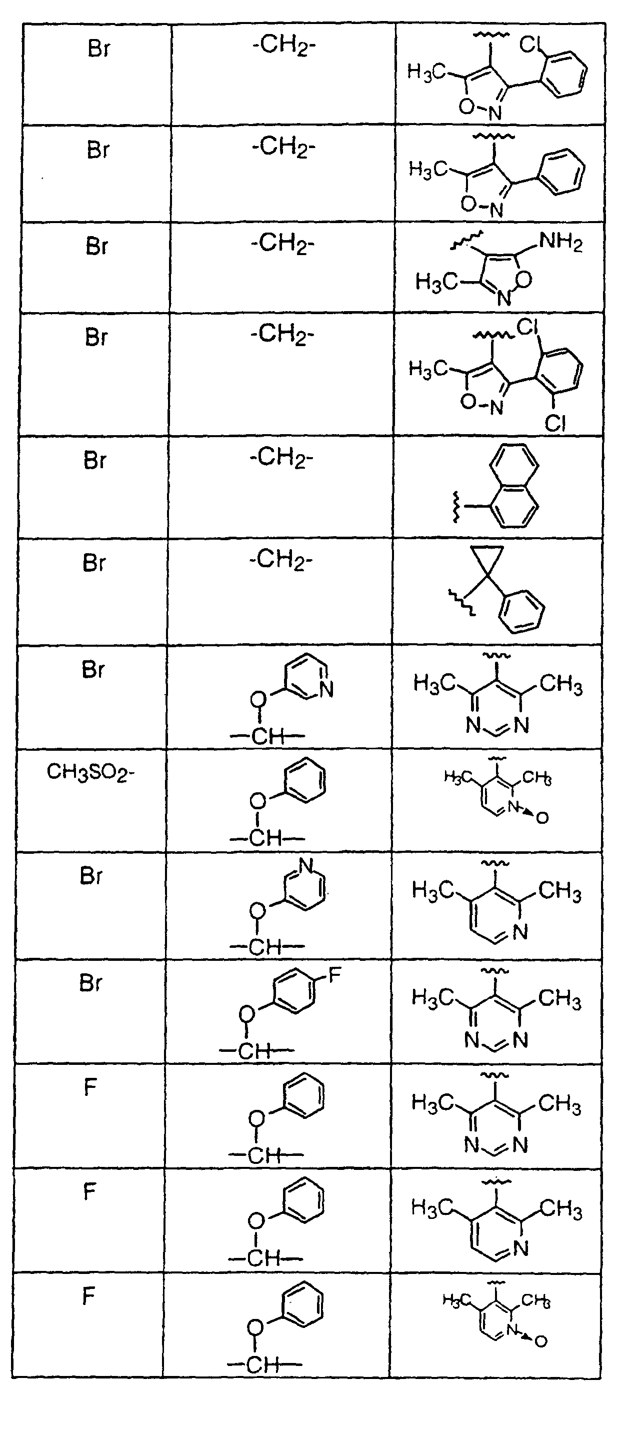

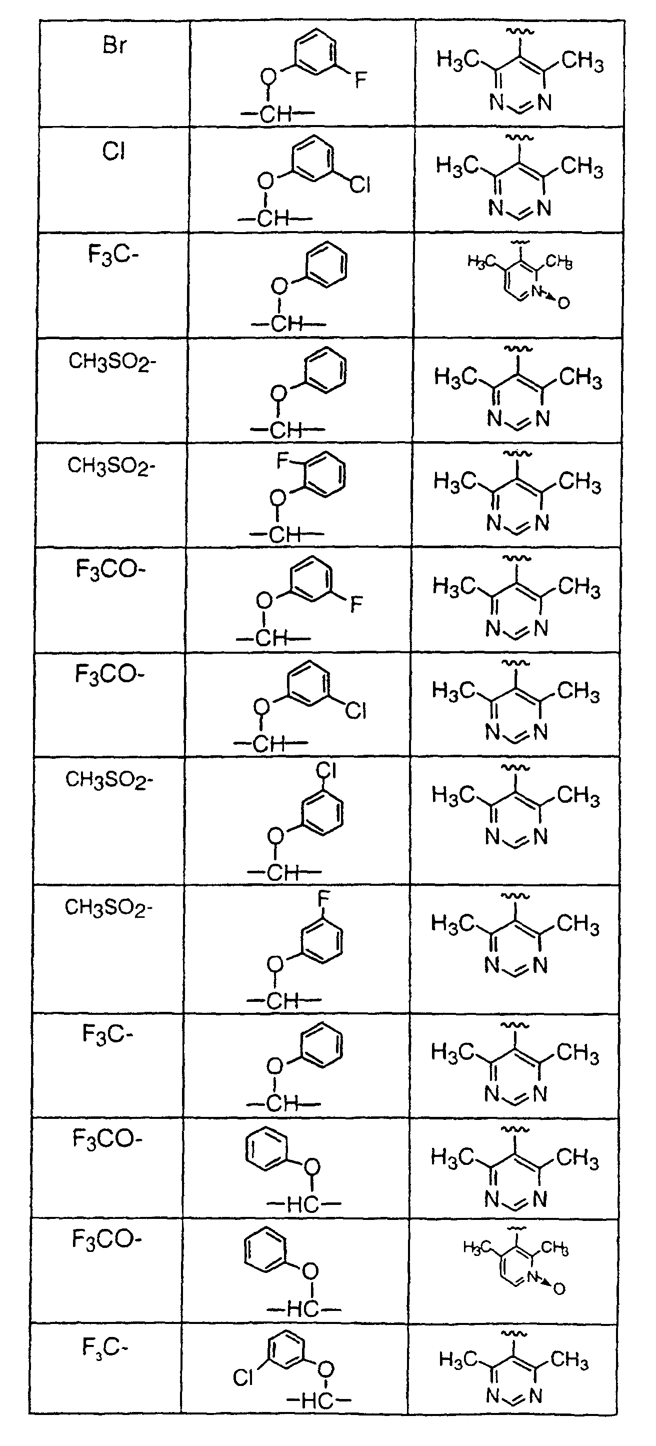

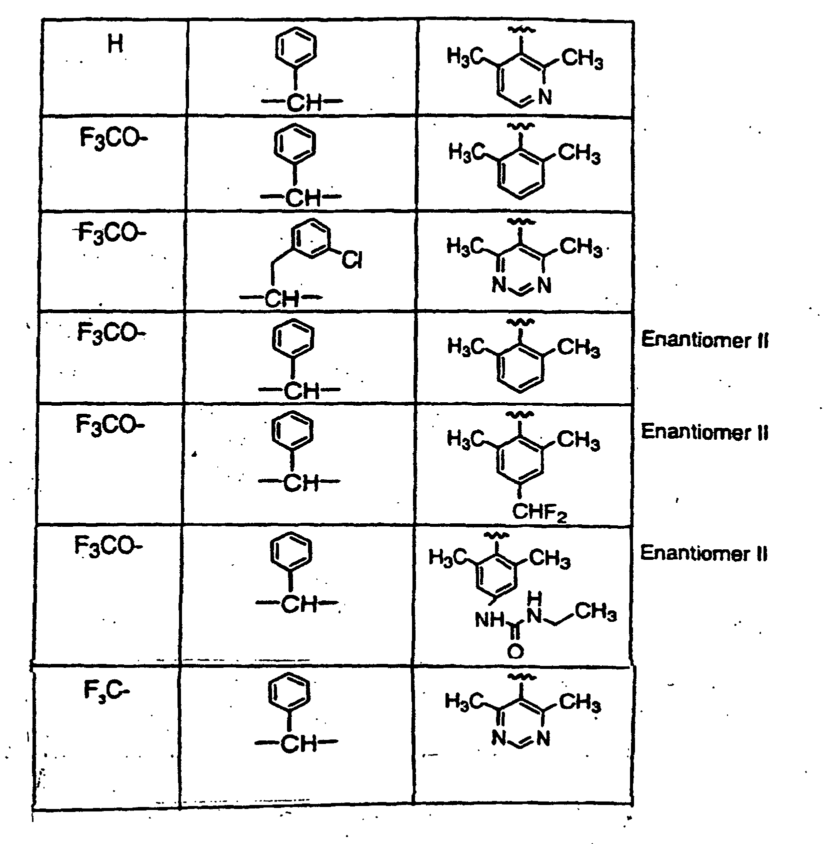

10. A compound according to Claim 9 selected from the group consisting of

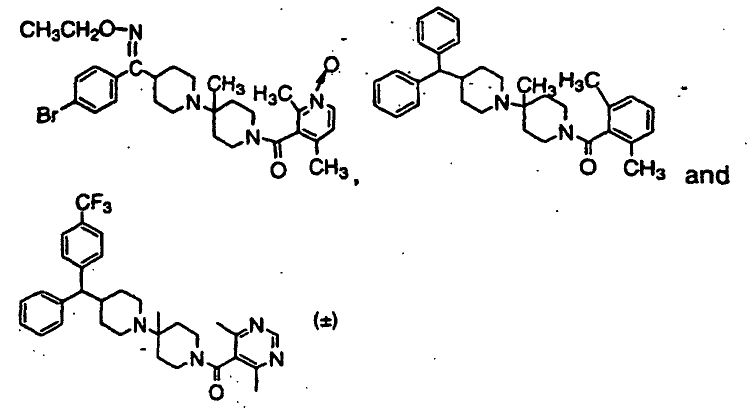

11. A compound having the formula

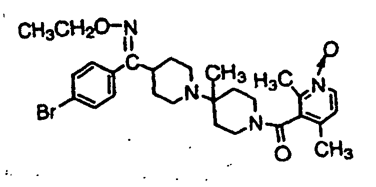

12. A pharmaceutical composition for the treatment of Human Immunodeficiency Virus, solid organ transplant rejection, graft v. host disease, arthritis, rheumatoid arthritis, inflammatory bowel disease, atopic dermatitis, psoriasis asthma, allergies or multiple sclerosis, comprising an effective amount of a CCR5 antagonist of any preceding claim in combination with a pharmaceutical carrier.

13. The use of a compound of any of claims 1 to 11 for the preparation of a medicament for treating Human Immunodeficiency Virus, solid organ transplant rejection, graft v. host disease, arthritis, rheumatoid arthritis, inflammatory bowel disease, atopic dermatitis, psoriasis, asthma, allergies or multiple sclerosis.

14. The use of a compound of any of claims 1 to 11 for the preparation of a medicament for combined use with one or more antiviral or other agents useful in the treatment of Human Immunodeficiency Virus.

15. The use of claim 14 wherein the antiviral agent is selected from the group consisting of nucleoside reverse transcriptase inhibitors, non-nucleoside reverse transcriptase inhibitors and protease inhibitors.

16. The use of a compound of any of claims 1 to 11 for the preparation of a medicament for combined use with one or more agents for treating solid organ transplant rejection, graft v. host disease, inflammatory bowel disease, rheumatoid arthritis or multiple sclerosis.

17. The use of a CCR5 antagonist of formula I for the preparation of a medicament for treating Human Immunodeficiency Virus, solid organ transplant rejection, graft v. host disease, arthritis, rheumatoid arthritis, inflammatory bowel disease, atopic dermatitis, psoriasis, asthma, allergies or multiple sclerosis, wherein the CCR5 antagonist is represented by the structural formula I: 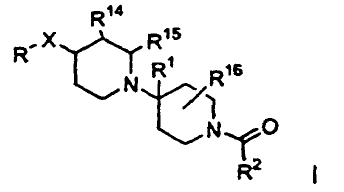 or a pharmaceutically acceptable salt thereof, wherein
X is -C(R¹³)₂-, -C(R¹³)(R¹⁹)-, -C(O)-, -O-, -NH-, -N((C₁-C₆)alkyl)-, 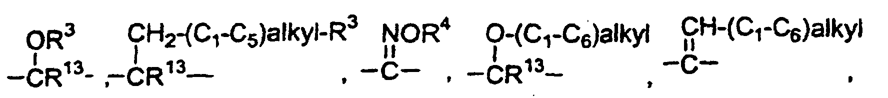 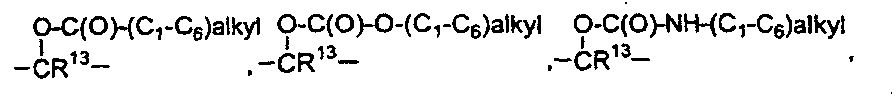 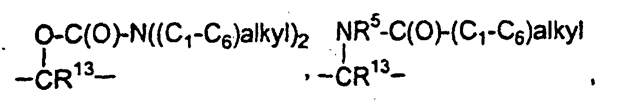 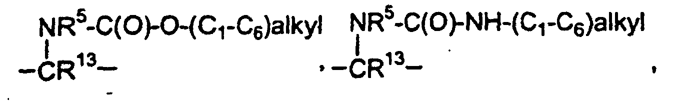 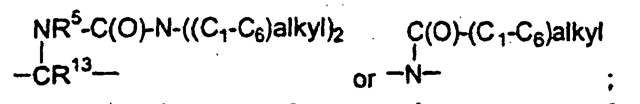 R is R⁶-phenyl, R⁶-pyridyl, R⁶-thiophenyl or R⁶-naphthyl;
R¹ is hydrogen, C₁-C₆ alkyl or C₂-C₆ alkenyl;
R² is 6-membered heteroaryl substituted by R⁷, R⁸ and R⁹; 6-membered heteroaryl N-oxide substituted by R⁷, R⁸ and R⁹; 5-membered heteroaryl substituted by R¹⁰ and R¹¹; naphthyl; fluorenyl; diphenylmethyl; 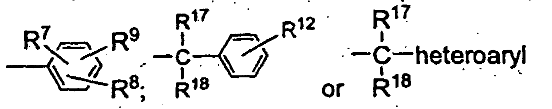 R³ is R⁶-phenyl, R⁶-heteroaryl or R⁶-naphthyl;
R⁴ is hydrogen, C₁-C₆ alkyl, fluoro-C₁-C₆ alkyl, cyclopropylmethyl, -CH₂CH₂OH, -CH₂CH₂-O-(C₁-C₆)alkyl, -CH₂C(O)-O-(C₁-C₆)alkyl, -CH₂C(O)NH₂, -CH₂C(O)-NH(C₁-C₆)alkyl or -CH₂C(O)-N((C₁-C₆)alkyl)₂;
R⁵ and R¹¹ are independently selected from the group consisting of hydrogen and (C₁-C₆)-alkyl;
R⁶ is 1 to 3 substituents independently selected from the group consisting of hydrogen, halogen, C₁-C₆ alkyl, C₁-C₆ alkoxy, -CF₃, CF₃O-, CH₃C(O)-, -CN, CH₃SO₂-, CF₃SO₂-, R¹⁴-phenyl, R¹⁴-benzyl, CH₃C(=NOCH₃)-, CH₃C(=NOCH₂CH₃)-, 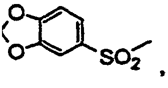 -NH₂, -NHCOCF₃, -NHCONH(C₁-C₆ alkyl), -NHCO(C₁-C₆ alkyl), -NHSO₂(C₁-C₆ alkyl), 5-membered heteroaryl and 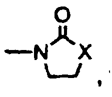 wherein X is -O-, -NH- or -N(CH₃)-;
R⁷ and R⁸ are independently selected from the group consisting of (C₁-C₆)alkyl, halogen, -NR²⁰R²¹, -OH, -CF₃, -OCH₃, -O-acyl, and -OCF₃;
R⁹ is R⁷, hydrogen, phenyl, -NO₂, -CN, -CH₂F, -CHF₂, -CHO, -CH=NOR²⁰, pyridyl, pyridyl N-oxide, pyrimidinyl, pyrazinyl, -N(R²⁰)CONR²¹R²², -NHCONH(chloro-(C₁-C₆)alkyl), -NHCONH((C₃-C₁₀)-cycloalkyl(C₁-C₆)alkyl), -NHCO(C₁-C₆)alkyl, -NHCOCF₃, -NHSO₂N((C₁-C₆)alkyl)₂, -NHSO₂(C₁-C₆)alkyl, -N(SO₂CF₃)₂, -NHCO₂(C₁-C₆)alkyl, C₃-C₁₀ cycloalkyl, -SR²³, -SOR²³, -SO₂R²³, -SO₂NH(C₁-C₆ alkyl), -OSO₂(C₁-C₆)alkyl, -OSO₂CF₃, hydroxy(C₁-C₆)alkyl, -CON R²⁰R²¹, -CON(CH₂CH₂-O-CH₃)₂, -OCONH(C₁-C₆)alkyl, -CO₂R²⁰, -Si(CH₃)₃ or -B(OC(CH₃)₂)₂;
R¹⁰ is (C₁-C₆)alkyl, -NH₂ or R¹²-phenyl;
R¹² is 1 to 3 substituents independently selected from the group consisting of hydrogen, (C₁-C₆) alkyl, -CF₃, -CO₂R₂₀, -CN, (C₁-C₆)alkoxy and halogen;
R¹³, R¹⁴, R¹⁵ and R¹⁶ are independently selected from the group consisting of hydrogen and (C₁-C₆)alkyl;
R¹⁷ and R¹⁸ are independently selected from the group consisting of hydrogen and C₁-C₆ alkyl, or R¹⁷ and R¹⁸ together are a C₂-C₅ alkylene group and with the carbon to which they are attached form a spiro ring of 3 to 6 carbon atoms;
R¹⁹ is R⁶-phenyl, R⁶-heteroaryl, R⁶-naphthyl, C₃-C₁₀ cycloalkyl, (C₃-C₁₀)cycloalkyl(C₁-C₆)alkyl or (C₁-C₆)alkoxy(C₁-C₆)alkyl;
R²⁰, R²¹ and R²² are independently selected from the group consisting of H and C₁-C₆ alkyl; and
R²³ is C₁-C₆ alkyl or phenyl.

18. The use of claim 17 wherein R is

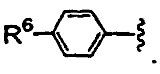

19. The use of claim 17 wherein X is -CHOR³, -C(R¹³)(R¹⁹)- or -C(=NOR⁴)-.

20. The use of claim 19 wherein R³ is pyridyl, R⁴ is (C₁-C₆)alkyl, or R¹³ is hydrogen and R¹⁹ is R⁶-phenyl.

21. The use of Claim 17 wherein R² is

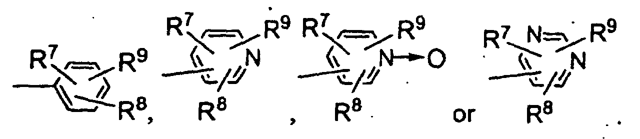

22. The use of Claim 21 wherein R² is selected from the group consisting of 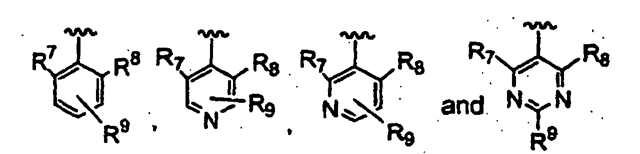 wherein R⁷ and R⁸ are selected from the group consisting of C₁-C₈)alkyl, halogen, and -NH₂, and R⁹ is hydrogen.

23. The use of claim 17 for the treatment of Human Immunodeficiency Virus, further comprisisng one or more entiviral or other agents useful in the treatment of Human Immunodeficiency Virus.

24. The use of claim 23 wherein the antiviral agent is selected from the group consisting of nucleotide reverse transcriptase inhibitors, non-nucleotide reverse transcriptase inhinitors and protease inhibitors.

25. The use of claim 17 for the treatment of solid organ transplant rejection, graft v. host disease, inflammatory bowel disease, rheumatoid arthritis or multiple sclerosis, further comprising one or more other agents useful in the treatment of said disease.

26. A kit comprising in separate containers in a single package pharmaceutical compositions for use in combination to treat Human Immunodeficiency Virus which comprises in one container a pharmaceutical composition comprising an effective amount of a CCR5 antagonist of claim 17 in a pharmaceutically acceptable carrier, and in separate containers, one or more pharmaceutical composition comprising an effective amount of a antiviral or other agent useful in the treatment of Human Immunodeficiency Virus in a pharmaceutically acceptable carrier.

27. A pharmaceutical composittion in the form of a cream for topical application comprising compound of Formula II as efined in any of claims 1 to 11 and a pharmaceutically acceptable carrier.

28. A pharmaceutical composition in the form of a cream for topical application comprising a compound of Formula 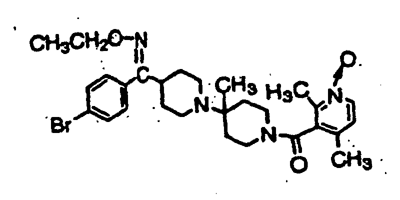 and a pharmaceutically acceptable carrier.

## Patentansprüche

1. Verbindung mit der Strukturformel II 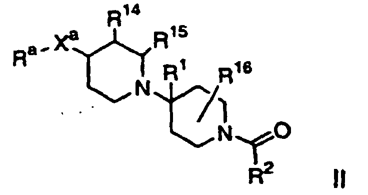 oder ein pharmazeutisch annehmbares Salz davon; worin
(1) X^{a} -C(R¹³)₂-, -C(R¹³)(R¹⁹)-, -C(O)-, -O-, -NH-, -N((C₁-C₆)-alkyl)-, 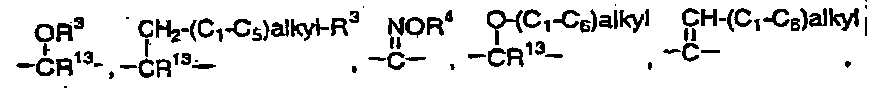 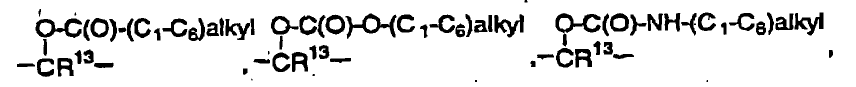 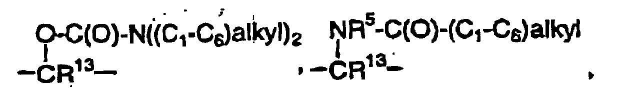 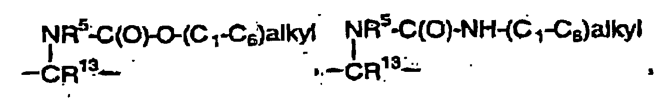 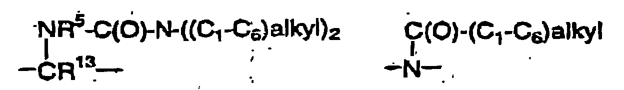 oder ist;
R^{a} R^{6a}-Phenyl, R^{6a}-Pyridyl, R^{6a}-Thiophenyl oder R⁶-Naphthyl ist;
R¹ Wasserstoff, C₁-C₆-Alkyl oder C₂-C₆-Alkenyl ist;
R² 6-gliedriges Heteroaryl, das mit R⁷, R⁸ und R⁹ substituiert ist; 6-gliedriges Heteroaryl-N-oxid, das mit R⁷, R⁸ und R⁹ substituiert ist; 5-gliedriges Heteroaryl, das mit R¹⁰ und R¹¹ substituiert ist; Naphthyl; Fluorenyl; Diphenylmethyl; 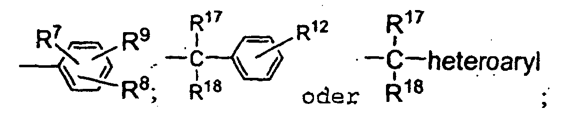 ist;
R³ R¹⁰-Phenyl, Pyridyl, Pyrimidyl, Pyrazinyl oder Thiazolyl ist;
R⁴ Wasserstoff, C₁-C₆-Alkyl, Fluor-C₁-C₆-alkyl, Cyclopropylmethyl, -CH₂CH₂OH, -CH₂CH₂-O-(C₁-C₆)alkyl, -CH₂C(O)-O-(C₁-C₆)alkyl, -CH₂C(O)NH₂, -CH₂C(O)-NH (C₁-C₆)alkyl oder -CH₂C(O)-N ((C₁-C₆)alkyl)₂ ist;
R⁵ und R¹¹ unabhängig ausgewählt sind aus der Gruppe bestehend aus Wasserstoff und (C₁-C₆)-Alkyl;
R^{6a} 1 bis 3 Substituenten ist, die unabhängig ausgewählt sind aus der Gruppe bestehend aus Wasserstoff, Halogen, -CF₃, CF₃O-, -CN, CF₃SO₂-, -NHCOCF₃, 5-gliedrigem Heteroaryl und 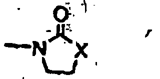 wobei X -O-, -NH- oder -N(CH₃)- ist;
R⁶ unabhängig ausgewählt ist aus der Gruppe bestehend aus R^{6a} und CH₃SO₂-;
R⁷ und R⁸ unabhängig ausgewählt sind aus der Gruppe bestehend aus (C₁-C₆)-Alkyl, Halogen, -NR²⁰R²¹, -OH, -CF₃, -OCH₃, -O-Acyl und -OCF₃;
R⁹ R⁷, Wasserstoff, Phenyl, -NO₂, -CN, -CH₂F, -CHF₂, -CHO, -CH=NOR²⁰, Pyridyl, Pyridyl N-oxid, Pyrimidinyl, Pyrazinyl, -N(R²⁰)CONR²¹R²², -NHCONH(Chlor-(C₁-C₆)alkyl), NHCONH((C₃-C₁₀)-cycloalkyl(C₁-C₆)alkyl), -NHCO(C₁-C₆)alkyl, -NHCOCF₃, -NHSO₂N((C₁-C₆)alkyl)₂, -NHSO₂(C₁-C₆)alkyl, -N(SO₂CF₃)₂, -NHCO₂(C₁-C₆)alkyl, C₃-C₁₀-Cycloalkyl, -SR²³, -SOR²³, -SO₂R²³, -SO₂NH(C₁-C₆-alkyl), -OSO₂(C₁-C₆)alkyl, -OSO₂CF₃, Hydroxy(C₁-C₆)alkyl, -CONR²⁰R²¹, -CON(CH₂CH₂-O-CH₃)₂, -OCONH(C₁-C₆)alkyl, -CO₂R²⁰, -Si(CH₃)₃ oder -B(OC(CH₃)₂)₂ ist;
R¹⁰ (C₁-C₆)-Alkyl, -NH₂ oder R¹²-Phenyl ist;
R¹² 1 bis 3 Substituenten ist, die unabhängig ausgewählt sind aus der Gruppe bestehend aus Wasserstoff, (C₁-C₆)-Alkyl, -CF₃, -CO₂R²⁰, -CN, (C₁-C₆)-Alkoxy und Halogen;
R¹³, R¹⁴, R¹⁵ und R¹⁶ unabhängig ausgewählt sind aus der Gruppe bestehend aus Wasserstoff und (C₁-C₆)-Alkyl;
R¹⁷ und R¹⁸ unabhängig ausgewählt sind aus der Gruppe bestehend aus Wasserstoff und C₁-C₆-Alkyl, oder R¹⁷ und R¹⁸ zusammen eine C₂-C₅-Akylengruppe sind und mit dem Kohlenstoff, an den sie gebunden sind, einen Spiroring mit 3 bis 6 Kohlenstoffatomen bilden;
R¹⁹ R⁶-Phenyl, R⁶-Heteroaryl, R⁶-Naphthyl, C₃-C₁₀-Cycloalkyl, (C₃-C₁₀) Cycloalkyl (C₁-C₆) alkyl oder (C₁-C₆)Alkoxy(C₁-C₆)alkyl ist;
R²⁰, R²¹ und R²² unabhängig ausgewählt sind aus der Gruppe bestehend aus H und C₁-C₆-Alkyl; und
R²³ C₁-C₆-Alkyl oder Phenyl ist; oder
(2): X^{a} -C(R¹³) (R¹⁹)-, -C(O)-, -O-, -NH-, -N((C₁-C₆)alkyl)- 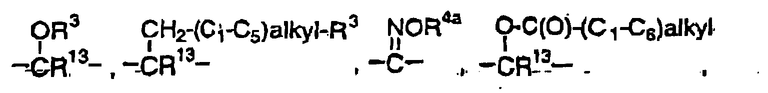 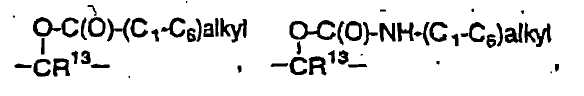 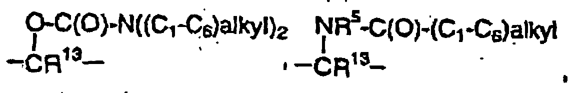 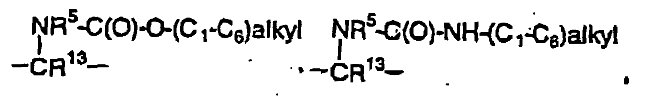 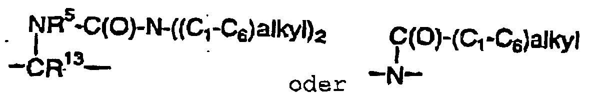 ist;
R^{a} R^{6b}-Phenyl, R^{6b}-Pyridyl oder R^{6b}-Thiophenyl ist;
R^{4a} Fluor-C₁-C₆-alkyl, Cyclopropylmethyl, -CH₂CH₂OH, -CH₂CH₂-O-(C₁-C₆)alkyl, -CH₂C(O) -O-(C₁-C₆)alkyl, -CH₂C(O)NH₂, -CH₂C(O)-NH(C₁-C₆)alkyl oder -CH₂C(O)-N((C₁-C₆)alkyl)₂ ist;
R^{6b} CH₃SO₂- ist; und
R¹, R², R³, R⁵, R¹⁴, R¹⁵, R¹⁶ und R¹⁹ wie in (1) definiert sind.

2. Verbindung nach Anspruch 1, bei der R^{a} 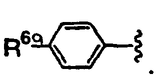 ist.

3. Verbindung nach Anspruch 1, Formel II(1), in der X^{a} -CHOR³, -C(R¹³) (R¹⁹)- oder -C(=NOR⁴)- ist.

4. Verbindung nach Anspruch 3, bei der R³ Pyridyl ist, R⁴ (C₁-C₆)-Alkyl ist oder R¹³ Wasserstoff ist und R¹⁹ R⁶-Phenyl ist.

5. Verbindung nach Anspruch 1, Formel II(2), in der X^{a} -CHOR³, -C(R¹³) (R¹⁹)- oder -C(=NOR⁴)- ist.

6. Verbindung nach Anspruch 5, bei der R³ Pyridyl ist, R^{4a} Cyclopropylmethyl oder Trifluorethyl ist, oder R¹³ Wasserstoff ist und R¹⁹ R⁶-Phenyl ist.

7. Verbindung nach Anspruch 1, bei der R² 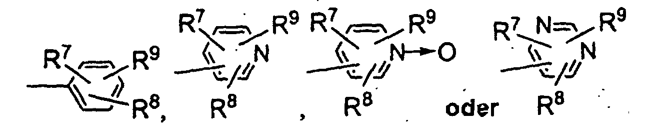 ist.

8. Verbindung nach Anspruch 7, bei der R² ausgewählt ist aus der Gruppe bestehend aus 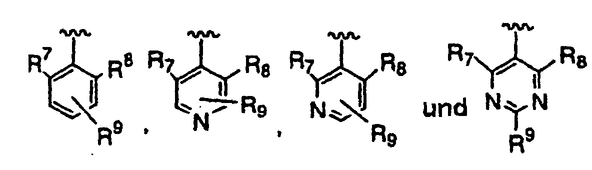 worin R⁷ und R⁸ ausgewählt sind aus der Gruppe bestehend aus (C₁-C₆)-Alkyl, Halogen und -NH₂, und R⁹ Wasserstoff ist.

9. Verbindung ausgewählt aus der Gruppe bestehend aus jenen, die durch die folgende Formel wiedergegeben werden: 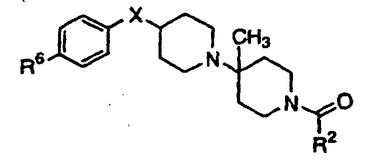 worin R⁶, X und R² wie in der folgenden Tabelle definiert sind:

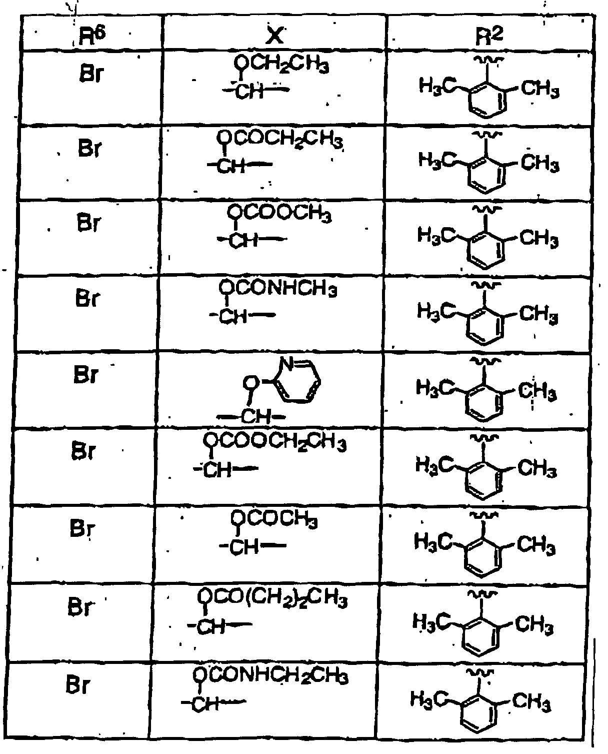

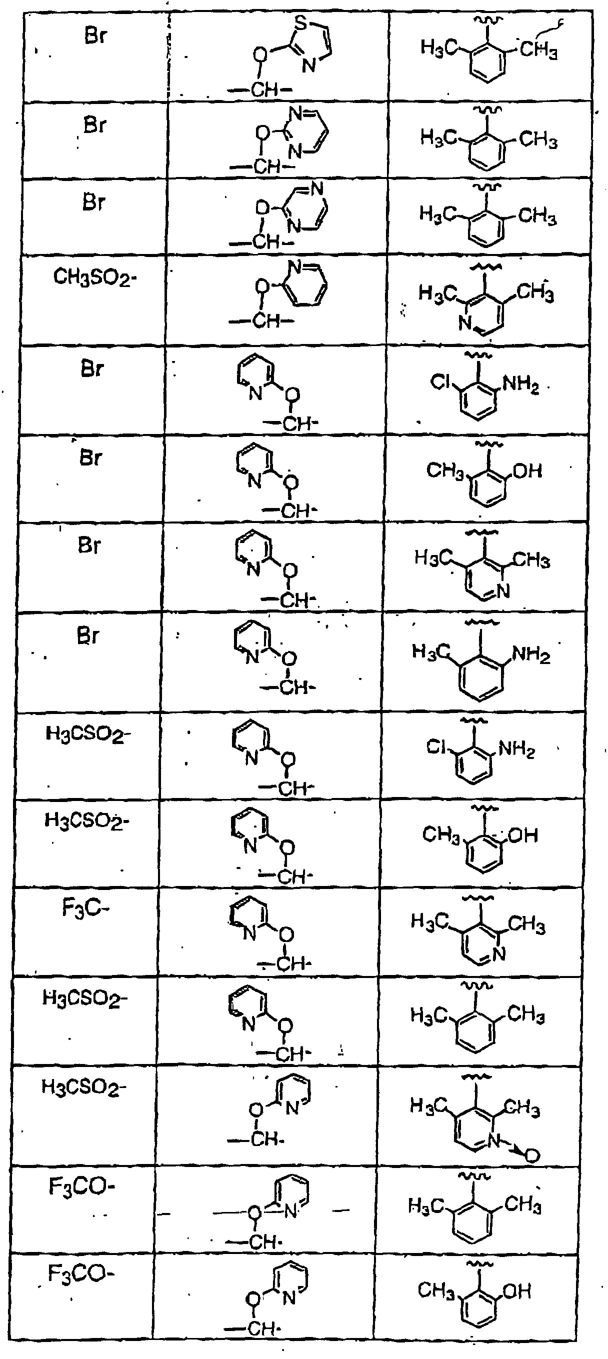

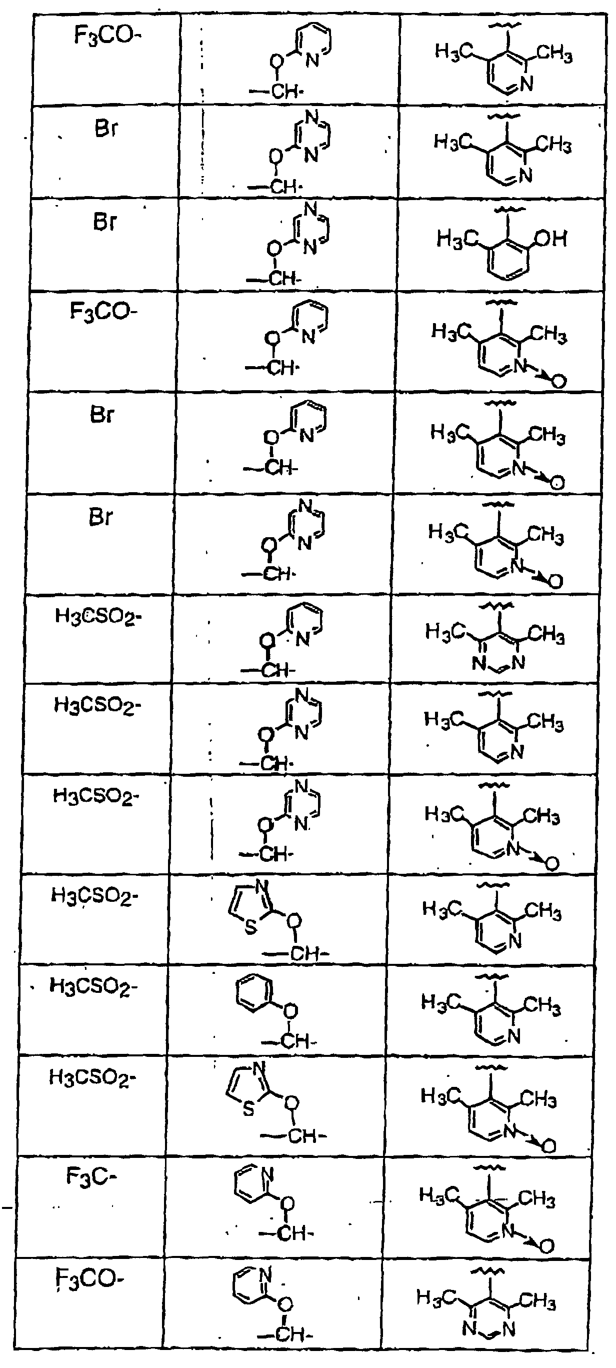

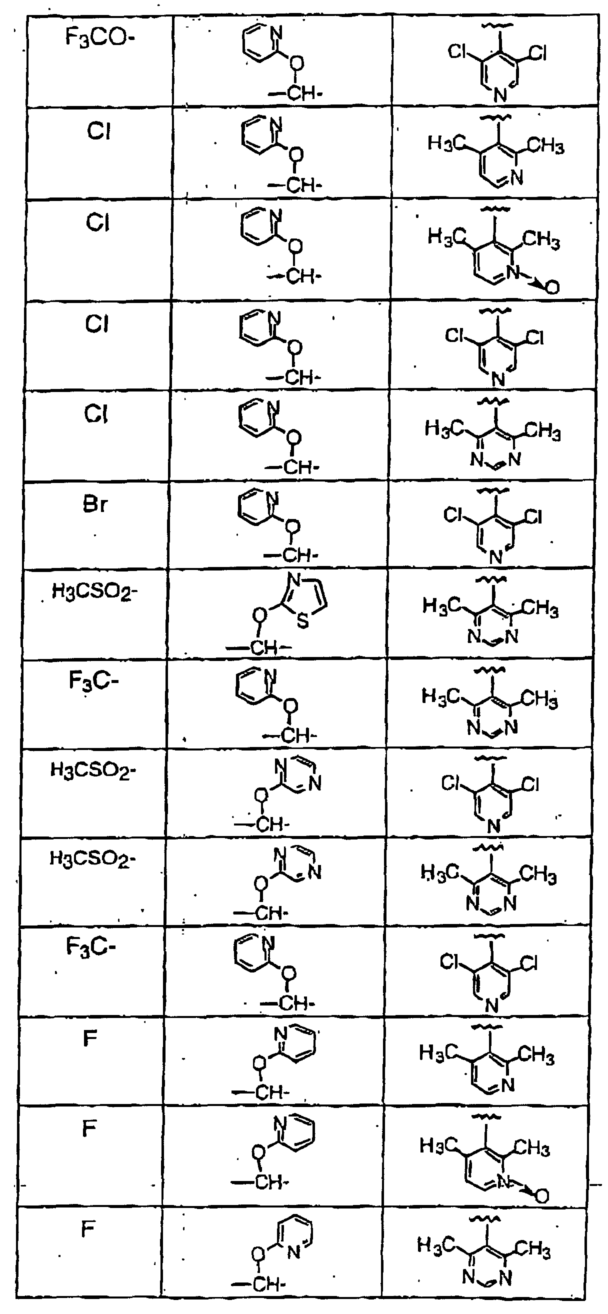

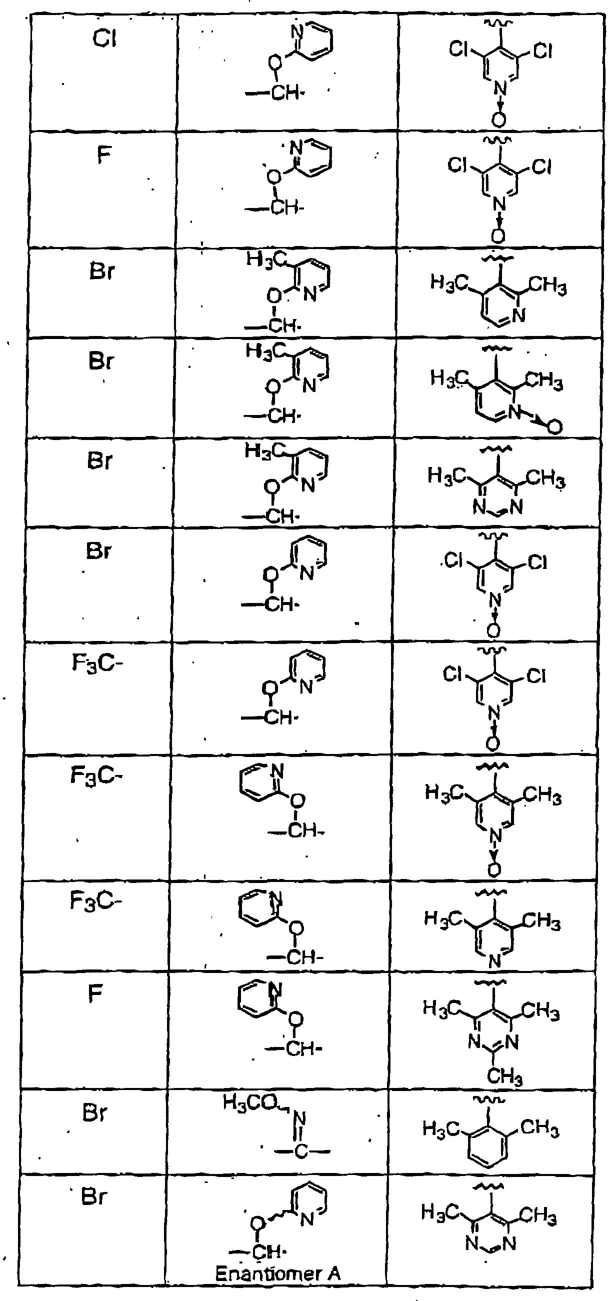

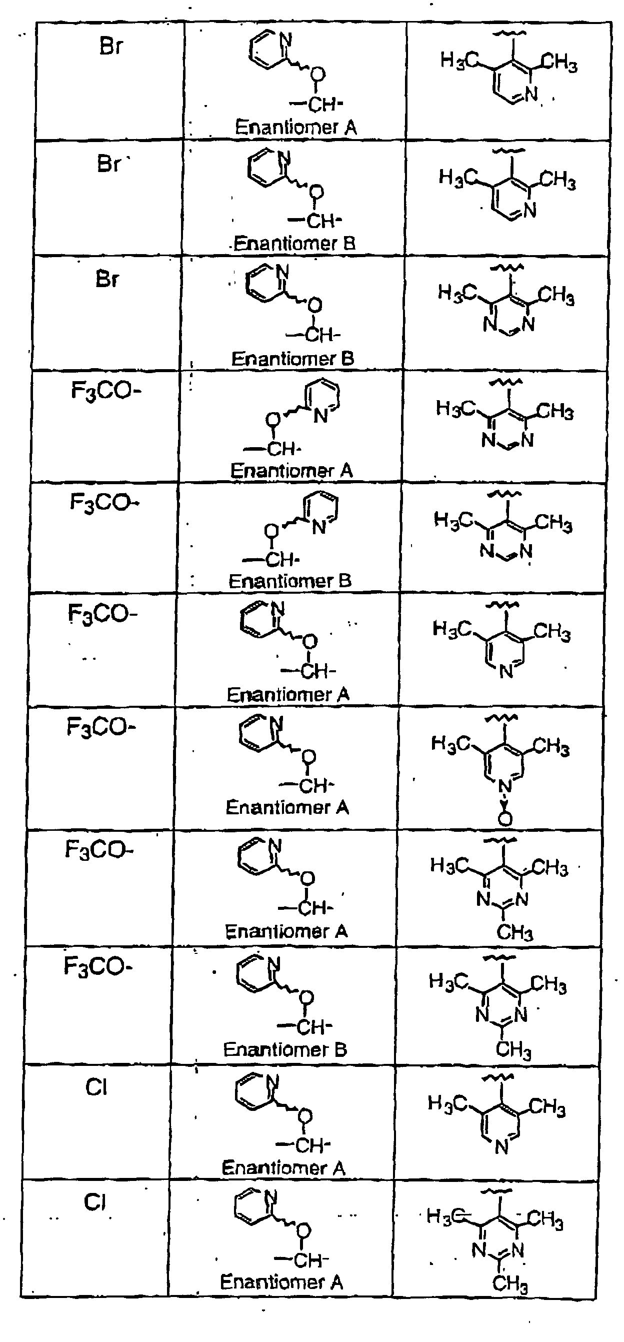

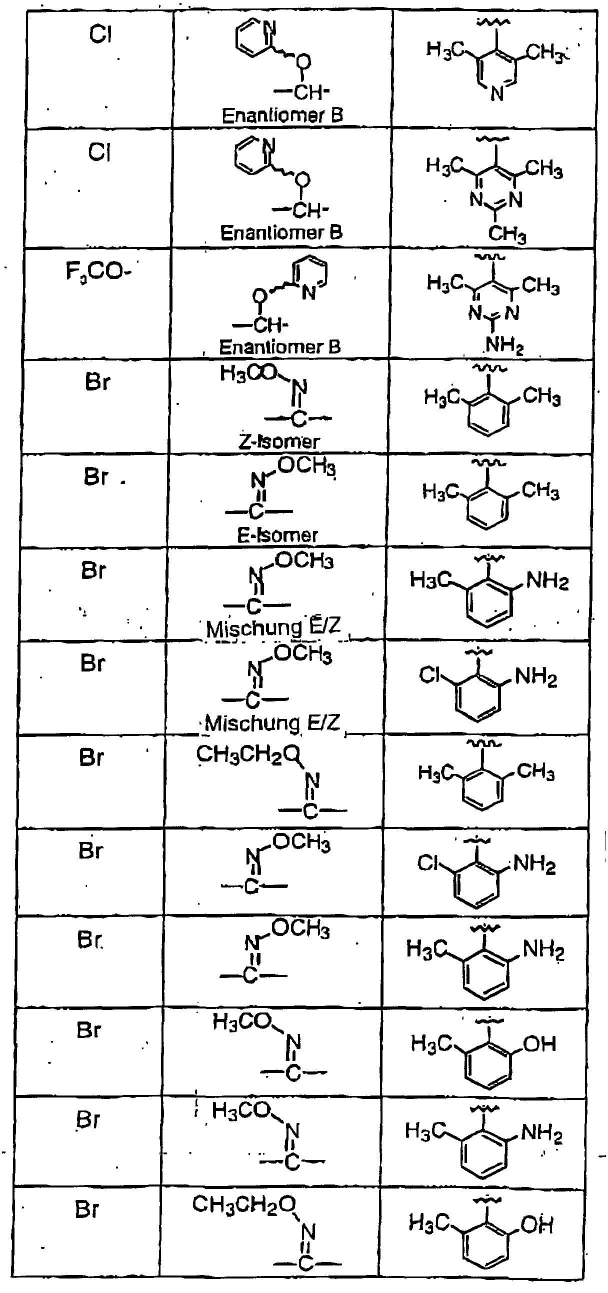

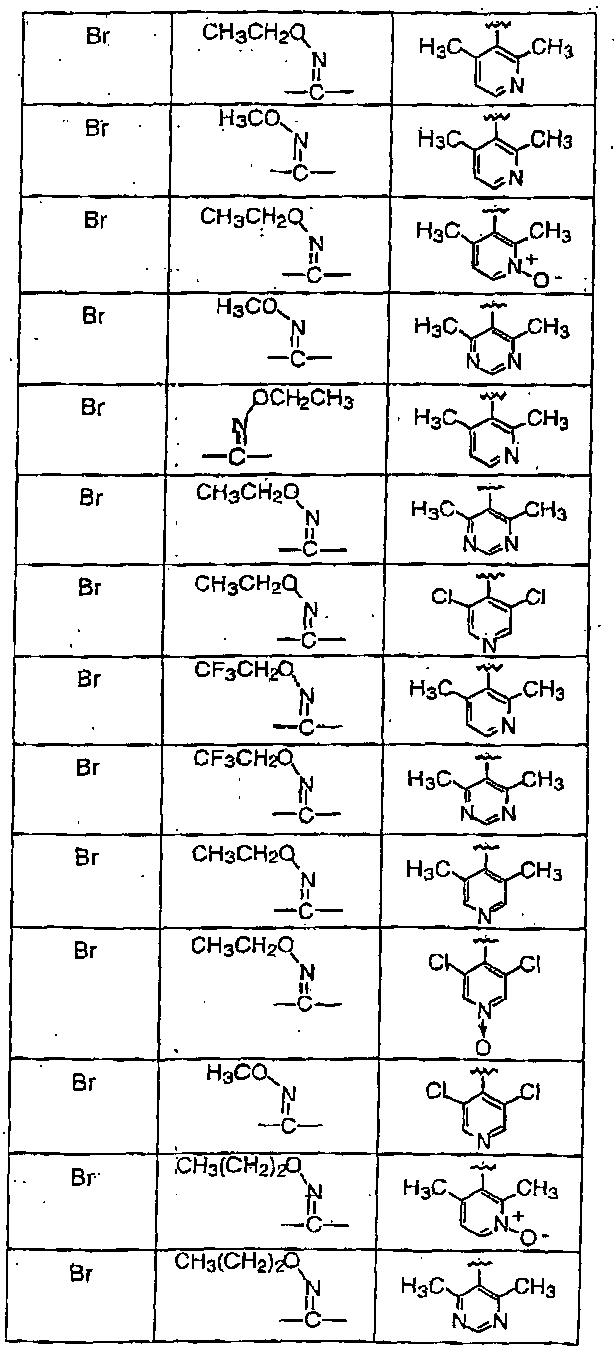

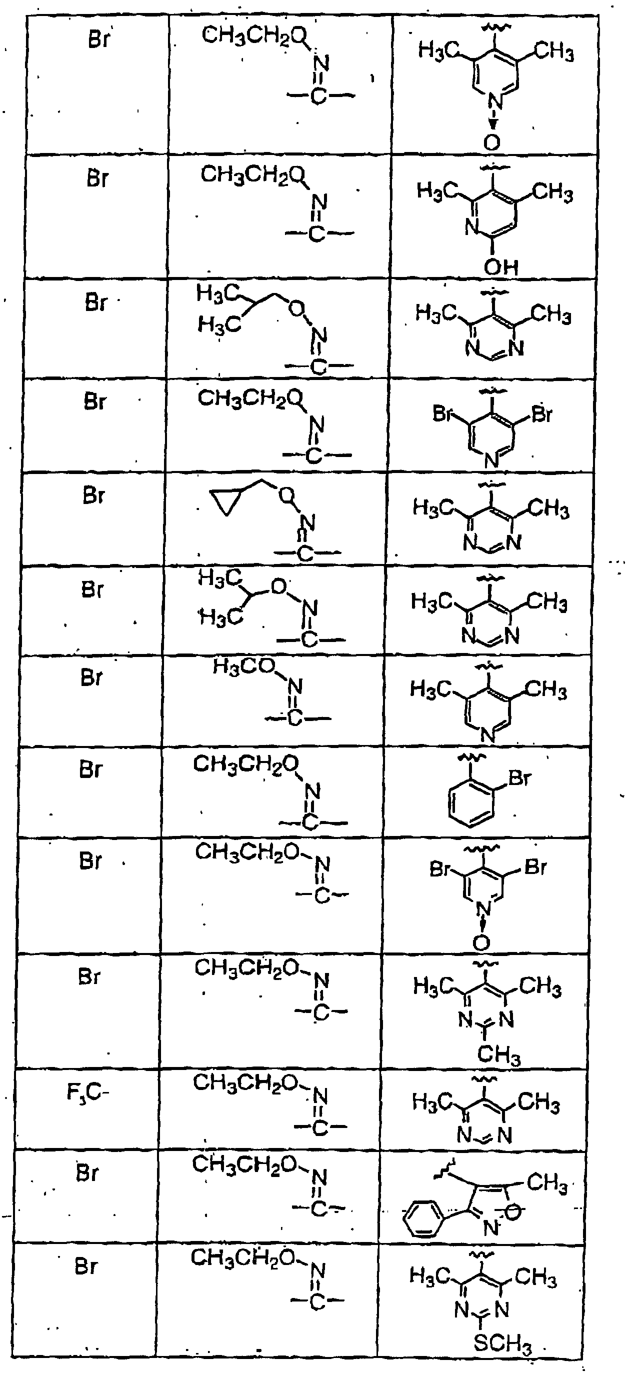

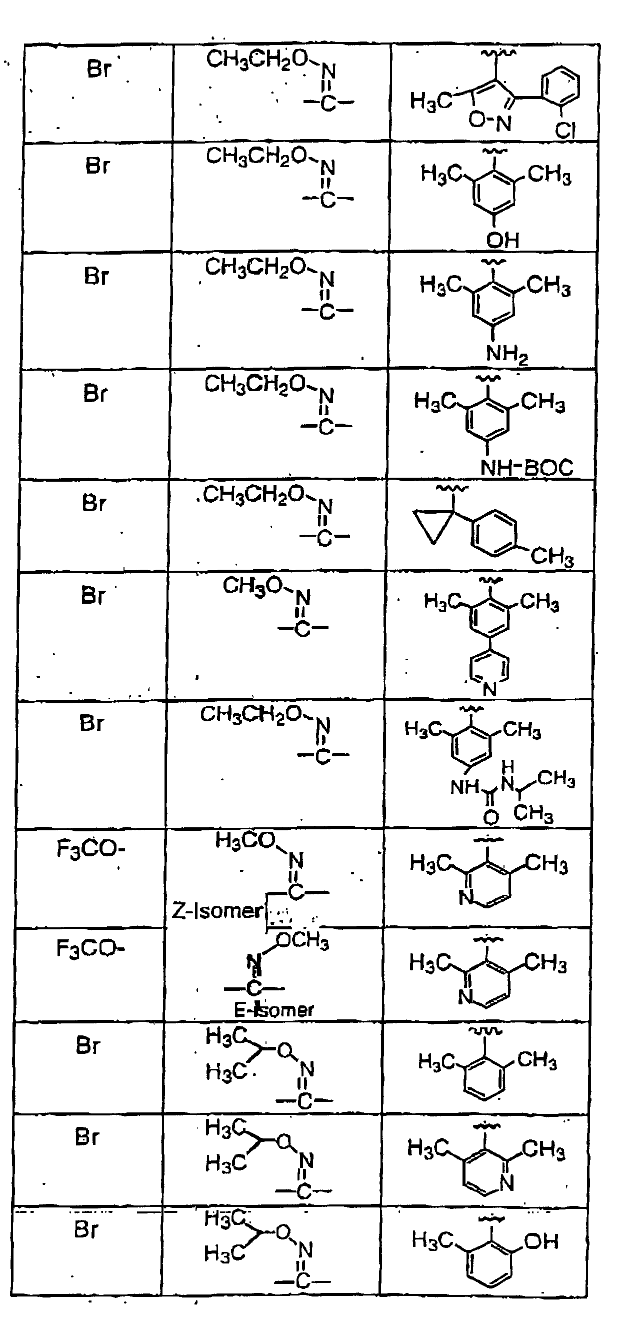

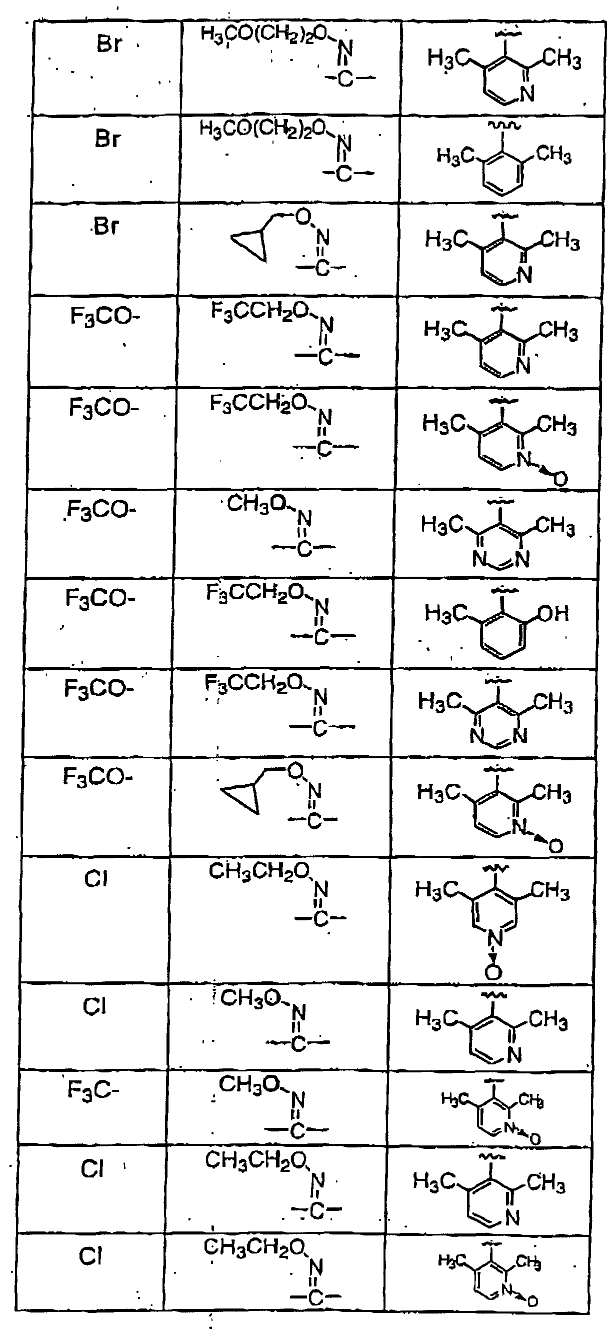

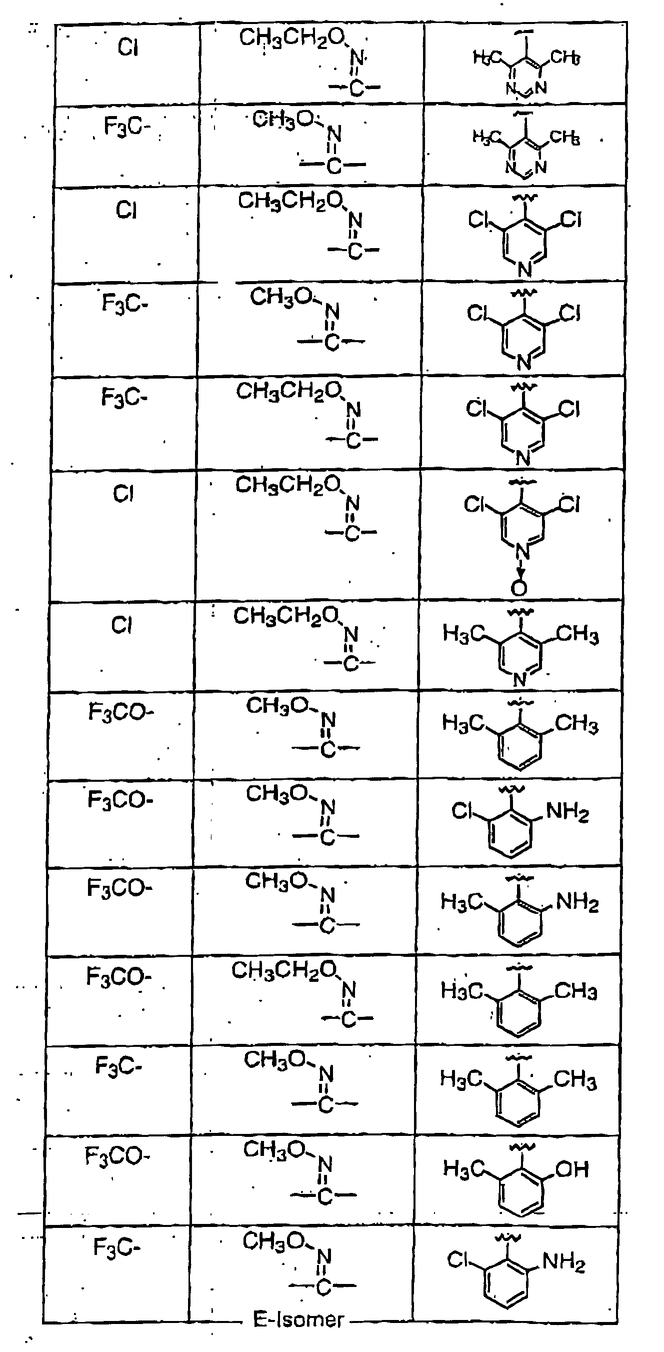

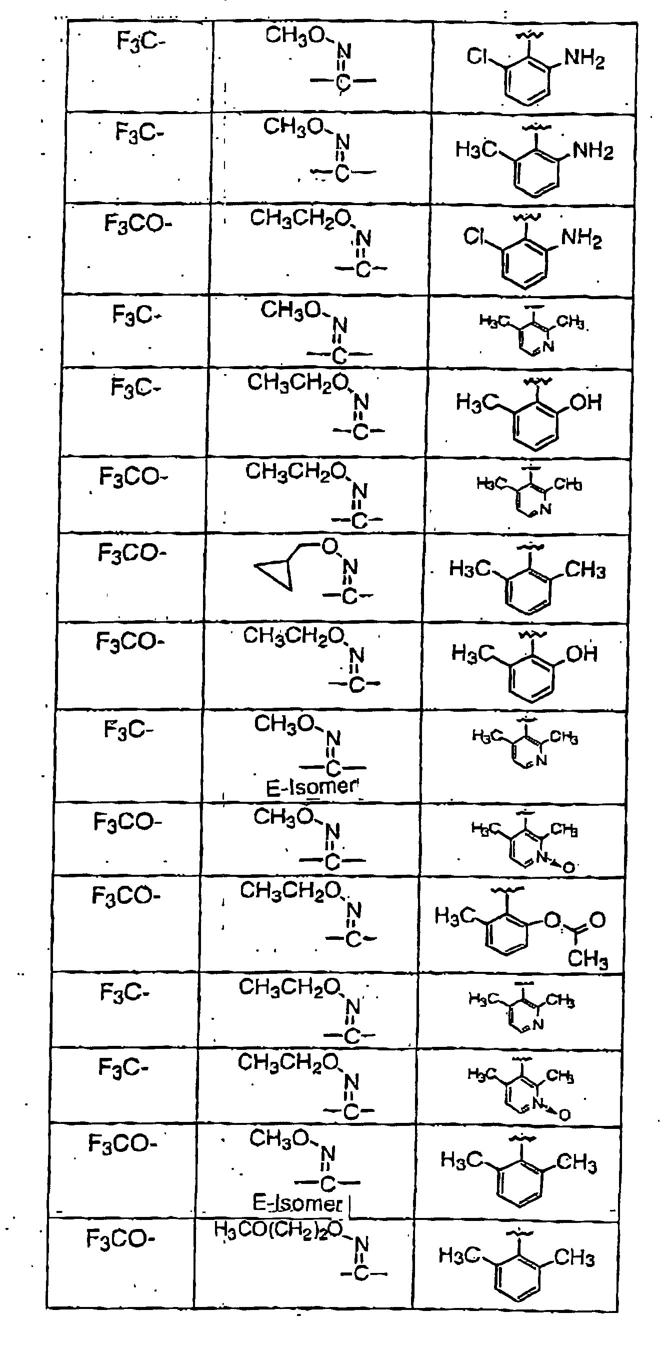

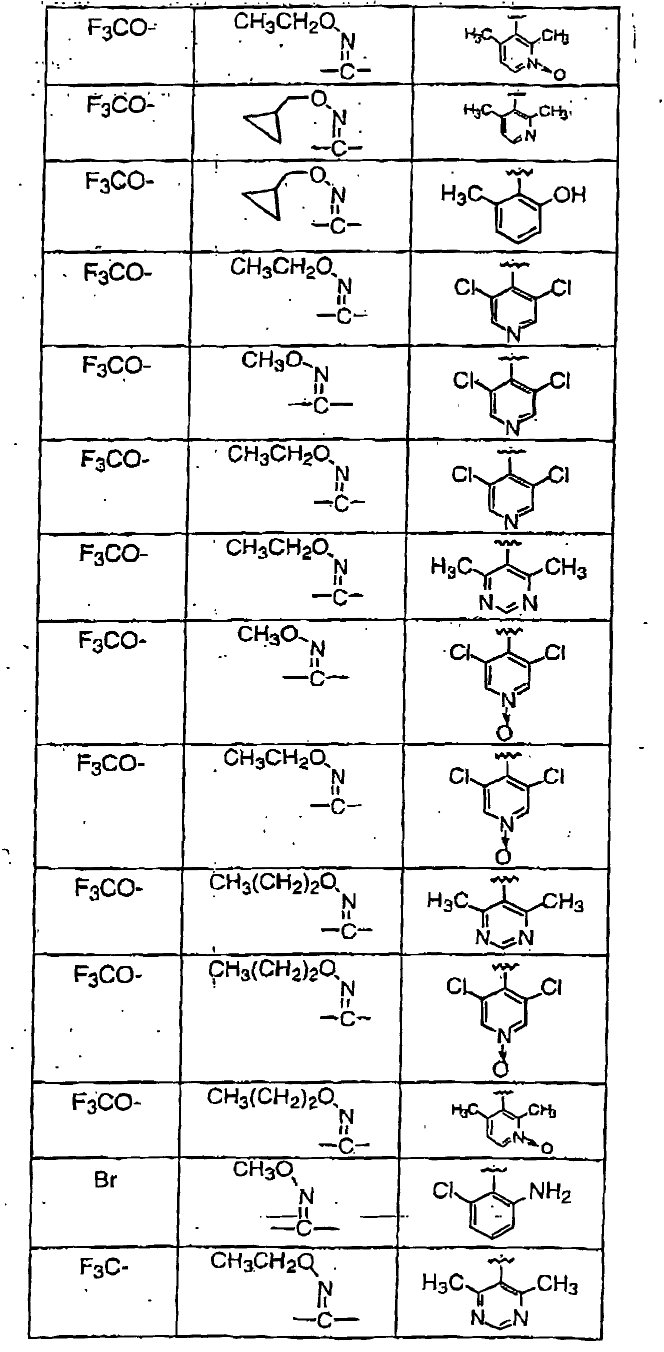

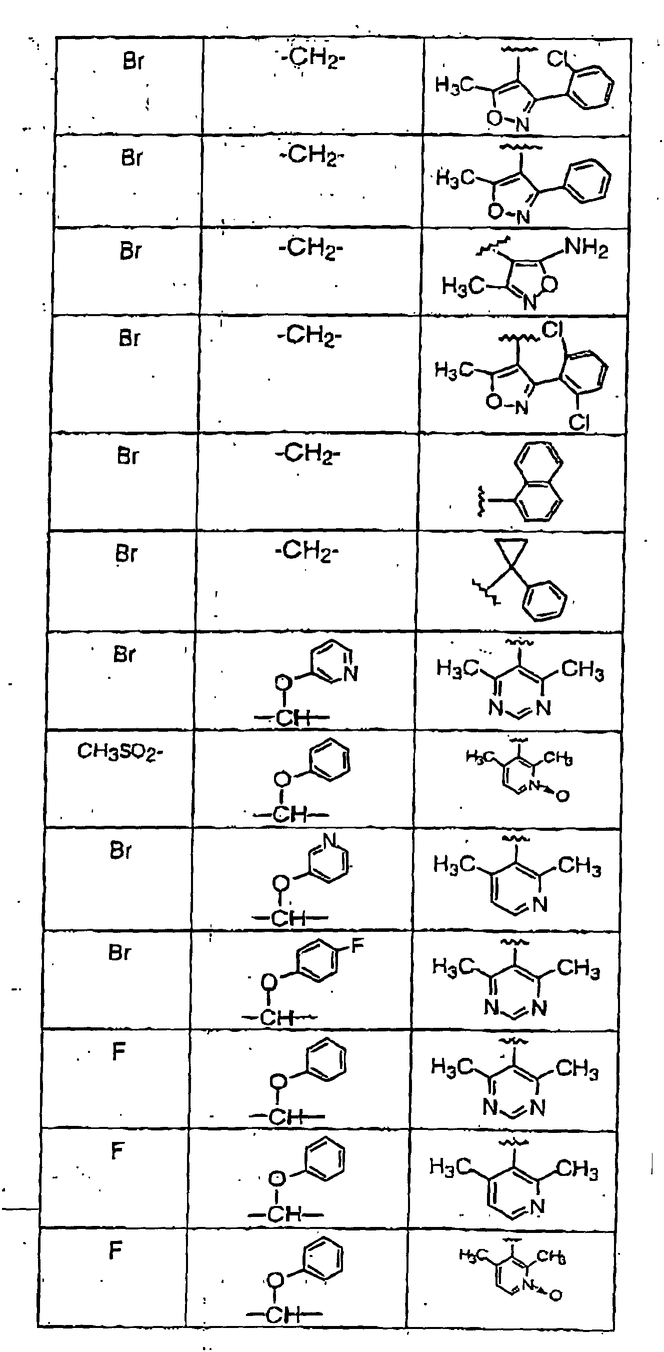

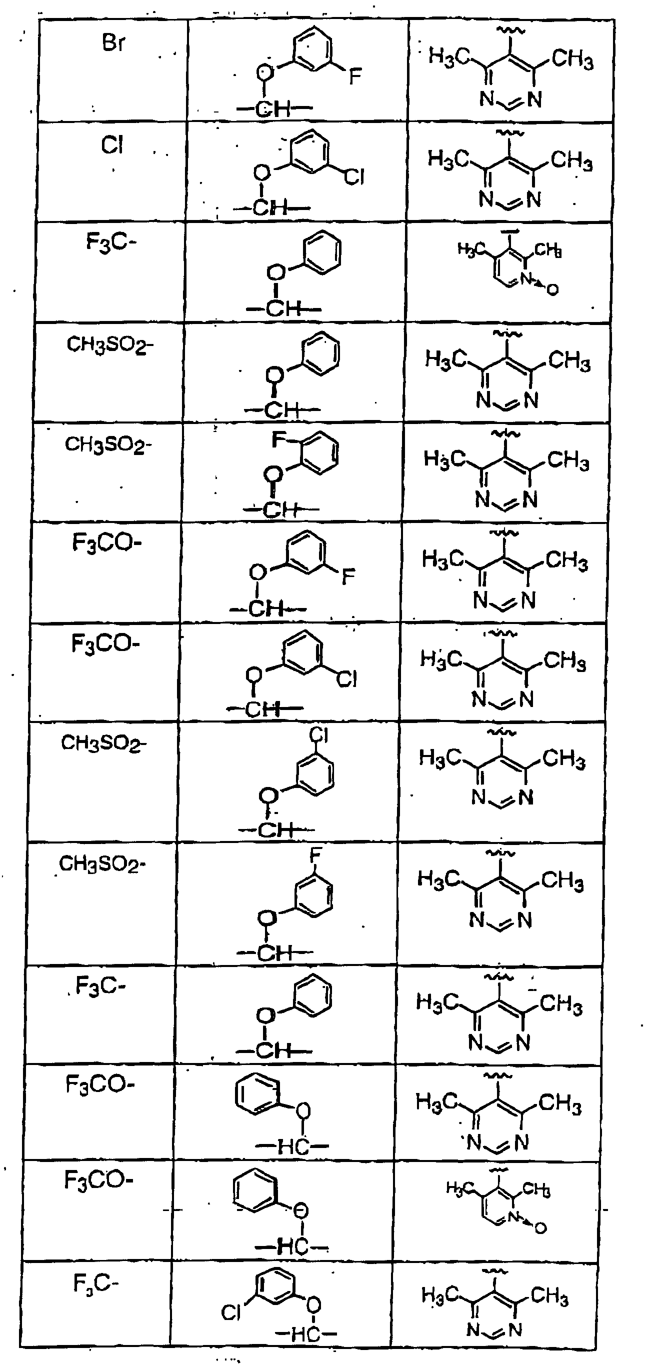

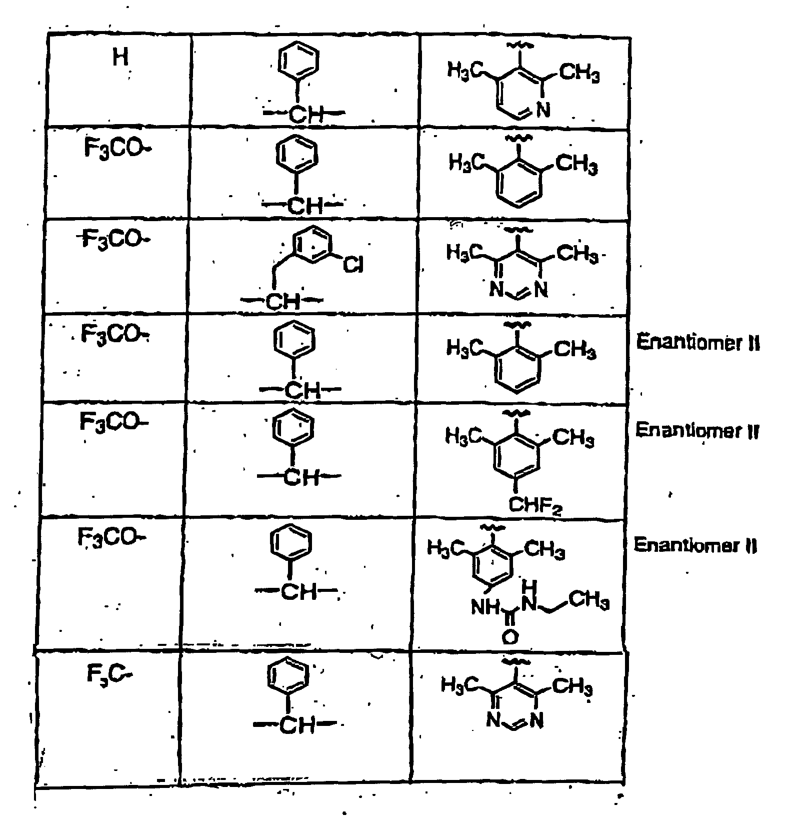

10. Verbindung nach Anspruch 9 ausgewählt aus der Gruppe bestehend aus

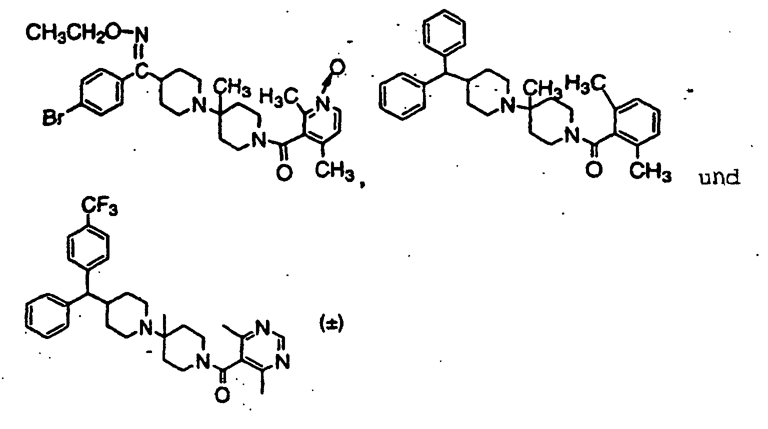

11. Verbindung mit der Formel

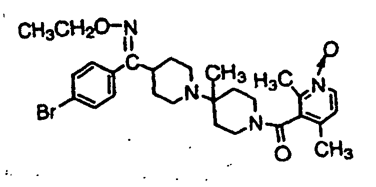

12. Pharmazeutische Zusammensetzung zur Behandlung von HIV-Virus, Abstoßung von soliden Organtransplantaten, Graft-versus-Host-Erkrankung, Arthritis, rheumatoider Arthritis, entzündlicher Darmerkrankung, atopischer Dermatitis, Psoriasis, Asthma, Allergien oder multipler Sklerose, die eine wirksame Menge eines CCR5-Antagonisten gemäß einem der vorhergehenden Ansprüche in Kombination mit einem pharmazeutisch annehmbaren Träger enthält.

13. Verwendung einer Verbindung nach einem der Ansprüche 1 bis 11 zur Herstellung eines Medikaments zur Behandlung von HIV-Virus, Abstoßung von soliden Organtransplantaten, Graft-versus-Host-Erkrankung, Arthritis, rheumatoider Arthritis, entzündlicher Darmerkrankung, atopischer Dermatitis, Psoriasis, Asthma, Allergien oder multipler Sklerose.

14. Verwendung einer Verbindung nach einem der Ansprüche 1 bis 11 zur Herstellung eines Medikaments zur kombinierten Verwendung mit einem oder mehreren Antivirus- oder anderen Mitteln, die zur Behandlung von HIV brauchbar sind.

15. Verwendung nach Anspruch 14, bei der das Antivirusmittel ausgewählt ist aus der Gruppe bestehend aus Nukleosid-reverse-Transkriptase-Inhibitoren, Nicht-Nukleosid-reverse-Transkriptase-Inhibitoren und Protease-Inhibitoren.

16. Verwendung einer Verbindung nach einem der Ansprüche 1 bis 11 zur Herstellung eines Medikaments zur kombinierten Verwendung mit einem oder mehreren Mitteln zur Behandlung von Abstoßung solider Organtransplantate, Graft-versus-Host-Erkrankung, entzündlicher Darmerkrankung, rheumatoider Arthritis oder multipler Sklerose.

17. Verwendung eines CCRS-Antagonisten der Formel I zur Herstellung eines Medikaments zur Behandlung von HIV-Virus, Abstoßung von soliden Organtransplantaten, Graft-versus Host-Erkrankung, Arthritis, rheumatoider Arthritis, entzündlicher Darmerkrankung, atopischer Dermatitis, Psoriasis, Asthma, Allergien oder multipler Sklerose, wobei der CCR5-Antagonist durch die Strukturformel I wiedergegeben wird: 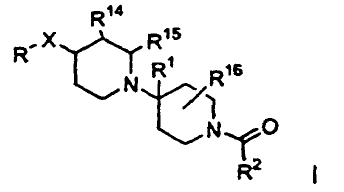 oder eines pharmazeutisch annehmbaren Salzes davon; worin X C(R¹³)₂-, -C(R¹³)(R¹⁹)-, -C(O)-, -O-, -NH-, -N((C₁-C₆)alkyl)-, 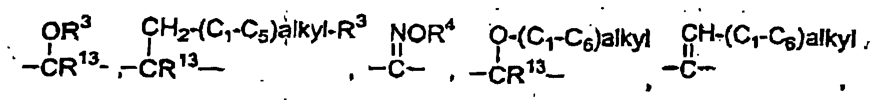 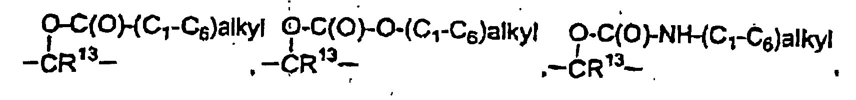 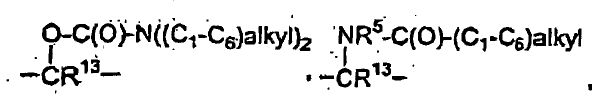 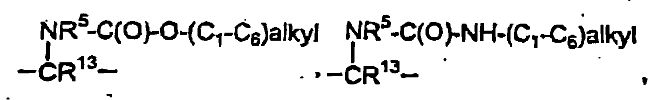 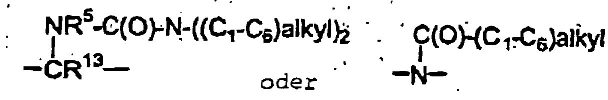 ist;
R R⁶-Phenyl, R⁶-Pyridyl, R⁶-Thiophenyl oder R⁶-Naphthyl ist;
R¹ Wasserstoff, C₁-C₆-Alkyl oder C₂-C₆-Alkenyl ist;
R² 6-gliedriges Heteroaryl, das mit R⁷, R⁸ und R⁹ substituiert ist; 6-gliedriges Heteroaryl-N-oxid, das mit R⁷, R⁸ und R⁹ substituiert ist; 5-gliedriges Heteroaryl, das mit R¹⁰ und R¹¹ substituiert ist; Naphthyl; Fluorenyl; Diphenylmethyl; 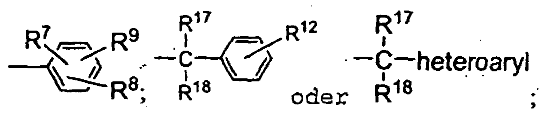 ist;
R³ R⁶-Phenyl, R⁶-Heteroaryl oder R⁶-Naphthyl ist;
R⁴ Wasserstoff, C₁-C₆-Alkyl, Fluor-C₁-C₆-alkyl, Cyclopropylmethyl, -CH₂CH₂OH, -CH₂CH₂-O-(C₁-C₆) alkyl, -CH₂C(O)-O-(C₁-C₆)alkyl, -CH₂C(O)NH₂, -CH₂C(O)-NH(C₁-C₆)alkyl oder -CH₂C(O)-N((C₁-C₆)alkyl)₂ ist;
R⁵ und R¹¹ unabhängig ausgewählt sind aus der Gruppe bestehend aus Wasserstoff und (C₁-C₆)-Alkyl;
R⁶ 1 bis 3 Substituenten ist, die unabhängig ausgewählt sind aus der Gruppe bestehend aus Wasserstoff, Halogen, C₁-C₆-Alkyl, C₁-C₆-Alkoxy, -CF₃, CF₃O-, CH₃C(O)-, -CN, CH₃SO₂-, CF₃SO₂-, R¹⁴-Phenyl, R¹⁴-Benzyl, CH₃C(=NOCH₃), CH₃C(=NOCH₂CH₃), 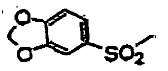 -NH₂, -NHCOCF₃, -NHCONH(C₁-C₆-alkyl), -NHCO(C₁-C₆-alkyl), -NHSO₂(C₁-C₆-alkyl), 5-gliedrigem Heteroaryl und 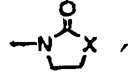 wobei X -O-, -NH- oder -N(CH₃)- ist;
R⁷ und R⁸ unabhängig ausgewählt sind aus der Gruppe bestehend aus (C₁-C₆)-Alkyl, Halogen, -NR²⁰R²¹, -OH, -CF₃, -OCH₃, -O-Acyl und -OCF₃;
R⁹ R⁷, Wasserstoff, Phenyl, -NO₂, -CN, -CH₂F, -CHF₂, -CHO, -CH=NOR²⁰, Pyridyl, Pyridyl-N-oxid, Pyrimidinyl, Pyrazinyl, -N(R²⁰)CONR²¹R²², -NHCONH(chlor-(C₁-C₆)alkyl), NHCONH((C₃-C₁₀)-cycloalkyl(C₁-C₆)alkyl), -NHCO(C₁-C₆) alkyl, -NHCOCF₃, -NHSO₂N((C₁-C₆)alkyl)₂, -NHSO₂(C₁-C₆) alkyl, -N(SO₂CF₃)₂, -NHCO₂(C₁-C₆)alkyl, C₃-C₁₀-Cycloalkyl, -SR²³, -SOR²³, -SO₂R²³, -SO₂NH(C₁-C₆-alkyl), -OSO₂(C₁-C₆)alkyl, -OSO₂CF₃, Hydroxy(C₁-C₆)alkyl, -CONR²⁰R²¹, -CON(CH₂CH₂-O-CH₃)₂, -OCONH(C₁-C₆) alkyl, -CO₂R²⁰, -Si(CH₃)₃ oder -B(OC(CH₃)₂)₂ ist;
R¹⁰ (C₁-C₆)-Alkyl, -NH₂ oder R¹²-Phenyl ist;
R¹² 1 bis 3 Substituenten ist, die unabhängig ausgewählt sind aus der Gruppe bestehend aus Wasserstoff, (C₁-C₆)-Alkyl, -CF₃, -CO₂R²⁰, -CN, (C₁-C₆)-Alkoxy und Halogen;
R¹³, R¹⁴, R¹⁵ und R¹⁶ unabhängig ausgewählt sind aus der Gruppe bestehend aus Wasserstoff und (C₁-C₆)-Alkyl;
R¹⁷ und R¹⁸ unabhängig ausgewählt sind aus der Gruppe bestehend aus Wasserstoff und C₁-C₆-Alkyl, oder R¹⁷ und R¹⁸ zusammen eine C₂-C₅-Akylengruppe sind und mit dem Kohlenstoff, an den sie gebunden sind, einen Spiroring mit 3 bis 6 Kohlenstoffatomen bilden;
R¹⁹ R⁶-Phenyl, R⁶-Heteroaryl, R⁶-Naphthyl, C₃-C₁₀-Cycloalkyl, (C₃-C₁₀)-Cycloalkyl(C₁-C₆)alkyl oder (C₁-C₆)-Alkoxy-(C₁-C₆)alkyl ist;
R²⁰, R²¹ und R²² unabhängig ausgewählt sind aus der Gruppe bestehend aus H und C₁-C₆-Alkyl; und
R²³ C₁-C₆-Alkyl oder Phenyl ist.

18. Verwendung nach Anspruch 17, bei der R 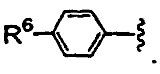 ist.

19. Verwendung nach Anspruch 17, in der X -CHOR³, -C(R¹³)(R¹⁹) - oder -C(=NOR⁴)- ist.

20. Verwendung nach Anspruch 19, bei der R³ Pyridyl ist, R⁴ (C₁-C₆)-Alkyl ist oder R¹³ Wasserstoff ist und R¹⁹ R⁶-Phenyl ist.

21. Verwendung nach Anspruch 17, bei der R² 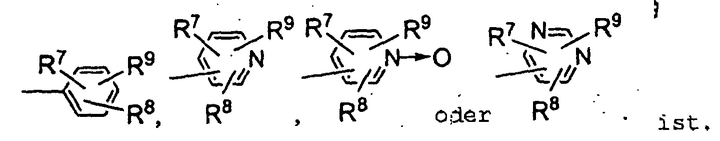 ist.

22. Verwendung nach Anspruch 21, bei der R² ausgewählt ist aus der Gruppe bestehend aus 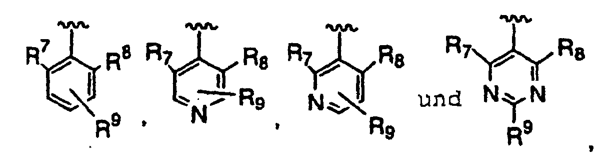 worin R⁷ und R⁸ ausgewählt sind aus der Gruppe bestehend aus (C₁-C₆)-Alkyl, Halogen und -NH₂, und R⁹ Wasserstoff ist.

23. Verwendung nach Anspruch 17 zur Behandlung des HIV-Virus, wobei ferner ein oder mehrere Antivirus- oder andere Mittel vorhanden sind, die zur Behandlung des HIV-Virus brauchbar sind.

24. Verwendung nach Anspruch 23, bei der das Antivirusmittel ausgewählt ist aus der Gruppe bestehend aus Nukleosid-reverse-Transkriptase-Inhibitoren, Nicht-Nukleosid-reverse-Transkriptase-Inhibitoren und Protease-Inhibitoren.

25. Verwendung nach Anspruch 17 zur Behandlung von Abstoßung von soliden Organtransplantaten, Graft-versus-Host-Erkrankung, entzündlicher Darmerkrankung, rheumatoider Arthritis oder multipler Sklerose, wobei ferner ein oder mehrere andere Mittel vorhanden sind, die zur Behandlung dieser Erkrankungen brauchbar sind.

26. Kit, der in separaten Behältern in einer Einzelpackung pharmazeutische Zusammensetzungen zur kombinierten Verwendung zur Behandlung des HIV-Virus enthält, der in einem Behälter eine pharmazeutische Zusammensetzung aufweist, die eine wirksame Menge eines CCR5-Antagonisten gemäß Anspruch 17 in einem pharmazeutisch annehmbaren Träger enthält, und in separaten Behältern eine oder mehrere pharmazeutische Zusammensetzung(en) aufweist, die eine wirksame Menge eines Antivirus- oder anderen Mittels, das zur Behandlung des HIV-Virus brauchbar ist, in einem pharmazeutisch annehmbaren Träger enthält.

27. Pharmazeutische Zusammensetzung in Form einer Creme zur topischen Aufbringung, die eine Verbindung der Formel II gemäß einem der Ansprüche 1 bis 11 und einen pharmazeutisch annehmbaren Träger enthält.

28. Pharmazeutische Zusammensetzung in Form einer Creme zur topischen Aufbringung, die eine Verbindung der Formel 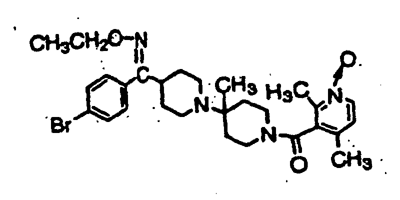 und einen pharmazeutisch annehmbaren Träger enthält.

## Revendications

1. Composé représenté par la formule développée II 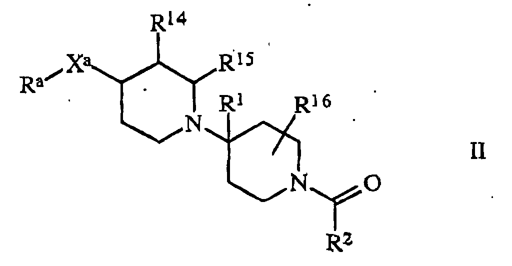 dans laquelle :
(1) X^{a} représente un groupe 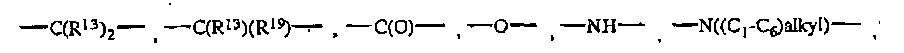 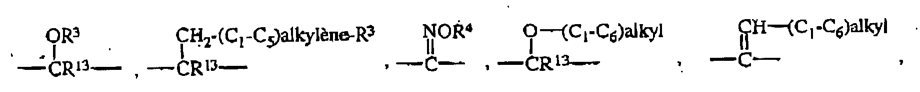 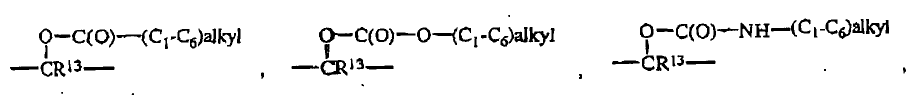 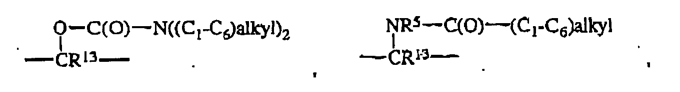 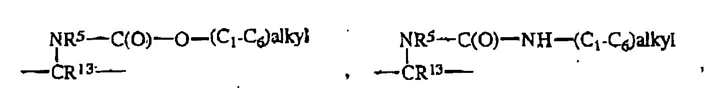 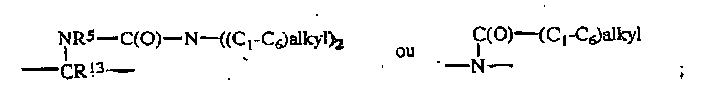 R^{a} représente un groupe R^{6a}-phényle, R^{6a}-pyridyle, R^{6a}-thiophényle ou R⁶-naphtyle,
R¹ représente un atome d'hydrogène, un groupe alkyle en C₁ à C₆ ou un groupe alcényle en C₂ à C₆,
R² représente un groupe hétéroaryle à 6 chaînons, substitué par R⁷, R⁸ et R⁹, un groupe hétéroaryle-N-oxyde à 6 chaînons, substitué par R⁷, R⁸ et R⁹, un groupe hétéroaryle à 5 chaînons, substitué par R¹⁰ et R¹¹, un groupe naphtyle, un groupe fluorényle, un groupe diphénylméthyle, ou un groupe 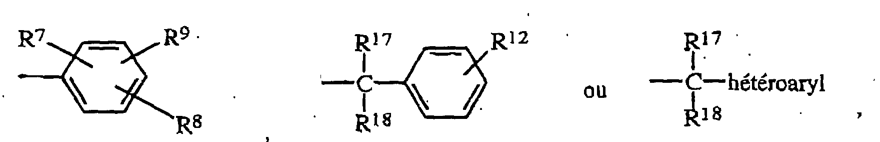 R³ représente un groupe R¹⁰-phényle, pyridyle, pyrimidyle, pyrazinyle, ou thiazolyle,
R⁴ représente un atome d'hydrogène ou un groupe alkyle en C₁ à C₆, fluoroalkyle en C₁ à C₆, cyclopropylméthyle, -CH₂CH₂OH, -CH₂CH₂-O-alkyl(C₁ à C₆), -CH₂C(O)-O-alkyl(C₁ à C₆), -CH₂C(O)NH₂, -CH₂C(O)-NHalkyl(C₁ à C₆) ou -CH₂C(O)-N(alkyl en C₁ à C₆)₂,
R⁵ et R¹¹ représentent chacun indépendamment un atome d'hydrogène ou un groupe alkyle en C₁ à C₆,
R^{6a} représente un à 3 substituants choisis indépendamment parmi les atomes d'hydrogène et d'halogène, et les groupes -CF₃, CF₃O-, -CN, CF₃SO₂-, -NHCOCF₃, hétéroaryle à 5 chaînons et 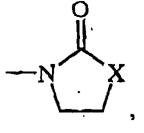 où X représente -O-, -NH- ou -N(CH₃)-,
R⁶ représente R^{6a} ou CH₃SO₂-,
R⁷ et R⁸ représentent chacun indépendamment un atome d'halogène ou un groupe alkyle en C₁ à C₆, -NR²⁰R²¹, -OH, -CF₃, -OCH₃, -O-acyl ou -OCF₃,
R⁹ représente R⁷, un atome d'hydrogène, ou un groupe phényle, -NO₂, -CN, -CH₂F, -CHF₂, -CHO, -CH=NOR²⁰, pyridyle, pyridyle-N-oxyde, pyrimidinyle, pyrazinyle, -N(R²⁰)CONR²¹R²², -NHCONH(chloroalkyl en C₁ à C₆), -NHCONH(cycloalkyl(C₃ à C₁₀)-alkyl(C₁ à C₆)), -NHCO(alkyl en C₁ à C₆), -NHCOCF₃, -NHSO₂N(alkyl en C₁ à C₆)₂, -NHSO₂(alkyl en C₁ à C₆), -N(SO₂CF₃)₂, -NHCO₂(alkyl en C₁ à C₆), cycloalkyle en C₃ à C₁₀, -SR²³, -SOR²³, -SO₂R²³, -SO₂NH(alkyl en C₁ à C₆), -OSO₂(alkyl en C₁ à C₆), -OSO₂CF₃, hydroxyalkyle en C₁ à C₆, -CONR²⁰R²¹, -CON(CH₂CH₂O-CH₃)₂, -OCONH(alkyl en C₁ à C₆), -CO₂R²⁰, -Si(CH₃)₃ ou -B(OC(CH₃)₃)₂,
R¹⁰ représente un groupe alkyle en C₁ à C₆, -NH₂ ou R¹²-phényle,
R¹² représente 1 à 3 substituants choisis indépendamment parmi les atomes d'hydrogène et d'halogène, et les groupes alkyle en C₁ à C₆, -CF₃, -CO₂R²⁰, -CN et alcoxy en C₁ à C₆,
R¹³, R¹⁴, R¹⁵ et R¹⁶ représentent chacun indépendamment un atome d'hydrogène ou un groupe alkyle en C₁ à C₆,
R¹⁷ et R¹⁸ représentent chacun indépendamment un atome d'hydrogène ou un groupe alkyle en C₁ à C₆, ou bien R¹⁷ et R¹⁸ forment ensemble un groupe alkylène en C₂ à C₅ et forment avec l'atome de carbone auquel ils sont liés un cycle spiro ayant 3 à 6 atomes de carbone,
R¹⁹ représente un groupe R⁶-phényle, R⁶-hétéroaryle, R⁶-naphtyle, cycloalkyle en C₃ à C₁₀, cycloalkyl(C₃ à C₁₀)alkyle(C₁ à C₆) ou alcoxy(C₁ à C₆)alkyle(C₁ à C₆),
R²⁰, R²¹ et R²² représentent chacun indépendamment un atome d'hydrogène ou un groupe alkyle en C₁ à C₆, et
R²³ représente un groupe alkyle en C₁ à C₆ ou un groupe phényle,
ou bien
(2)
X^{a} représente un groupe 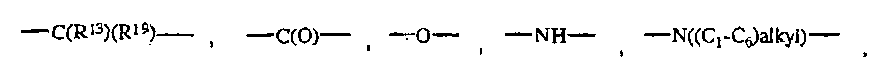 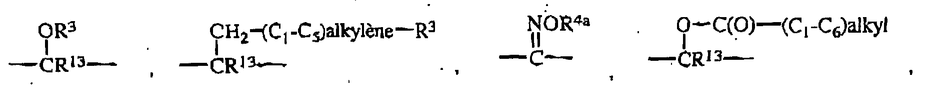 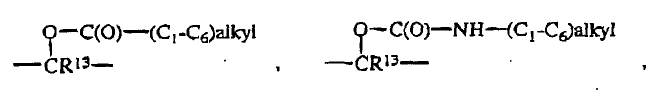 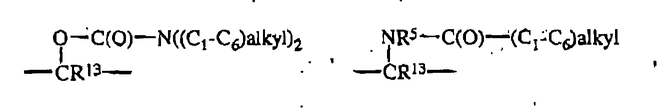 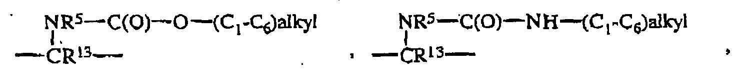 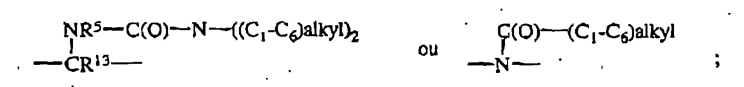 R^{a} représente un groupe R^{6b}-phényle, R^{6b}-pyridyle ou R^{6b}-thiophényle,
R^{4a} représente un groupe fluoroalkyle en C₁ à C₆, cyclopropylméthyle, -CH₂CH₂OH, -CH₂CH₂-O-alkyl(C₁ à C₆), -CH₂C(O)-O-alkyl(C₁ à C₆), -CH₂C(O)NH₂, -CH₂C(O)-NH-alkyl(C₁ à C₆) ou -CH₂C(O)-N(alkyl en C₁ à C₆)₂,
R^{6b} représente un groupe CH₃SO₂-, et
R¹, R², R³, R⁵, R¹⁴, R¹⁵, R¹⁶ et R¹⁹ sont tels que définis dans la partie (1),
ou un sel pharmaceutiquement acceptable d'un tel composé.

2. Composé selon la revendication 1, pour lequel R^{a} représente

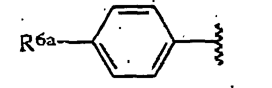

3. Composé selon la revendication 1, de formule II (1), pour lequel X^{a} représente un groupe -CH(OR³)-, -C(R¹³)(R¹⁹)- ou -C(=NOR⁴)-.

4. Composé selon la revendication 3, pour lequel R³ représente un groupe pyridyle, R⁴ représente un groupe alkyle en C₁ à C₆, ou R¹³ représente un atome d'hydrogène et R¹⁹ représente un groupe R⁶-phényle.

5. Composé selon la revendication 1, de formule II (2), pour lequel X^{a} représente un groupe -CH(OR³)-, -C(R¹³)(R¹⁹)- ou -C(=NOR^{4a})-.

6. Composé selon la revendication 5, pour lequel R³ représente un groupe pyridyle, R^{4a} représente un groupe cyclopropylméthyle ou trifluoroéthyle, ou R¹³ représente un atome d'hydrogène et R¹⁹ représente un groupe R⁶-phényle.

7. Composé selon la revendication 1, pour lequel R² représente

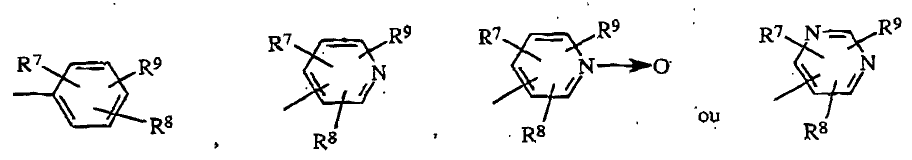

8. Composé selon la revendication 7, pour lequel R² représente un groupe 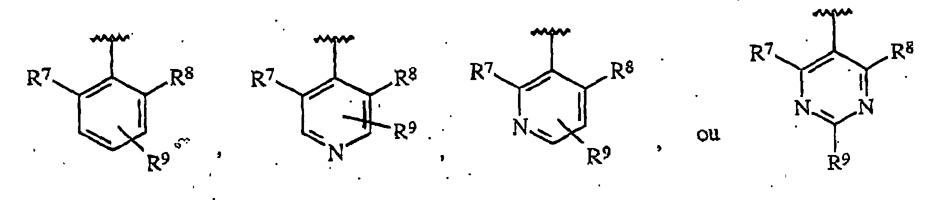 où R⁷ et R⁸ représentent chacun indépendamment un atome d'halogène ou un groupe alkyle en C₁ à C₆ ou -NH₂, et R⁹ représente un atome d'hydrogène.

9. Composé qui est choisi parmi les composés représentés par la formule 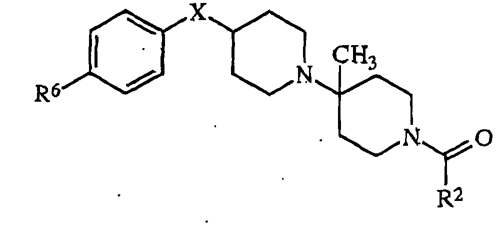 dans laquelle R⁶, X et R² ont les significations indiquées dans le tableau suivant :

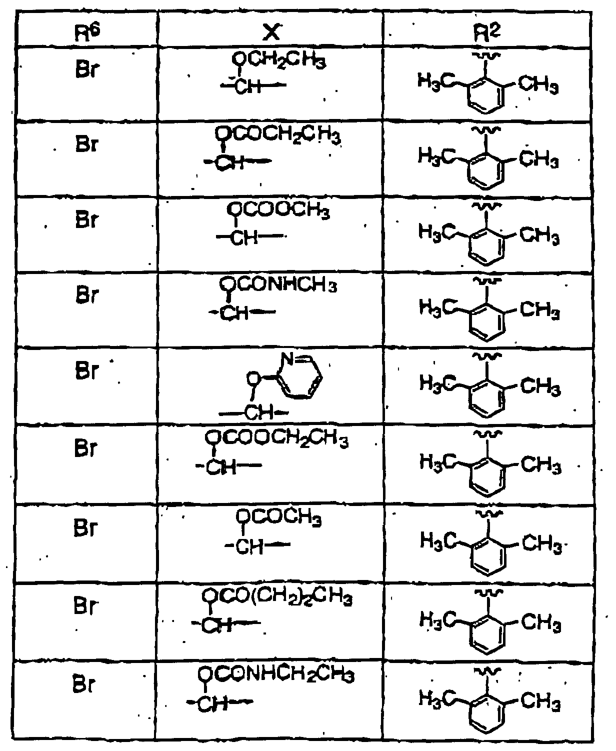

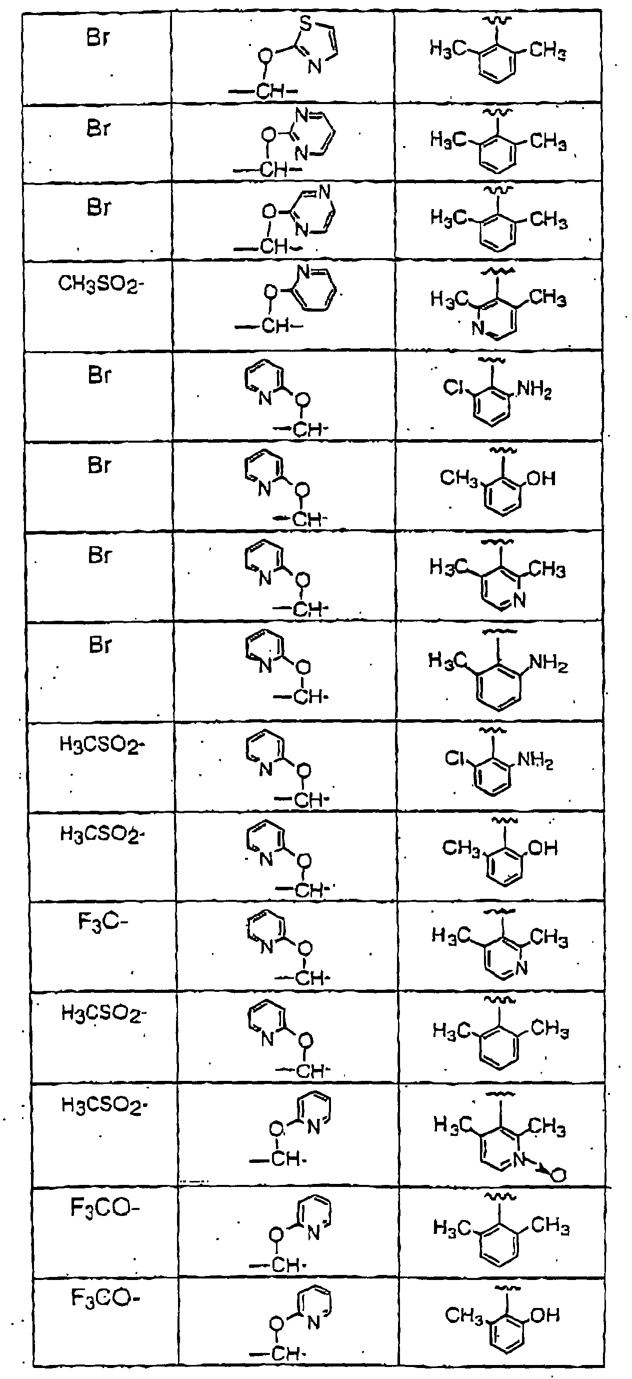

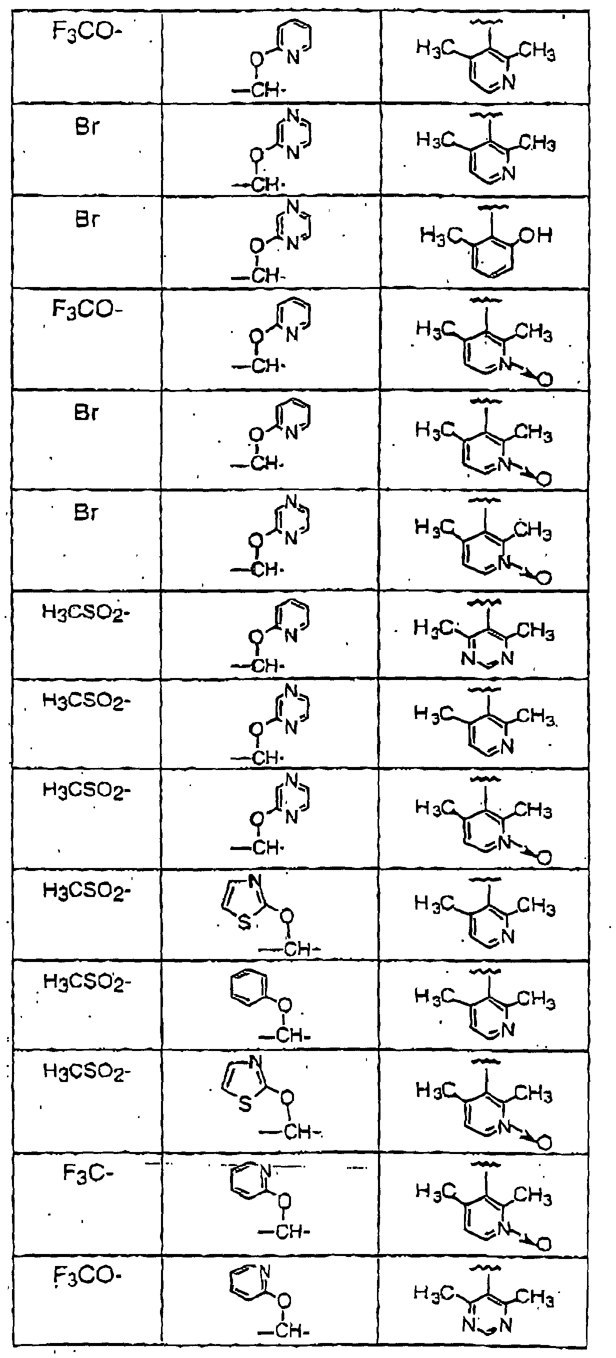

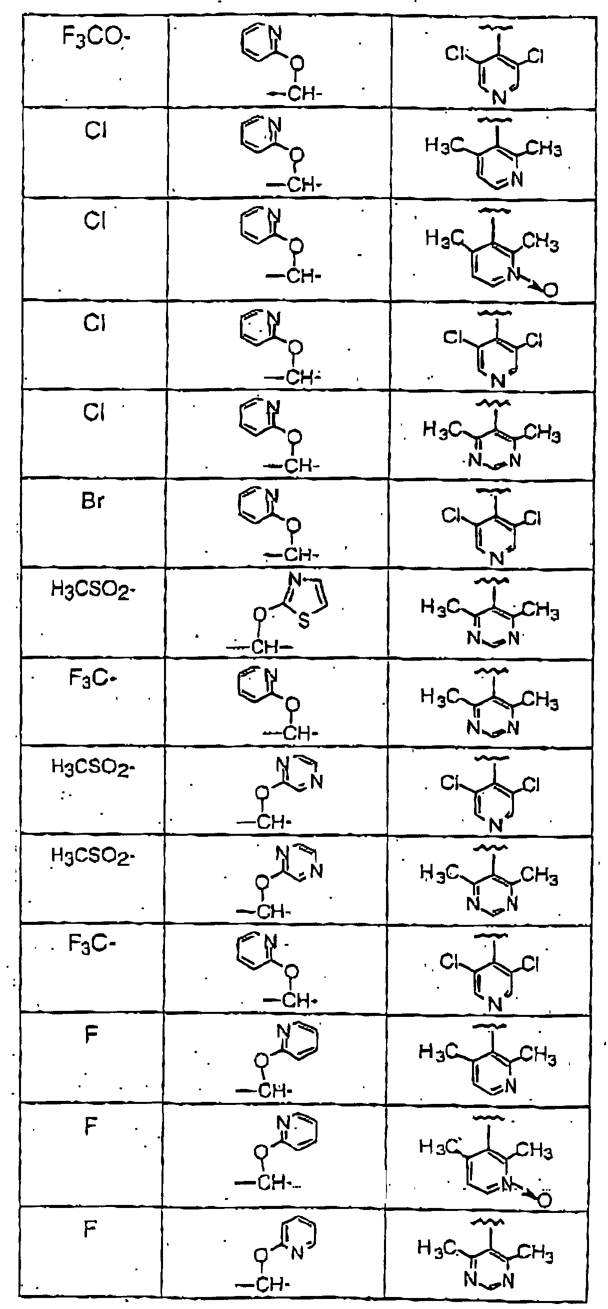

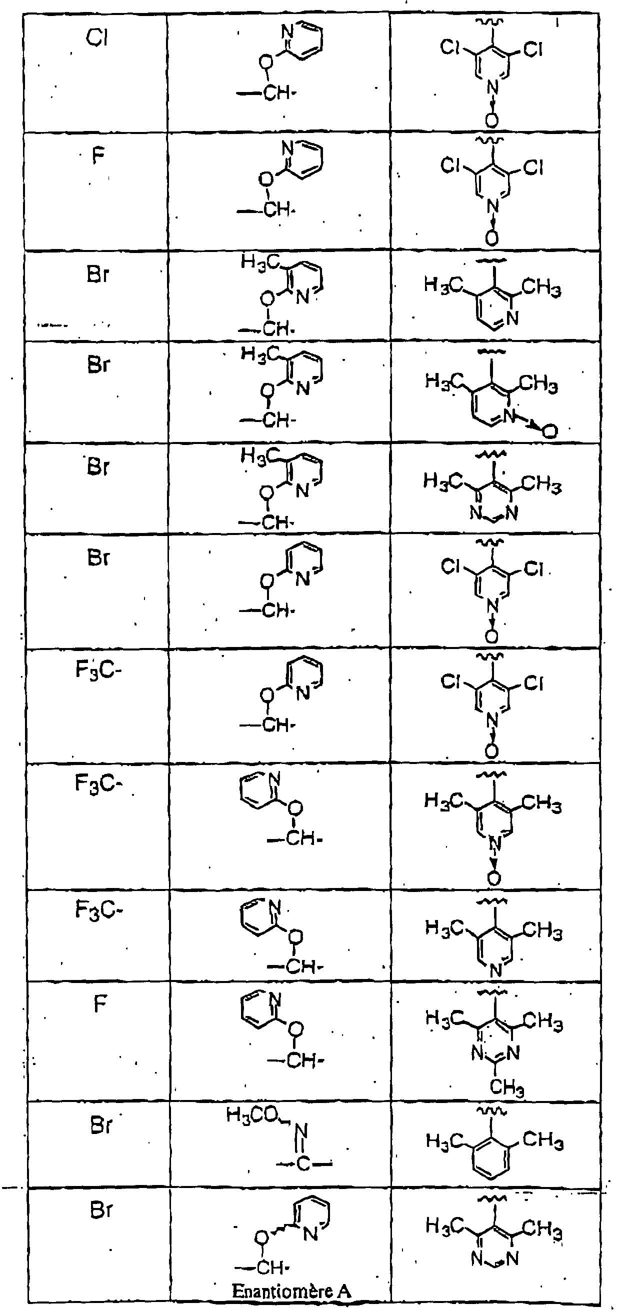

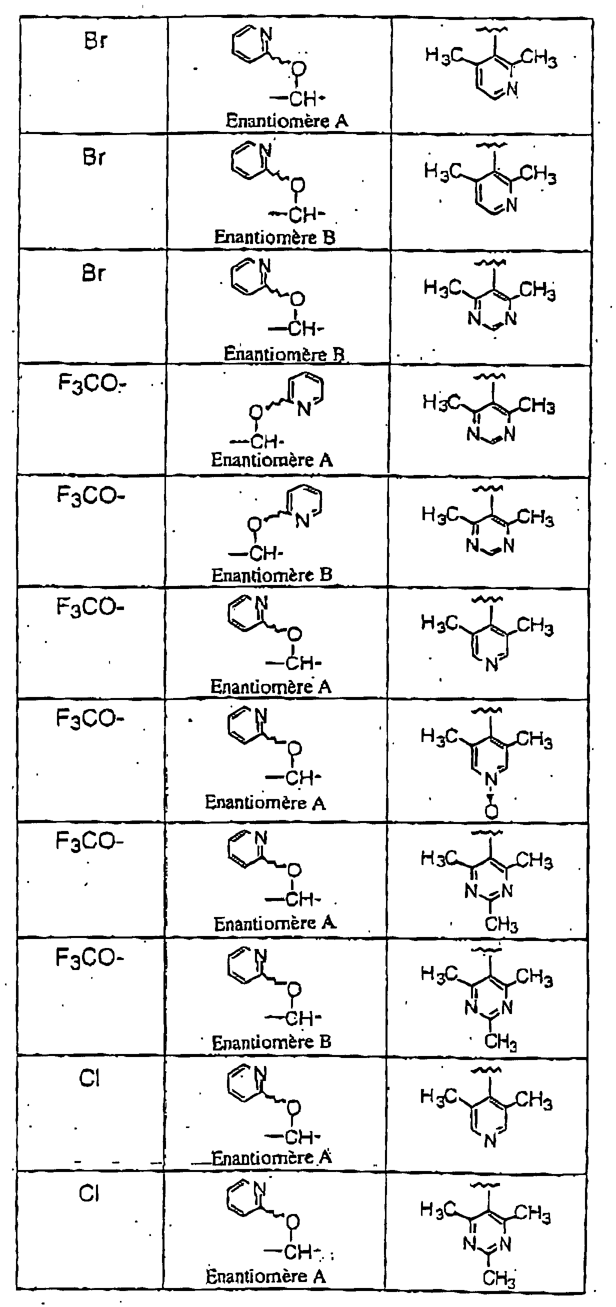

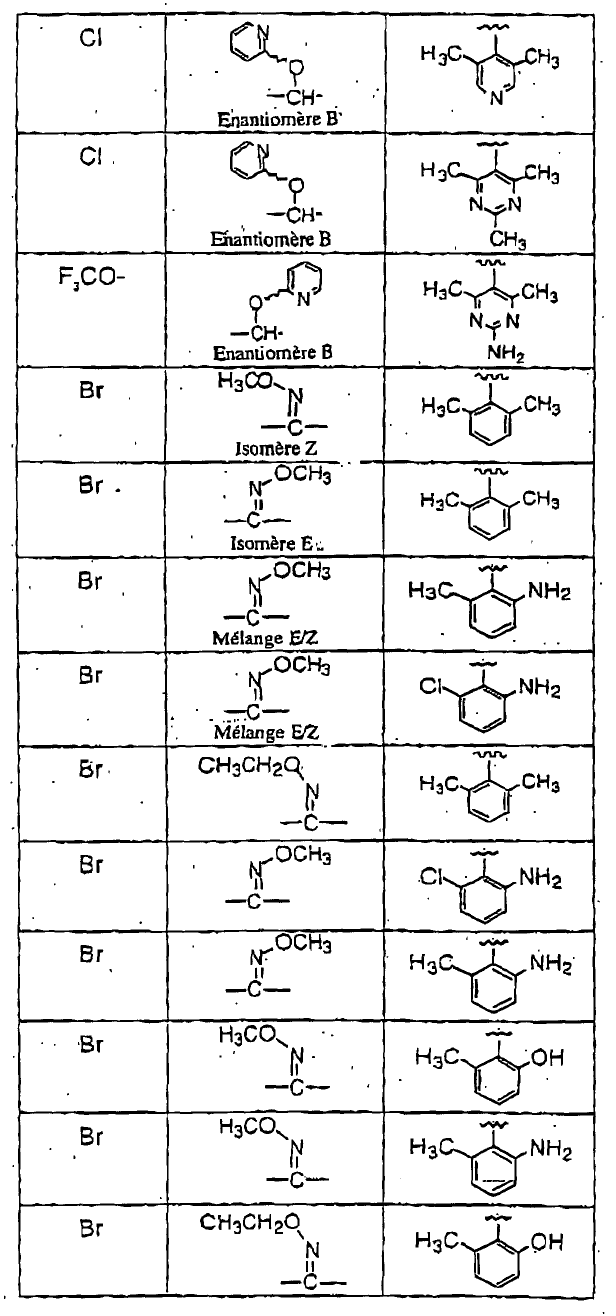

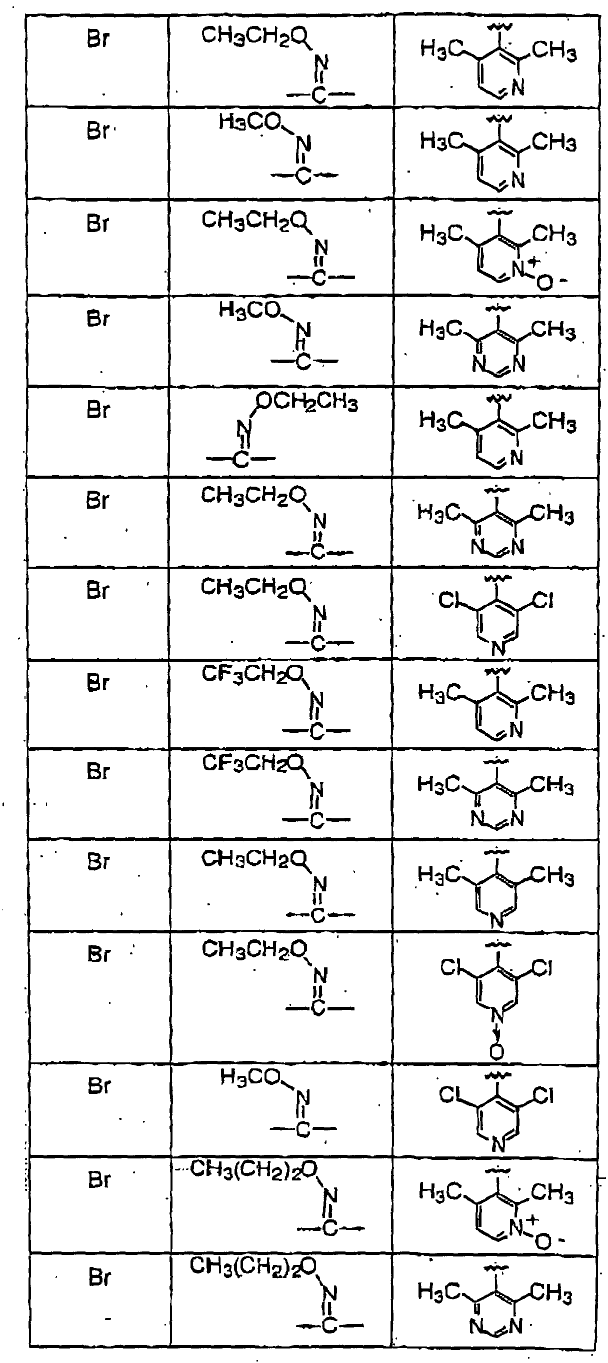

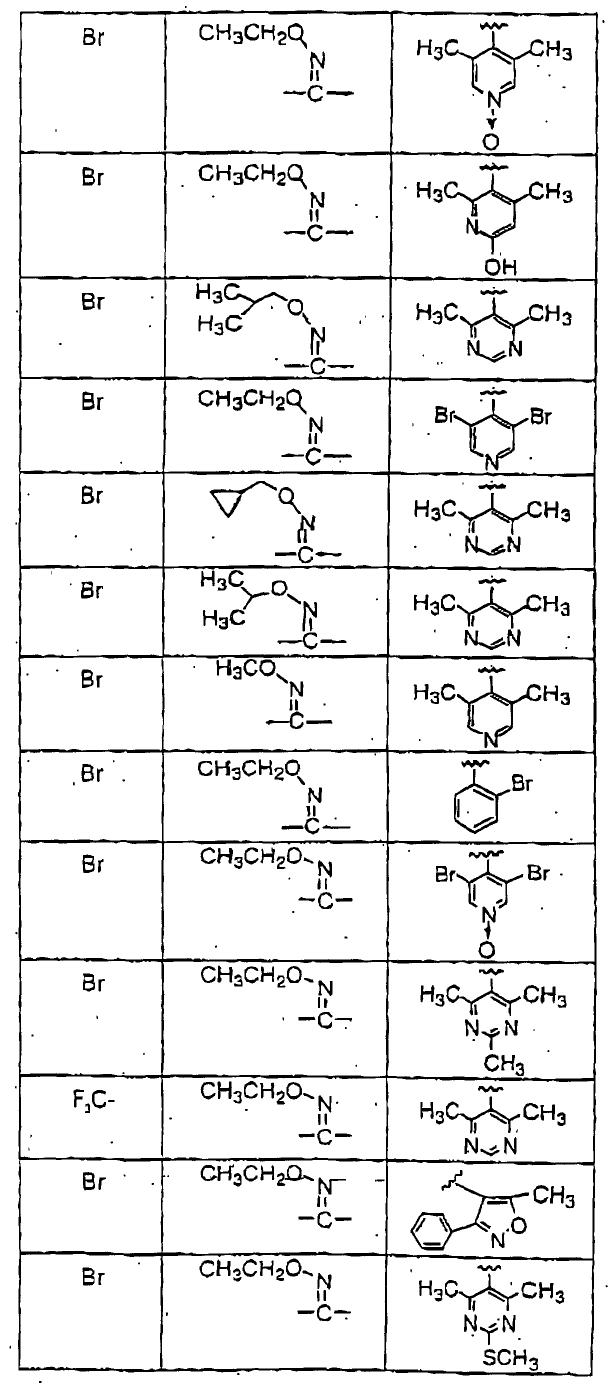

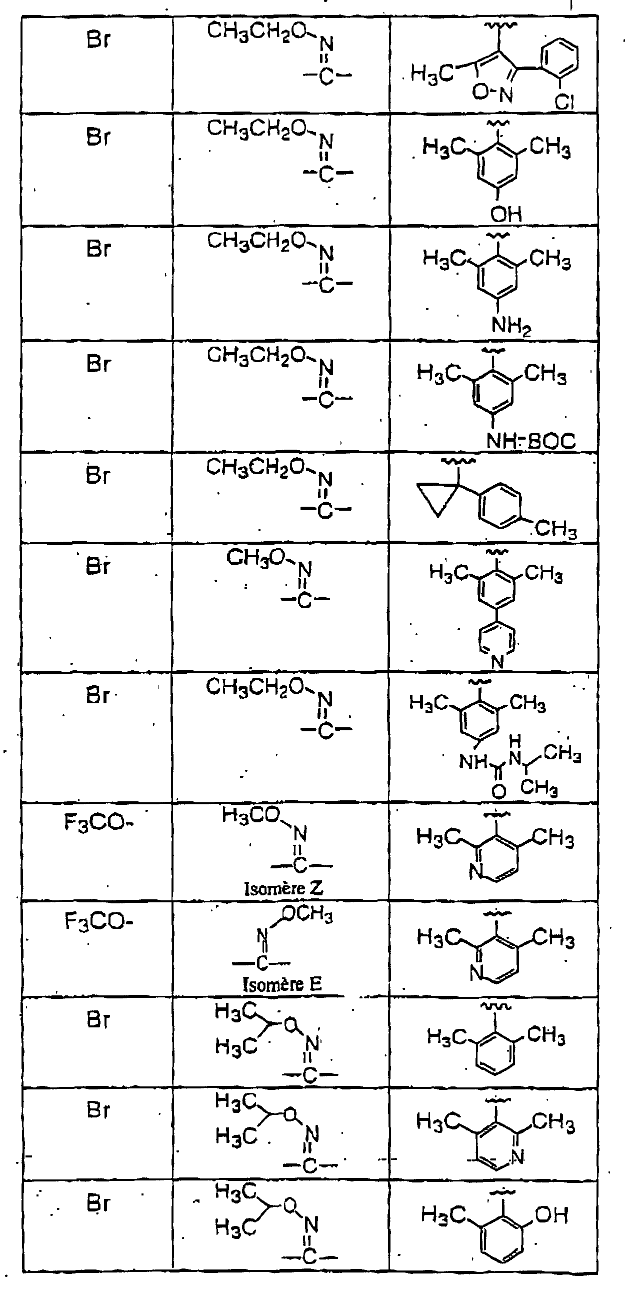

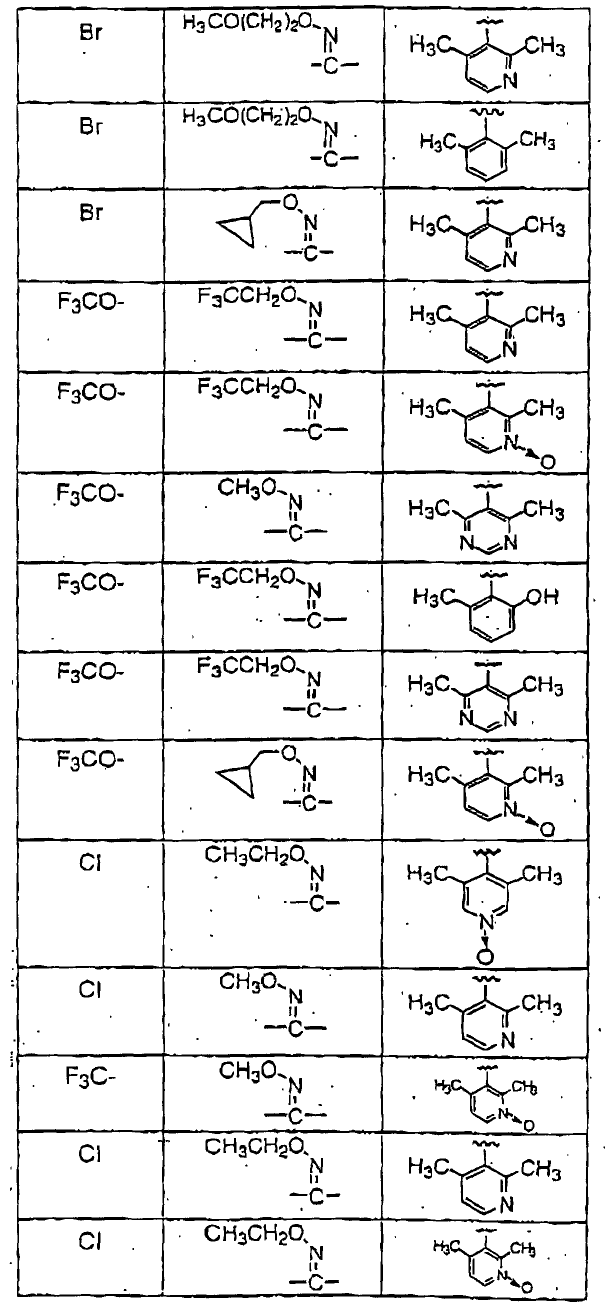

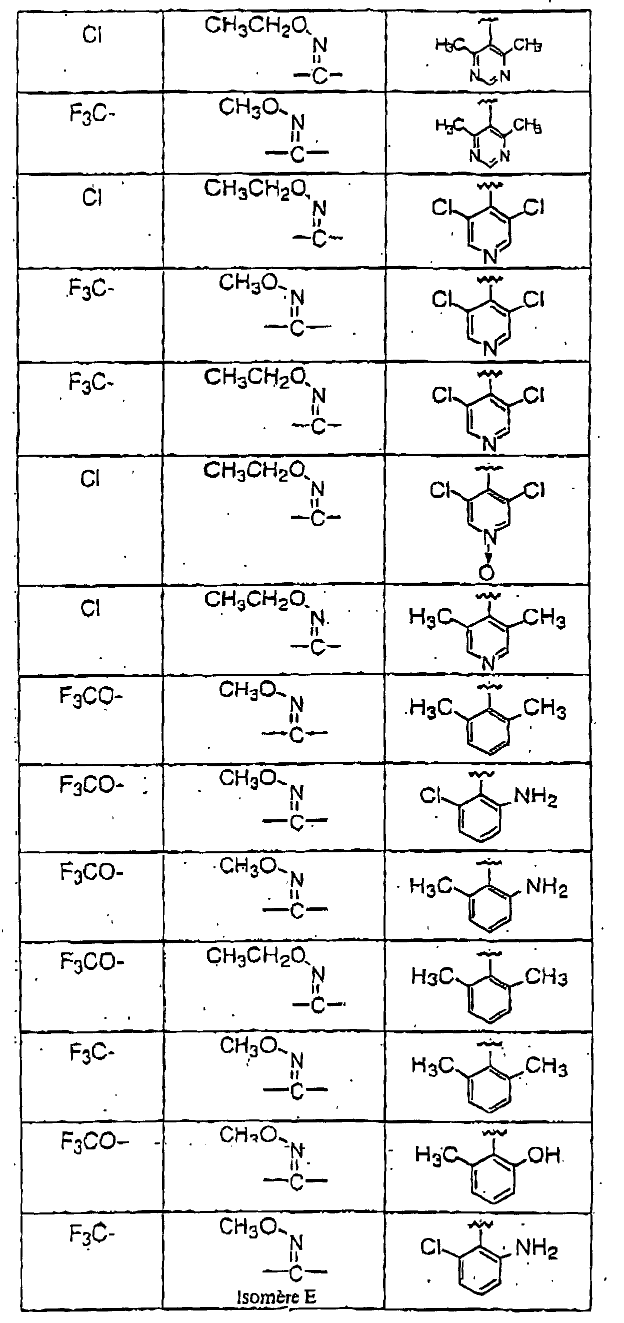

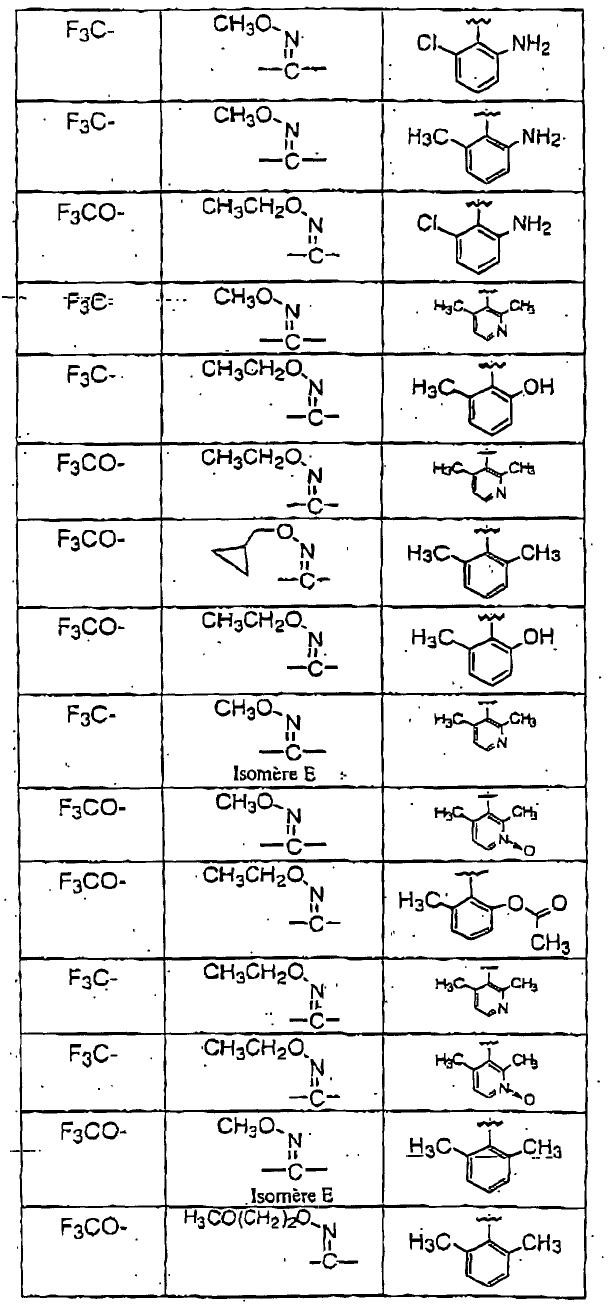

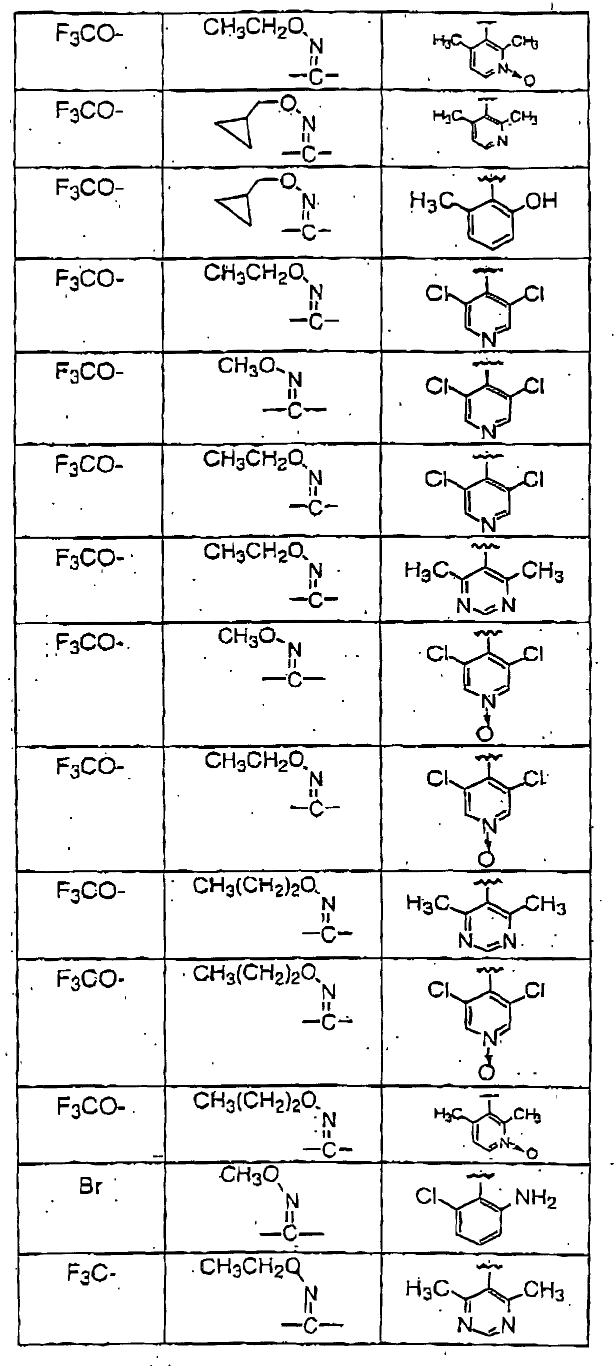

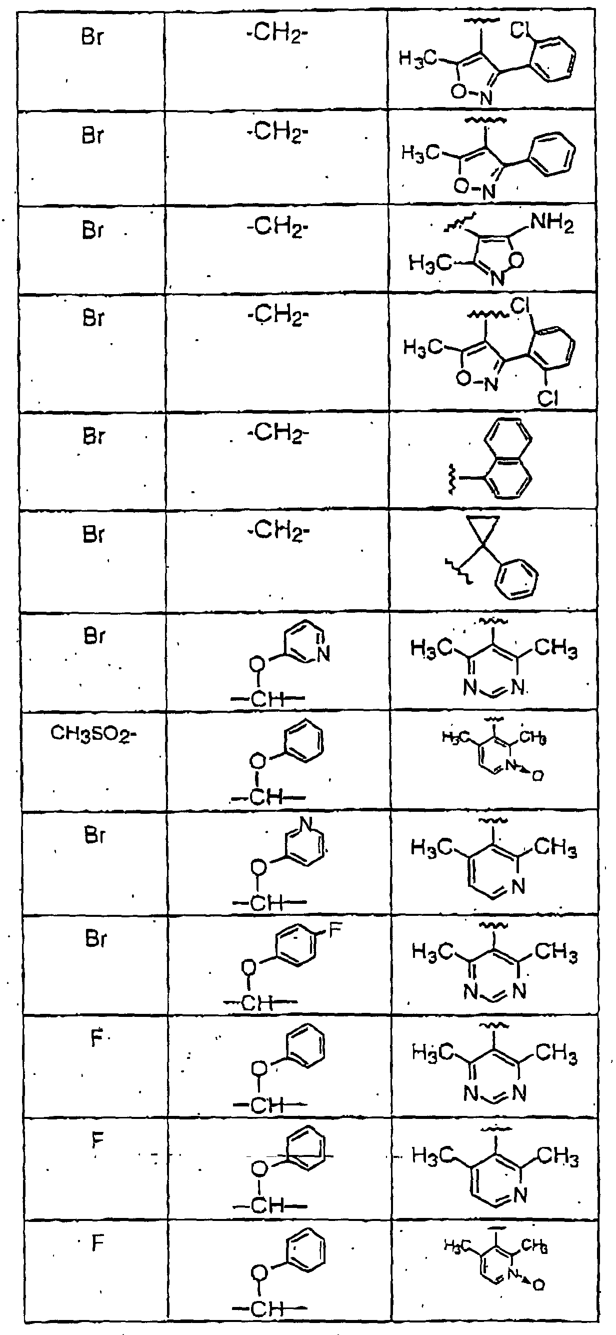

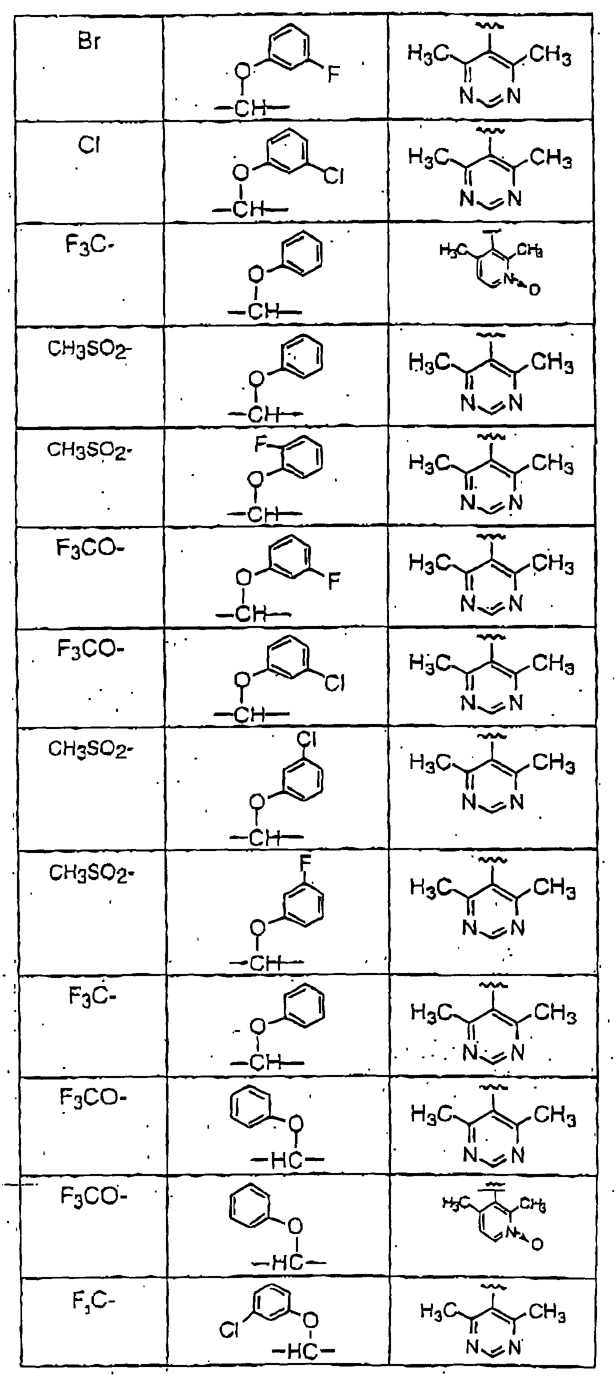

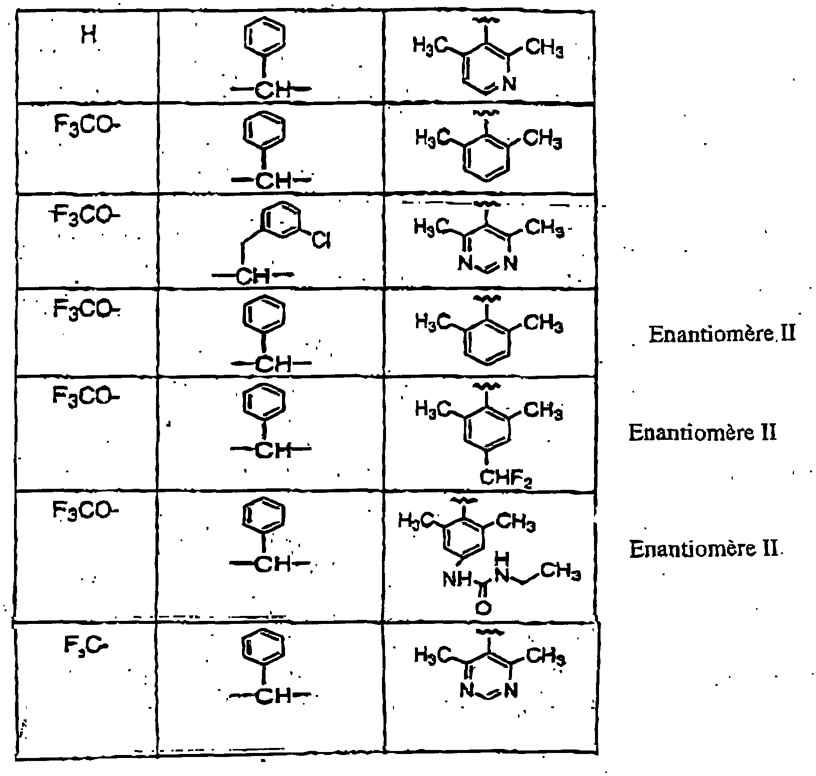

10. Composé selon la revendication 9, qui est choisi parmi les composés suivants :

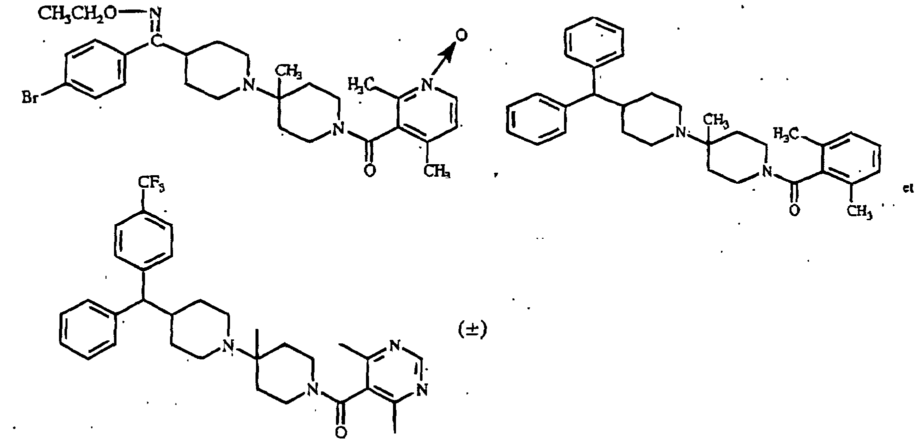

11. Composé de formule

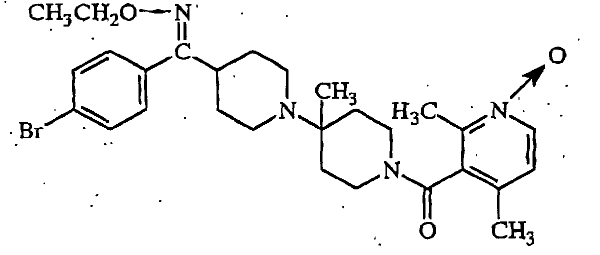

12. Composition pharmaceutique pour le traitement du virus d'immunodéficience humaine, du rejet de greffe d'organe solide, des maladies dues à l'opposition entre le greffon et l'hôte, de l'arthrite, de l'arthrite rhumatoïde, des maladies inflammatoires des intestins, de la dermatite atopique, du psoriasis, de l'asthme, des allergies ou de la sclérose multiple, qui comprend une quantité efficace d'un antagoniste de CCR5 selon l'une quelconque des revendications précédentes, en combinaison avec un support pharmaceutique.

13. Utilisation d'un composé selon l'une quelconque des revendications 1 à 11, pour' la préparation d'un médicament destiné au traitement du virus d'immunodéficience humaine, du rejet de greffe d'organe solide, des maladies dues à l'opposition entre le greffon et l'hôte, de l'arthrite, de l'arthrite rhumatoïde, des maladies inflammatoires des intestins, de la dermatite atopique, du psoriasis, de l'asthme, des allergies ou de la sclérose multiple.

14. Utilisation d'un composé selon l'une quelconque des revendications 1 à 11, pour la préparation d'un médicament destiné à une utilisation combinée avec un ou plusieurs agents antiviraux ou autres agents utiles dans le traitement du virus d'immunodéficience humaine.

15. Utilisation selon la revendication 14, dans laquelle l'agent antiviral est un agent choisi parmi les inhibiteurs de transcriptase inverse de type nucléoside, les inhibiteurs de transcriptase inverse de type non-nucléoside.et les inhibiteurs de protéase.

16. Utilisation d'un composé selon l'une quelconque des revendications 1 à 11, pour la préparation d'un médicament destiné à une utilisation combinée avec un ou plusieurs agents pour le traitement du rejet de greffe d'organe solide, des maladies dues à l'opposition entre le greffon et l'hôte, des maladies inflammatoires des intestins, de l'arthrite rhumatoïde ou de la sclérose multiple.

17. Utilisation d'un antagoniste de CCR5 pour la préparation d'un médicament destiné au traitement du virus d'immunodéficience humaine, du rejet de greffe d'organe solide, des maladies dues à l'opposition entre le greffon et l'hôte, de l'arthrite, de l'arthrite rhumatoïde, des maladies inflammatoires des intestins, de la dermatite atopique, du psoriasis, de l'asthme, des allergies ou de la sclérose multiple, ledit antagoniste de CCR5 étant représenté par la formule développée I suivante : 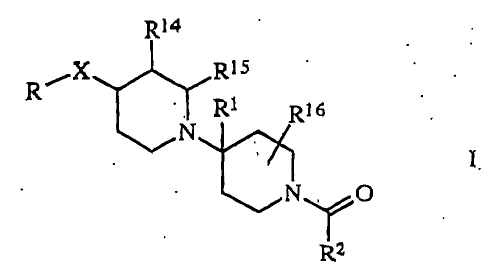 dans laquelle X représente un groupe 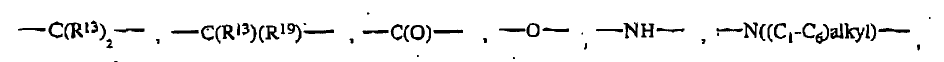 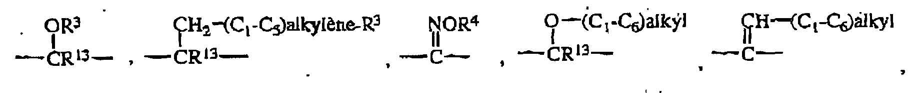 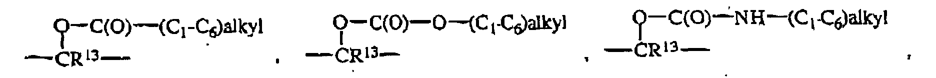 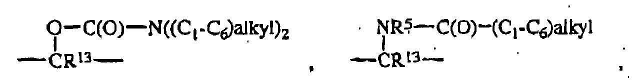 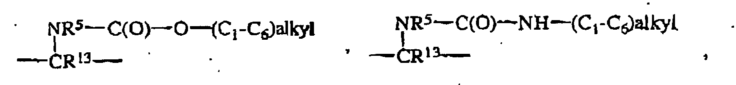 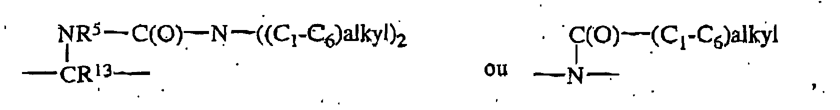 R représente un groupe R⁶-phényle, R⁶-pyridyle, R⁶-thiophényle ou R⁶-naphtyle,
R¹ représente un atome d'hydrogène, un groupe alkyle en C₁ à C₆ ou un groupe alcényle en C₂ à C₆,
R² représente un groupe hétéroaryle à 6 chaînons, substitué par R⁷, R⁸ et R⁹, un groupe hétéroaryle-N-oxyde à 6 chaînons, substitué par
R⁷, R⁸ et R⁹, un groupe hétéroaryle à 5 chaînons, substitué par R¹⁰ et R¹¹, un groupe naphtyle, un groupe fluorényle, un groupe diphénylméthyle ou un groupe 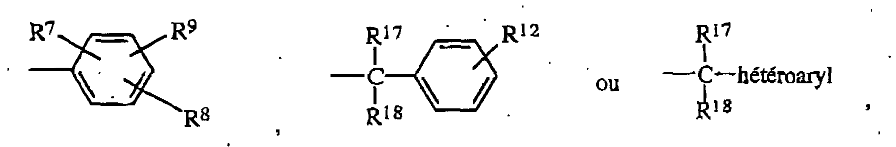 R³ représente un groupe R⁶-phényle, R⁶-hétéroaryle ou R⁶-naphtyle,
R⁴ représente un atome d'hydrogène ou un groupe alkyle en C₁ à C₆, fluoroalkyle en C₁ à C₆, cyclopropylméthyle, -CH₂CH₂OH, -CH₂CH₂-O-alkyl(C₁ à C₆), -CH₂C(O)-O-alkyl(C₁ à C₆), -CH₂C(O)NH₂, -CH₂C(O)-NHalkyl(C₁ à C₆) ou -CH₂C(O)-N(alkyl en C₁ à C₆)₂,
R⁵ et R¹¹ représentent chacun indépendamment un atome d'hydrogène ou un groupe alkyle en C₁ à C₆,
R⁶ représente 1 à 3 substituants choisis indépendamment parmi les atomes d'hydrogène et d'halogène, et les groupes alkyle en C₁ à C₆, alcoxy en C₁ à C₆, 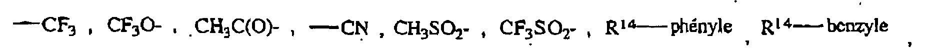 CH₃C(=NOCH₃)- , CH₃C(=NOCH₂CH₃)- , 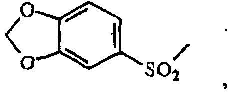 -NH₂, -NHCOCF₃ , -NHCONH(alkyl en C₁-C₆) , -NHCO(alkyl en C₁-C₆) , -NHSO₂(alkyl en C₁-C₆) , hétéroaryle à 5 chaînons et 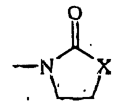 où X représente -O-, -NH- ou -N(CH₃)-,
R⁷ et R⁸ représentent chacun indépendamment un atome d'halogène ou un groupe alkyle en C₁ à C₆, -NR²⁰R²¹, -OH, -CF₃, -OCH₃, -O-acyl ou -OCF₃,
R⁹ représente R⁷, un atome d'hydrogène, ou un groupe phényle, -NO₂, -CN, -CH₂F, -CHF₂, -CHO, -CH=NOR²⁰, pyridyle, pyridyle-N-oxyde, pyrimidinyle, pyrazinyle, -N(R²⁰)CONR²¹R²², -NHCONH(chloroalkyl en C₁ à C₆), -NHCONH(cycloalkyl(C₃ à C₁₀)-alkyl(C₁ à C₆)), -NHCOalkyl(C₁ à C₆), -NHCOCF₃, -NHSO₂N(alkyl en C₁ à C₆)₂, -NHSO₂alkyl(C₁ à C₆), -N(SO₂CF₃)₂, -NHCO₂alkyl(C₁ à C₆), cycloalkyle en C₃ à C₁₀, -SR²³, -SOR²³, -SO₂R²³, -SO₂NHalkyl(C₁ à C₆), -OSO₂alkyl(C₁ à C₆), -OSO₂CF₃, hydroxyalkyle en C₁ à C₆, -CONR²⁰R²¹, -CON(CH₂CH₂-O-CH₃)₂, -OCONHalkyl(C₁ à C₆), -CO₂R²⁰, -Si(CH₃)₃ ou -B(OC(CH₃)₃)₂,
R¹⁰ représente un groupe alkyle en C₁ à C₆, -NH₂ ou R¹²-phényle,
R¹² représente 1 à 3 substituants choisis indépendamment parmi les atomes d'hydrogène et d'halogène, et les groupes alkyle en C₁ à C₆, -CF₃, -CO₂R²⁰, -CN et alcoxy en C₁ à C₆,
R¹³, R¹⁴, R¹⁵ et R¹⁶ représentent chacun indépendamment un atome d'hydrogène ou un groupe alkyle en C₁ à C₆,
R¹⁷ et R¹⁸ représentent chacun indépendamment un atome d'hydrogène ou un groupe alkyle en C₁ à C₆, ou bien R¹⁷ et R¹⁸ forment ensemble un groupe alkylène en C₂ à C₅ et forment avec l'atome de carbone auquel ils sont liés un cycle spiro ayant 3 à 6 atomes de carbone,
R¹⁹ représente un groupe R⁶-phényle, R⁶-hétéroaryle, R⁶-naphtyle, cycloalkyle en C₃ à C₁₀, cycloalkyl(C₃ à C₁₀)alkyle(C₁ à C₆) ou alcoxy(C₁ à C₆)alkyle(C₁ à C₆),
R²⁰, R²¹ et R²² représentent chacun indépendamment un atome d'hydrogène ou un groupe alkyle en C₁ à C₆, et
R²³ représente un groupe alkyle en C₁ à C₆ ou un groupe phényle,
ou d'un sel pharmaceutiquement acceptable d'un tel antagoniste de CCR5.

18. Utilisation selon la revendication 17, dans laquelle R représente

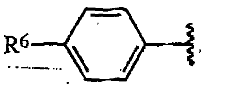

19. Utilisation selon la revendication 17, dans laquelle X représente un groupe -CH(OR³)-, -C(R¹³)(R¹⁹)- ou -C(=NOR⁴)-.

20. Utilisation selon la revendication 19, dans laquelle R³ représente un groupe pyridyle, R⁴ représente un groupe alkyle en C₁ à C₆, ou R¹³ représente un atome d'hydrogène et R¹⁹ représente un groupe R⁶-phényle.

21. Utilisation selon la revendication 17, dans laquelle R² représente

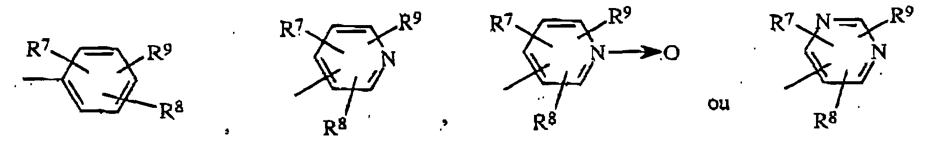

22. Utilisation selon la revendication 21, dans laquelle R² représente un groupe pris parmi les groupes suivants : 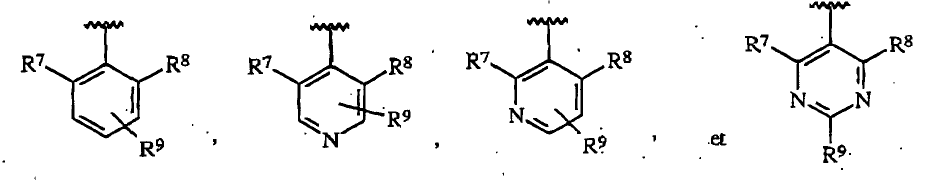 dans lesquels R⁷ et R⁸ représentent chacun un atome d'halogène ou un groupe alkyle en C₁ à C₆ ou -NH₂, et R⁹ représente un atome d'hydrogène.

23. Utilisation selon la revendication 17, pour le traitement du virus d'immunodéficience humaine, qui comprend en outre l'utilisation d'un ou plusieurs agents antiviraux ou autres agents utiles dans le traitement du virus d'immunodéficience humaine.

24. Utilisation selon la revendication 23, dans laquelle l'agent antiviral est un agent choisi parmi les inhibiteurs de transcriptase inverse de type nucléoside, les inhibiteurs de transcriptase inverse de type non-nucléoside, et les inhibiteurs de protéase.

25. Utilisation selon la revendication 17, pour le traitement du rejet de greffe d'un organe solide, des maladies dues à l'opposition entre le greffon et l'hôte, des maladies inflammatoires des intestins, de l'arthrite rhumatoïde ou de la sclérose multiple, qui comprend en outre l'utilisation d'un ou plusieurs autres agents utiles dans le traitement de ces maladies.

26. Nécessaire comprenant dans des récipients séparés et dans un emballage unique, des compositions pharmaceutiques destinées à être utilisées en combinaison pour le traitement du virus d'immunodéficience humaine, qui comprend, dans un récipient, une composition pharmaceutique contenant une quantité efficace d'un antagoniste de CCR5 selon la revendication 17, dans un support pharmaceutiquement acceptable, et, dans des récipients séparés, une ou plusieurs compositions pharmaceutiques contenant une quantité efficace d'un agent antiviral ou d'un autre agent utile dans le traitement du virus d'immunodéficience humaine, dans un support pharmaceutiquement acceptable.

27. Composition pharmaceutique sous la forme d'une crème pour application topique, qui comprend un composé de formule II tel que défini dans l'une quelconque des revendications 1 à 11, et un support pharmaceutiquement acceptable.

28. Composition pharmaceutique sous la forme d'une crème pour application topique, qui comprend un composé de formule 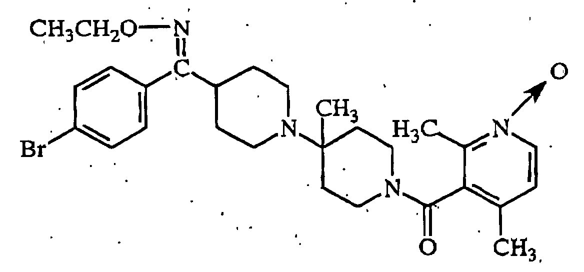 et un support pharmaceutiquement acceptable.
